# EUROPEAN PATENT APPLICATION

(11) **EP 4 174 068 A1**
(43) Date of publication of application: **03.05.2023**
(21) Application number: 21828669.8
(22) Date of filing: 26.06.2021
(51) Int. Cl.: C07D 487/04, A61K 31/4985, A61P 35/00, C07D 487/14, C07D 471/14

(54) **NOVEL COMPOUND, AND PHARMACEUTICAL COMPOSITION FOR PREVENTING OR TREATING RESISTANT CANCER, COMPRISING SAME**

(30) Priority: 26.06.2020 KR 20200078242
(71) Applicant: Holosmedic, Anyang-si, Gyeonggi-do 14058 (KR)
(72) Inventor: YUN, Yeo Jin, Anyang-si, Gyeonggi-do 14058 (KR); KIM, Hyeong Nam, Anyang-si, Gyeonggi-do 14058 (KR); LEE, In Hye, Anyang-si Gyeonggi-do 14058 (KR)
(74) Representative: BCKIP Part mbB
(86) International application number: PCT/KR2021/008051
(87) International publication number: WO 2021/261970

(57) **Abstract**

The present application relates to a novel compound, and a pharmaceutical composition for preventing or treating resistant cancer, the composition comprising the novel compound or a pharmaceutically acceptable salt thereof. When a composition according to the present application and an anticancer agent are administered together, an excellent anticancer effect may be exhibited.

## Description

### [Technical Field]

This application claims priority to and the benefit of Korean Patent Application No. 10-2020-0078242, filed on June 26, 2020, the disclosure of which is incorporated herein by reference in its entirety.

The present application relates to a novel compound and a pharmaceutical composition for preventing or treating cancer, including the same.

### [Background Art]

Cancer is one of the most common causes of death around the world, and accounts for approximately 12% of all deaths. Chemotherapy, which is a typical anticancer therapy, is currently being used as the most efficient therapeutic method of treating cancer alone or in combination with different therapeutic methods such as radiation therapy. However, in chemotherapy, the efficacy of a cancer therapeutic drug depends on its ability to kill cancer cells, but there is a problem of not only acting on cancer cells but also acting on normal cells when the drug is used.

Meanwhile, cancer stem cells are cancer cells with unlimited regenerative ability, and the hypothesis that tumors originate from stem cells was confirmed as it was announced in the late 1990s that human leukemia was reproduced in mice by transplanting cells that can become cancer stem cells from acute myeloid leukemia into immunosuppressed mice, and as cancer stem cells are identified in breast cancer, the presence of stem cells was also confirmed even in solid carcinoma.

Cancer stem cells are cells that have a self-renewal ability and also have the ability to differentiate into other cells, and act as the cause of cancer recurrence and metastasis. A specific patient group is classified as intractable cancer patients, who are difficult to treat with existing anticancer therapy because cancer stem cells are activated and exhibit strong anticancer agent resistance. Diverse heterogeneities shown by malignant tumors are consistent with various differentiative potentials of stem cells, and the drug resistance of cancer cells, which is constantly expressed despite many targeted therapies, is consistent with the basic characteristics of stem cells.

Cancer stem cells may be used in a novel targeted therapy field, and in order to efficiently perform treatment targeting only cancer stem cells without damaging normal stem cells, knowledge and understanding of molecular and biological characteristics important for the maintenance and regulation of cancer stem cells and regulatory pathways thereof are required.

Several treatment methods have been devised based on the cancer stem cell hypothesis, and the most well-known method is a method using the self-renewal pathway of cancer stem cells. An important point in such treatment is to target the self-renewal of cancer stem cells while maintaining the self-renewal of normal stem cells. For example, Notch signaling is carried out by an enzyme called gamma secretase, and an inhibitor thereof (gamma secretase inhibitor) is used for Notch1-overexpressing breast cancer, so a tumor suppression effect can be achieved. There is a recent report that targeting a Hedgehog signaling system also shows an anticancer effect, and when the hedgehog inhibitor cyclopamine was administered to a tumor-xenografted animal, the tumor dramatically atrophied.

In addition, it is known to be involved in PI3K/AKT, MAPK, and JAK2/STAT3 signaling pathways.

As described above, a number of studies are being conducted to inhibit cancer stem cells by inhibiting cancer stem cells using an experiment of directly inhibiting a target gene of cancer stem cells or by inhibiting cancer stem cells by inhibiting an upstream signaling protein of cancer stem cells. However, to date, there have been few studies on anticancer drugs or natural product-derived extracts that directly target cancer stem cells, and in most tumor patients, there are many difficulties in targeting experiments due to mutations in oncogenes or proteins.

Therefore, it is necessary to overcome conventional anticancer drug resistance by mutations in oncogenes or proteins, and to develop new drugs with an anticancer effect.

### [Disclosure]

### [Technical Problem]

One aspect of the present application provides a novel compound.

In addition, another aspect of the present application provides a pharmaceutical composition for preventing or treating cancer, which includes a novel compound.

In addition, still another aspect of the present application provides a pharmaceutical composition for preventing or treating resistant cancer, which includes a novel compound.

In addition, yet another aspect of the present application provides the use of a novel compound for preventing or treating resistant cancer.

In addition, yet another aspect of the present application provides a method of treating cancer having resistance to oncological therapy by administering a novel compound to a subject with resistant cancer.

However, technical problems to be solved in the present application are not limited to the above-described problems, and other problems which are not described herein will be fully understood by those of ordinary skill in the art from the following descriptions.

### [Technical Solution]

One embodiment of the present application provides a compound represented by Formula 1 below or a pharmaceutically acceptable salt thereof.

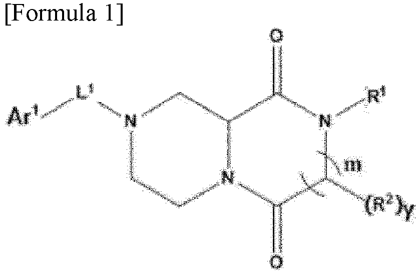

In Formula 1,
L¹ is a direct bond or C₁₋₁₀ alkylene;
Ar¹ is C₁₋₁₂ alkyl, or 5- to 16-membered monocyclic, bicyclic, tricyclic, or tetracyclic ring which contains 0 to 3 heteroatoms independently selected from O, N or S, wherein the ring may be unsubstituted or substituted with groups independently selected from C₁₋₆ haloalkyl, halogen, oxo, -OCHF₂, -CN, nitro, -C(=O)NZ^{a}Z^{a}, -C(=O)Z^{b}, -C(=O)OZ^{b}, -C(=NZ^{a})NZ^{a}Z^{a}, -OZ^{a}, - OC(=O)Z^{b}, -OC(=O)NZ^{a}Z^{a}, -O-C₁₋₆ alkyl N(Z^{a})C(=O)OZ^{b}, -OC(=O)N(Z^{a})S(=O)₂Z^{b}, -OC₂₋₆ alkyl NZ^{a}Z^{a}, -OC₂₋₆ alkyl OZ^{a}, -SZ^{a}, -S(=O)Z^{b}, -S(=O)₂Z^{b}, -S(=O)₂NZ^{a}Z^{a}, - S(=O)₂N(Z^{a})C(=O)Z^{b}, -S(=O)₂N(Z^{a})C(=O)OZ^{b}, -S(=O)₂N(Z^{a})C(=O)NZ^{a}Z^{a}, -NZ^{a}Z^{a}, -NZ^{c}Z^{c}, - N(Z^{a})C(=O)Z^{b}, -N(Z^{a})C(=O)OZ^{b}, -N(Z^{a})C(=O)NZ^{a}Z^{a}, -N(Z^{a})C(=NZ^{a})NZ^{a}Z^{a}, -N(Z^{a})S(=O)₂Z^{b}, - N(Z^{a})S(=O)₂NZ^{a}Z^{a}, -NZ^{a}C₂₋₆ alkyl NZ^{a}Z^{a}, -NZ^{a}C₂₋₆ alkyl OZ^{a}, C₆₋₁₀ aryl, C₅₋₁₀ heteroaryl, -C₁₋₆ alkyl, -C₃₋₁₀ cycloalkyl, -C₂₋₆ alkenyl or -C₂₋₆ alkynyl (here, -C₁₋₆ alkyl, -C₃₋₁₀ cycloalkyl, -C₂₋₆ alkenyl or -C₂₋₆ alkynyl is substituted with 0 to 3 substituents independently selected from C₁₋₆ haloalkyl, halogen, cyano, nitro, C₆₋₁₂ aryl or C₅₋₁₂ heteroaryl);
Z^{a} is each independently hydrogen or Z^{b};
Z^{b} is each independently phenyl, benzyl, C₁₋₆ alkyl, C₄₋₈ heterocycloalkyl, or C₃₋₈ cycloalkyl, wherein the phenyl, benzyl, C₁₋₆ alkyl, C₄₋₈ heterocycloalkyl, or C₃₋₈ cycloalkyl is substituted with 0 to 3 substituents independently selected from halogen, -OH, -S(=O)₂Z^{b}, -OC₂₋₆ alkyl OZ^{a}, C₁₋₆ alkyl, C₁₋₆ haloalkyl, -OC₁₋₄ alkyl, -NH₂, -CN, or -NZ^{a}Z^{a};
Z^{c} may each be independently hydrogen or C₁₋₆ alkyl, or the group CZ^{c}Z^{c} may form C₃₋₈ cycloalkyl ring;
m and y are each independently an integer of 0 to 2; and
R¹ or R² is each independently hydrogen, C₁₋₆ alkyl, C₁₋₆ alkoxy, C₁₋₆ alkoxycarbonyl, halo C₁₋₆ alkyl, C₂₋₆ alkenyl, or C₄₋₁₅ monocyclic or bicyclic ring (which is C₃₋₁₀ cycloalkyl; C₆₋₁₂ aryl; 5- to 6-membered saturated or partially saturated hetero ring containing 0 to 3 heteroatoms, independently selected from N, O and S; 5- or 6-membered aromatic hetero ring containing 0 to 3 heteroatoms (provided that two or more of the heteroatoms are not O or S), independently selected from N, O and S; or 7- to 15-membered saturated, partially saturated, or unsaturated hetero ring containing 0 to 3 heteroatoms, independently selected from N, O and S),
wherein R¹ and R² may be connected to form 5- to 12-membered monocyclic or bicyclic ring.

One embodiment of the present application provides a pharmaceutical composition for preventing or treating cancer, which includes the compound or a pharmaceutically acceptable salt thereof, or a hydrate, solvate, structural isomer, optical isomer or stereoisomer thereof, or a combination thereof.

One embodiment of the present application provides a pharmaceutical composition for preventing or treating resistant cancer, which includes the compound or a pharmaceutically acceptable salt thereof, or hydrate, solvate, structural isomer, optical isomer or stereoisomer thereof, or combination thereof.

In one embodiment of the present application, the composition may further include a pharmaceutically acceptable carrier, excipient, diluent or adjuvant.

In one embodiment of the present application, the composition may inhibit SERCA protein expression.

In one embodiment of the present application, the resistant cancer may be resistant to an anticancer drug, or resistant to radiation.

In one embodiment of the present application, the anticancer drug may be at least one selected from the group consisting of nitrogen mustard, imatinib, oxaliplatin, rituximab, erlotinib, neratinib, lapatinib, gefitinib, vandetanib, nilotinib, semaxanib, bosutinib, axitinib, masitinib, cediranib, lestaurtinib, trastuzumab, gefitinib, bortezomib, sunitinib, pazopanib, toceranib, nintedanib, regorafenib, semaxanib, tivozanib, ponatinib, cabozantinib, carboplatin, sorafenib, lenvatinib, bevacizumab, cisplatin, cetuximab, viscum album, asparaginase, tretinoin, hydroxycarbamide, dasatinib, estramustine, gemtuzumab ozogamicin, ibritumomab tiuxetan, heptaplatin, methylaminolevulinic acid, amsacrine, alemtuzumab, procarbazine, alprostadil, holmium nitrate chitosan, gemcitabine, doxifluridine, pemetrexed, tegafur, capecitabine, gimeracil, oteracil, azacytidine, methotrexate, uracil, cytarabine, 5-fluorouracil, fludarabine, enocitabine, flutamide, capecitabine, decitabine, mercaptopurine, thioguanine, cladribine, carmofur, raltitrexed, docetaxel, paclitaxel, cabazitaxel, irinotecan, belotecan, topotecan, vinorelbine, etoposide, vincristine, vinblastine, teniposide, doxorubicin, idarubicin, epirubicin, mitoxantrone, mitomycin, bleomycin, daunorubicin, dactinomycin, pirarubicin, aclarubicin, pepromycin, temsirolimus, temozolomide, busulfan, ifosfamide, cyclophosphamide, melphalan, altretamine, dacabazine, thiotepa, nimustine, chlorambucil, mitolactol, leucovorin, tretonin, exmestane, aminoglutethimide, anagrelide, olaparib, navelbine, padrazol, tamoxifen, toremifene, testolactone, anastrozole, letrozole, borozol, bicalutamide, lomustine, vorinostat, entinostat, and carmustine.

In one embodiment of the present application, the anticancer drug may be included in a molar concentration ratio of 1: 0.001 to 1:1000 with the compound represented by Formula 1 or a pharmaceutically acceptable salt thereof.

In one embodiment of the present application, the resistant cancer may be at least one selected from the group consisting of ovarian cancer, colorectal cancer, pancreatic cancer, gastric cancer, liver cancer, breast cancer, cervical cancer, thyroid cancer, parathyroid cancer, lung cancer, non-small cell lung cancer, prostate cancer, gallbladder cancer, biliary tract cancer, blood cancer, bladder cancer, kidney cancer, melanoma, colon cancer, bone cancer, skin cancer, head and neck cancer, uterine cancer, rectal cancer, brain cancer, anal cancer, fallopian tube carcinoma, endometrial carcinoma, vaginal cancer, vulvar carcinoma, esophagus cancer, small intestine cancer, endocrine carcinoma, adrenal cancer, soft tissue sarcoma, urethral cancer, penile cancer, ureteral cancer, renal cell carcinoma, renal pelvic carcinoma, blood cancer, leukemia, a central nervous system (CNS) tumor, a spinal cord tumor, brainstem glioma, and pituitary adenoma.

One embodiment of the present application provides a method of treating cancer, which includes administering a therapeutically effective amount of the compound or a pharmaceutically acceptable salt thereof, or a hydrate, solvate, structural isomer, optical isomer or stereoisomer thereof, or a combination thereof to a subject with cancer.

One embodiment of the present application provides a method of treating cancer having resistance to oncological therapy, which includes administering a therapeutically effective amount of the compound or a pharmaceutically acceptable salt thereof, or a hydrate, solvate, structural isomer, optical isomer or stereoisomer thereof, or a combination thereof to a subject with resistant cancer.

One embodiment of the present application provides a method of treating cancer resistant to oncological therapy, which includes simultaneously, individually or sequentially administering a chemotherapeutic agent useful for treatment of cancer or a proliferative disease.

One embodiment of the present application provides a use of the compound or a pharmaceutically acceptable salt thereof for preparing a drug for treatment of cancer or resistant cancer.

One embodiment of the present application provides a use of the compound or a pharmaceutically acceptable salt thereof, or a hydrate, solvate, structural isomer, optical isomer or stereoisomer thereof, or a combination thereof for preparation of a drug for treating stem cell cancer.

### [Advantageous Effects]

A composition containing a compound according to one embodiment of the present application or a pharmaceutically acceptable salt thereof can improve the anticancer activity of an anticancer agent or radiation, and can effectively treat cancer by inducing cancer cell proliferation inhibition and cancer cell apoptosis.

A composition containing a compound according to one embodiment of the present application or a pharmaceutically acceptable salt thereof can overcome the resistance of cancer to an anticancer agent or radiation, and effectively treat resistant cancer.

The composition containing a compound according to one embodiment of the present application or a pharmaceutically acceptable salt thereof has an effect of preventing or treating cancer when used alone.

The effects according to the present application are not intended to limit the contents exemplified above, and various additional effects are included in the specification of the present specification.

### [Description of Drawings]

FIG. 1(a) shows IC50 values of anticancer agents, paclitaxel and docetaxel, in SKOV3, and FIG. 1(b) shows IC50 values of the anticancer agents, paclitaxel and docetaxel, in SKOV3-TR.
FIGS. 2 to 13 show the results of WST-1 assays of SKOV3-TR and SKOV3 for paclitaxel.
FIGS. 14 to 25 shows the results of WST-1 assays of SKOV3-TR and SKOV3 for docetaxel.

### [Modes of the Invention]

Advantages and features of the present application and methods for achieving them will be apparent with reference to the embodiments described below in detail with the accompanying drawings. However, the present application is not limited to embodiments disclosed below and may be implemented in various different forms. Therefore, it should be understood that the embodiments disclosed herein are merely provided to make the disclosure of the present application complete and fully inform persons having ordinary skill in the art to which the present application belongs of the scope of the present application, which is defined only by the scope of the claims.

Hereinafter, embodiments of the present application will be described.

One embodiment of the present application provides a compound represented by Formula 1 below or a pharmaceutically acceptable salt thereof.

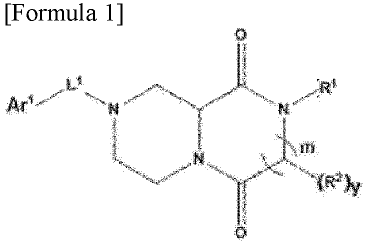

In one embodiment of the present application, the compound represented by Formula 1 may be a compound represented by any one of Formulas 2 to 10, or a pharmaceutically acceptable salt thereof.

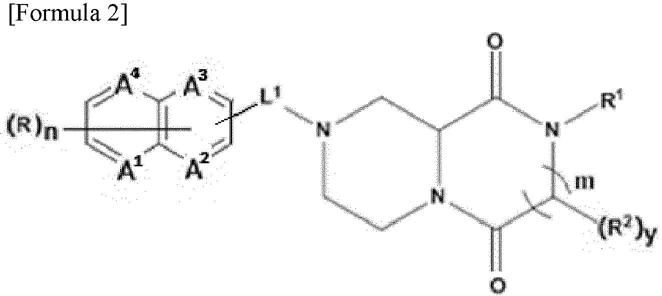

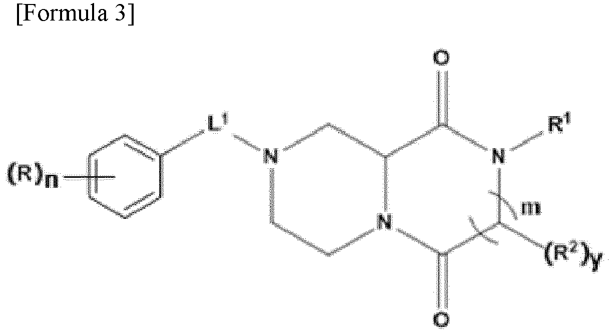

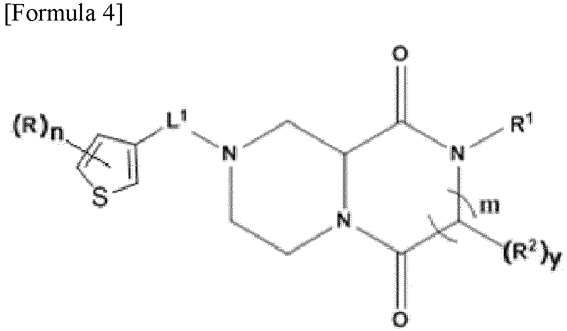

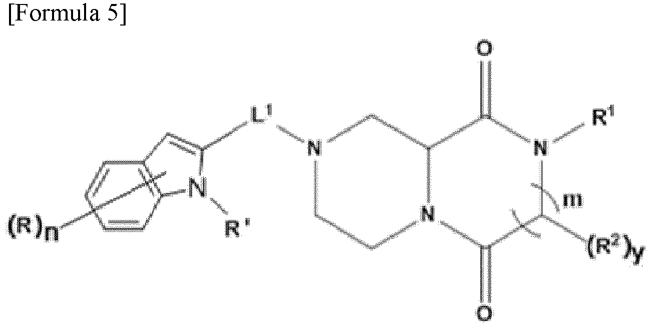

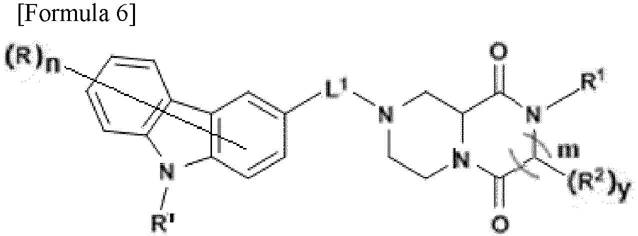

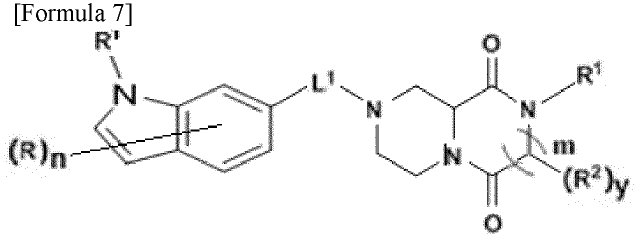

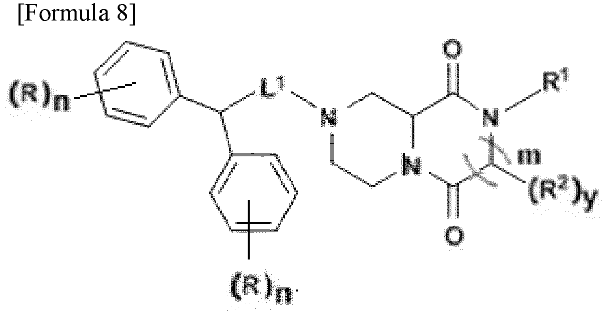

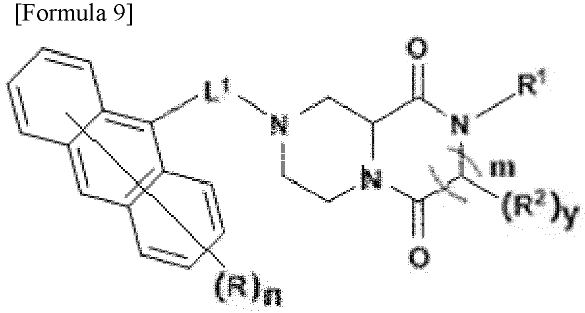

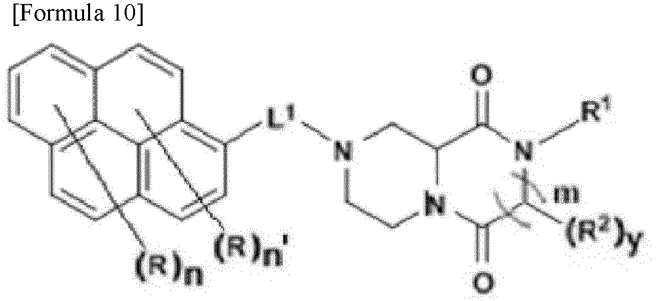

The compound represented by Formula 2 may be a compound represented by any one of Formulas 2a to 2c.

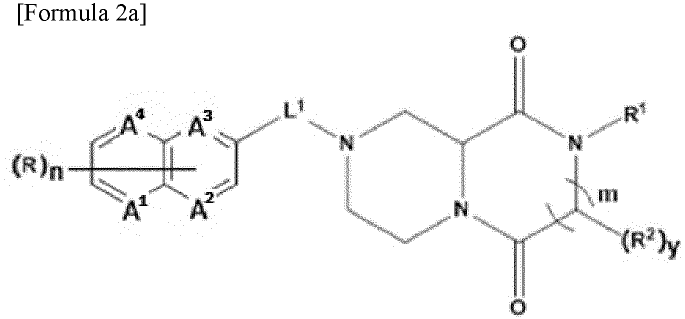

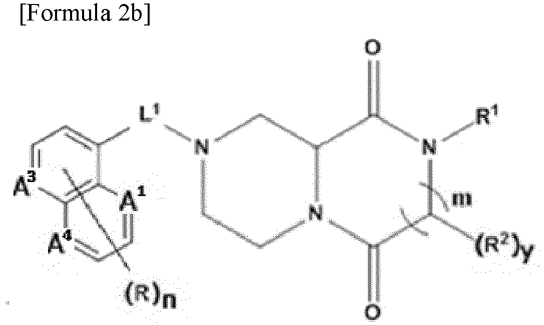

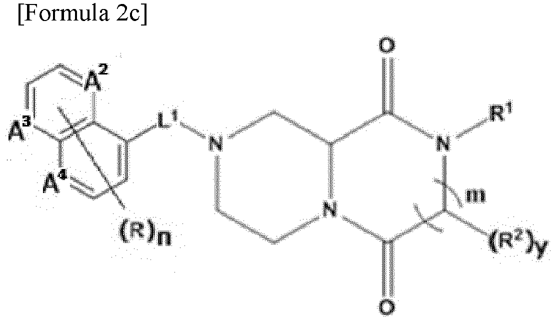

In Formula 1, Ar¹ may be C₁₋₁₂ alkyl, or 5- to 16-membered monocyclic, bicyclic, tricyclic, or tetracyclic ring which contains 0 to 3 heteroatoms independently selected from O, N or S, wherein the ring may be unsubstituted or substituted with groups independently selected from C₁₋₆ haloalkyl, halogen, oxo, -OCHF₂, -CN, nitro, -C(=O)NZ^{a}Z^{a}, -C(=O)Z^{b}, -C(=O)OZ^{b}, - C(=NZ^{a})NZ^{a}Z^{a}, -OZ^{a}, -OC(=O)Z^{b}, -OC(=O)NZ^{a}Z^{a}, -O-C₁₋₆ alkyl N(Z^{a})C(=O)OZ^{b}, - OC(=O)N(Z^{a})S(=O)₂Z^{b}, -OC₂₋₆ alkyl NZ^{a}Z^{a}, -OC₂₋₆ alkyl OZ^{a}, -SZ^{a}, -S(=O)Z^{b}, -S(=O)₂Z^{b}, - S(=O)₂NZ^{a}Z^{a}, -S(=O)₂N(Z^{a})C(=O)Z^{b}, -S(=O)₂N(Z^{a})C(=O)OZ^{b}, -S(=O)₂N(Z^{a})C(=O)NZ^{a}Z^{a}, - NZ^{a}Z^{a}, -NZ^{c}Z^{c}, -N(Z^{a})C(=O)Z^{b}, -N(Z^{a})C(=O)OZ^{b}, -N(Z^{a})C(=O)NZ^{a}Z^{a}, -N(Z^{a})C(=NZ^{a})NZ^{a}Z^{a}, - N(Z^{a})S(=O)₂Z^{b}, -N(Z^{a})S(=O)₂NZ^{a}Z^{a}, -NZ^{a}C₂₋₆ alkyl NZ^{a}Z^{a}, -NZ^{a}C₂₋₆ alkyl OZ^{a}, C₆₋₁₀ aryl, C₅₋₁₀ heteroaryl, -C₁₋₆ alkyl, -C₃₋₁₀ cycloalkyl, -C₂₋₆ alkenyl or -C₂₋₆ alkynyl (here, -C₁₋₆ alkyl, -C₃₋₁₀ cycloalkyl, -C₂₋₆ alkenyl or -C₂₋₆ alkynyl is substituted with 0 to 3 substituents independently selected from C₁₋₆ haloalkyl, halogen, cyano, nitro, C₆₋₁₂ aryl or C₅₋₁₂ heteroaryl);
Z^{a} is each independently hydrogen or Z^{b};
Z^{b} is each independently phenyl, benzyl, C₁₋₆ alkyl, C₄₋₈ heterocycloalkyl, or C₃₋₈ cycloalkyl, wherein the phenyl, benzyl, C₁₋₆ alkyl, C₄₋₈ heterocycloalkyl, or C₃₋₈ cycloalkyl is substituted with 0 to 3 substituents independently selected from halogen, -OH, -S(=O)₂Z^{b}, -OC₂₋₆ alkyl OZ^{a}, C₁₋₆ alkyl, C₁₋₆ haloalkyl, -OC₁₋₄ alkyl, -NH₂, -CN, or -NZ^{a}Z^{a}; and
Z^{c} may each be independently hydrogen or a C₁₋₆ alkyl, or the group CZ^{c}Z^{c} may form a C₃₋₈ cycloalkyl ring.

Specifically, Ar¹ may be substituted with at least one of a linear or branched C₁₋₆ alkyl, naphthyl, phenyl, naphthyl, anthracenyl, phenanthrenyl, pyrenyl, furanyl, indole, chromone, quinoline, carbazole or thiophenyl, which may unsubstituted or substituted with at least one of hydroxyl, halogen, C₁₋₆ alkyl, C₃₋₁₀cycloalkyl, C₁₋₆ alkoxy, C₁₋₆ haloalkyl, C₁₋₆ haloalkoxy, C₂₋₆ alkenyl, -C₂₋₆ alkynyl, C₆₋₁₂ aryl, or C₅₋₁₂ heteroaryl.

More specifically, Ar¹ may be C₁₋₆ linear or branched alkyl, 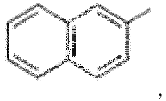 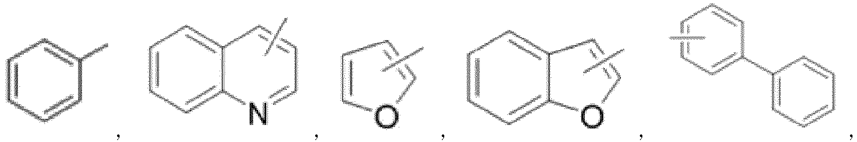 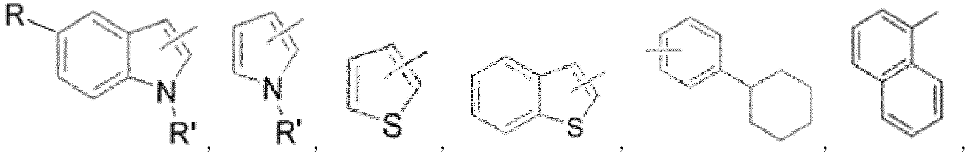 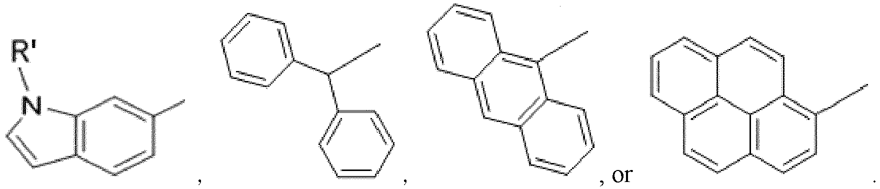 In the above formulas,
L¹ may be a direct bond or C₁₋₁₀ alkylene. Specifically, L¹ may be C₁₋₆ alkylene, and more specifically, C₁₋₃ alkylene.
A¹, A², A³, or A⁴ may each be independently CR or N.
R or R' may each be independently hydrogen, hydroxyl, halogen, C₆₋₁₂ aryl, C₅₋₁₂ heteroaryl, -C₁₋₆ alkyl, -C₃₋₁₀ cycloalkyl, -C₁₋₆ alkoxy, -C₂₋₆ alkenyl or -C₂₋₆ alkynyl (here, -C₁₋₆ alkyl, -C₃₋₁₀cycloalkyl, -C₂₋₆ alkenyl or -C₂₋₆ alkynyl may be substituted with 0 to 3 substituents independently selected from C₁₋₆ haloalkyl, halogen, cyano, nitro, C₆₋₁₂ aryl, or C₅₋₁₂ heteroaryl).
n and n' may each be independently an integer of 0 to 13.
m and y may each be independently an integer of 0 to 2. Specifically, m may be 1, and y may be 1 or 2.
R¹ or R² may each be independently hydrogen, C₁₋₆ alkyl, C₁₋₆ alkoxy, C₁₋₆ alkoxycarbonyl, halo C₁₋₆ alkyl, C₂₋₆ alkenyl, C₄₋₁₅ monocyclic or bicyclic ring (which is C₃₋₁₀ cycloalkyl; C₆₋₁₀ aryl; 5- or 6-membered saturated or partially saturated hetero ring containing 0 to 3 heteroatoms, independently selected from N, O and S; 5- or 6-membered aromatic hetero ring containing 0 to 3 heteroatoms (provided that two or more of the heteroatoms are not O or S), independently selected from N, O and S; or 7- to 15-membered saturated, partially saturated, or unsaturated hetero ring containing 0 to 3 heteroatoms, independently selected from N, O and S);
wherein R¹ and R² may be connected to form 5- to 12-membered monocyclic or bicyclic ring.

Specifically, R₁ or R₂ may each be independently hydrogen, C₁₋₆ alkyl, benzyl, naphthylalkyl, benzofuranylalkyl, quinolinylalkyl, pyridinylalkyl, cyclohexylalkyl, thiophenylalkyl, pyrrolylalkyl, furanylalkyl or benzothiophenylalkyl, which may be unsubstituted or may each be independently substituted with at least one of C₁₋₆ alkyl, C₃₋₆ cycloalkyl, C₆₋₁₂ aryl, hydroxyl, halogen, C₁₋₆ alkoxy, C₁₋₆ haloalkyl, or C₁₋₆ haloalkoxy; wherein R¹ and R² may be connected to form a 5- to 12-membered monocyclic or bicyclic ring.

More specifically, R₁ or R₂ is each independently hydrogen, C₁₋₆ linear or branched alkyl, 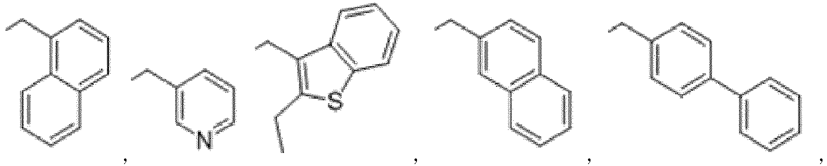 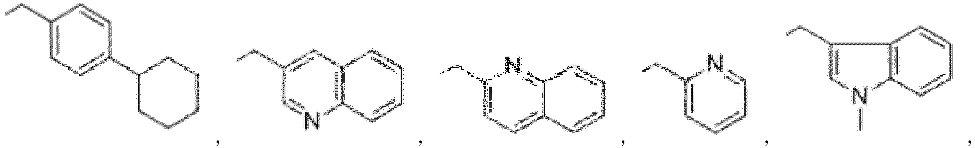 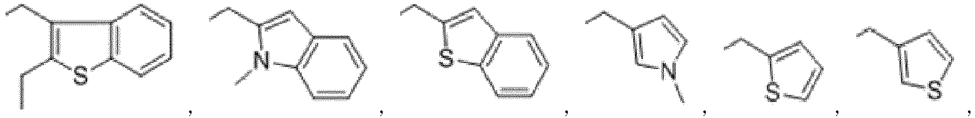 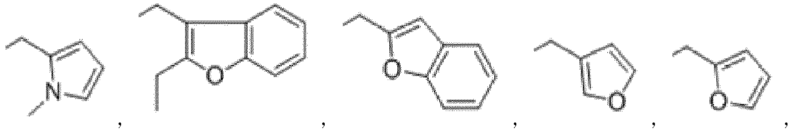 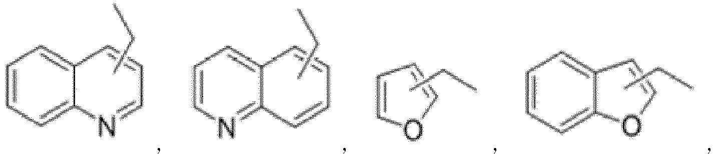 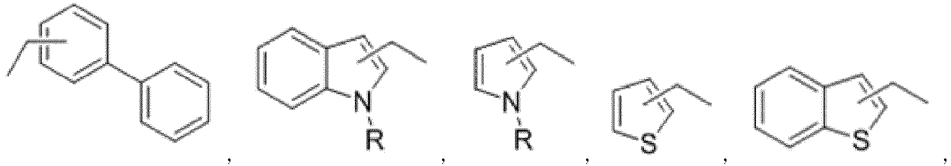 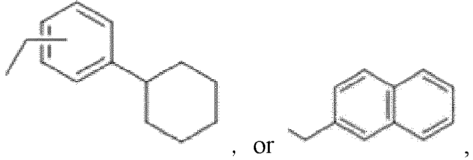 wherein R¹ and R² may be connected to form a 5-to 12-membered monocyclic or bicyclic ring.

The term "independently" used herein means that independently applied variables vary independently from application to application. Therefore, for a compound such as R^{a}XYR^{a}, when R^{a} is "independently carbon or nitrogen," R^{a} at both sides may be carbon, R^{a} at both sides may be nitrogen, or one R^{a} may be carbon, and the other R^{a} may be nitrogen.

The term "alkyl" used herein typically refers to, unless specified otherwise, a C₁ to C₁₀ saturated linear or branched hydrocarbon chain, and specifically includes methyl, ethyl, propyl, isopropyl, butyl, isobutyl, t-butyl, pentyl, isopentyl, neopentyl, hexyl, isohexyl, cyclohexyl, cyclohexylmethyl, 3-methylpentyl, 2,2-dimethylbutyl, 2,3-dimethylbutyl, and the like. The term includes both substituted and unsubstituted alkyl groups. The alkyl group may be optionally substituted with one or more moieties selected from the group consisting of hydroxyl, amino, alkylamino, arylamino, alkoxy, aryloxy, nitro, cyano, sulfonic acid, sulfate, phosphoric acid, phosphate, or phosphonate. One or more hydrogen atoms attached to the carbon atoms of the alkyl may be substituted with one or more halogen atoms such as fluorine or chlorine, such as trifluoromethyl, difluoromethyl, fluorochloromethyl and so on. The hydrocarbon chain may also include a heteroatom such as N, O or S in the middle.

The term "cycloalkyl" used herein refers to, unless specified otherwise, a saturated hydrocarbon ring having 3 to 8 carbon atoms, like cyclopropyl, cyclobutyl, cyclopentyl and cyclohexyl and preferably 3 to 6 carbon atoms. The cycloalkyl group may have a ring substituted with an alkyl group like cyclopropylmethyl and so on.

The term "alkylamino" or "arylamino" refers to an amino group having one or two alkyl or aryl substituents.

The term "protected" used herein refers to, unless defined otherwise, a group added to an oxygen, nitrogen or phosphorous atom for preventing additional reaction or for a different purpose. A wide variety of oxygen and nitrogen protecting groups are known to those of ordinary skill in the art in the field of organic synthesis. Non-limiting examples include C(O)-alkyl, C(O)Ph, C(O)aryl, CH₃, CH₂-alkyl, CH₂- alkenyl, CH₂Ph, CH₂-aryl, CH₂O-alkyl, CH₂O-aryl, SO₂-alkyl, SO₂-aryl, tert-butyldimethylsilyl, tert-butyldiphenylsilyl, and 1,3-(1,1,3,3-tetraisopropyldisiloxanylidene).

The "aryl" used herein refers to a C₆~C₂₀ aryl group, and specifically, a C₆~C₁₂ aryl group, and includes an aromatic ring group having 6 to 20 carbon atoms and that does not contain a heteroatom in the ring. The aryl group refers to, specifically, phenyl, biphenyl, naphthyl, anthracenyl, phenanthrenyl, pyrenyl, chrysenyl or picenyl, and more specifically, phenyl, biphenyl, naphthyl, anthracenyl, phenanthrenyl or pyrenyl. This term includes both substituted and unsubstituted moieties. The aryl group may be substituted with one or more substituents including unprotected or protected hydroxyl, halogen, amino, alkylamino, arylamino, alkoxy, aryloxy, nitro, cyano, sulfonic acid, sulfate, phosphoric acid, phosphate, or phosphonate as needed, but the present invention is not limited thereto.

The "alkaryl" or "alkylaryl" refers to an alkyl group having an aryl substituent. The term "aralkyl" or "arylalkyl" refers to an aryl group having an alkyl substituent, for example, benzyl.

The term "halo" used herein includes chloro, bromo, iodo, and fluoro.

The term "acyl ester" or "O-linked ester" used herein refers to a carboxylic acid ester of C(O)R', in which the non-carbonyl moiety R' of the ester group is linear or branched alkyl, or cycloalkyl or lower alkyl, alkoxyalkyl including methoxymethyl, aralkyl including benzyl, aryloxyalkyl such as phenoxymethyl, aryl including phenyl optionally substituted with a halogen(F, Cl, Br, I), C₁ to C₄ alkyl or C₁ to C₄ alkoxy, a sulfonate ester such as alkyl including methanesulfonyl, mono-, di- or triphosphate ester, or aralkyl sulfonyl, trityl or monomethoxytrityl, substituted benzyl, trialkylsilyl (e.g., dimethyl-t-butylsilyl), or diphenylmethylsilyl. The aryl group in the ester optionally includes a phenyl group.

The term "acyl" refers to a group of R"C(O)-, wherein R" is linear or branched alkyl, or cycloalkyl, amino acid, aryl including phenyl, alkylaryl, aralkyl including benzyl, alkoxyalkyl including methoxymethyl, aryloxylalkyl such as phenoxymethyl; or substituted alkyl (including lower alkyl), aryl including phenyl optionally substituted with chlorine, bromine, fluorine or iodine, C₁ to C₄ alkyl or C₁ to C₄ alkoxy, sulfonate esters such as alkyl or aralkyl sulfonyl including methanesulfonyl, the mono-, di- or triphosphate ester, trityl or monomethoxy-trityl, substituted benzyl, alkaryl, aralkyl including benzyl, alkoxyalkyl including methoxymethyl, aryloxyalkyl such as phenoxymethyl. Aryl groups in the esters optimally include a phenyl group. Particularly, the acyl groups include acetyl, trifluoroacetyl, methylacetyl, cyclopropylacetyl, cyclopropyl carboxy, propionyl, butyryl, isobutyryl, hexanoyl, heptanoyl, octanoyl, neo-heptanoyl, phenylacetyl, 2-acetyoxy-2-phenylacetyl, diphenylacetyl, α-methoxy-α-trifluoromethyl-phenylacetyl, bromoacetyl, 2-nitro-benzeneacetyl, 4-chloro-benzeneacetyl, 2-chloro-2,2-diphenylacetyl, 2-chloro-2-phenylacetyl, trimethylacetyl, chlorodifluoroacetyl, perfluoroacetyl, fluoroacetyl, bromodifluoroacetyl, methoxyacetyl, 2-thiopheneacetyl, chlorosulfonylacetyl, 3-methoxyphenylacetyl, phenoxyacetyl, tert-butylacetyl, trichloroacetyl, monocloro-acetyl, dichloroacetyl, 7H-dodecafluoro-heptanoyl, perfluoro-heptanoyl, 7H-dodeca-fluoroheptanoyl, 7-chlorododecafluoro-heptanoyl, 7-chloro-dodecafluoro-heptanoyl, 7H-dodecafluoroheptanoyl, 7H-dodeca-fluoroheptanoyl, nona-fluoro-3,6-dioxa-heptanoyl, nonafluoro-3,6-dioxaheptanoyl, perfluoroheptanoyl, methoxybenzoyl, methyl 3-amino-5-phenylthiophene-2-carboxyl, 3,6-dichloro-2-methoxy-benzoyl, 4-(1,1,2,2-tetrafluoro-ethoxy)-benzoyl, 2-bromo-propionyl, omega-aminocapryl, decanoyl, n-pentadecanoyl, stearyl, 3-cyclopentyl-propionyl, 1-benzene-carboxyl, O-acetylmandelyl, pivaloyl acetyl, 1-adamantane-carboxyl, cyclohexane-carboxyl, 2,6-pyridinedicarboxyl, cyclopropane-carboxyl, cyclobutane-carboxyl, perfluorocyclohexyl carboxyl, 4-methylbenzoyl, chloromethyl isoxazolyl carbonyl, perfluorocyclohexyl carboxyl, crotonyl, 1-methyl-1H-indazole-3-carbonyl, 2-prophenyl, isovaleryl, 1-pyrrolidinecarbonyl, and 4-phenylbenzoyl. When the term acyl is used, it is meant to specifically and independently disclose acetyl, trifluoroacetyl, methylacetyl, cyclopropylacetyl, propionyl, butyryl, isobutyryl, hexanoyl, heptanoyl, octanoyl, neo-heptanoyl, phenylacetyl, diphenylacetyl, ct-trifluoromethyl-phenylacetyl, bromoacetyl, 4-chloro-benzylacetyl, 2-chloro-2,2-diphenylacetyl, 2-chloro-2-phenylacetyl, trimethylacetyl, chlorodifluoroacetyl, perfluoroacetyl, fluoroacetyl, bromodifluoroacetyl, 2-thiopheneacetyl, tert-butylacetyl, trichloroacetyl, monochloro-acetyl, dichloroacetyl, methoxybenzoyl, 2-bromo-propionyl, decanoyl, n-pentadecanoyl, stearyl, 3-cyclopentyl-propionyl, 1-benzene-carboxyl, pivaloyl acetyl, 1-adamantane-carboxyl, cyclohexane-carboxyl, 2,6-pyridinedicarboxy, cyclopropane-carboxyl, cyclobutane-carboxyl, 4-methylbenzoyl, crotonyl, 1-methyl-1H-indazole-3-carbonyl, 2-propenyl, isovaleryl, or 4-phenylbenzoyl.

The term "C₁~C₆ alkyl" used herein refers to linear or branched alkyl having 1 to 6 carbon atoms, and includes methyl, ethyl, propyl, isopropyl, butyl, isobutyl, sec-butyl, tert-butyl, amyl, hexyl, and the like, but the present invention is not limited thereto, and it is preferably ethyl, propyl, isopropyl, butyl, isobutyl, sec-butyl, or tert-butyl.

The term "C₁~C₆ alkoxy" refers to linear or branched alkoxy having 1 to 6 carbon atoms, and includes methoxy, ethoxy, propoxy, isopropoxy, butoxy, and the like, but the present invention is not limited thereto.

The term "C₂~C₆ alkenyl" used herein refers to linear or branched alkenyl that has 2 to 6 carbon atoms and contains one double bond, and includes vinyl, propenyl, butenyl, isobutenyl, pentenyl, hexenyl, and the like, but the present invention is not limited thereto.

The term "C₂~C₆ alkynyl" used herein refers to linear or branched alkynyl that has 2 to 6 carbon atoms and contains one triple bond, and includes ethnyl, propynyl, butynyl, isobutynyl, pentynyl, hexynyl, and the like, but the present invention is not limited thereto.

The term "C₃~C₁₀ cycloalkyl" used herein refers to cyclic alkyl that has 3 to 10 carbon atoms in the ring, and includes cyclopropyl, cyclobutyl, cyclopentyl, cyclohexyl, cycloheptyl, cyclooctyl, cyclodecyl, and the like. The terms "C₃~C₈ cycloalkyl," "C₃~C₇ cycloalkyl" and "C₃~C₆ cycloalkyl" have similar connotations.

The term "C₃~C₁₀ cycloalkenyl" used herein refers to cyclic alkenyl that has 3 to 10 carbon atoms in the ring, and includes cyclopropenyl, cyclobutenyl, cyclopentenyl, cyclohexenyl, cycloheptene, cyclooctene, cyclodecene, and the like, but the present invention is not limited thereto. The term "C₃~C₇ cycloalkenyl" has a similar connotation.

The term "3- to 12-membered heterocyclic" used herein refers to a saturated or unsaturated 3- to 12-membered ring group containing 1 to 3 heteroatoms selected from oxygen, sulfur, and nitrogen in the ring, and for example, is dioxol. The term "3~7-membered heterocyclic" has a similar connotation.

The term "alkylene" used herein refers to a divalent hydrocarbyl group. This is because it is divalent and thus can be linked with two other groups. Generally, it is -(CH₂)ₙ-, wherein n is 1 to 8, and preferably 1 to 4, but in specific cases, the alkylene may also be substituted with other groups and may have different lengths, and open valences does not necessarily need to be on opposite sides of the chain.

In general, any alkyl, alkenyl, alkynyl, acyl, aryl or arylalkyl group included in the substituent may itself be optionally substituted with different additional substituents. Unless otherwise described herein, the nature of these substituents is similar to that recited for the primary substituents themselves.

The term "heteroatom" used herein refers to an atom other than carbon or hydrogen, for example, nitrogen, oxygen or sulfur. When the heteroatom is a part of the backbone or skeleton of a chain or a ring, the heteroatom should be at least divalent, and may be generally selected from N, O, P, and S.

The term "may be substituted" used in this specification indicates that a specific group(s) said to be optionally substituted may not have non-hydrogen substituents, or the specific group(s) may have one or more non-hydrogen substituents whose chemical and pharmacological activities correspond to those of the resulting molecules. Unless otherwise stated, the total number of these substituents that may be present in the specific group(s) is identical to the total number of hydrogen atoms present in an unsubstituted form of the group(s) being described; and may be present in a smaller number than the maximum number of these substituents. When an optional substituent is bound through a double bond, for example, carbonyl oxygen (C = O), this group takes two available valences on carbon atoms to which the optional substituent is attached. Therefore, the total number of the substituents that may be included in the group decreases with the number of available valences. In the present application, the term "substituted" indicates that one or more hydrogen atoms are each independently replaced by substituent(s) which are the same or different from each other when the term is used to modify specified groups, moieties or radicals, whether it is used as a portion of the term "may be substituted" or used otherwise.

Non-limiting examples of substituents useful for substituting saturated carbon atoms in specific groups, moieties or radicals may include -Z^{a}, =O, -OZ^{b}, -SZ^{b}, =S-, -NZ^{c}Z^{c}, =NZ^{b}, =N-OZ^{b}, trihalomethyl, -CF₃, -CN, -OCN, -SCN, -NO, -NO₂, =N₂, -N₃, -S(O)₂Z^{b}, -S(O)₂NZ^{b}, - S(O₂)O-, -S(O₂)OZ^{b}, -OS(O₂)OZ^{b}, -OS(O₂)O-, -OS(O₂)OZ^{b}, -P(O)(O-)₂, -P(O)(OZ^{b})(O-), - P(O)(OZ^{b})(OZ^{b}), -C(O)Z^{b}, -C(S)Z^{b}, -C(NZ^{b})Z^{b}, -C(O)O-, -C(O)OZ^{b}, -C(S)OZ^{b}, -C(O)NZ^{c}Z^{c}, - C(NZb)NZ^{c}Z^{c}, -OC(O)Zb, -OC(S)Z^{b}, -OC(O)O-, -OC(O)OZ^{b}, -OC(S)OZ^{b}, -NZ^{bC}(O)Z^{b}, - NZ^{bC}(S)Z^{b}, -NZ^{bC}(O)O-, -NZ^{bC}(O)OZ^{b}, -NZ^{bC}(S)OZ^{b}, -NZ^{bC}(O)NZ^{c}Z^{c}, -NZ^{bC}(NZ^{b})Z^{b}, and - NZ^{bC}(NZ^{b})NZ^{c}Z^{c}, wherein Z^{a} is selected from the group consisting of alkyl, cycloalkyl, heteroalkyl, cycloheteroalkyl, aryl, arylalkyl, heteroaryl and heteroarylalkyl; Z^{b} is independently hydrogen or Z^{a}; and Z^{c} is independently Z^{b}, or two Z^{c}' may form a 4-, 5-, 6- or 7-membered cycloheteroalkyl ring structure with nitrogen atoms binding thereto, and the ring structure may optionally include 1 to 4 heteroatoms, which are the same or different from any one selected from the group consisting of N, O, and S. As a special example, -NZ^{c}Z^{c} is intended to include, but not limited to, -NH₂, -NH-alkyl, -N-pyrrolidinyl, and -N-morpholinyl, and includes other substituents known in the art. Likewise, as other specific examples, a substituted alkyl is intended to include, but not limited to, -alkylene-O-alkyl, -alkylene-heteroaryl, -alkylene-cycloheteroaryl, -alkylene-C(O)OZ^{b}, -alkylene-C(O)NZ^{b}Z^{b}, and -CH₂-CH₂-C(O)-CH₃, and includes other alternatives known in the art. One or more substituents, together with the atoms to which they are bound, may form a cyclic ring including cycloalkyl and cycloheteroalkyl as non-limiting examples.

Likewise, non-limiting examples of substituents useful for substituting unsaturated carbon atoms in specific groups, moieties or radicals may include -Z^{a}, halo, -O-, -OZ^{b}, -SZ^{b}, -S-, -NZ^{c}Z^{c}, trihalomethyl, -CF₃, -CN, -OCN, -SCN, -NO, -NOz, -N₃, -S(O)2Z^{b}, -S(Oz)O-, - S(O₂)OZ^{b}, -OS(O₂)OZ^{b}, -OS(O₂)O-, -P(O)(O-)₂, -P(O)(OZ^{b})(O-), -P(O)(OZ^{b})(OZ^{b}), -C(O)Z^{b}, - C(S)Z^{b}, -C(NZ^{b})Z^{b}, -C(O)O-, -C(O)OZ^{b}, -C(S)OZ^{b}, -C(O)NZ^{c}Z^{c}, -C(NZ^{b})NZ^{c}Z^{c}, -OC(O)Z^{b}, - OC(S)Z^{b}, -OC(O)O-, -OC(O)OZ^{b}, -OC(S)OZ^{b}, -NZ^{bc}(O)OZ^{b}, -NZ^{bc}(S)OZ^{b}, -NZ^{bc}(O)NZ^{c}Z^{c}, - NZ^{bc}(NZ^{b})Z^{b}, and -NZ^{bc}(NZb)NZ^{c}Z^{c}, wherein Z^{a}, Z^{b}, and Z^{c} are defined as above.

Likewise, non-limiting examples of substituents useful for substituting nitrogen atoms in heteroalkyl and cycloheteroalkyl groups may include-Z^{a}, halo, -O-, -OZ^{b}, -SZ^{b}, -S-, -NZ^{c}Z^{c}, trihalomethyl, -CF₃, -CN, -OCN, -SCN, -NO, -NOz, -N₃, -S(O)2Z^{b}, -S(Oz)O-, -S(O₂)OZ^{b}, - OS(O₂)OZ^{b}, -OS(O₂)O-, -P(O)(O-)₂, -P(O)(OZ^{b})(O-), -P(O)(OZ^{b})(OZ^{b}), -C(O)Z^{b}, -C(S)Z^{b}, - C(NZ^{b})Z^{b}, -C(O)O-, -C(O)OZ^{b}, -C(S)OZ^{b}, -C(O)NZ^{c}Z^{c}, -C(NZ^{b})NZ^{c}Z^{c}, -OC(O)Z^{b}, -OC(S)Z^{b}, - OC(O)O-, -OC(O)OZ^{b}, -OC(S)OZ^{b}, -NZ^{bc}(O)OZ^{b}, -NZ^{bc}(S)OZ^{b}, -NZ^{bc}(O)NZ^{c}Z^{c}, -NZ^{bc}(NZ^{b})Z^{b}, and -NZ^{bc}(NZb)NZ^{c}Z^{c}, wherein Z^{a}, Z^{b}, and Z^{c} are defined as above.

Because the compounds described in the present application may have one or more chiral centers and/or double bonds, the compounds may be present as stereoisomers, for example double-bond isomers (i.e., geometrical isomers, for example, E and Z), enantiomers or diastereomers. Also, the present application encompasses each isolated stereoisomeric form (for example, enantiomerically pure isomers, E and Z isomers, and other alternatives to the stereoisomers) and mixtures of stereoisomers having different chiral purities and E and Z percentages (unless otherwise specified as specific stereoisomers). Therefore, the chemical structures described in the present application encompass all possible enantiomers and stereoisomers of the described compounds, including stereoisomerically pure forms (for example, geometrically pure, enantiomerically pure, or diastereomerically pure forms), enantiomeric mixtures and stereoisomeric mixtures. The enantiomeric mixtures and the stereoisomeric mixtures may be divided into their corresponding enantiomeric or stereoisomeric components using separation technology or chiral synthesis technology well-known in the art. The present application also encompasses the individually isolated stereoisomeric forms, and a mixture of stereoisomers having different chiral purities, including a racemic mixture. The present application also encompasses various diastereomers. Other structures may appear to depict a specific isomer, but this is only for the sake of convenience. Therefore, it should be understood that the present application is not intended to be limited to such isomers specifically described as such. When a chemical name does not specify the isomeric form of a compound, it refers to all possible isomeric forms of the compound, or a mixture thereof.

Also, the words "comprise," "comprising," "include," "including," "encompass," and "encompassing" used herein and in the following claims are intended to specify the presence of stated features, integers, components, or steps, but do not preclude the presence or addition of one or more other features, integers, components, steps, or groups.

The time, when used with temperature, the term "approximately" refers to ±5 hours, for example, ±1 hour.

The temperature, when used with temperature, the term "approximately" refers to ±5 °C, for example, ±1 °C. The percentage or other values, when used with temperature, the term "approximately" indicates ±10% of the stated percentage or value, for example, ±5%.

The term "treating" or "treatment" used herein refers to a therapeutic agent, or a preventive, palliative or prophylactic method. For the purposes of the present application, beneficial or desired clinical outcomes include, but are not limited to, detectable or undetectable, symptom relief, attenuation of the severity of a disease, a stable (i.e., non-attenuated) state of the disease, a slow progression or delay in the progression of the disease, or mitigation, improvement, temporary treatment, or alleviation of the disease or remission (whether partial or total). The term "treatment" may also mean prolonging survival as compared to the expected survival without any treatment. The subjects in need of treatment include those already suffering from the condition or disorder, or those in which the condition or disorder has been prevented, as well as those predisposed to having the condition or disorder.

The terms "cancer" and "cancerous" used herein typically refer to or describe the physiological condition of a mammal, which is characterized by abnormal or uncontrolled cell growth. A "tumor" includes one or more cancerous cells. Examples of the cancer include, but are not limited to, a carcinoma, a lymphoma, a blastoma, a sarcoma, and leukemia, or a malignant lymphoid tumor. More specific examples of such cancer include squamous cell carcinoma (for example, epithelial squamous cell carcinoma), lung cancer (including small-cell lung cancer, non-small cell lung cancer ("NSCLC"), lung adenocarcinoma, and lung squamous carcinoma), peritoneal cancer, hepatocellular cancer, stomach or gastric cancer including stomach cancer, pancreatic cancer, glioblastoma, cervical cancer, ovarian cancer, liver cancer, bladder cancer, hepatoma, breast cancer, colon cancer, rectal cancer, colorectal cancer, endometrial or uterine carcinoma, salivary gland carcinoma, kidney or nephritic cancer, prostate cancer, vulvar cancer, thyroid cancer, hepatocarcinoma, anal carcinoma, penile carcinoma, skin cancer including melanoma, and head and neck cancer.

The expression "pharmaceutically acceptable" indicates that the material or composition is chemically and/or toxicologically compatible with a mammal treated therewith and/or other components included in a formulation.

The expression "pharmaceutically acceptable salt" used herein refers to a pharmaceutically acceptable organic or inorganic salt of the compound of the present invention.

The expression "pharmaceutically acceptable salt" used herein refers to a pharmaceutically acceptable organic or inorganic salt of the compound described in the specification. Exemplary salts include, but are not limited to, sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucuronate, saccharate, formate, benzoate, glutamate, methanesulfonate, esylate, ethanesulfonate, ethanedisulfonate, benzenesulfonate, p-toluenesulfonate, and pamoate (i.e., 1,1'-methylene-bis-(2-hydroxy-3-naphthoate)) salts. The pharmaceutically acceptable salt may include an inclusion body of another molecule, for example, an acetate ion, a succinate ion, or a different counterion. The counterions may be any organic or inorganic moiety that stabilizes the charge of a parent compound. Furthermore, the pharmaceutically acceptable salt may have more than one charged atom in the structure thereof. When multiple charged atoms are included in a portion of the pharmaceutically acceptable salt, the pharmaceutically acceptable salt may have multiple counterions. Therefore, the pharmaceutically acceptable salt may have one or more charged atoms and/or one or more counterions.

The compound according to the present application may be, but is not limited to, at least one selected from the group consisting of compounds represented by the following formulas.

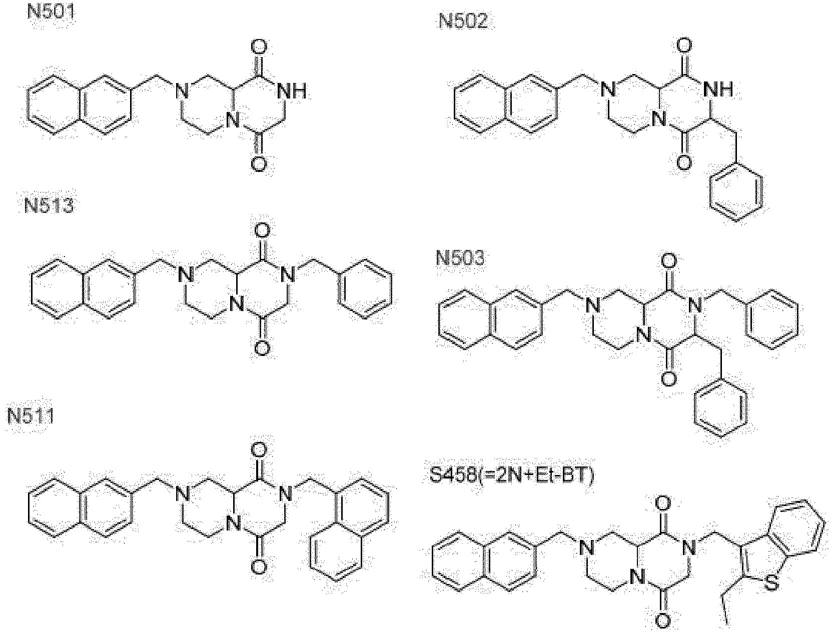

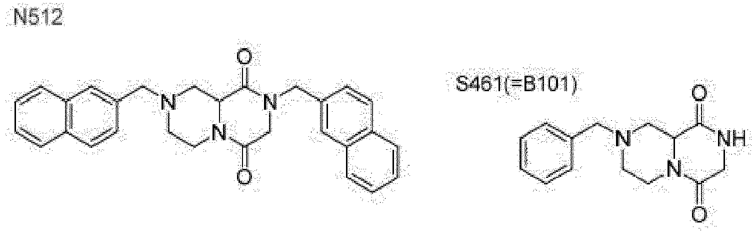

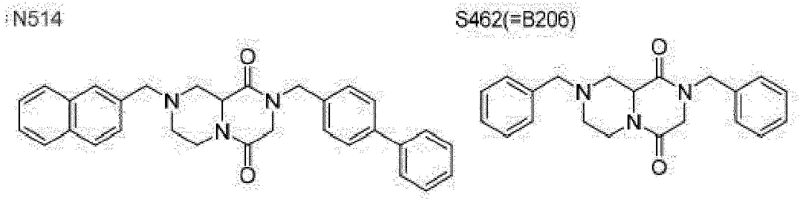

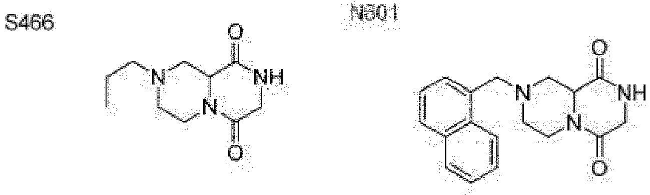

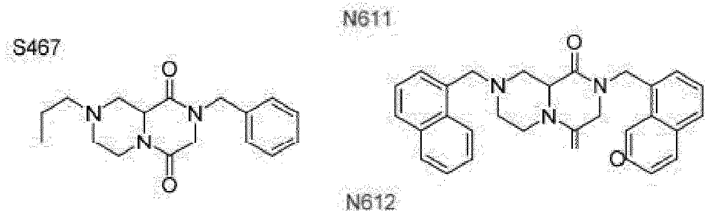

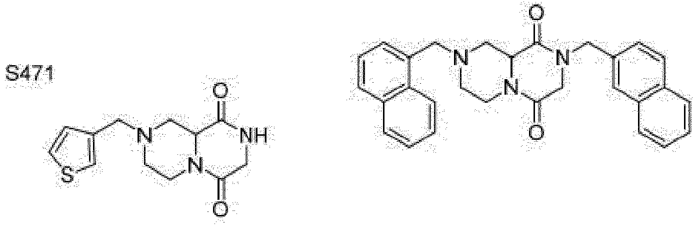

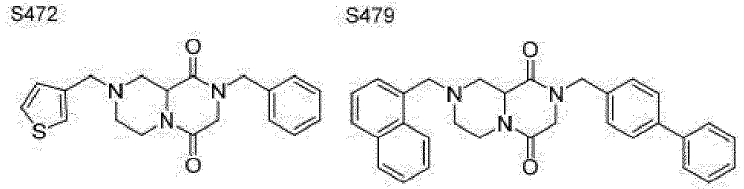

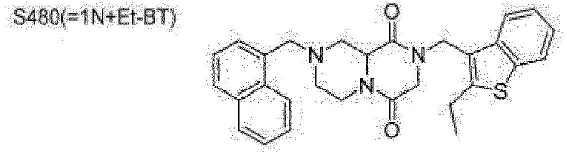

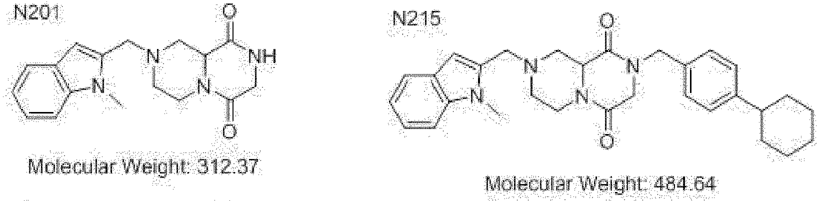

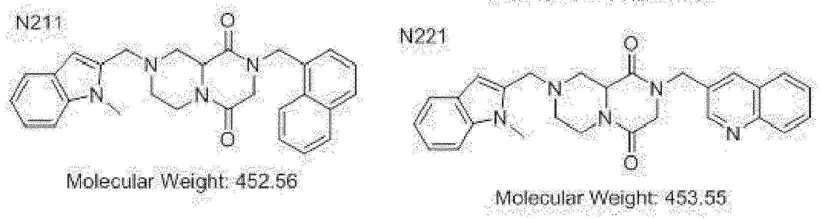

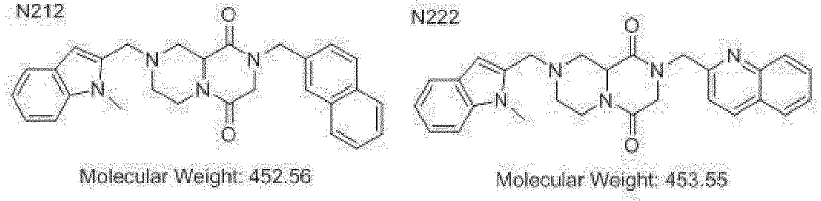

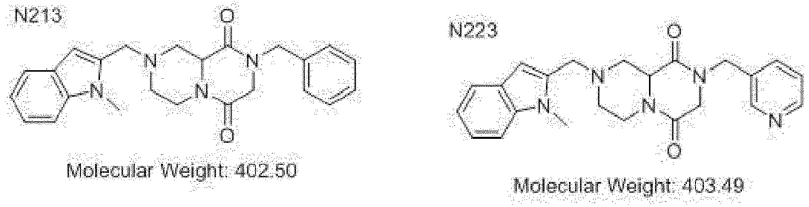

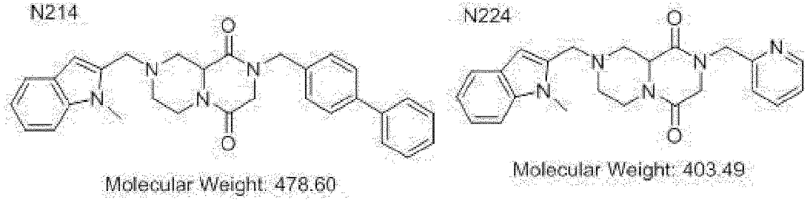

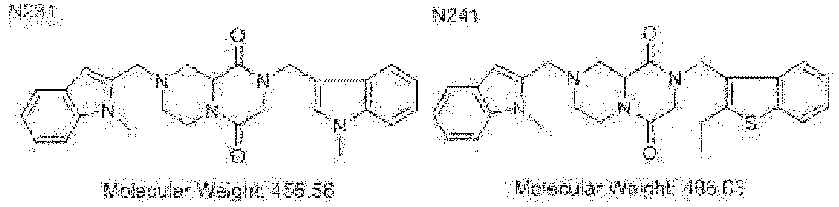

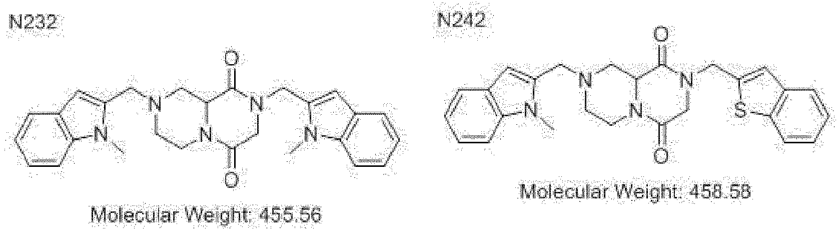

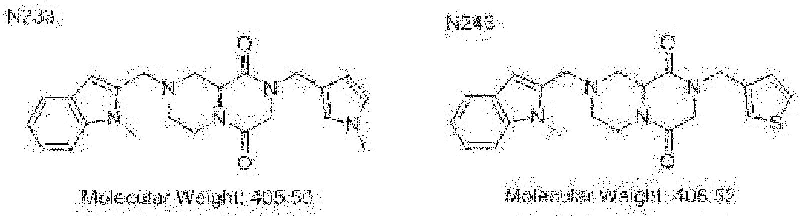

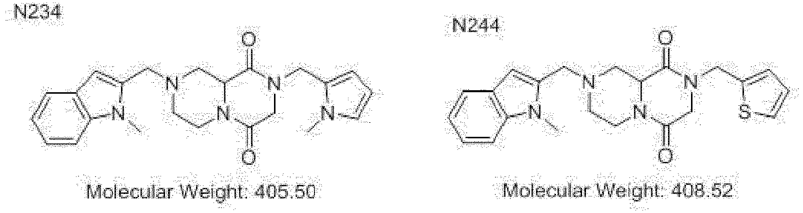

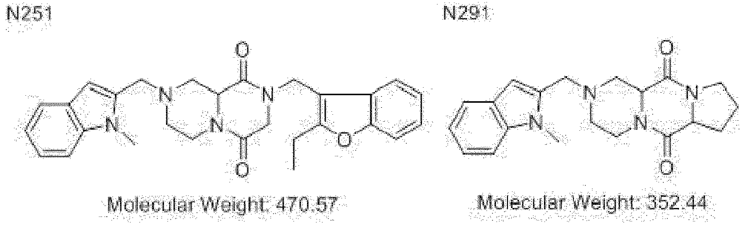

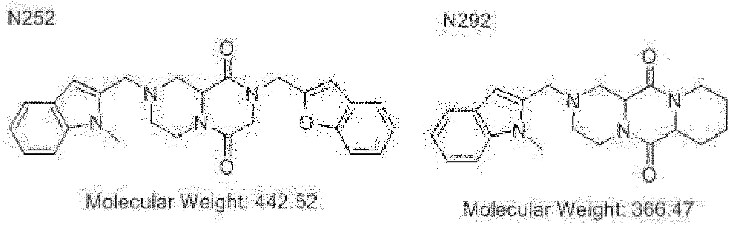

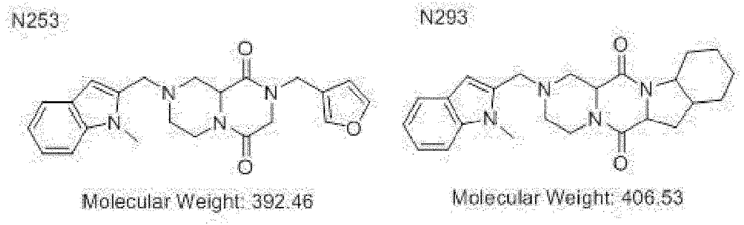

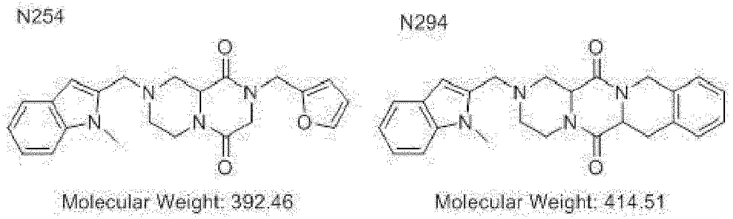

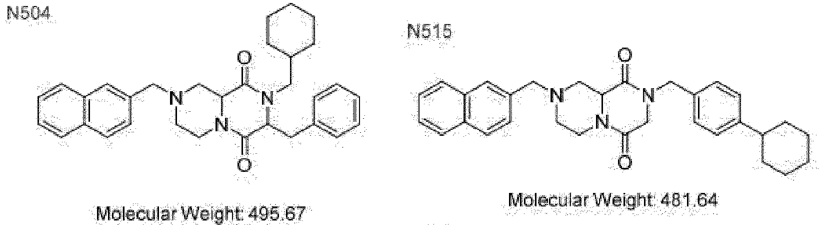

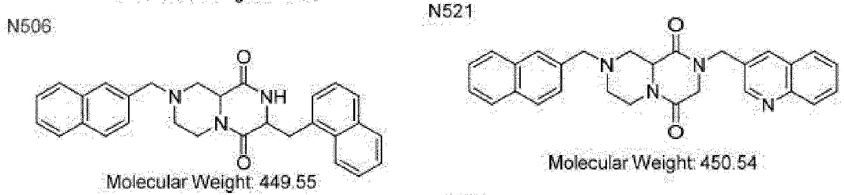

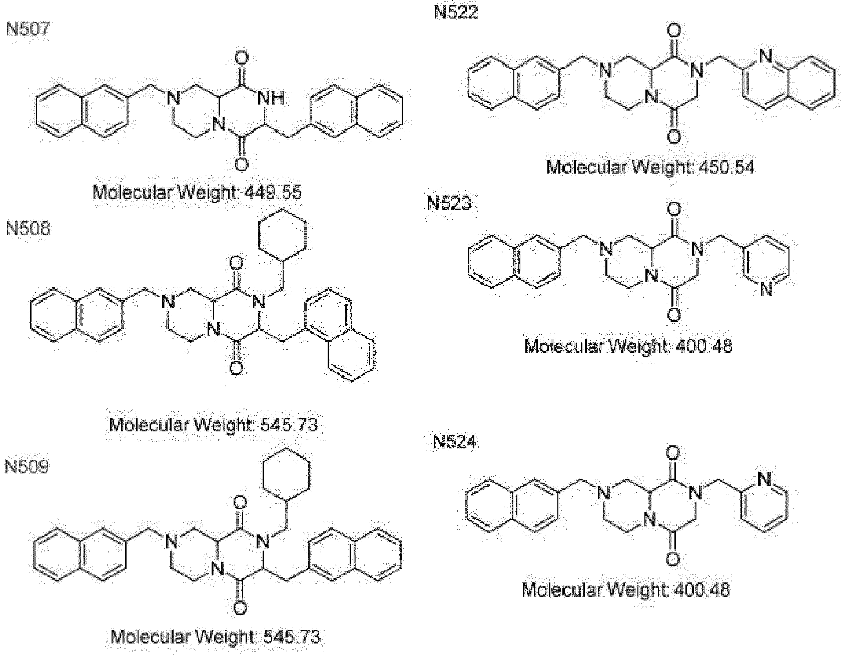

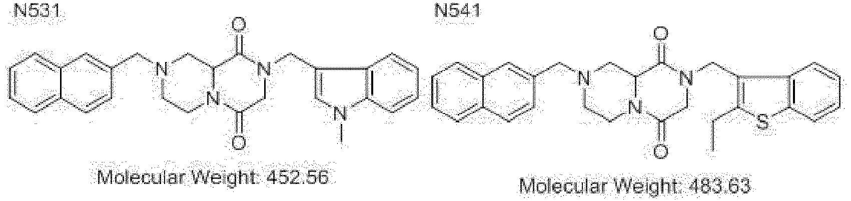

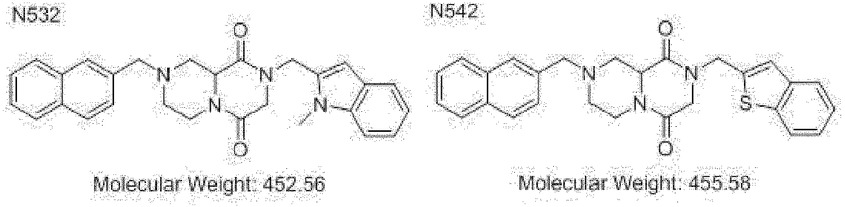

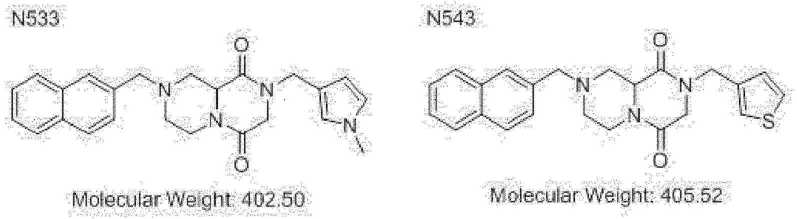

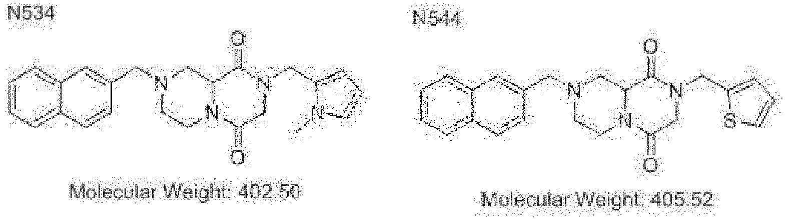

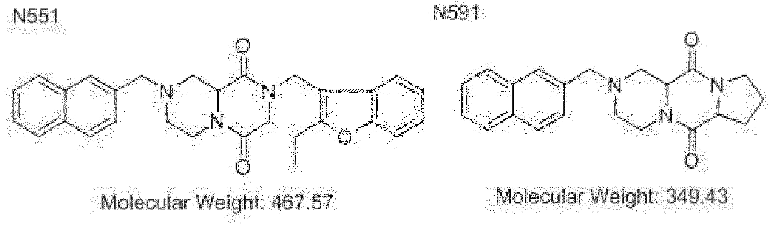

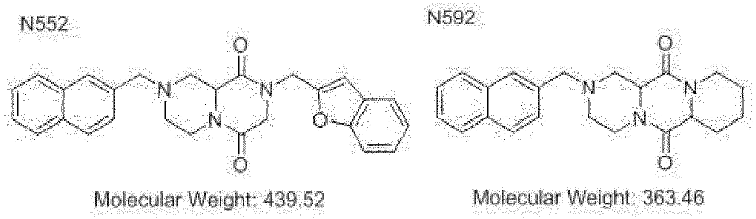

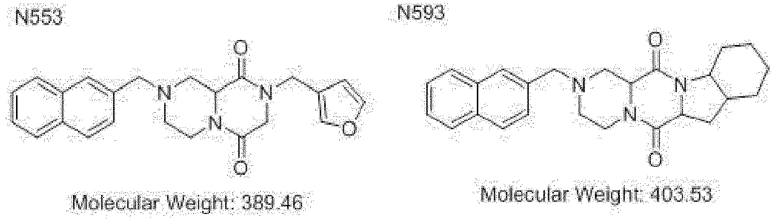

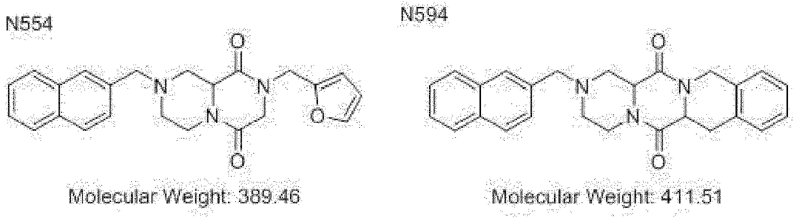

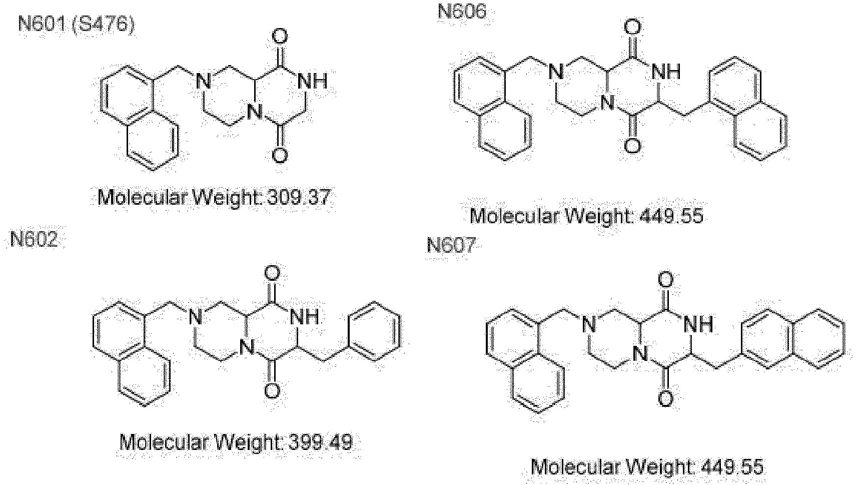

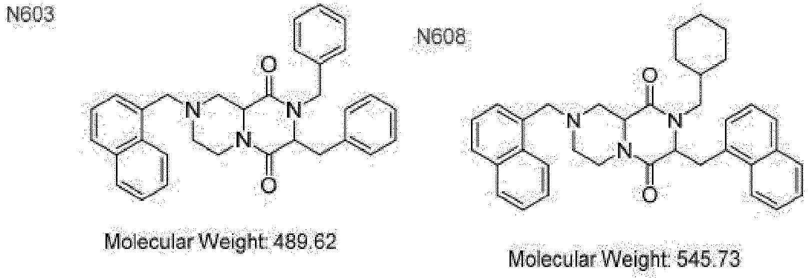

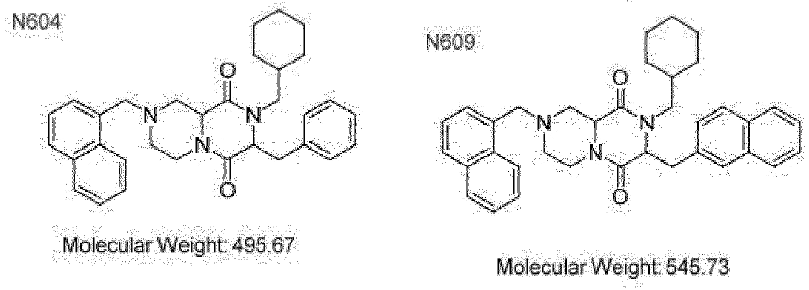

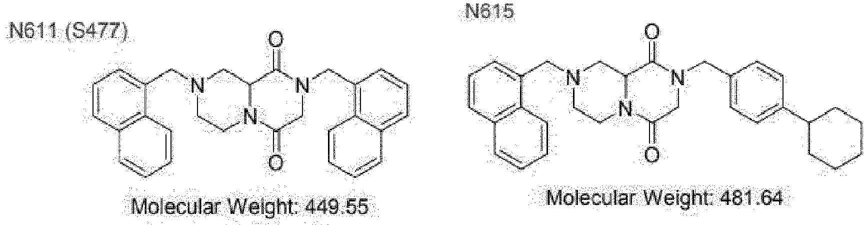

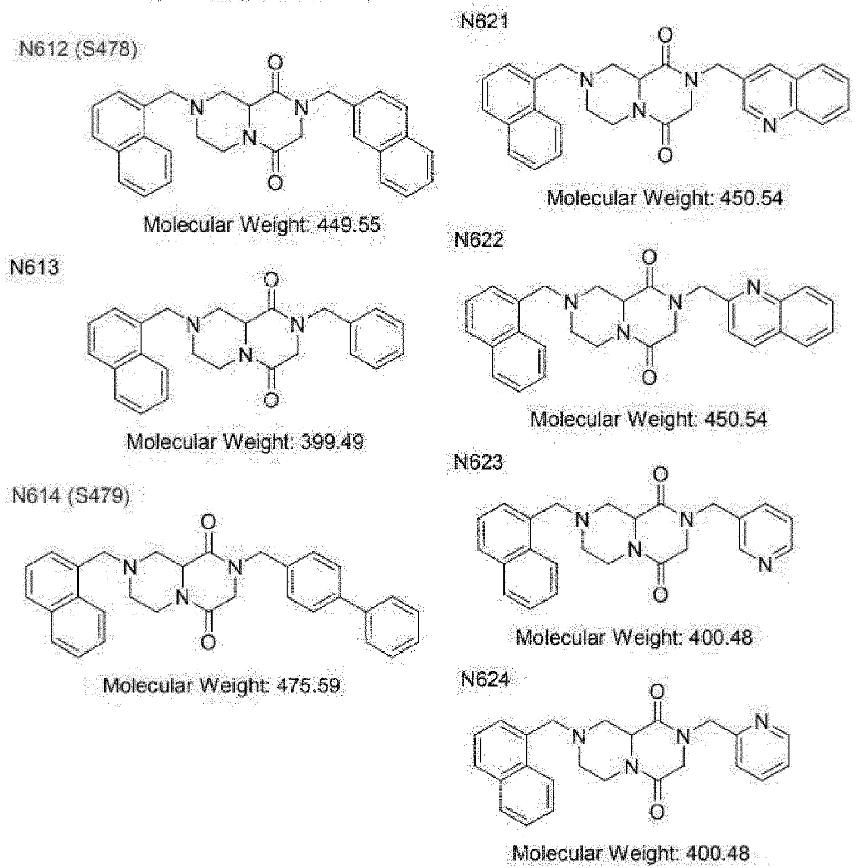

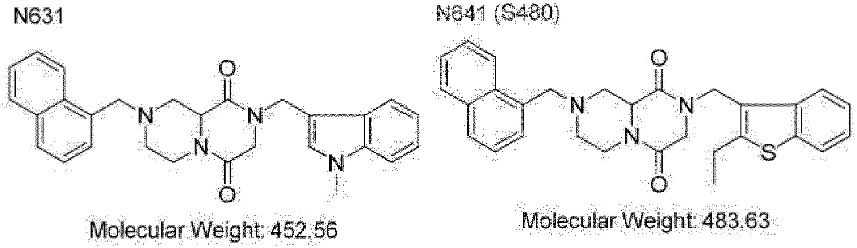

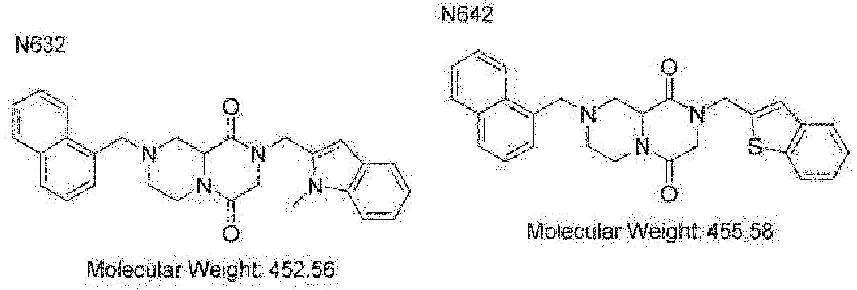

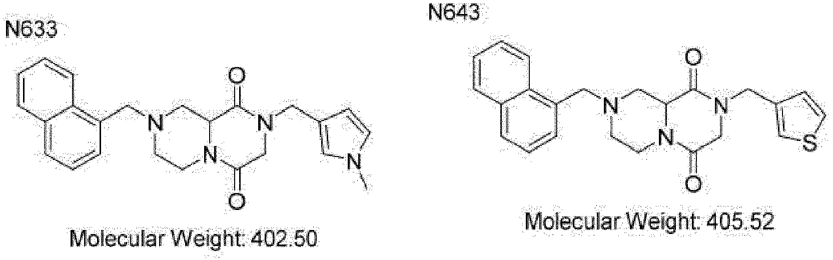

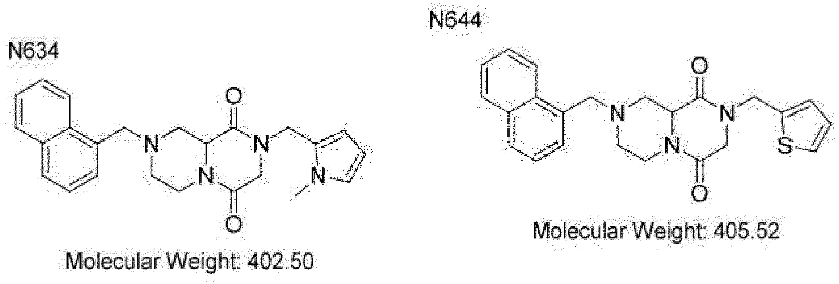

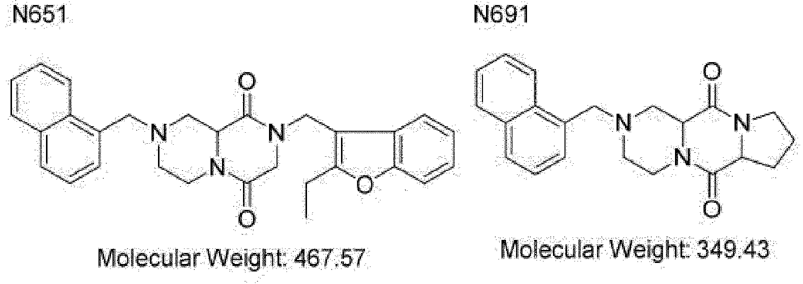

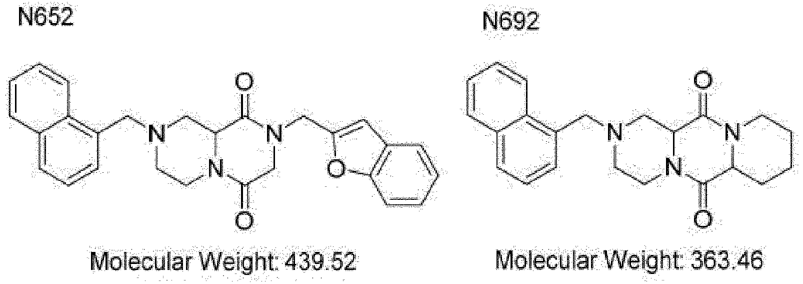

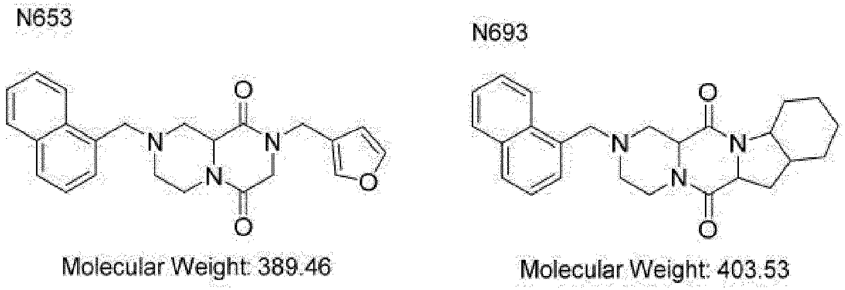

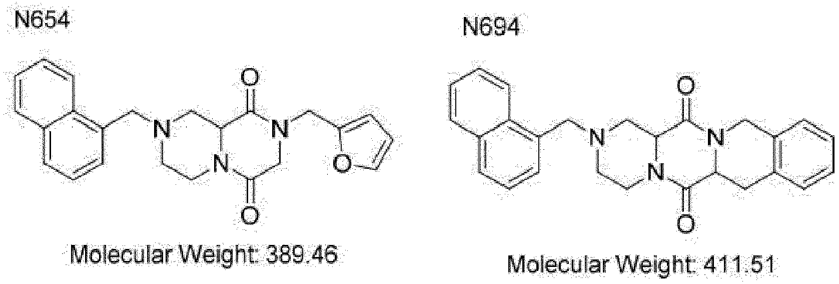

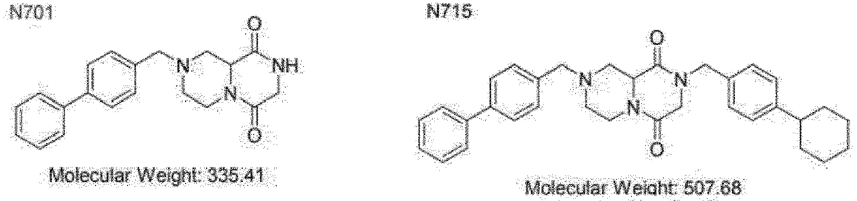

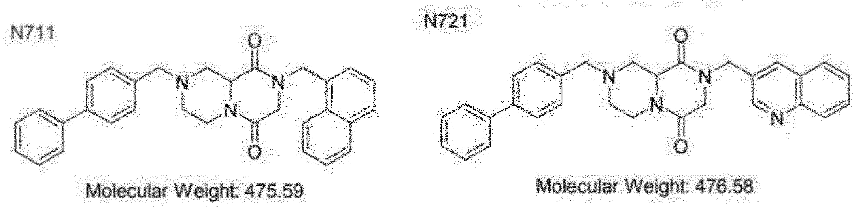

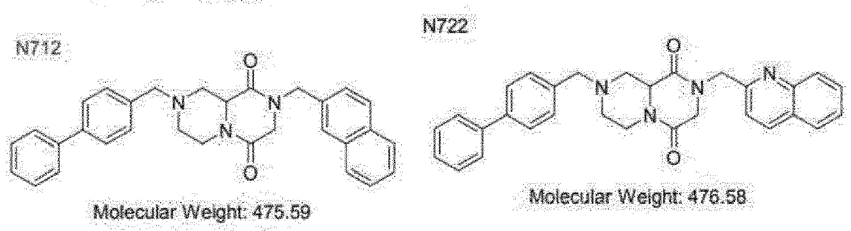

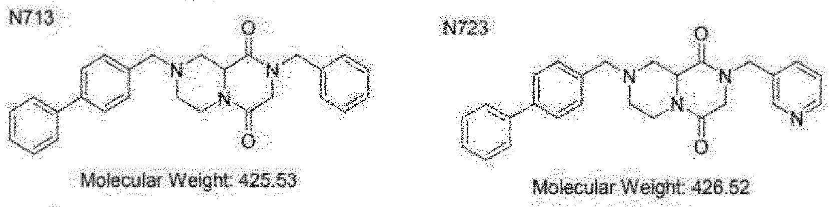

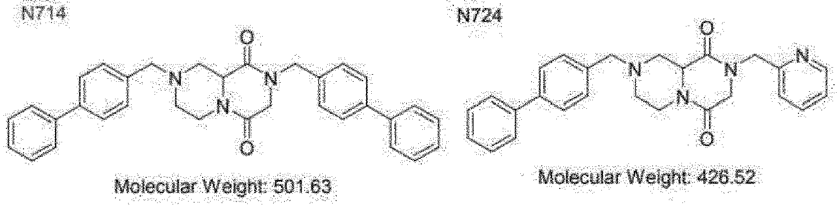

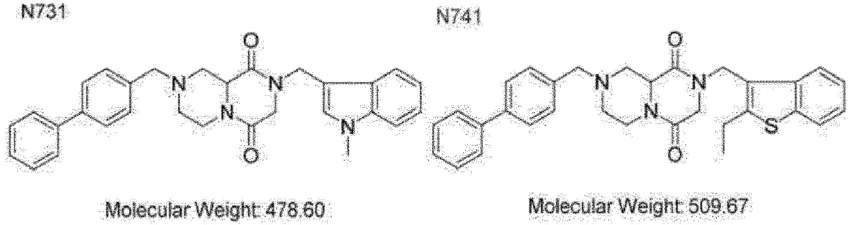

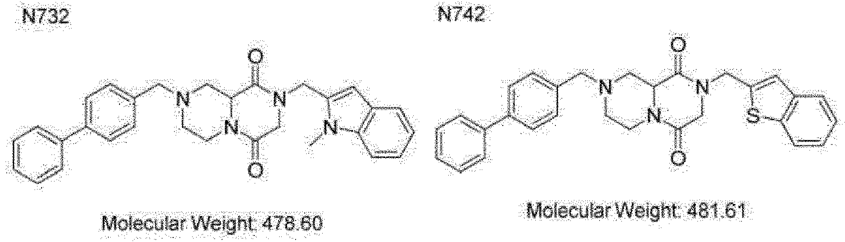

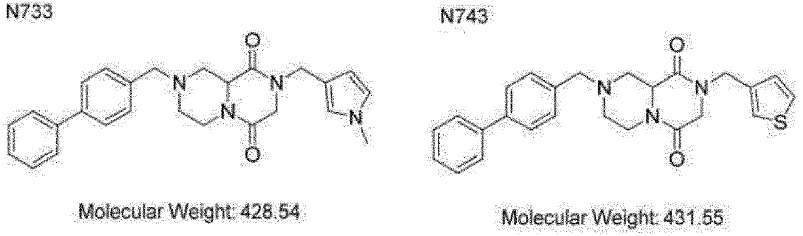

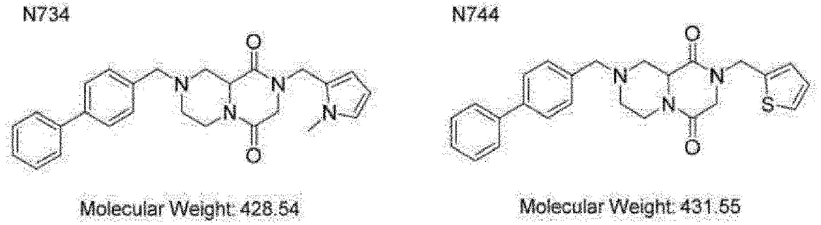

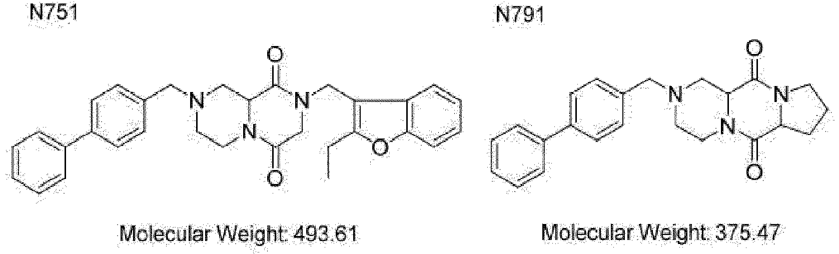

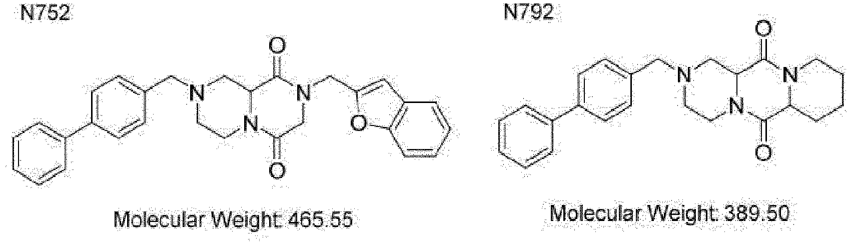

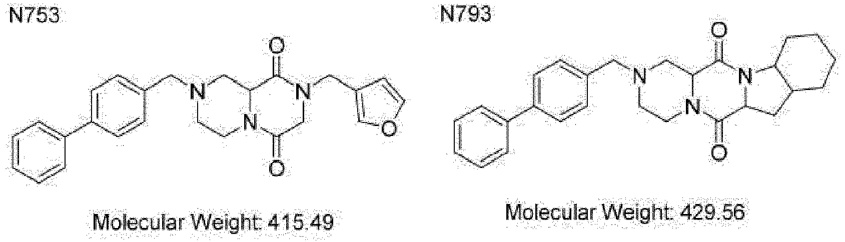

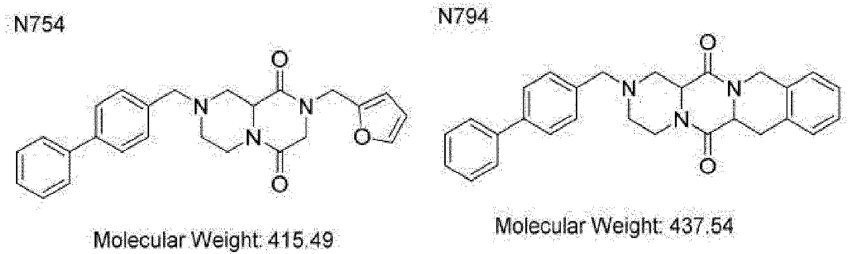

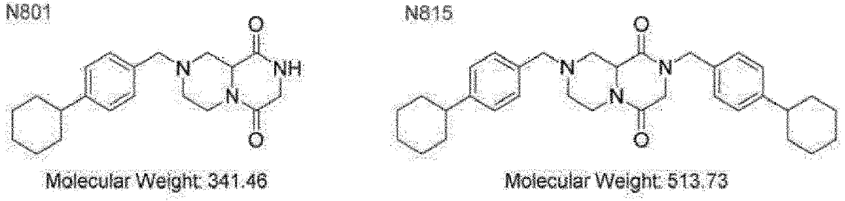

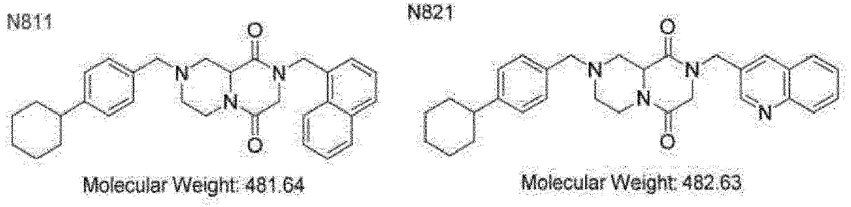

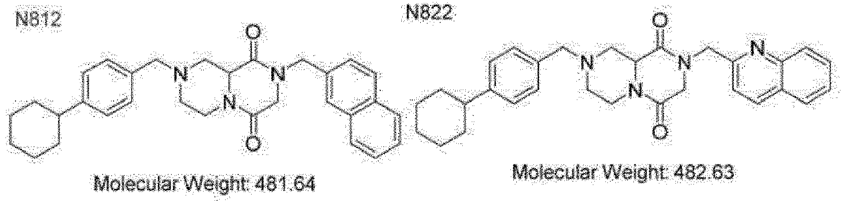

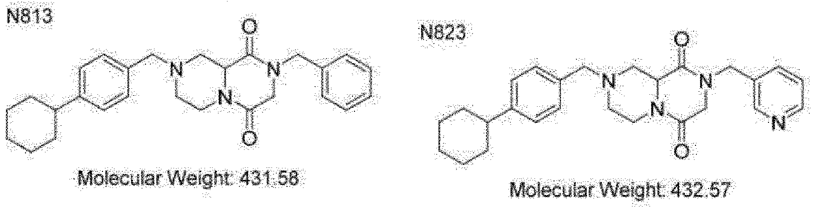

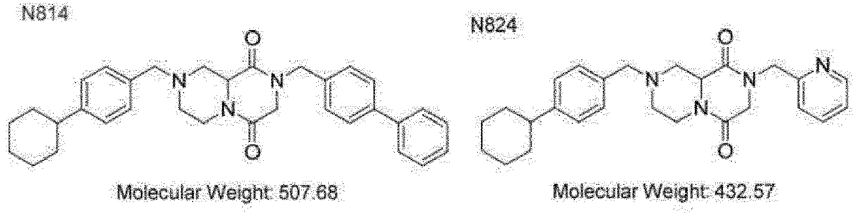

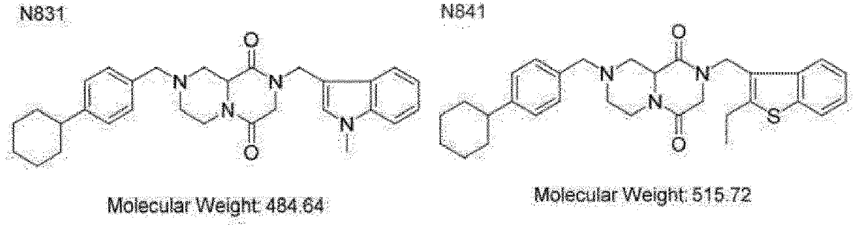

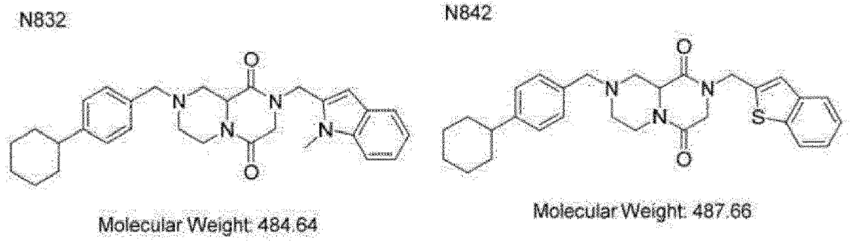

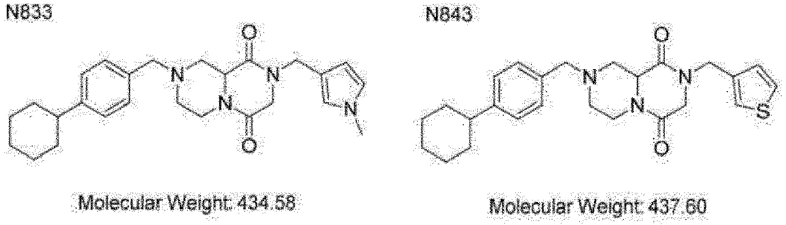

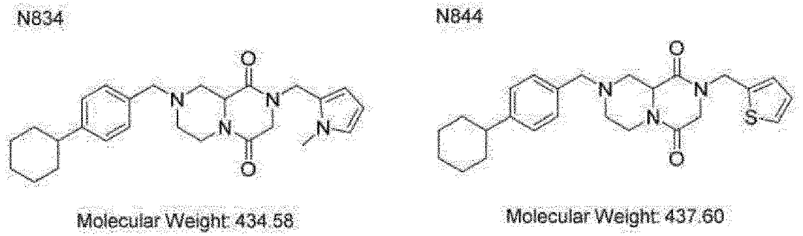

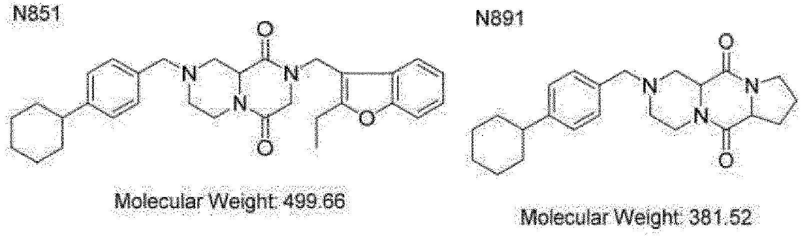

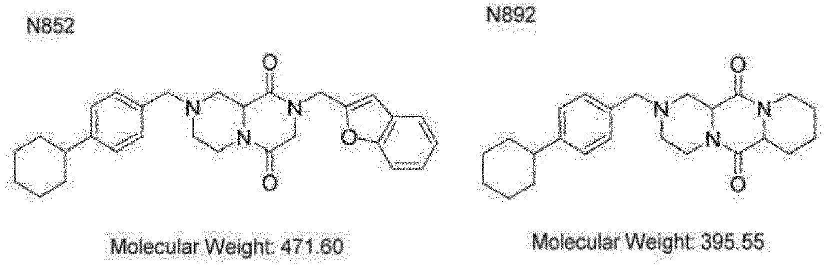

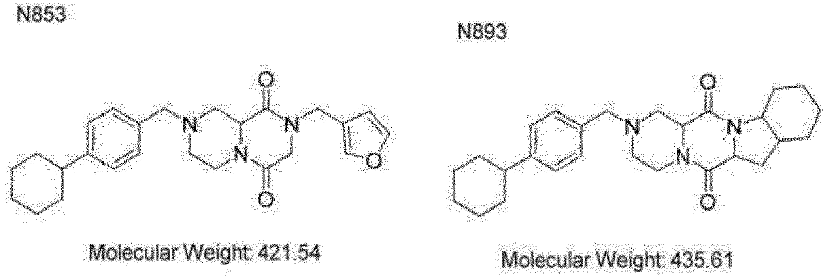

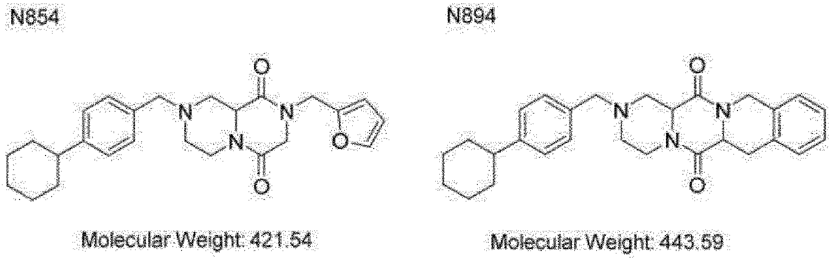

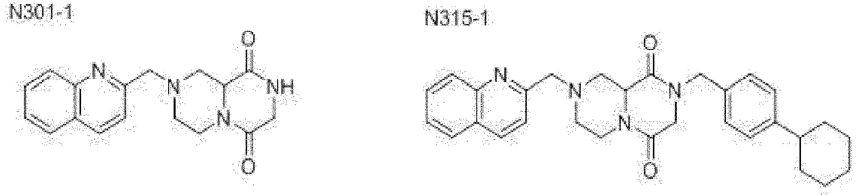

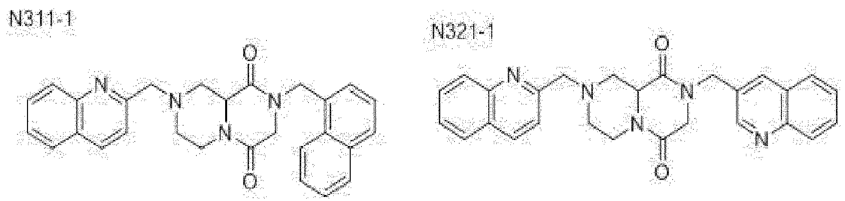

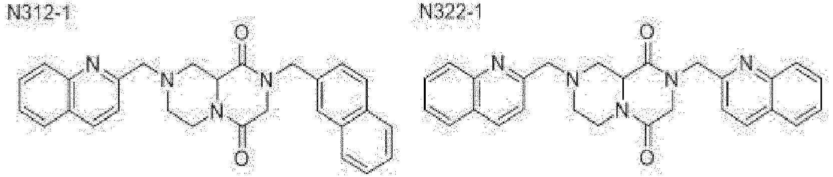

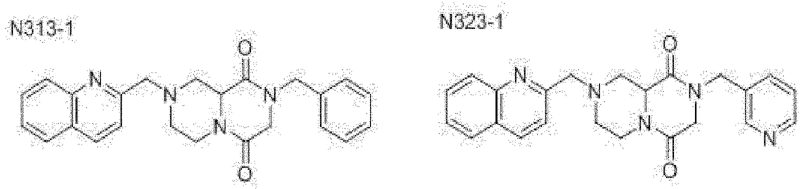

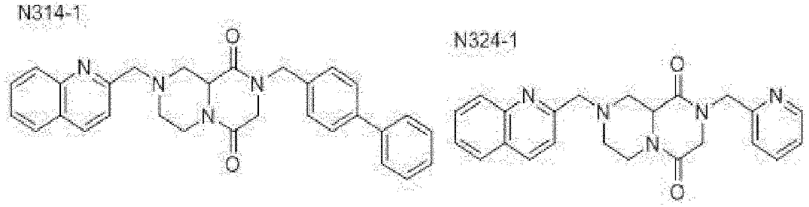

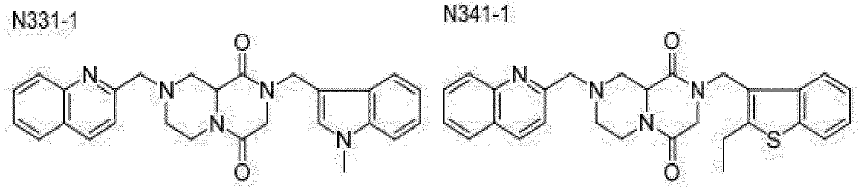

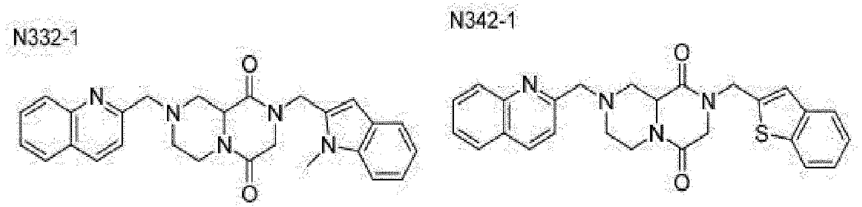

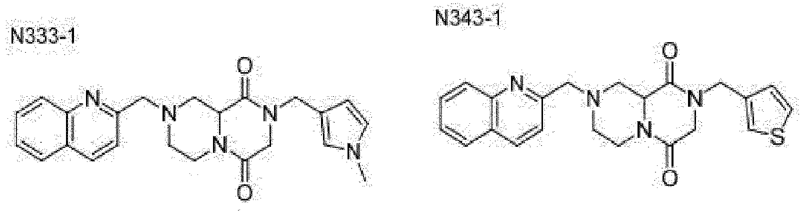

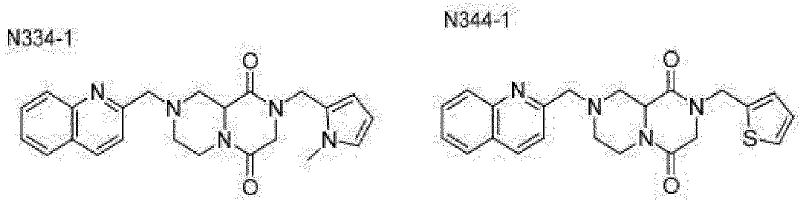

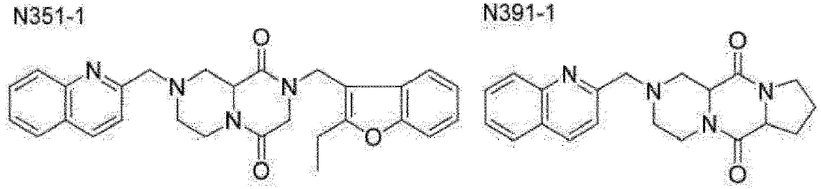

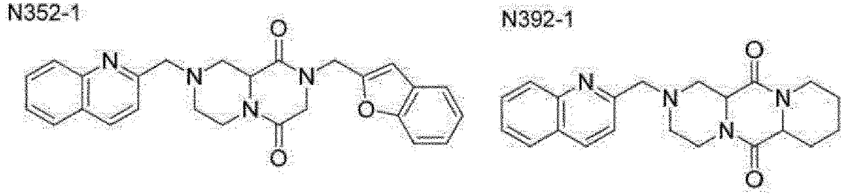

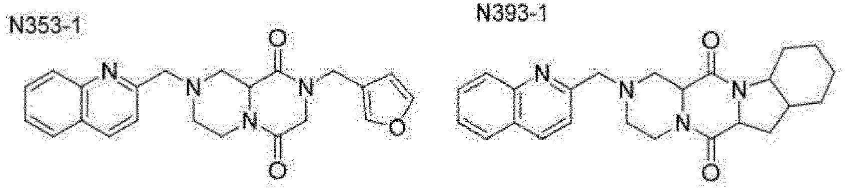

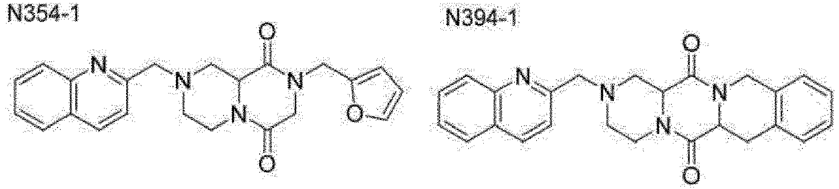

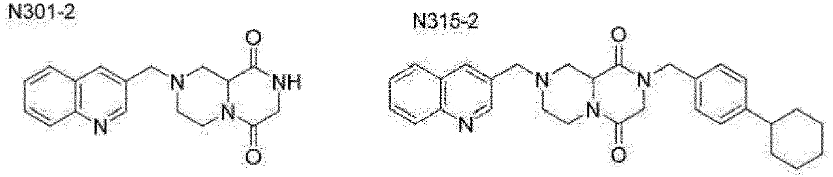

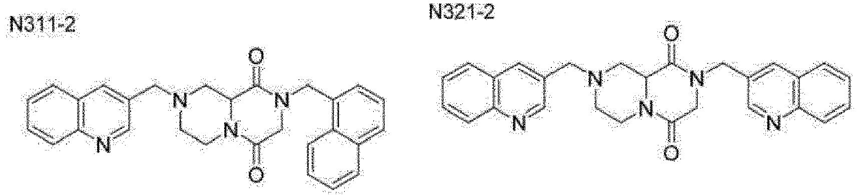

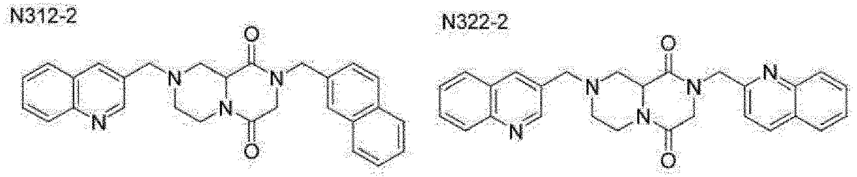

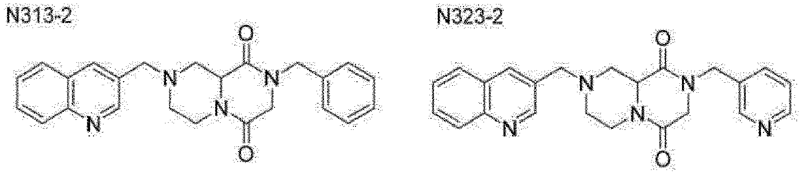

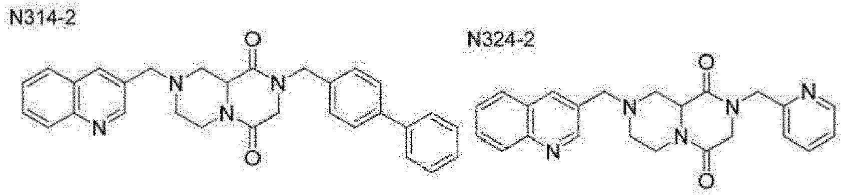

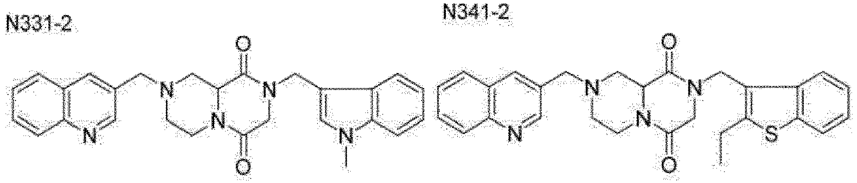

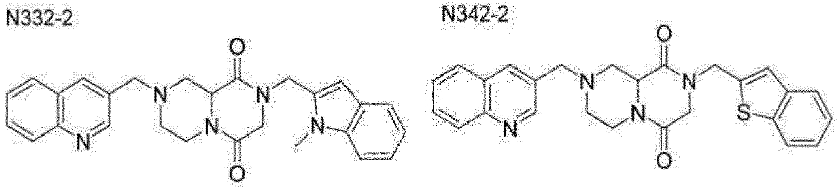

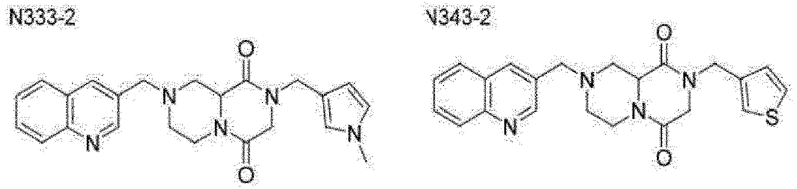

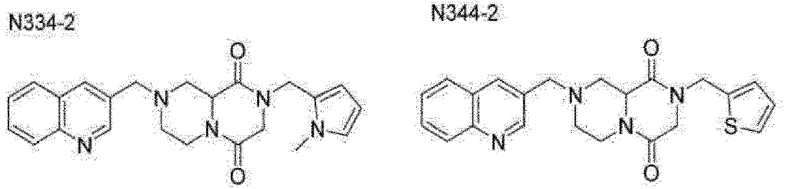

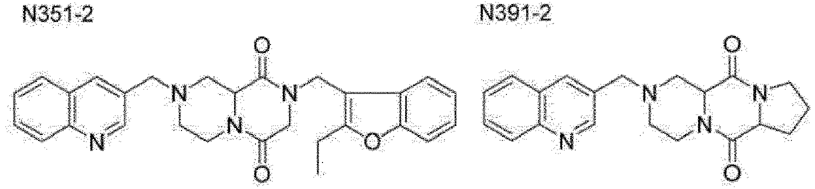

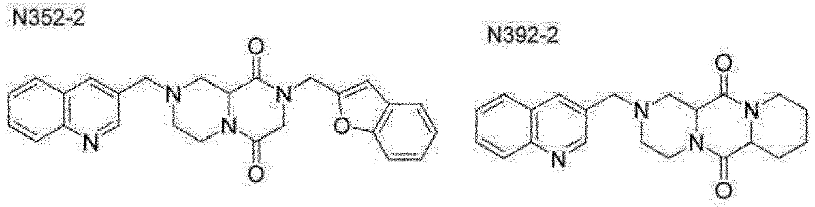

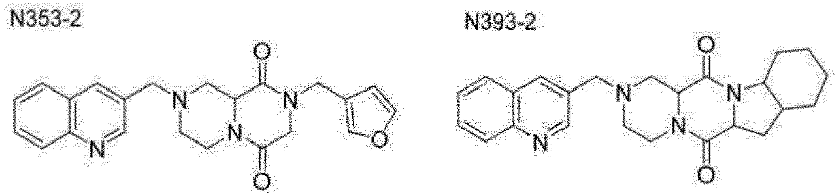

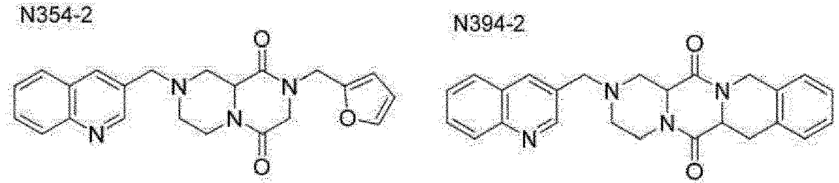

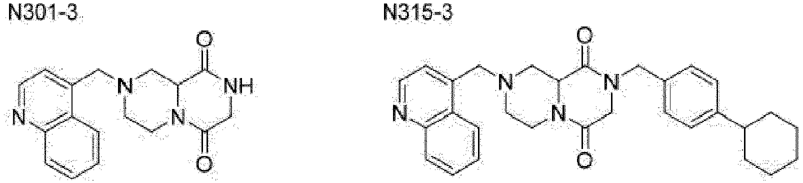

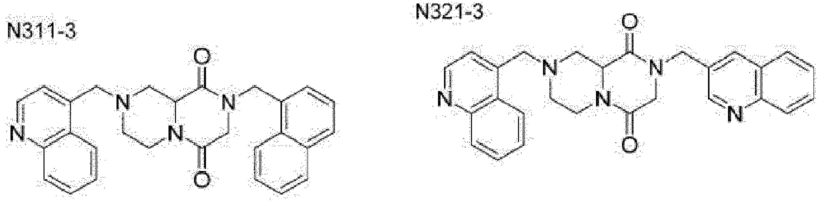

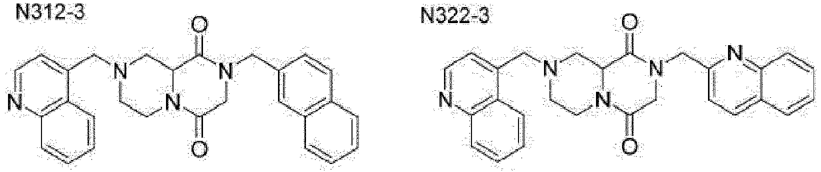

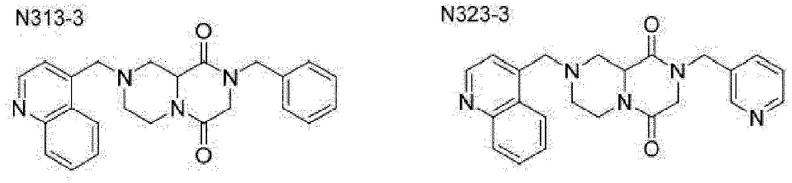

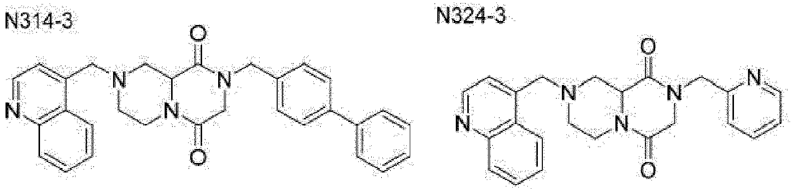

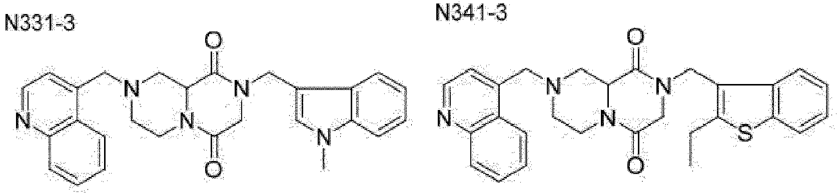

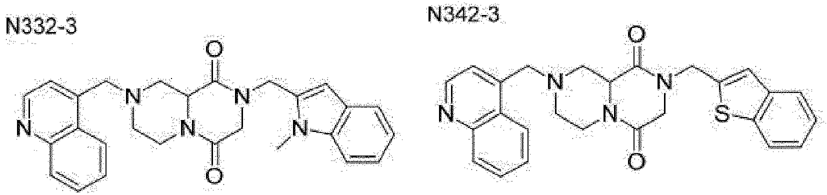

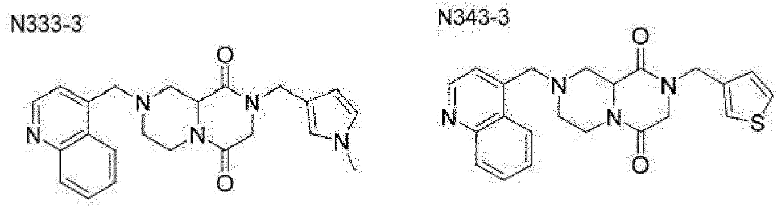

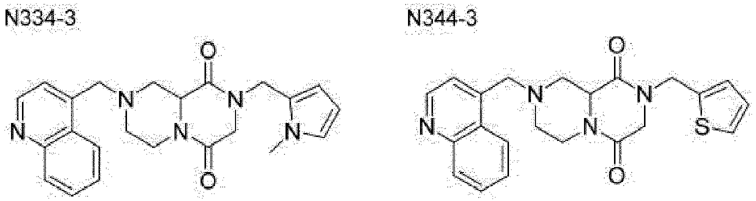

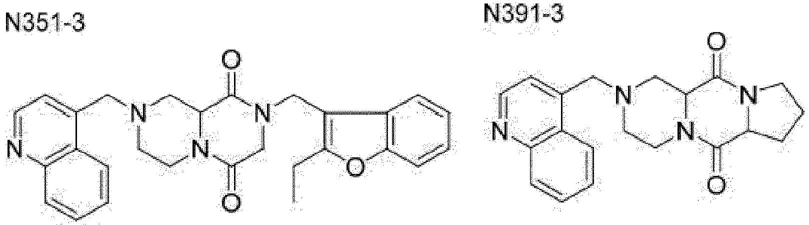

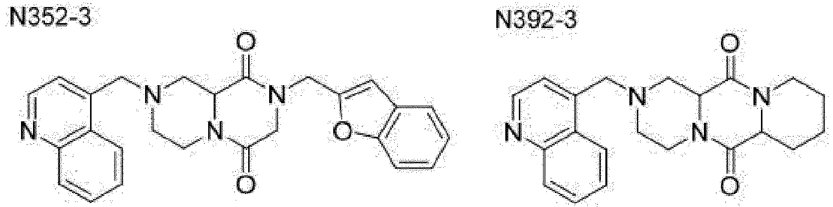

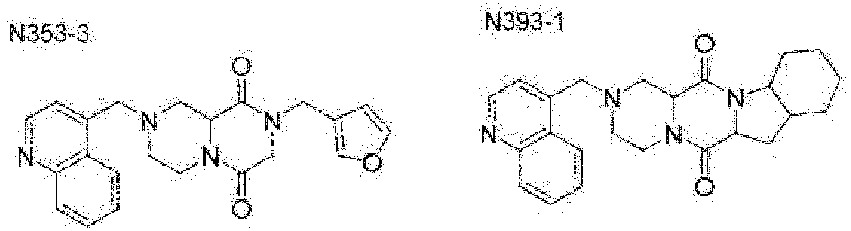

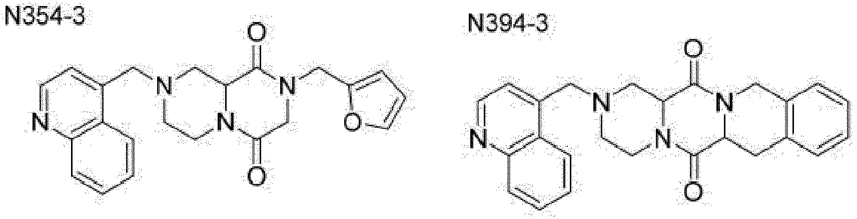

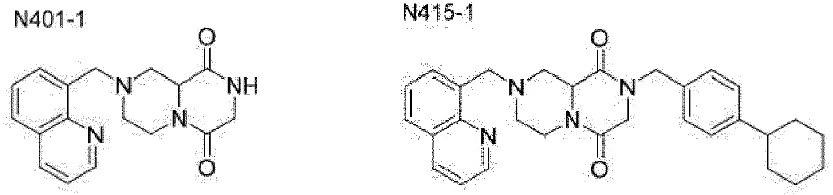

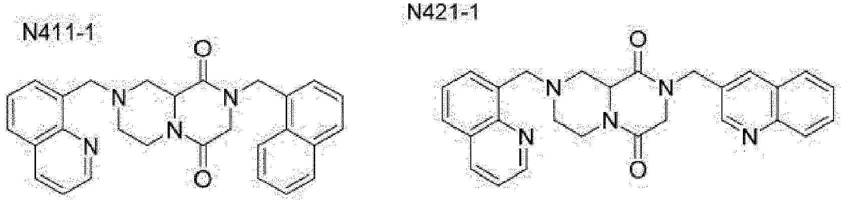

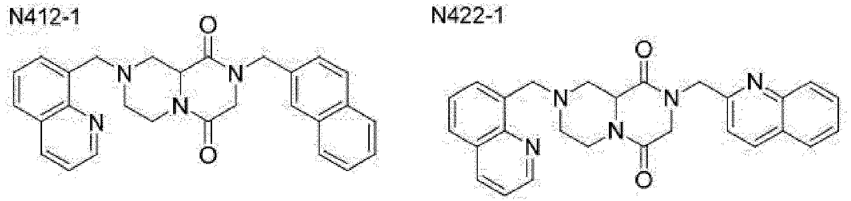

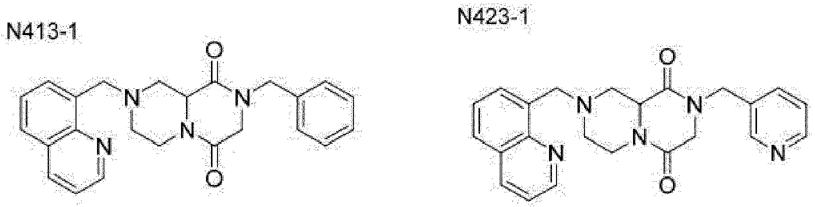

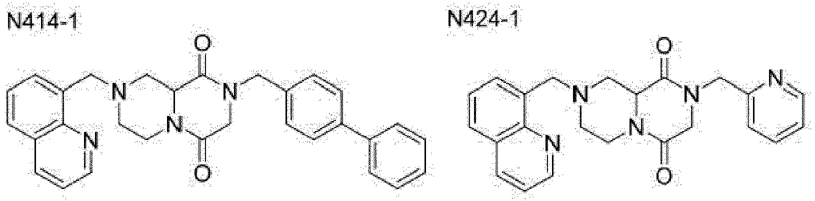

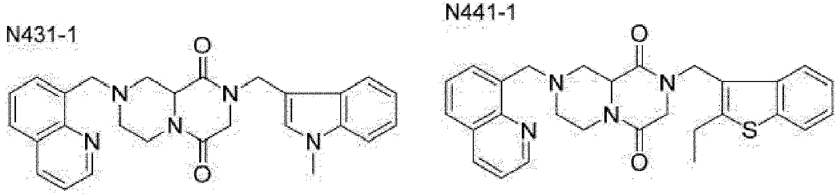

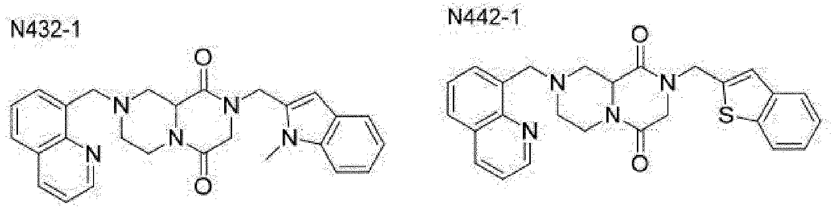

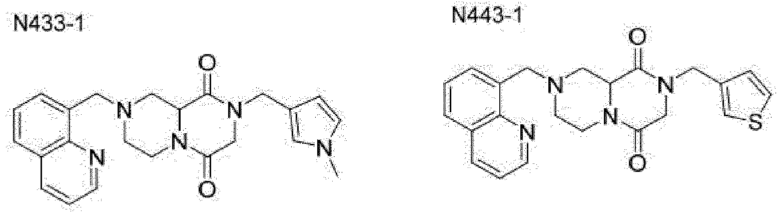

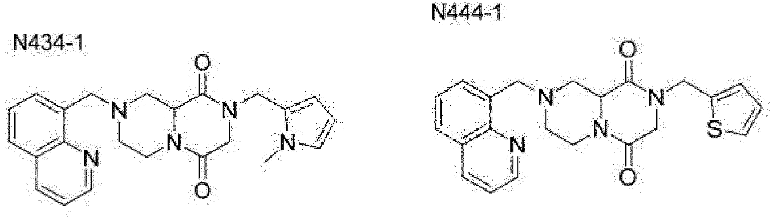

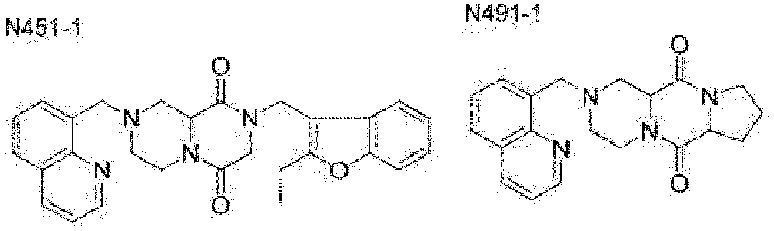

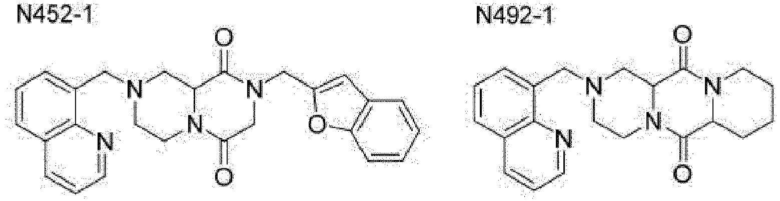

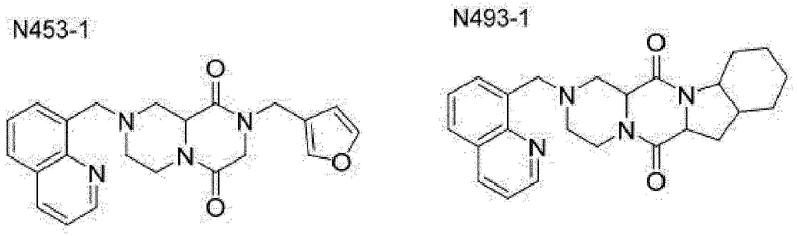

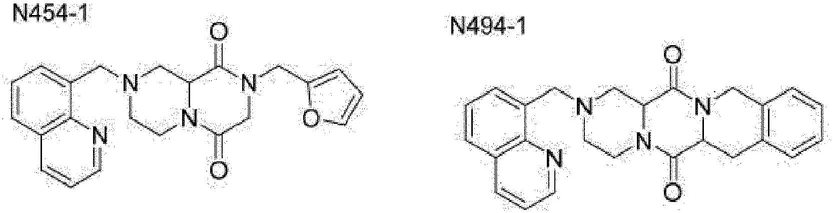

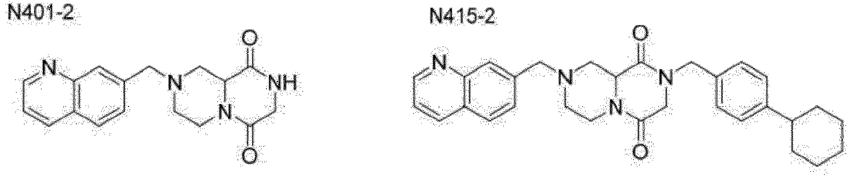

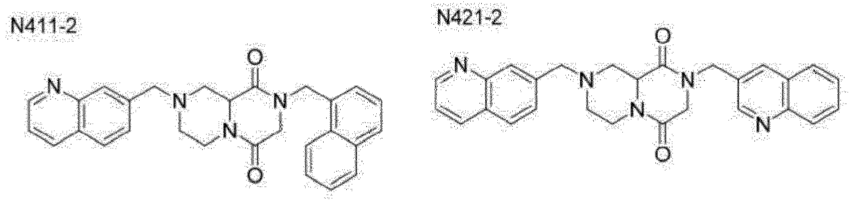

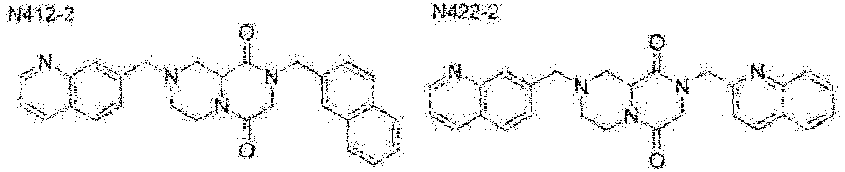

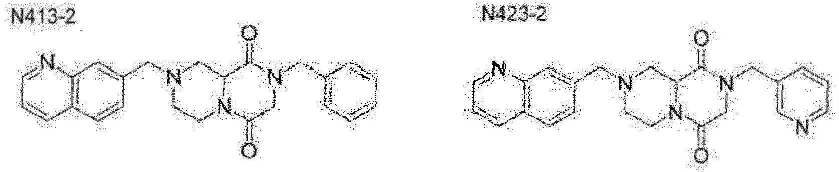

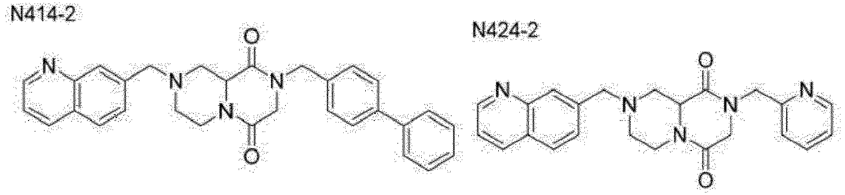

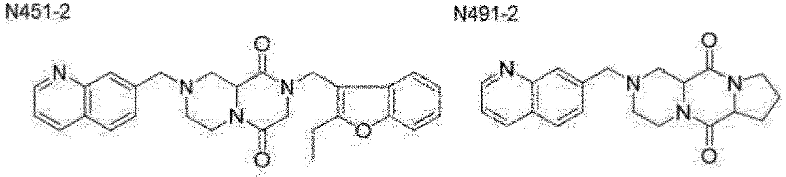

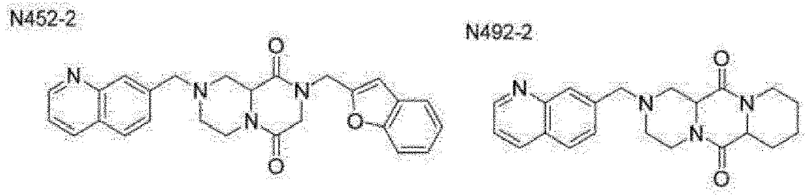

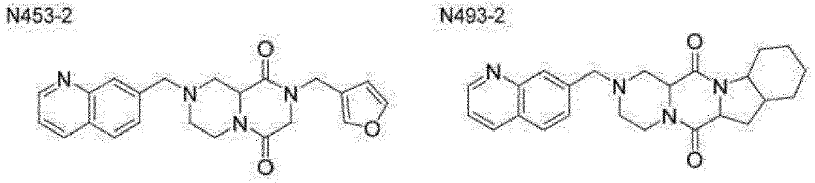

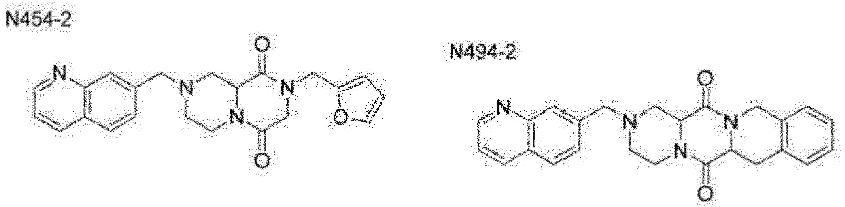

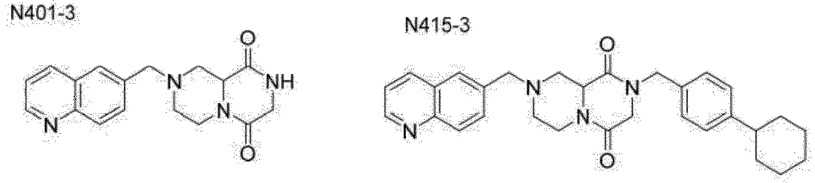

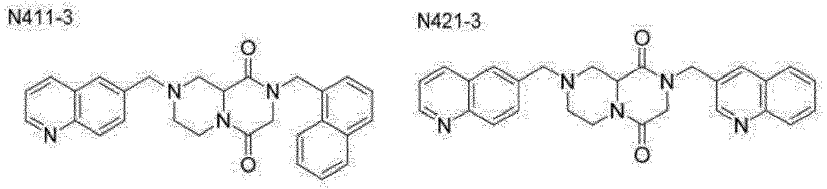

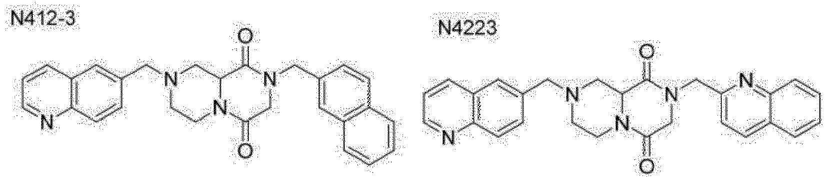

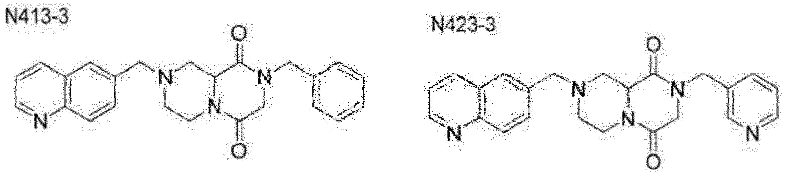

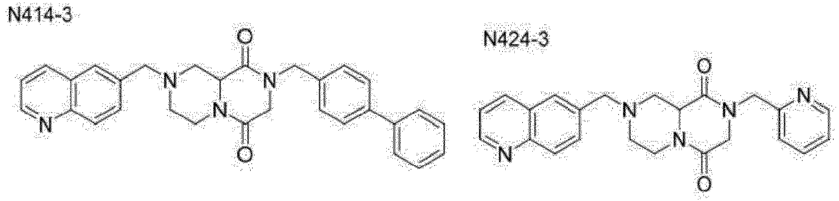

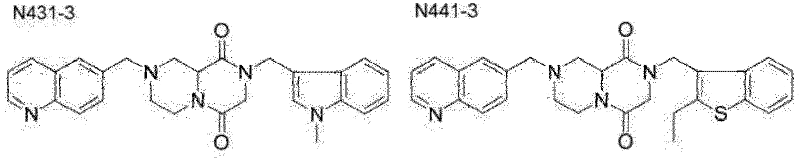

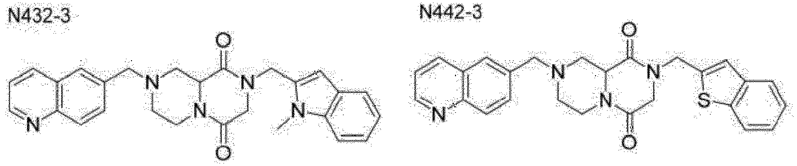

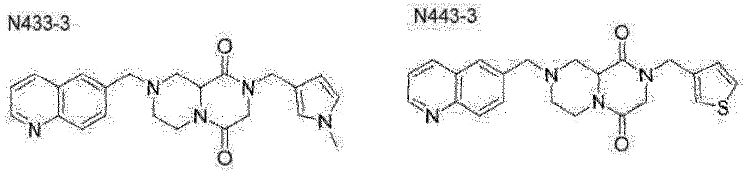

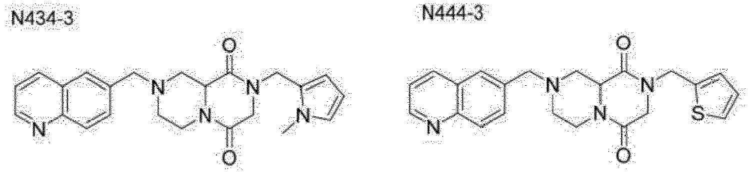

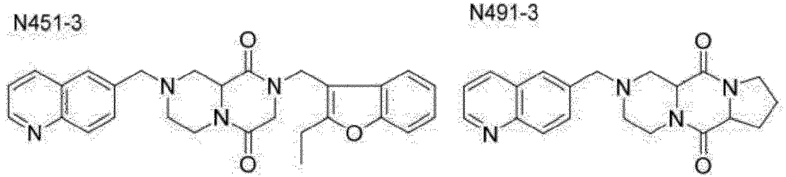

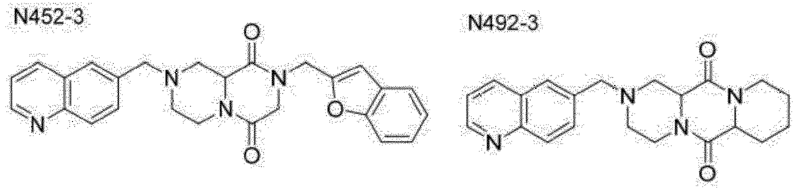

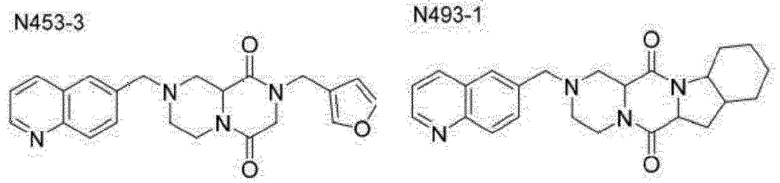

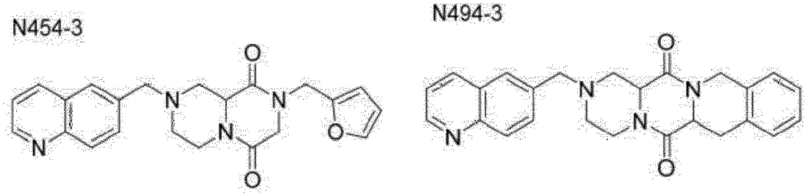

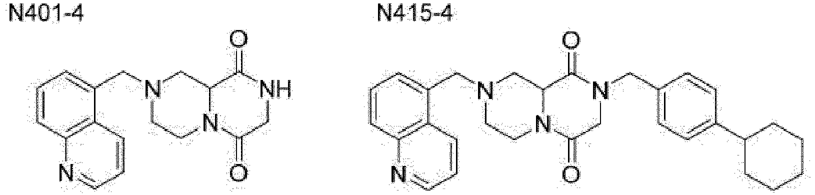

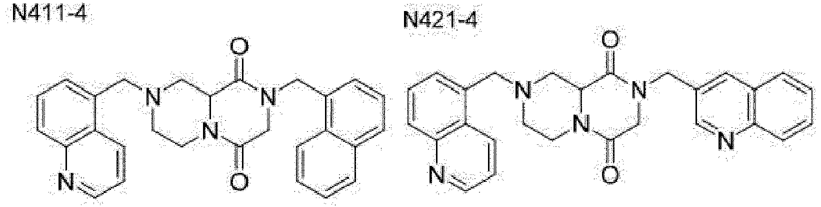

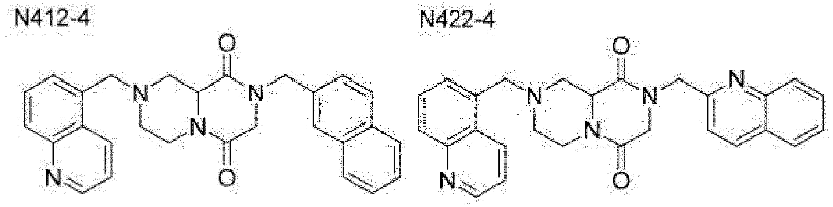

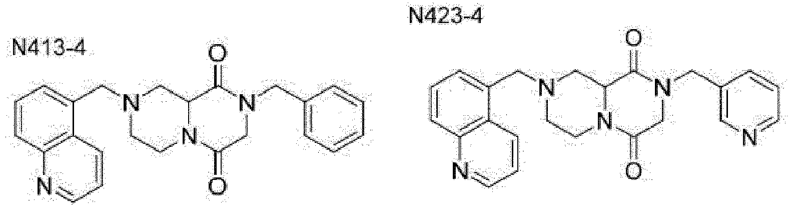

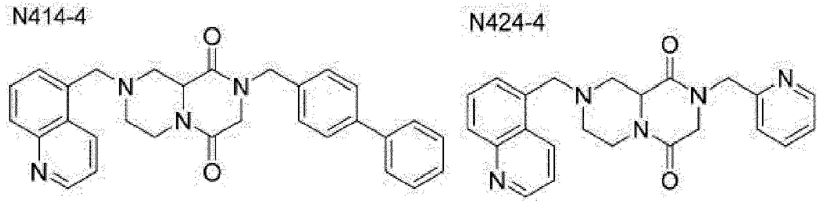

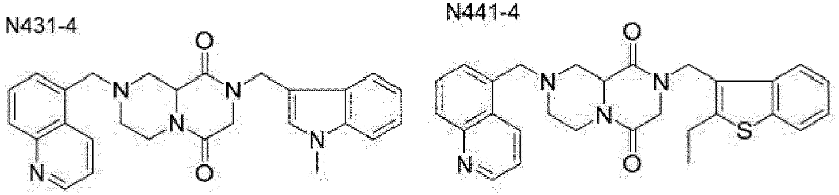

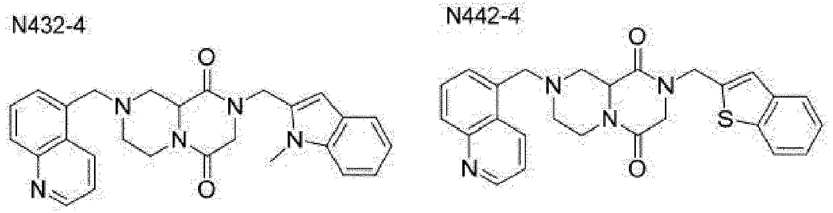

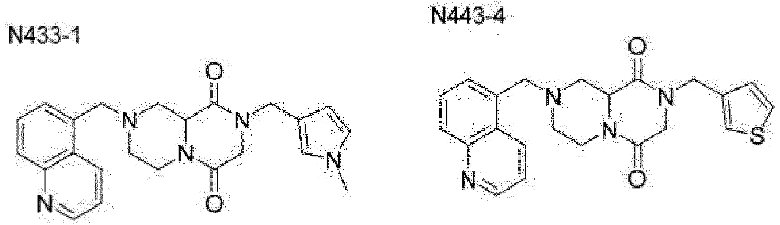

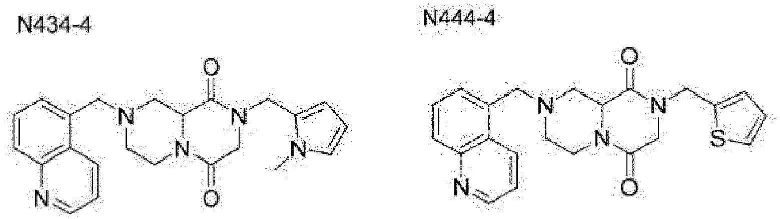

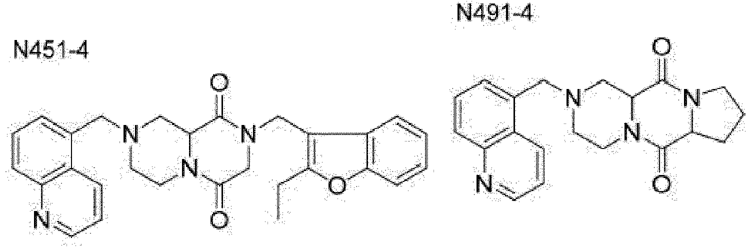

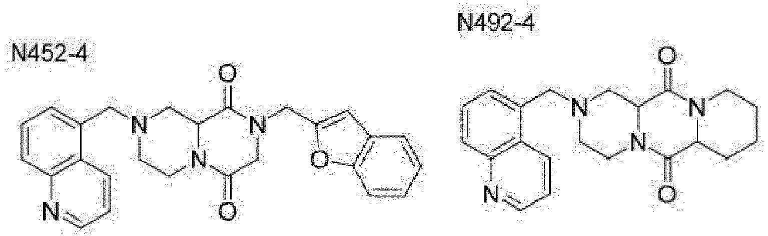

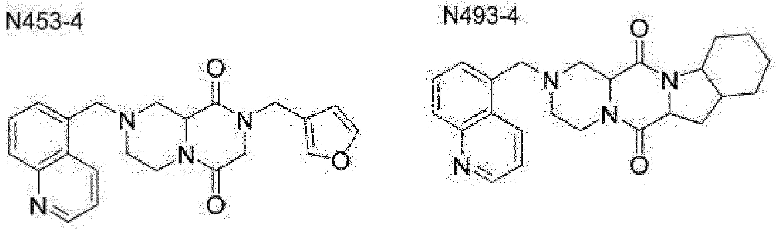

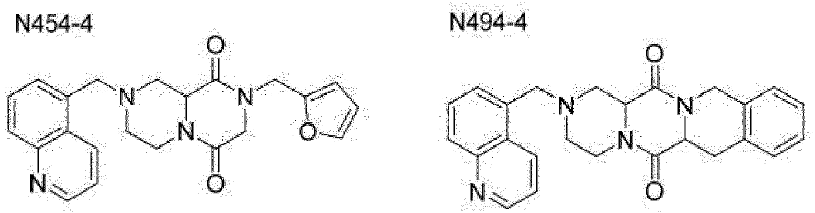

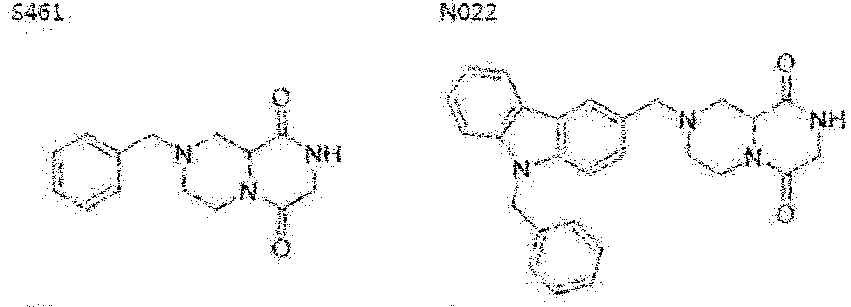

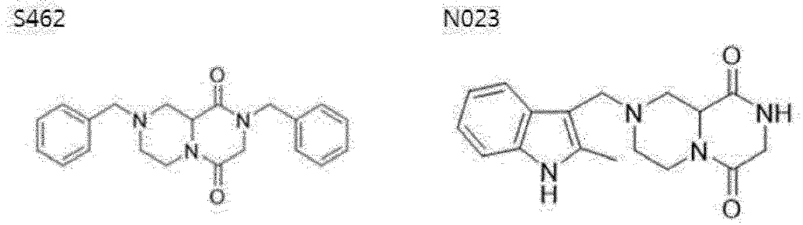

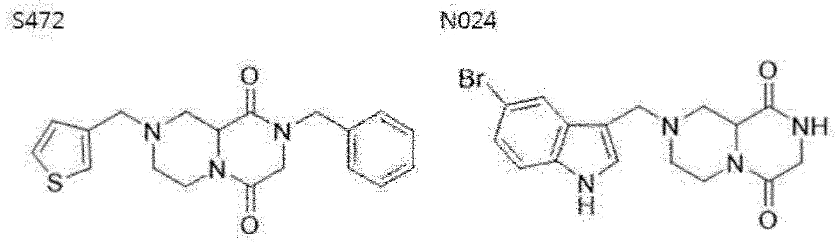

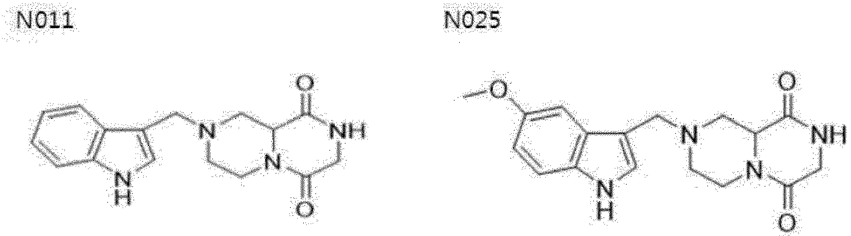

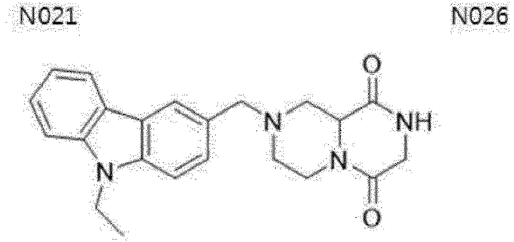

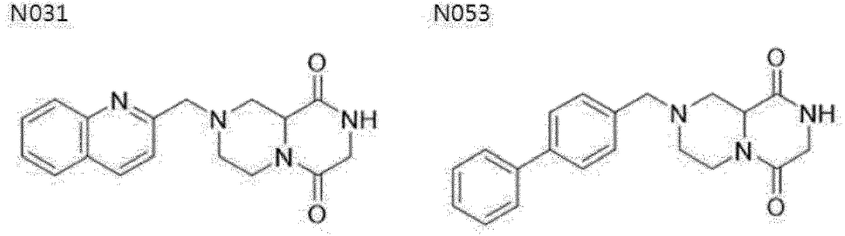

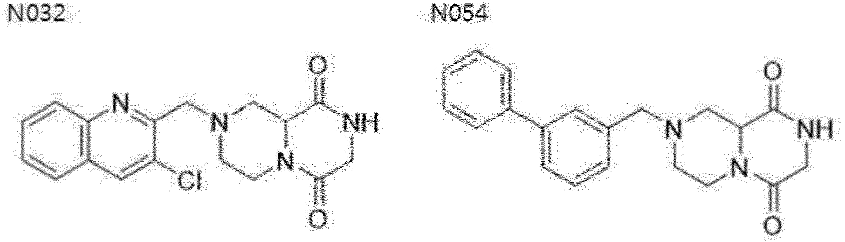

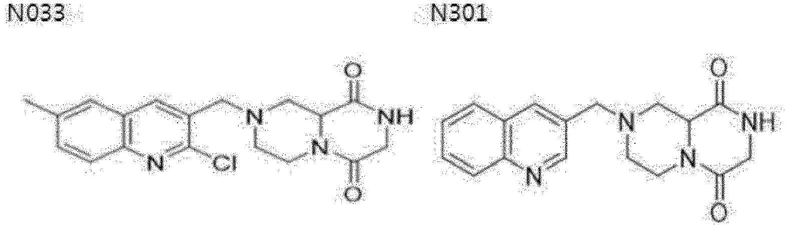

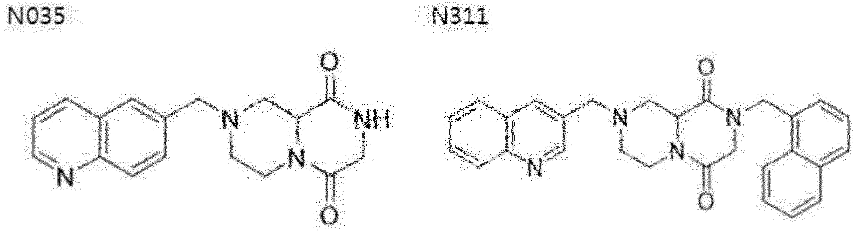

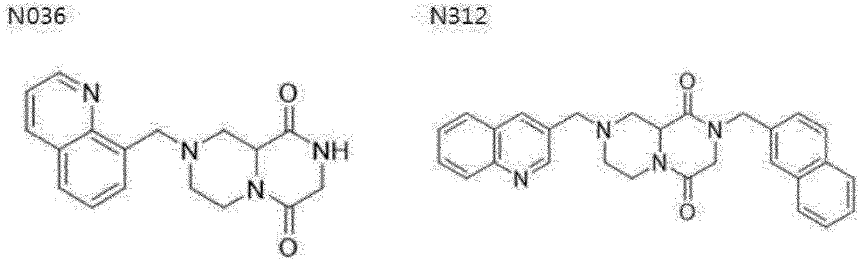

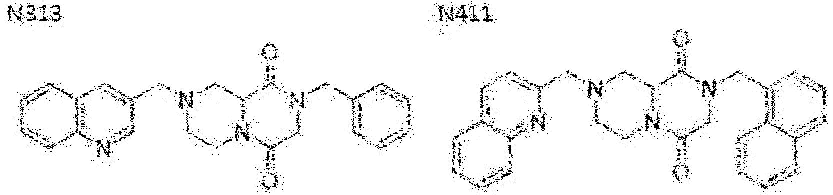

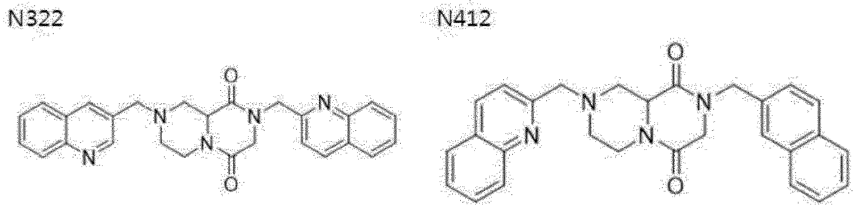

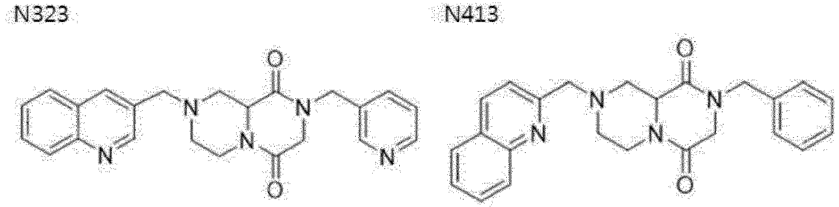

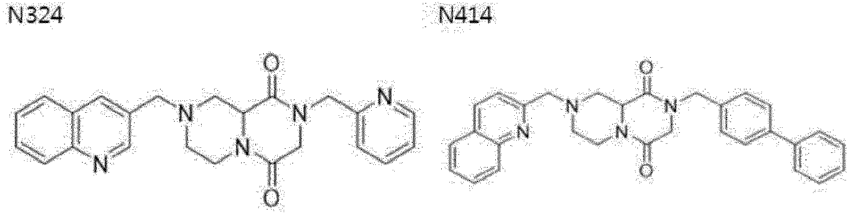

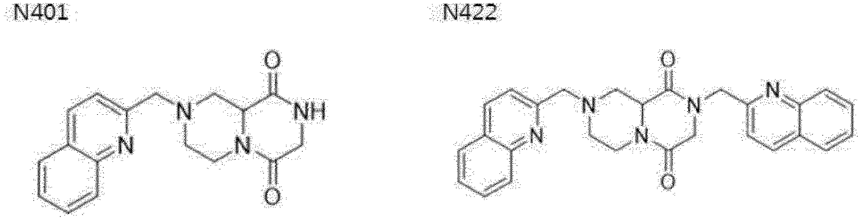

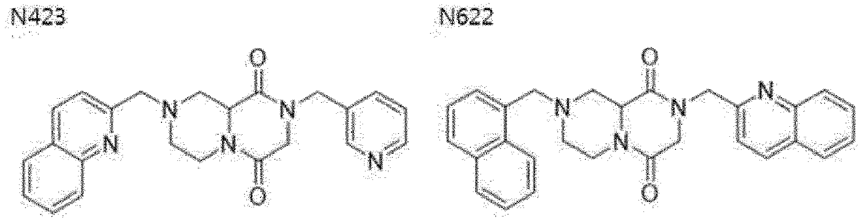

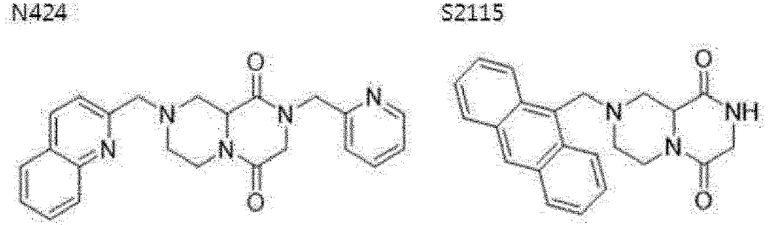

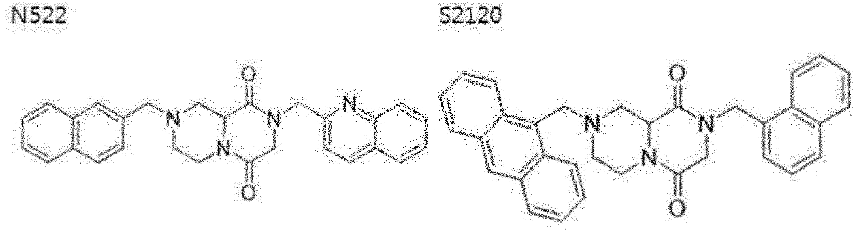

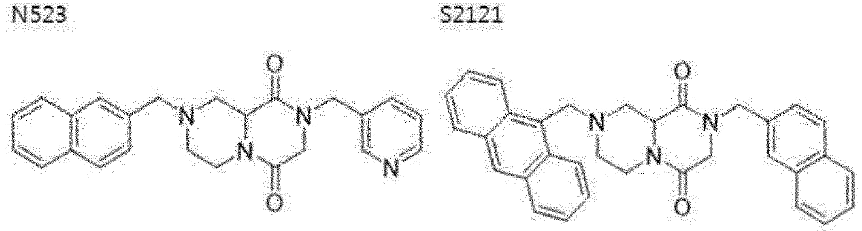

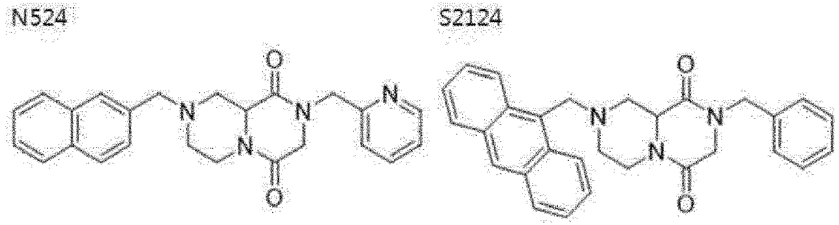

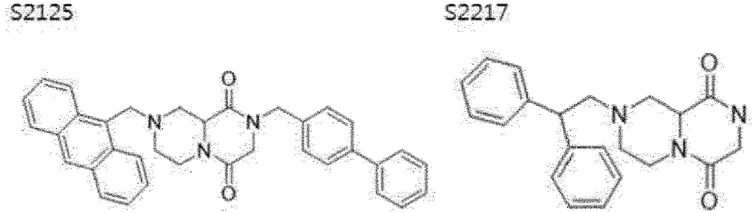

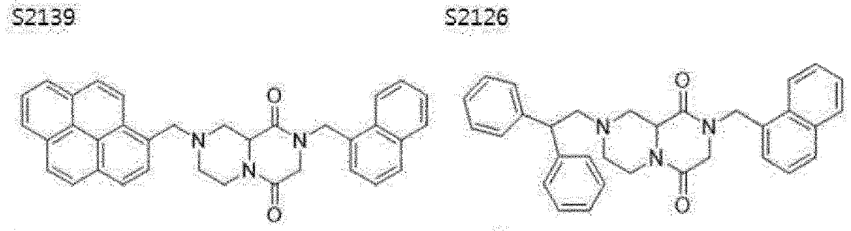

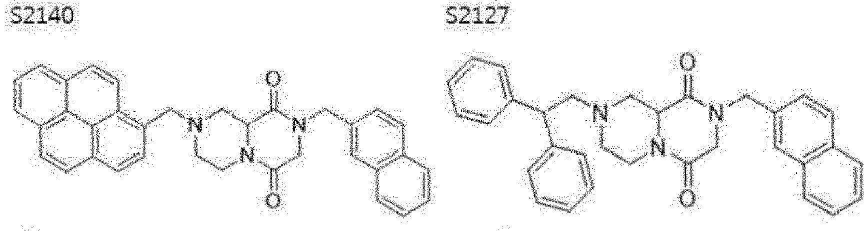

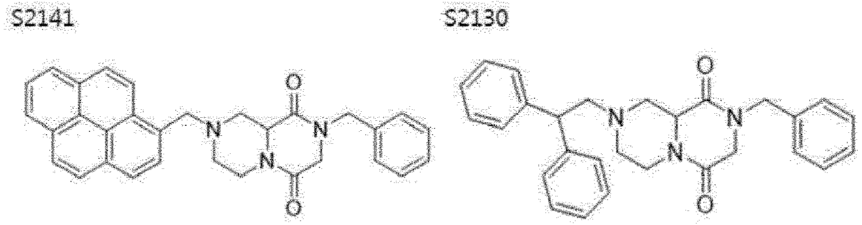

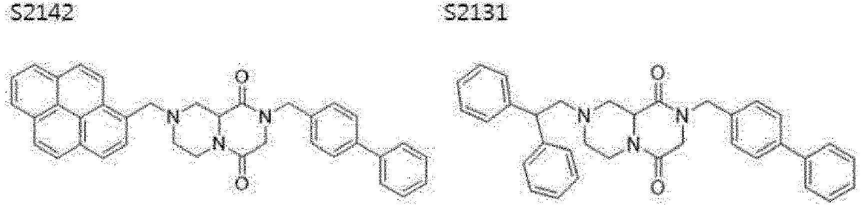

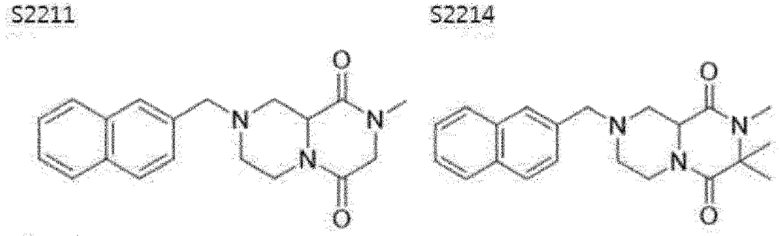

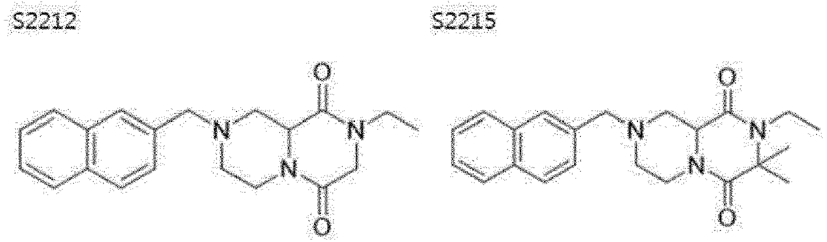

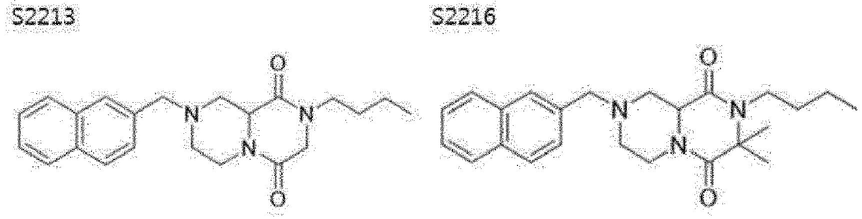

One embodiment of the present application provides a pharmaceutical composition for preventing or treating cancer, which includes the compound or a pharmaceutically acceptable salt thereof, a hydrate, a solvate, a constitutional isomer, an optical isomer, a stereoisomer, or a combination thereof; and a pharmaceutically acceptable carrier, excipient, diluent or adjuvant. When the compound represented by Formula 1 or a pharmaceutically acceptable salt thereof is administered alone, it has an effect of inhibiting cancer activity.

One embodiment of the present application provides a pharmaceutical composition for preventing or treating resistant cancer, which includes the compound or a pharmaceutically acceptable salt thereof, a hydrate, solvate, constitutional isomer, optical isomer, stereoisomer, or combination thereof; and a pharmaceutically acceptable carrier, excipient, diluent or adjuvant. When the compound represented by Formula 1 or a pharmaceutically acceptable salt thereof is administered in combination with an anticancer drug against resistant cancer or with radiation therapy and the like, it has an effect of inhibiting cancer activity.

One embodiment of the present application provides a pharmaceutical composition for preventing or treating resistant cancer, which includes a compound represented by Formula 1 or a pharmaceutically acceptable salt thereof. When the compound represented by Formula 1 or a pharmaceutically acceptable salt thereof is administered in combination with an anticancer drug against resistant cancer or with radiation therapy and the like, it has an effect of inhibiting cancer activity.

In one embodiment of the present application, the cancer or resistant cancer may be at least one selected from the group consisting of ovarian cancer, colorectal cancer, pancreatic cancer, gastric cancer, liver cancer, breast cancer, cervical cancer, thyroid cancer, parathyroid cancer, lung cancer, non-small cell lung cancer, prostate cancer, gallbladder cancer, biliary tract cancer, blood cancer, bladder cancer, kidney cancer, melanoma, colon cancer, bone cancer, skin cancer, head and neck cancer, uterine cancer, rectal cancer, brain cancer, anal cancer, fallopian tube carcinoma, endometrial carcinoma, vaginal cancer, vulvar carcinoma, esophagus cancer, small intestine cancer, endocrine carcinoma, adrenal cancer, soft tissue sarcoma, urethral cancer, penile cancer, ureteral cancer, renal cell carcinoma, renal pelvic carcinoma, blood cancer, leukemia, a central nervous system (CNS) tumor, a spinal cord tumor, brainstem glioma, and pituitary adenoma.

The pharmaceutical composition according to one embodiment of the present application may include the compound represented by Formula 1 or a pharmaceutically acceptable salt thereof, and an anticancer agent in a molar concentration ratio of 1:0.001 to 1:1000, 1:0.01 to 1:100, 1:0.1 to 1:50, or 1:0.1 to 1:20.

The pharmaceutical composition according to one embodiment of the present application may be prepared in a capsule, tablet, granule, injection, ointment, powder, or drink, and may be used in an oral formulation, an external-use formulation, a suppository formulation, and an injectable formulation.

The pharmaceutical composition of the present application may be prepared in various formulations by mixing with a pharmaceutically acceptable carrier, and may be orally or parenterally administered. The pharmaceutically acceptable carrier may be a binder, a glidant, a disintegrant, an excipient, a solubilizer, a dispersant, a stabilizer, a suspending agent, a colorant, a flavoring agent, and the like for oral administration. For an injection, a mixture of a buffer, a preservative, a pain relief agent, a solubilizer, an isotonic agent, a stabilizer, and the like, may be used, and for topical administration, a base, an excipient, a lubricant, a preservative, and the like, may be used.

For example, for oral administration, the pharmaceutical composition of the present application may be prepared in the form of a tablet, a troche, a capsule, an elixir, a suspension, a syrup, a wafer, a tablet, a powder, a granule, a capsule, and the like. Among the above formulations, a solid formulation may be prepared by mixing at least one of excipients such as starch, calcium carbonate, sucrose, lactose, gelatin, and the like. In addition, in addition to the excipients, lubricants such as magnesium stearate and talc may be used. For a liquid formulation, in addition to a simple diluent, such as water or liquid paraffin, a wetting agent, a sweetening agent, a fragrance, a preservative, and the like, may be used, but the present invention is not limited thereto.

For example, formulations for parenteral administration may include a non-aqueous solvent, a suspension, an emulsion, a freeze-dried preparation, a suppository, and the like. An injection may be prepared in a unit dose ampoule or multi-dose form, and may be injected by intraperitoneal injection, intrarectal injection, subcutaneous injection, intravenous injection, intramuscular injection, or intrathoracic injection.

The carrier, excipient, and diluent for preparation of a pharmaceutical composition may be lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, mineral oil, a filler, an anticoagulant, a lubricant, a wetting agent, a flavoring agent, an emulsifier, a preservative, or the like.

The pharmaceutical composition of the present application is administered via an administration route, but not limited to, an oral, intravenous, intramuscular, intraarterial, intramedullary, intrathecal, intracardiac, transdermal, subcutaneous, intraperitoneal, intranasal, enteric, topical, sublingual, or rectal route.

As an adjuvant that can be additionally used, a conventional adjuvant may be used without limitation. In the present application, the adjuvant refers to a substance that may cause, intensify or modify a specific response to the active ingredient of the present application when administered simultaneously, concurrently or consecutively. For example, known adjuvants for an injectable solution include an aluminum composition (for example, aluminum hydroxide or aluminum phosphate), saponins (for example, QS21), muramyl dipeptide or muramyl tripeptide, proteins (for example, gamma-interferon, TNF, or M59), squalene, or a polyol.

The dose of the pharmaceutical composition of the present application may be selected by those of ordinary skill in the art according to a patient's condition and body weight, severity of a disease, a dosage form, an administration route and duration. For example, the pharmaceutical composition of the present application may be administered daily at 0.0001 to 1000 mg/kg or 0.001 to 500 mg/kg. The pharmaceutical composition of the present application may be administered once a day or several times in divided portions. The dose does not limit the scope of the present application in any way.

In addition, the present application provides a use of the compound represented by Formula 1 or a pharmaceutically acceptable salt thereof for treating resistant cancer.

The resistant cancer, and the compound represented by Formula 1 or a pharmaceutically acceptable salt thereof are the same as described above, and thus the detailed description thereof will be omitted.

The present application provides a method of treating cancer, which includes administering a therapeutically effective amount of the above-described compound represented by Formula 1 or a pharmaceutically acceptable salt, hydrate, solvate, structural isomer, optical isomer or stereoisomer thereof, or a combination thereof to a subject with cancer. In the administration, a chemotherapeutic agent useful in the treatment of cancer or a proliferative disease may be administered simultaneously, separately, or sequentially.

In addition, the present application provides a method of treating cancer resistant to oncological therapy, which includes administering a therapeutically effective amount of the above-described compound represented by Formula 1 or a pharmaceutically acceptable salt, hydrate, solvate, structural isomer, optical isomer or stereoisomer thereof, or a combination thereof to a subject with resistant cancer. In the administration, a chemotherapeutic agent useful in the treatment of cancer or a proliferative disease may be administered simultaneously, separately, or sequentially.

The term "therapeutically effective amount" refers to an amount of a compound of Formula 1 that is sufficient (i) to treat or prevent a specific disease, condition, or disorder, (ii) to weaken, improve or alleviate one or more symptoms of a specific disease, condition, or disorder, or (iii) to prevent or delay of the initiation of one or more symptoms of a specific disease, condition, or disorder, described in the present invention, when the compound of Formula 1 is administered to a mammal in need of such treatment. The amount of compound corresponding to such an effective amount will depend on factors such as the particular compound, the state of a disease and its severity, a characteristic (e.g., weight) of the mammal in need of treatment. Nevertheless, the amount of the compound may be routinely determined by those skilled in the art.

The term "administration" refers to introduction of a specific material to a subject by an appropriate method.

The resistant cancer, and the compound represented by Formula 1 or a pharmaceutically acceptable salt thereof are the same as described above, and thus the detailed description thereof will be omitted.

The "subject with resistant cancer" refers to a subject who has developed resistant cancer or has a high possibility of developing resistant cancer and thus is in need of suitable treatment, and may be a subject who has received anticancer therapy, for example, surgical resection therapy, chemotherapy using an anticancer drug, radiation therapy or immunotherapy, but has relapsed due to the resistance thereto.

The subject with resistant cancer may be a human, a cow, a dog, a guinea pig, a rabbit, a chicken, an insect, or the like.

In addition, the present application provides a radiation therapy method including administering the above-described compound represented by Formula 1 or a pharmaceutically acceptable salt thereof to a subject with resistant cancer; and irradiating the subject with radiation.

The resistant cancer, and the compound represented by Formula 1 or a pharmaceutically acceptable salt thereof are the same as described above, and thus the detailed description thereof will be omitted.

For irradiation, any irradiation method conventionally used for radiation therapy or an irradiation method for cancer, which will be developed in the future, may be applied.

When the compound represented by Formula 1 according to the present application or a pharmaceutically acceptable salt thereof is administered in combination with radiation therapy, a synergistic effect on the inhibition of the growth of cancer cells or cancer stem cells and/or induction of cell death may be imparted, thereby not only effectively preventing or treating cancer but also preventing resistance to radiation or the metastasis or recurrence of cancer.

The compound represented by Formula 1 of the present application or a pharmaceutically acceptable salt thereof can act as an inhibitor targeting the SERCA protein. The compound represented by Formula 1 of the present application or a pharmaceutically acceptable salt thereof may inhibit SERCA protein expression.

Previous study results reveal that the key cause of anticancer drug resistance of cancer stem cells lies in a protein, which is 'sarco/endoplasmic reticulum calcium ATPase (SERCA),' involved in the transport and storage of intercellular calcium ions in cells.

When an anticancer agent is administered to normal cancer cells, excessive stress is induced, calcium ions are excessively secreted from the endoplasmic reticulum (ER), and the secreted calcium ions accumulate in mitochondria, leading to the suicide of cancer cells. On the other hand, it has been found that cancer stem cells survive while controlling the concentration of calcium ions by reducing the excessive secretion of calcium ions when an anticancer agent is administered and increasing the expression of SERCA, which can return the excessively secreted calcium ions back to the endoplasmic reticulum. That is, the SERCA protein can play a role in survival signaling in the endoplasmic reticulum stress signaling process.

The compound represented by Formula 1 of the present application or a pharmaceutically acceptable salt thereof has an effect of acting as an inhibitor targeting the SERCA protein, which causes the anticancer agent resistance of cancer stem cells. Therefore, it is possible to exhibit an excellent anti-cancer effect even with a lower dose of drug by increasing the efficacy of chemotherapy with an anti-cancer drug.

### [Examples]

### Preparation Example

Compounds according to the present application were prepared by methods of the following Preparation Examples.

### 1. Preparation Example 1: Tetrahydro-2H-pyrazino[1,2-a]pyrazine-1,4(3H,6H)-dione

Tetrahydro-2*H*-pyrazino[1,2-*a*]pyrazine-1,4(3*H*,6*H*)-dione (PPD) having the skeleton of Formula 8 was prepared through a 5-step process described below. Each step of the synthesis method is described in detail below.

### 1) Step 1: 1,4-bis(tert-butoxycarbonyl)piperazine-2-carboxylic acid

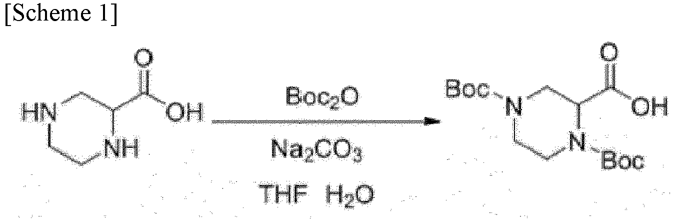

30 g (231 mmol) of piperazine-2-carboxylic acid, 150 mL of tetrahydrofuran, and 150 mL of distilled water were input. The resulting mixture was cooled, and 26.8 g (254 mmol) of sodium carbonate was added thereto. The resulting mixture was cooled, and 110.6 g (506 mmol) of di-tert-butyl dicarbonate was added thereto, followed by stirring at room temperature. The resulting mixture was concentrated under reduced pressure. 500 mL of dichloromethane was added to the concentrated residue. The resulting mixture was cooled and 4M hydrochloric acid was added dropwise. An organic layer was extracted. An aqueous layer was extracted with 60 mL of dichloromethane. Organic layers were combined, followed by washing with 300 mL of distilled water. The resulting organic layer was dried over anhydrous magnesium sulfate and filtered, and then the reaction solution was concentrated under reduced pressure. 60 mL of dichloromethane and 210 mL of n-heptane were added to the residue, and stirred at room temperature. 210 mL of n-heptane was further added, and then the resulting mixture was stirred at room temperature. The resulting product was filtered and dried in hot air at 60 °C, thereby obtaining 1,4-bis(tert-butoxycarbonyl)piperazine-2-carboxylic acid as a solid (70 g, yield: 92%).

### 2) Step 2: Di-tert-butyl 2-((2-methoxy-2-oxoethyl)carbamoyl)piperazine-1,4-dicarboxylate

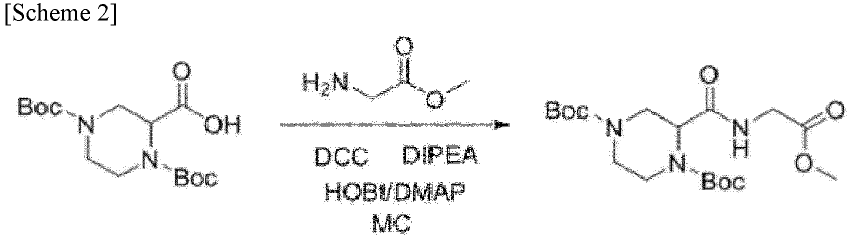

70 g (212 mmol) 1,4-bis(tert-butoxycarbonyl)piperazine-2-carboxylic acid, 28.6 g (228 mmol) of glycine methyl ester hydrochloride, 30.8 g (228 mmol) of hydroxybenzotriazole, 2.5 g (21 mmol) of 4-dimethylaminopyridine, 1,400 mL of dichloromethane, 39 mL (229 mmol) of N,N-diisopropylethylamine, and 47.2 g (229 mmol) of N,N'-dicyclohexylcarbodiimide were input, and then the resulting mixture was stirred at room temperature. The resulting mixture was filtered, and the filtrate was washed three times with 700 mL of distilled water. An organic layer was dried over anhydrous magnesium sulfate and then filtered. The reaction solution was concentrated under reduced pressure to yield di-tert-butyl 2-((2-methoxy-2-oxoethyl)carbamoyl)piperazine-1,4-dicarboxylate as a final product, which was used in the subsequent step.

### 3) Step 3: Methyl[(piperazine-2-carbonyl)amino]acetate 2-trifluoroacetate

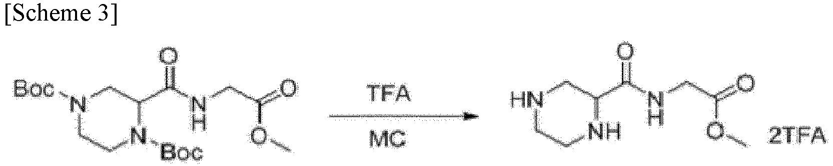

95.4g of the concentrated di-tert-butyl 2-((2-methoxy-2-oxoethyl)carbamoyl)piperazine-1,4-dicarboxylate residue, 700 mL of dichloromethane, and 200 mL of trifluoroacetic acid (TFA) were input, and the resulting mixture was stirred at reflux at 45 °C and concentrated under reduced pressure with dichloromethane. 300 mL of isopropyl alcohol was added to the concentrated residue and stirred at 45 °C for 30 minutes. 100 mL of isopropyl alcohol was added, and then the reaction solution was cooled to room temperature. 600 mL of n-heptane was added to the resulting product, and stirred at room temperature. 600 mL of n-heptane was further added to the resulting product and stirred at room temperature. After filtering the resulting product, a solid was recovered and dried in hot air at 60 °C, thereby obtaining methyl[(piperazine-2-carbonyl)amino]acetate 2-trifluoroacetate as a solid. (76 g, total yield of two steps: 85%)

### 4) Step 4: Tetrahydro-2H-pyrazino[1,2-a]pyrazine-1,4(3H,6H)-dione trifluoroacetate

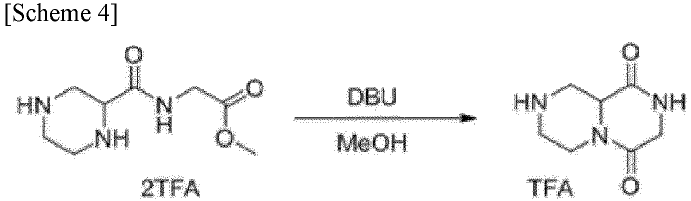

76 g (177 mmol) of methyl[(piperazine-2-carbonyl)amino]acetate 2-trifluroacetate, 460 ml methyl alcohol, and 79 mL (531 mmol) of 1,8-diazabicyclo(5.4.0)undec-7-ene (DBU) were input and stirred at room temperature for 2 hours, and the reaction solution was concentrated under reduced pressure. 230 mL of ethyl acetate was added to the concentrated residue, and the resulting product was cooled to 10 °C or less. 122 mL of trifluoroacetic acid (TFA) was added dropwise. 684 mL of isopropylether was added at room temperature and stirred for 3 hours, followed by filtering the resulting mixture. A solid was recovered and dried in hot air at 60 °Cm thereby obtaining tetrahydro-2H-pyrazino[1,2-a]pyrazine-1,4(3H,6H)-dione trifluoroacetate as a solid. (48 g, yield: 95%)

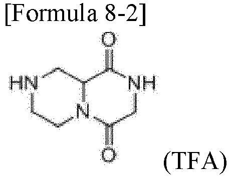

### tetrahydro-2H-pyrazino[1,2-a]pyrazine-1,4(3H,6H)-dione

¹HNMR (700 MHz, D₂O) δ 4.64 - 4.61 (m, 1H), 4.51 - 4.49 (m, 1H), 4.07 (d, *J=* 1.0 Hz, 2H), 3.80 - 3.78 (m, 1H), 3.53 - 3.50 (m, 1H), 3.29 - 3.25 (m, 1H), 3.13 - 3.08 (m, 1H), 3.07 - 3.03 (m, 1H).

¹³C NMR (176 MHz, D₂O) δ 164.62, 164.43, 53.47, 44.48, 43.73, 42.13, 38.04.

MS (ESI) m/z for C₇H₁₁N₃O₂ [M+H]⁺ : calcd 170.0930, found 170.0917.

### 2. Preparation Example 2: 8-[(naphthalen-2-yl)methyl]tetrahydro-2H-pyrazino[1,2-a]pyrazine-1,4(3H,6H)-dione (Formula N501)

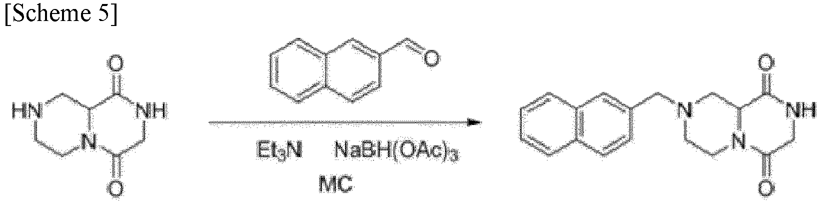

1g (3.53 mmol) of the tetrahydro-2*H*-pyrazino[1,2-*a*]pyrazine-1,4(3*H*,6*H*)-dione trifluoroacetate yielded from Preparation Example 1, 10 mL of dichloromethane, 1.0 mL of triethylamine, and 0.61 g (3.88 mmol) of 2-naphthaldehyde were input at room temperature. 1.5 g (7.06 mmol) of sodium triacetoxyborohydride was added, and then stirred at room temperature for 4 hours. The reaction solution was washed with 10 mL of distilled water. The reaction solution was washed with an aqueous solution of sodium hydrogen carbonate and 10 mL of purified water in this order. An organic layer was dried over anhydrous magnesium sulfate and filtered, followed by concentrating the reaction solution. 6 mL of dichloromethane was added to the concentrated residue, and the reaction solution was dissolved. 6 mL of n-heptane was slowly added dropwise to generate crystals and stirred at room temperature for 2 hours, followed by filtering the resulting product. The solid was dried in hot air at 60 °C, thereby obtaining 8-[(naphthalen-2-yl)methyl]tetrahydro-2*H*-pyrazino[1,2-*a*]pyrazine-1,4(3*H*,6*H*)-dione as a white solid (0.95 g, yield: 87%).

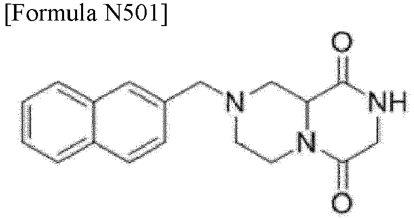

### 8-[(naphthalen-2-yl)methyl]tetrahydro-2H-pyrazino[1,2-a]pyrazine-1,4(3H,6H)-dione (Expressed as N501)

¹H NMR (500 MHz, CDCl₃)δ7.84-7.80(m, 3H), 7.72 (s, 1H), 7.49-7.45 (m, 3H), 6.43 (s, 1H), 4.53-4.50 (m, 1H), 4.12-4.04 (m, 1H), 4.04 (d, *J=* 1.5 Hz, 2H), 3.81 (d, *J=* 13 Hz, 1H), 3.69 (d, *J =* 13.5 Hz, 1H), 3.49-3.47 (m, 1H), 2.91 (d, *J=* 11.5 Hz, 1H), 2.85-2.79 (m, 1H), 2.17 (t, *J=* 11.3 Hz, 1H), 2.13-2.08 (m, 1H).

¹³C NMR (176 MHz, CDCl₃) δ163.41, 161.66, 134.91, 132.92, 128.93, 128.51, 128.17, 127.80, 127.65, 127.11, 126.09, 125.82, 62.69, 57.31, 56.71, 51.30, 48.53, 41.26.

MS (ESI) m/z for C18H19N3O2[M+H]+:calcd310.1556,found310.1550.

### 3. Preparation Example 3: Formula N513

A compound of Formula N513 was obtained by further performing the step of Scheme 6 below using the compound obtained in Preparation Example 2 (Formula N501) as a starting material.

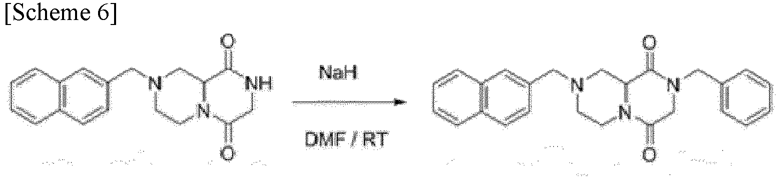

0.3 g (0.97 mmol) of 8-[(naphthalen-2-yl)methyl]tetrahydro-2*H*-pyrazino[1,2-*a*]pyrazine-1,4(3*H*,6*H*)-dione, 0.08 g (1.9 mmol) of sodium hydride, and dimethylformamide were input and stirred for 1 hour, 0.18 mL (1.5 mmol) of benzylbromide was added and stirred at room temperature overnight. The reaction was terminated with 10 mL of an aqueous ammonium chloride solution and 15 mL of ethyl acetate. An organic layer was extracted and washed with 20 mL of an aqueous sodium chloride solution. The organic layer was dried over anhydrous magnesium sulfate and filtered. The reaction solution was concentrated and purified by silica chromatography, thereby obtaining 2-benzyl-8-(naphthalen-2-ylmethyl)hexahydro-1*H-*pyrazino[1,2-*a*]pyrazine-1,4(6*H*)-dione (0.15 g, yield: 39%).

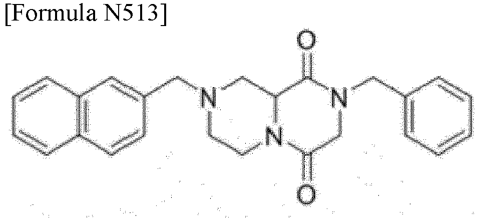

### 2-benzyl-8-(naphthalen-2-ylmethyl)hexahydro-1H-pyrazino[1,2-a]pyrazine-1,4(6H)-dione (Expressed as N513)

¹H NMR (500 MHz, CDCl₃) δ 7.83-7.80(m,3H), 7.72(s,1H), 7.49-7.45(m,3H), 7.35-7.30(m,3H), 7.24(d,J= 7.0 Hz, 2H), 4.59 - 4.52 (m, 2H), 4.47 (d, *J=* 13.5 Hz, 1H), 4.19-4.16 (m, 1H), 3.86 (s, 2H), 3.82 (d, *J=* 13.0 Hz, 1H), 3.67 (d, *J=* 13.0 Hz, 1H), 3.59 (d, *J=* 11.5 Hz, 1H), 2.87 (d, *J=* 11.5 Hz, 1H), 2.81-2.76 (m, 1H), 2.16 (t, *J=* 11.3 Hz, 1H), 2.09-2.04 (m, 1H).

¹³C NMR (176 MHz, CDCl₃) δ165.84, 162.76, 134.79, 134.57, 133.23, 132.86, 130.73, 130.04, 128.68, 128.08, 127.77, 127.64, 127.35, 127.11, 126.08, 125.80, 62.67, 57.39, 55.94, 55.76, 51.09, 41.50, 41.12.

MS (ESI) m/z for C₂₅H₂₅N₃O₂[M+H]+:calcd400.2025,found400.2018.

### 4. Preparation Example 4: Formula S461

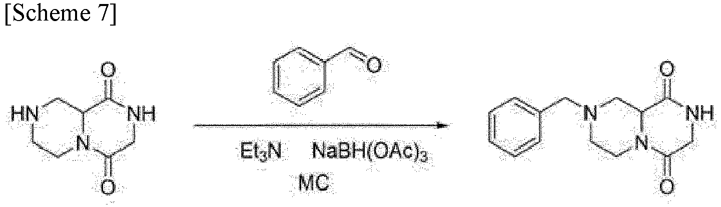

1 g (3.53 mmol) of the tetrahydro-2H-pyrazino[1,2-a]pyrazine-1,4(3H,6H)-dione trifluoroacetate obtained in Preparation Example 1, 10 mL of dichloromethane, 1.0 mL of triethylamine, and 0.6 g (5.9 mmol) of benzaldehyde were input at room temperature. 1.5g (7.06 mmol) of sodium triacetoxyborohydride was added and stirred at room temperature for 4 hours, and the reaction solution was washed with 8 mL of distilled water. The reaction solution was washed with sodium bicarbonate aqueous solution and 8 mL of distilled water in this order. An organic layer was dried over anhydrous magnesium sulfate and filtered, and the reaction solution was concentrated. 6 mL of dichloromethane was added to the concentrated residue, and the reaction solution was dissolved. 6 mL of n-heptane was slowly added dropwise to generate crystals, and the resulting product was stirred at room temperature for 2 hours, followed by filtering the reaction product. The solid was dried in hot air at 60 °C, thereby obtaining 8-benzyltetrahydro-2H-pyrazino[1,2-a]pyrazine-1,4(3H,6H)-dione as a white solid (0.78 g, yield: 51%).

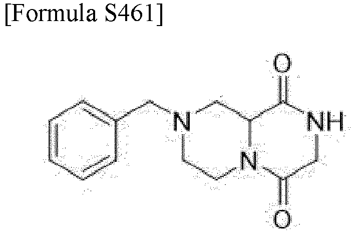

### 8-benzyltetrahydro-2H-pyrazino[1,2-a]pyrazine-1,4(3H,6H)-dione

¹H NMR (500 MHz, CDCl₃) δ 7.35 - 7.27 (m, 5H), 7.01 (s, 1H), 4.53 - 4.50 (m, 1H), 4.11 - 4.09 (m, 1H), 4.03 (s, 2H), 3.65 (d, 1H), 3.53 (d, 1H), 3.44 - 3.42 (m, 1H), 2.87 - 2.85 (m, 1H), 2.83 - 2.77 (m, 1H), 2.11 (t, *J=* 11.5Hz, 1H), 2.08 - 2.03 (m, 1H).

¹³C NMR (176 MHz, CDCl₃) δ 163.41, 161.64, 137.89, 134.91, 128.94, 128.51, 128.32, 128.19, 57.27, 56.44, 51.22, 49.31, 48.53, 41.23.

Mass 260.13

### 5. Preparation Example 5: Formula S462

A compound of Formula S462 was obtained by further performing the step of Scheme 8 below using the compound (Formula S461) obtained in Preparation Example 4 as a starting material.

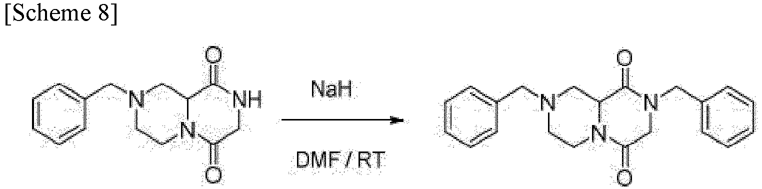

0.5 g (1.95 mmol) of 8-benzyltetrahydro-2*H*-pyrazino[1,2-*a*]pyrazine-1,4(3*H*,6*H*)-dione, 0.16 g (3.9 mmol) of sodium hydride, and 10 mL of dimethylformamide were input and stirred for 1 hour, and 0.35 mL (2.9 mmol) of benzylbromide was added, followed by stirring at room temperature overnight. The reaction was terminated with 20 mL of an ammonium chloride aqueous solution and 20 mL of ethyl acetate. An organic layer was extracted and washed with 20 mL of sodium chloride aqueous solution. The organic layer was dried over anhydrous magnesium sulfate and filtered, and the reaction solution was concentrated and purified by silica chromatography, thereby obtaining 2,8-dibenzyltetrahydro-2*H*-pyrazino[1,2-*a*]pyrazine-1,4(3*H*,6*H*)-dione (0.49 g, yield: 72%).

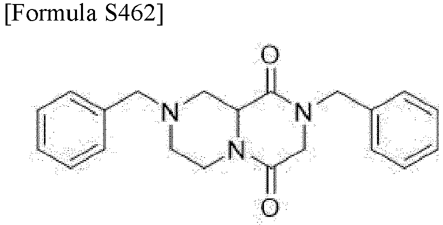

### 2,8-dibenzyltetrahydro-2H-pyrazino[1,2-a]pyrazine-1,4(3H,6H-dione

¹H NMR (500 MHz, CDCl₃) δ 7.36 - 7.25 (m, 10H), 4.61 - 4.54 (m, 2H), 4.47 (d, *J=* 13.0Hz, 1H), 4.15 (d, *J=* 9.5Hz, 1H), 3.90 - 3.83 (m, 2H), 3.66 (d, *J=* 13.0Hz, 1H), 3.55 - 3.52 (m, 2H), 2.85 - 2.75 (m, 2H), 2.11 (t, *J=* 11.5Hz, 1H), 2.06 - 2.00 (m, 1H).

¹³C NMR (176 MHz, CDCl₃) δ 163.43, 161.64, 137.02, 134.93, 129.07, 128.93, 128.50, 128.41, 128.18, 127.44, 62.54, 57.31, 56.61, 51.27, 49.30, 48.53, 41.26.

Mass 350.18

### 6. Preparation Example 6: Formula S471

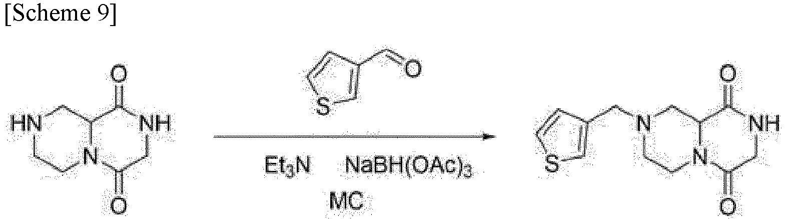

1 g (3.53 mmol) of tetrahydro-2*H*-pyrazino[1,2-*a*]pyrazine-1,4(3*H*,6*H*)-dione trifluoroacetate, 10 mL of dichloromethane, 1.0 mL of trimethylamine, and 0.66 g (5.9 mmol) of 3-thiophenecarboxaldehyde were input at room temperature. 1.5 g (7.06 mmol) of sodium triacetoxyborohydride was added, followed by stirring at room temperature for 4 hours. The reaction solution was washed with 8 mL of distilled water, and then washed with a sodium bicarbonate aqueous solution and 8 mL of distilled water in this order. An organic layer was dried over anhydrous magnesium sulfate and filtered. The reaction solution was concentrated. 6 mL of dichloromethane was added to the concentrated residue, and the reaction solution was dissolved. 6 mL of n-heptane was slowly added dropwise to generate crystals, and the resulting solution was stirred at room temperature for 2 hours, followed by filtering the reaction product. The solid was dried in hot air at 60 °C, thereby obtaining 8-[(thiophen-3-yl)methyl]tetrahydro-2*H*-pyrazino[1,2-*a*]pyrazine-1,4(3*H*,6*H*)-dione (0.75 g, yield: 48%).

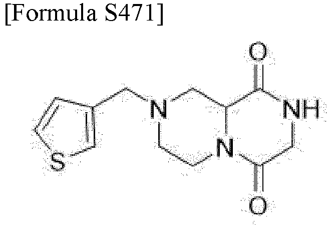

### 8-[(thiophen-3-yl)methyl]tetrahydro-2H-pyrazino[1,2-a]pyrazine-1,4(3H,6H)-dione

¹H NMR (500 MHz, CDCl₃) δ 7.31 - 7.29 (m, 1H), 7.13 (m, 1H), 7.05 - 7.04 (m, 2H), 4.53 - 4.51 (m, 1H), 4.10 - 3.99 (m, 3H), 3.66 (d, *J=* 13.5Hz, 1H), 3.59 (d, *J=* 13.5Hz), 3.44 - 3.42 (m, 1H), 2.88 (d, *J=* 11.5Hz, 1H), 2.83 - 2.77 (m, 1H), 2.11 - 2.02 (m, 2H).

¹³C NMR (176 MHz, CDCl₃) δ 166.09, 161.86, 137.81, 128.30, 125.90, 123.27, 57.09, 56.83, 55.88, 51.35, 44.50, 41.42.

Mass 266.09

### 7. Preparation Example 7: Formula S472

A compound of Formula S472 was obtained by further performing the step of Scheme 8 below using the compound (Formula S471) obtained in Preparation Example 6 as a starting material.

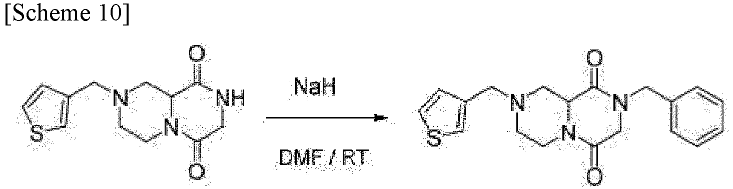

0.5 g (1.87 mmol) of 8-[(thiophen-3-yl)methyl]tetrahydro-2*H*-pyrazino[1,2-*a*]pyrazine-1,4(3*H*,6*H*)-dione, 0.15 g (3.75 mmol) of sodium hydride, and 10 mL of dimethylformamide were input and stirred for 1 hour, 0.33 mL (2.81 mmol) of benzyl bromide was added and stirred at room temperature overnight. The reaction was terminated with 30 mL of an ammonium chloride aqueous solution and 30 mL of ethyl acetate. An organic layer was extracted and washed with 20 mL of a sodium chloride aqueous solution. The organic layer was dried over anhydrous magnesium sulfate and filtered. The reaction solution was concentrated and purified by silica chromatography, thereby obtaining 2-benzyl-8-[(thiophen-3-yl)methyl]tetrahydro-2*H-*pyrazino[1,2-*a*]pyrazine-1,4(3*H*,6*H*)-dione (0.42 g, yield: 62%).

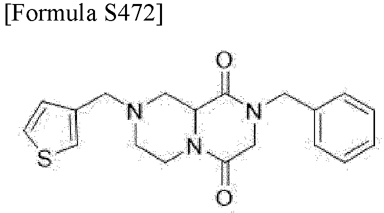

¹H NMR (500 MHz, CDCl₃) δ 7.36 - 7.29 (m, 4H), 7.26 - 7.25 (m, 2H), 7.14 (m, 1H), 7.06 - 7.05 (m, 1H), 4.62 - 4.53 (m, 2H), 4.50 - 4.47 (m, 1H), 4.15 - 4.14 (m, 1H), 3.90 - 3.83 (m, 2H), 3.67 (d, *J=* 13.5Hz, 1H), 3.59 (d, *J=* 13.5Hz, 1H), 3.56 - 3.54 (m, 1H), 2.87 - 2.85 (m, 1H), 2.81 - 2.76 (m, 1H), 2.08 (t, *J=* 11.5Hz, 1H), 2.05 - 2.00 (m, 1H).

¹³C NMR (176 MHz, CDCl₃) δ 166.03, 161.88, 134.54, 133.25, 132.90, 128.18, 127.80, 127.71, 127.66, 127.10, 126.13, 125.85, 62.69, 56.85, 56.12, 51.50, 50.58, 44.48, 41.45.

Mass 356.14

### 8. Preparation Example 8: Formula N021

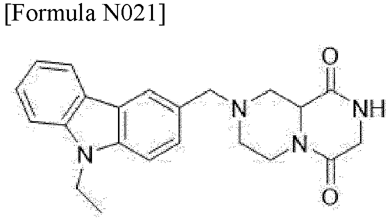

¹H NMR (400 MHz, CDCl₃) δ 8.08 (d, *J=* 7.7 Hz, 1H), 7.99 (m, 1H), 7.48 - 7.44 (m, 1H), 7.41 - 7.35 (m, 3H), 7.24 - 7.20 (m, 1H), 6.61 (s, 1H), 4.50 (ddd, *J=* 13.1, 2.9, 1.8 Hz, 1H), 4.36 (q, *J* = 7.2 Hz, 2H), 4.14 - 4.10 (m, 1H), 4.06 - 3.97 (m, 2H), 3.83 (d, *J=* 12.8 Hz, 1H), 3.70 (d, *J* = 12.8 Hz, 1H), 3.52 - 3.48 (m, 1H), 2.94 - 2.91 (m, 1H), 2.81 (td, *J* = 12.8, 3.2 Hz, 1H), 2.15 (t, *J=* 11.2 Hz, 1H), 2.11 - 2.50 (m, 1H), 1.45 - 1.41 (m, 3H).

¹³C NMR (101 MHz, CDCl₃) δ 165.78, 161.62, 140.19, 139.45, 127.09, 127.02, 125.71, 122.88, 122.64, 121.20, 120.45, 118.80, 108.50, 108.32, 77.34, 77.03, 76.71, 63.01, 56.93, 56.13, 51.31, 44.63, 41.54, 37.59, 13.85.

Mass 377.19

### 9. Preparation Example 9: Formula N022

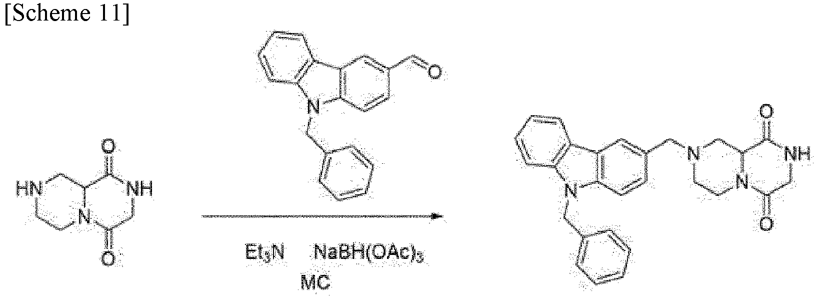

0.15 g (0.53 mmol) of tetrahydro-2*H*-pyrazino[1,2-*a*]pyrazine-1,4(3*H*,6*H*)-dione trifluoroacetate, 15 mL of dichloromethane, 1.5 mL of triethylamine, and 0.15 g (0.53 mmol) of 9-benzyl-9*H*-carbazole-3-carbaldehyde were input at room temperature. 0.22 g (1.06 mmol) of sodium triacetoxyborohydride was added, and the resulting mixture was stirred at room temperature overnight. The reaction solution as washed with 10 mL of distilled water. The reaction solution was washed with a sodium bicarbonate aqueous solution, and 10 mL of distilled water in this order. An organic layer was dried over anhydrous magnesium sulfate and filtered. The reaction solution was concentrated. 2 mL of dichloromethane was added to the concentrated residue, and the reaction solution was dissolved. 6 mL of n-heptane was slowly added dropwise to generate crystals, and the reaction product was filtered. The solid was dried, thereby obtaining 2-((9-benzyl-9*H*-carbazol-3-yl)methyl)hexahydro-2*H*-pyrazino[1,2-a]pyrazine-6,9-dione (0.126 g, yield: 54%).

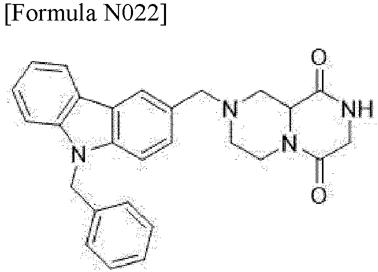

### 2-((9-benzyl-9H-carbazol-3-yl)methyl)hexahydro-2H-pyrazino[1,2-a]pyrazine-6,9-dione

¹H NMR (400 MHz, CDCl₃) δ 8.11 (d, *J=* 7.7 Hz, 1H), 8.03 (s, 1H), 7.44 - 7.21 (m, 8H), 7.16 - 7.14 (m, 2H), 7.00 (s, 1H), 5.50 (s, 2H), 4.52 - 4.49 (m, 1H), 4.13 - 4.09 (m, 1H), 4.00 (s, 2H), 3.82 (d, *J=* 12.8 Hz, 1H), 3.69 (d, *J=* 12.9 Hz, 1H), 3.51 - 3.48 (m, 1H), 2.93 (d, *J* = 11.6 Hz, 1H), 2.80 (td, *J=* 12.8, 3.2 Hz, 1H), 2.15 (t, *J=* 11.3 Hz, 1H), 2.11 - 2.05 (m, 1H).

¹³C NMR (101 MHz, CDCl₃) δ 166.16, 161.87, 141.12, 140.37, 137.28, 128.98, 127.78, 127.67, 127.48, 126.59, 126.16, 123.19, 122.93, 121.31, 120.60, 119.46, 109.17, 108.95, 63.16, 57.10, 56.32, 51.56, 46.82, 44.77, 41.70.

Mass 439.21

### 10. Preparation Example 10: Formula N024

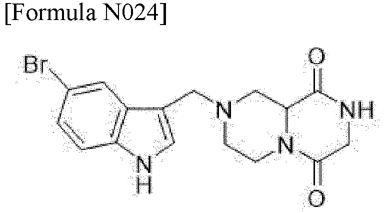

¹H NMR (500 MHz, CDCl₃) δ 8.11 (brs, 1H), 7.86 (m, 1H), 7.30 - 7.24 (m, 2H), 7.12 (m, 1H), 5.89 (s, 1H), 4.54 - 4.50 (m, 1H), 4.13 - 4.10 (m, 1H), 4.06 - 4.05 (m, 2H), 3.73 (dd, *J=* 30.4, 13.4 Hz, 2H), 3.51 - 3.48 (m, 1H), 2.96 - 2.94 (m, 1H), 2.82 (td, *J* = 12.7, 3.2 Hz, 1H), 2.11 - 2.04 (m, 2H).

Mass 377.06

### 11. Preparation Example 11: Formula N025

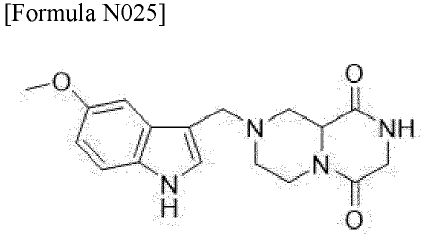

¹H NMR (400 MHz, MeOD) δ 7.20 (d, *J=* 8.8 Hz, 1H), 7.13 (m, 2H), 6.73 (dd, *J=* 8.8, 2.4 Hz, 1H), 4.41 (ddd, *J* = 13.2, 3.1, 1.8 Hz, 1H), 4.07 - 4.03 (m, 1H), 3.93 - 3.90 (m, 2H), 3.78 (s, 3H), 3.74 (d, *J=* 4.7 Hz, 2H), 3.44 - 3.40 (m, 1H), 2.99 - 2.96 (m, 1H), 2.78 (td, *J* = 12.8, 3.2 Hz, 1H), 2.09 - 2.02 (m, 2H).

¹³C NMR (101 MHz, MeOD) δ 167.61, 164.81, 155.34, 133.54, 129.58, 126.83, 113.09, 112.92, 110.95, 102.02, 58.15, 56.84, 56.40, 54.33, 52.71, 45.24, 42.54.

Mass 329.16

### 12. Preparation Example 12: Formula N026

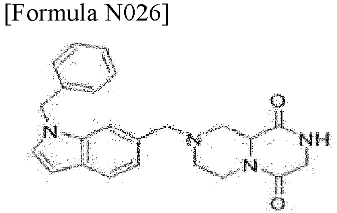

¹H NMR (500 MHz, CDCl₃) δ 7.58 (d, *J=* 8.1 Hz, 1H), 7.30 - 7.24 (m, 3H), 7.20 (s, 1H), 7.11 - 7.09 (m, 3H), 7.05 - 7.04 (m, 1H), 6.52 (m, 1H), 5.31 (s, 2H), 4.46 - 4.30 (m, 1H), 4.07 - 4.05 (m, 1H), 3.98 (m, 1H), 3.74 (d, *J=* 12.9 Hz, 1H), 3.58 (d, *J=* 12.9 Hz, 1H), 3.42 (d, *J* = 11.1 Hz, 1H), 2.82 (m, 1H), 2.76 - 2.70 (m, 1H), 2.11 - 2.06 (m, 1H), 2.01 - 1.96 (m, 1H).

¹³C NMR (126 MHz, CDCl₃) δ 166.08, 161.84, 137.53, 136.50, 130.39, 128.85, 128.68, 128.28, 127.71, 126.89, 121.24, 120.91, 110.38, 101.69, 63.22, 56.96, 56.17, 51.27, 50.16, 44.65, 41.54.

Mass 389.19

### 13. Preparation Example 13: Formula N032

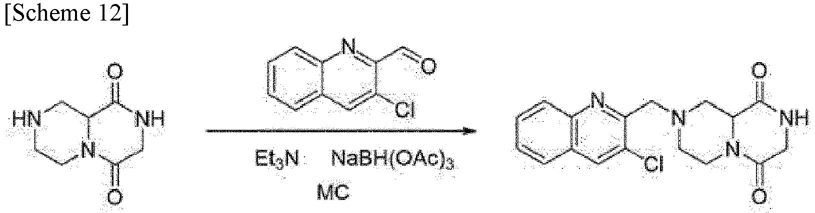

1.41g of the tetrahydro-2*H*-pyrazino[1,2-*a*]pyrazine-1,4(3*H*,6*H*)-dione trifluoroacetate (1.2 equivalents), 10 mL of methyl alcohol, 1 mL of triethylamine, 0.8 g of 2-chloro-3-quinolinecarboxaldehyde (1 equivalent) were input at room temperature, and stirred at room temperature overnight. 3 mL of dichloromethane and 2.65 g of sodium triacetoxyborohydride were added, and stirred at room temperature for 10 minutes. The reaction solution was washed with 100 mL of a sodium bicarbonate aqueous solution, dried over anhydrous magnesium sulfate and filtered. The reaction solution was concentrated and purified by silica chromatography, thereby obtaining 2-((3-chloroquinolin-2-yl)methyl)hexahydro-2*H*-pyrazino[1,2-*a*]pyrazine-6,9-dione (0.04 g, yield: 3%).

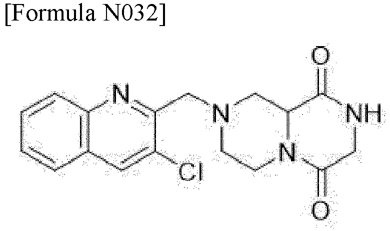

### 2-((3-chloroquinolin-2-yl)methyl)hexahydro-2H-pyrazino[1,2-a]pyrazine-6,9-dione

¹H NMR (500 MHz, CDCl₃) δ 8.30 - 8.25 (m, 1H), 8.02 - 7.96 (m, 1H), 7.91 - 7.85 (m, 1H), 7.79 - 7.72 (m, 1H), 7.64 - 7.55 (m, 1H), 4.58 - 4.50 (m, 1H), 4.23 - 4.14 (m, 1H), 4.05 - 4.00 (m, 2H), 3.87 - 3.82 (m, 1H), 3.52 - 3.45 (m, 1H), 3.05 - 2.85 (m, 2H), 2.38 - 2.26 (m, 2H).

¹³C NMR (126 MHz, CDCl₃) δ 165.74, 162.98, 151.30, 147.00, 139.24, 130.90, 129.35, 127.85, 127.81, 127.64, 127.51, 59.04, 57.09, 56.11, 51.96, 44.44, 41.71.

Mass 345.11

### 14. Preparation Example 14: Formula N033

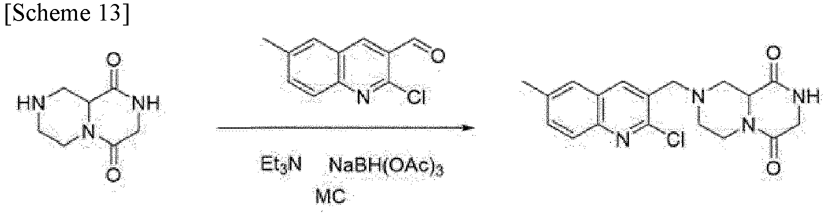

0.3 g (1.06 mmol) of tetrahydro-2*H*-pyrazino[1,2-*a*]pyrazine-1,4(3*H*,6*H*)-dione·trifluoroacetate, 3 mL of dichloromethane, 0.3 mL of triethylamine, and 0.24 g (1.17 mmol) of 6-chloro-2-methylquinoline-3-carboxaldehyde were input at room temperature. 0.449 g (2.12 mmol) of sodium triacetoxyborohydride was added to the resulting mixture, and stirred at room temperature for 1 hour. Acetic acid was added to the resulting mixture to adjust the pH to 4 to 5, and then stirred at room temperature for 5 hours. The reaction solution was washed with 24 mL of distilled water. The reaction solution was washed with 24 mL of a sodium bicarbonate aqueous solution and 24 mL of distilled water in this order. An organic layer was dried over anhydrous magnesium sulfate and filtered. The reaction solution was concentrated and purified by silica chromatography, thereby 2-((2-chloro-6-mrthylquinolin-3-yl)methyl)hexahydro-2*H*-pyrazino[1,2-*a*]pyrazine-6,9-dione (0.234 g, yield: 56%).

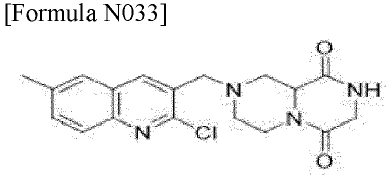

### 2-((2-chloro-6-mrthylquinolin-3-yl)methyl)hexahydro-2H-pyrazino[1,2-a]pyrazine-6,9-dione

¹H NMR (400 MHz, DMSO) δ 8.37 - 8.23 (m, 2H), 7.87 - 7.803 (m, 2H), 7.66 - 7.64 (m, 1H), 4.31 (d, *J=* 13.0 Hz, 1H), 4.07 - 4.04 (m, 1H), 3.91 - 3.78 (m, 4H), 3.26 - 3.24 (m, 1H), 2.94 - 2.92 (m, 1H), 2.75 (t, *J=* 11.7 Hz, 1H), 2.17 - 2.15 (m, 2H).

¹³C NMR (101 MHz, DMSO) δ 164.68, 162.63, 149.60, 144.82, 138.34, 137.07, 132.70, 129.40, 127.29, 126.97, 126.64, 58.05, 56.04, 55.31, 51.41, 43.90, 40.66, 21.13.

Mass 359.2

### 15. Preparation Example 15: Formula N034

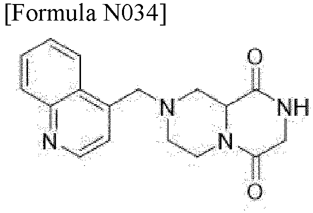

¹H NMR (500 MHz, CDCl₃) δ 8.81 - 8.79 (m, 1H), 8.27 - 8.24 (m, 1H), 8.10 - 8.07 (m, 1H), 7.79 - 7.76 (m, 1H), 7.64 - 7.61 (m, 1H), 7.50 - 7.48(m, 1H), 4.53 - 4.50 (m, 1H), 4.15 - 4.10 (m, 2H), 4.03 - 4.00 (m, 3H), 3.49 - 3.46 (m, 1H), 2.95 - 2.93 (m, 1H), 2.88 - 2.82 (m, 1H), 2.31 - 2.22 (m, 2H)

¹³C NMR (126 MHz, CDCl₃) δ 165.77, 163.08, 149.87, 148.00, 144.04, 130.08, 129.23, 127.87, 127.17, 124.46, 121.92, 59.37, 57.15, 56.49, 52.15, 44.45, 41.73.

Mass 311.14

### 16. Preparation Example 16: Formula N035

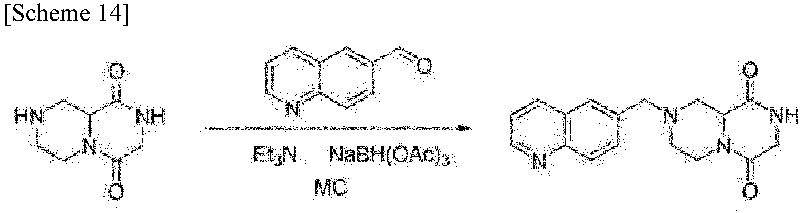

0.2 g (0.70 mmol) of tetrahydro-2H-pyrazino[1,2-a]pyrazine-1,4(3H,6H)-dione·trifluoroacetate, 2 mL of dichloromethane, 0.2 mL of triethylamine, and 0.122 g (0.78 mmol) of 6-quinolinecarboxaldehyde were input at room temperature. 0.3g (1.42 mmol) of sodium triacetoxyborohydride was added to the resulting mixture, and then stirred at room temperature for 1 hour. Acetic acid was added to adjust the pH to 4 to 5, and the resulting mixture was stirred at room temperature for 5 hours. The reaction solution was washed with 16 mL of distilled water. The reaction solution was washed with 16 mL of a sodium bicarbonate aqueous solution and 16 mL of distilled water in this order. An organic layer was dried over anhydrous magnesium sulfate and filtered. The reaction solution was concentrated and purified by silica chromatography, thereby 2-(quinoline-6-ylmethyl)hexahydro-2*H*-pyrazino[1,2-*a*]pyrazine-6,9-dione (0.114 g, yield: 52%).

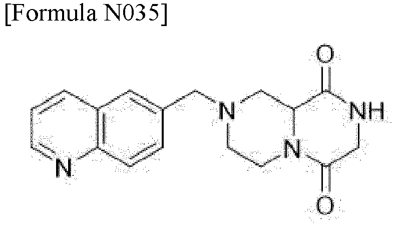

### 2-(quinoline-6-ylmethyl)hexahydro-2H-pyrazino[1,2-a]pyrazine-6,9-dione

¹H NMR (400 MHz, CDCl₃) δ 8.91 (dd, *J* = 4.2, 1.6 Hz, 1H), 8.18 - 8.05 (m, 2H), 7.78 - 7.67 (m, 2H), 7.41 (dd, *J* = 8.3, 4.2 Hz, 1H), 7.21 (s, 1H), 4.65 - 4.44 (m, 1H), 4.19 - 4.09 (m, 1H), 4.04 (m, 2H), 3.77 (dd, *J=* 35.8, 13.3 Hz, 2H), 3.47 - 3.44 (m, 1H), 2.92 - 2.80 (m, 2H), 2.21 - 2.09 (m, 2H).

¹³C NMR (101 MHz, CDCl₃) δ 165.99, 161.95, 150.51, 148.03, 136.11, 135.77, 130.85, 129.82, 128.24, 127.63, 121.50, 62.51, 57.00, 56.30, 51.78, 44.74, 41.63.

Mass 311.2

### 17. Preparation Example 17: Formula N036

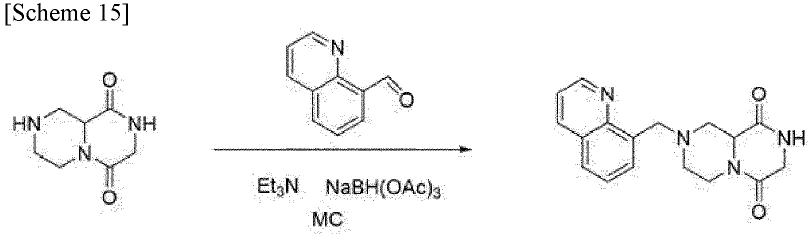

0.3 g (1.06 mmol) of tetrahydro-2*H*-pyrazino[1,2-*a*]pyrazine-1,4(3*H*,6*H*)-dione·trifluoroacetate, 3 mL of dichloromethane, 0.3 mL of triethylamine, and 0.166 g (1.06 mmol) of 8-quinolinecarboxaldehyde were input at room temperature. 0.449 g (2.12 mmol) of sodium triacetoxyborohydride was added to the resulting mixture, and then stirred at room temperature for 1 hour. Acetic acid was added to adjust the pH to 4 to 5, and then stirred at room temperature for 5 hours. The reaction solution was washed with 24 mL of distilled water. The reaction solution was washed with 24 mL of a sodium bicarbonate aqueous solution and 24 mL of distilled water in this order. An organic layer was dried over anhydrous magnesium sulfate and filtered. The reaction solution was concentrated and purified by silica chromatography, thereby 2-(quinoline-8-ylmethyl)hexahydro-2*H*-pyrazino[1,2-*a*]pyrazine-6,9-dione (0.141 g, yield: 43%).

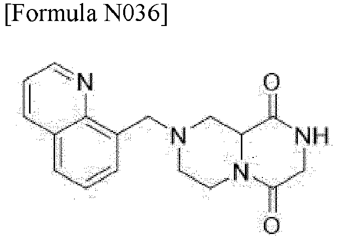

### 2-(quinoline-8-ylmethyl)hexahydro-2H-pyrazino[1,2-a]pyrazine-6,9-dione

¹H NMR (400 MHz, CDCl₃) δ 8.92 (dd, *J* = 4.2, 1.8 Hz, 1H), 8.17 (dd, *J=* 8.3, 1.8 Hz, 1H), 7.85 (d, *J* = 7.0 Hz, 1H), 7.76 (dd, *J* = 8.1, 1.1 Hz, 1H), 7.56 (dd, *J=* 7.9, 7.3 Hz, 1H), 7.42 (dd, *J* = 8.3, 4.2 Hz, 1H), 7.15 (s, 1H), 4.54 (ddd, *J* = 13.1, 2.9, 1.8 Hz, 1H), 4.43 - 4.31 (m, 2H), 4.21 - 4.14 (m, 1H), 4.03 (s, 2H), 3.55 (ddd, *J* = 11.5, 3.0, 1.8 Hz, 1H), 3.07 - 2.99 (m, 1H), 2.89 (td, *J* = 12.7, 3.3 Hz, 1H), 2.34 - 2.22 (m, 2H).

¹³C NMR (101 MHz, CDCl₃) δ 166.20, 161.87, 149.72, 146.98, 136.58, 135.43, 129.46, 128.51, 127.41, 126.52, 121.22, 57.20, 56.75, 56.50, 52.26, 44.76, 41.77.

Mass 311.2

### 18. Preparation Example 18: Formula N054

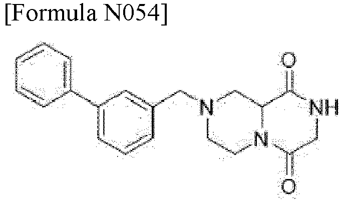

¹H NMR (500 MHz, CDCl₃) δ 7.60 - 7.58 (m, 2H), 7.54 - 7.49 (m, 2H), 7.47 - 7.33 (m, 4H), 7.29 (d, *J* = 7.5 Hz, 1H), 6.55 (s, 1H), 4.53 - 4.50 (m, 1H), 4.13 - 4.11 (m, 1H), 4.04 (s, 2H), 3.71 (d, *J* = 13.2 Hz, 1H), 3.60 (d, *J* = 13.2 Hz, 1H), 3.51 - 3.44 (m, 1H), 2.92 - 2.90 (m, 1H), 2.82 (td, *J* = 12.7, 3.2 Hz, 1H), 2.18 - 2.03 (m, 2H).

¹³C NMR (126 MHz, CDCl₃) δ 165.87, 161.90, 141.56, 140.99, 129.02, 128.87, 128.19, 128.05, 127.49, 127.27, 126.58, 62.63, 56.77, 56.06, 51.48, 44.66, 41.40.

Mass 336.17

### 19. Preparation Example 19: Formula N301

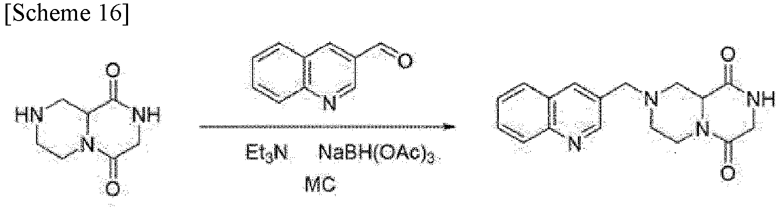

5.4 g (19.1 mmol) of tetrahydro-2*H*-pyrazino[1,2-*a*]pyrazine-1,4(3*H*,6*H*)-dione trifluoroacetate, 5.5 mL of dichloromethane, 5.5 mL of triethylamine, and 3.0 g (19.1 mmol) of 3-quinolinecarboxaldehyde were input at room temperature. 8.1 g (38.2 mmol) of sodium triacetoxyborohydride was added, and then stirred at room temperature for 6 hours. The reaction solution was washed with 10 mL of distilled water. The reaction solution was washed with a sodium bicarbonate aqueous solution and 10 mL of distilled water in this order. An organic layer was dried over anhydrous magnesium sulfate and filtered. The reaction solution was concentrated and purified by silica chromatography, thereby 2-(quinolin-3-ylmethyl)hexahydro-2*H*-pyrazino[1,2-*a*]pyrazine-6,9-dione.

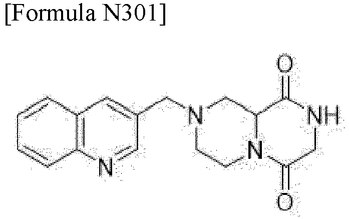

### 2-(quinolin-3-ylmethyl)hexahydro-2H-pyrazino[1,2-a]pyrazine-6,9-dione

¹H NMR (500 MHz, CDCl₃) δ 8.90 (d, *J* = 2.0 Hz, 1H), 8.11 (d, *J* = 8.4 Hz, 1H), 8.06 (s, 1H), 7.81 (d, *J=* 7.9 Hz, 1H), 7.75 - 7.68 (m, 1H), 7.60 - 7.53 (m, 1H), 7.49 (s, 1H), 4.55 - 4.49 (m, 1H), 4.17 - 4.08 (m, 1H), 4.02 (s, 2H), 3.77 (dd, *J=* 36.4, 13.5 Hz, 2H), 3.47 - 3.42 (m, 1H), 2.90 (d, *J* = 11.6 Hz, 1H), 2.82 (td, *J* = 12.9, 3.2 Hz, 1H), 2.26 - 2.10 (m, 2H).

¹³C NMR (126 MHz, CDCl₃) δ 165.78, 161.95, 151.71, 147.61, 135.86, 129.88, 129.48, 129.12, 127.80, 127.65, 126.93, 60.04, 56.75, 56.00, 51.59, 44.52, 41.37.

Mass 451.2

### 20. Preparation Example 20: Formula N311

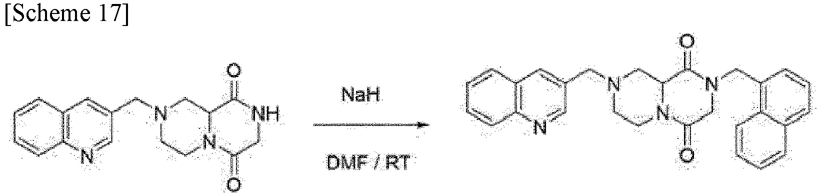

0.25 g (0.65 mmol) of 2-(quinolin-3-ylmethyl)hexahydro-2H-pyrazino[1,2-a]pyrazine-6,9-dione, 0.039 g (0.97 mmol) of sodium hydride, and dimethylformamide were input, stirred at room temperature for 30 minutes, stirred at room temperature for 30 minutes, and then 0.171 g (0.77 mmol) of 1-(bromomethyl)naphthalene was added, followed by stirring at room temperature overnight. 10 mL of an ammonium chloride aqueous solution and 20 mL of ethyl acetate were added and stirred, and then an organic layer was extracted and washed with 10 mL of a sodium chloride aqueous solution. An organic layer was dried over anhydrous magnesium sulfate and filtered. The reaction solution was concentrated and purified by silica chromatography, thereby 8-(naphthalen-1-yl)-2-(quinolin-3-ylmethyl)hexahydro-2*H-*pyrazino[1,2-*a*]pyrazine-6,9-dione (0.212 g, yield: 73%).

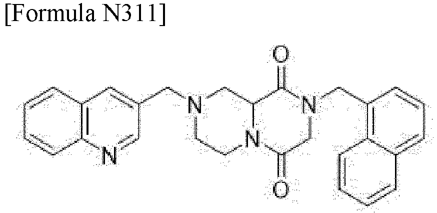

### 8-(naphthalen-1-yl)-2-(quinolin-3-ylmethyl)hexahydro-2H-pyrazino[1,2-a]pyrazine-6,9-dione

¹H NMR (500 MHz, CDCl₃) δ 8.91 (d, *J* = 2.2 Hz, 1H), 8.14 - 8.10 (m, 1H), 8.06 - 7.98 (m, 2H), 7.90 - 7.79 (m, 3H), 7.71 (ddd, *J* = 8.4, 6.9, 1.4 Hz, 1H), 7.58 - 7.49 (m, 3H), 7.44 - 7.36 (m, 2H), 5.06 (dd, *J* = 35.8, 14.5 Hz, 2H), 4.43 (ddd, *J* = 13.2, 3.1, 1.7 Hz, 1H), 4.21 - 4.16 (m, 1H), 3.84 - 3.69 (m, 4H), 3.62 (ddd, *J=* 11.3, 3.2, 1.7 Hz, 1H), 2.86 - 2.81 (m, 1H), 2.78 - 2.73 (m, 1H), 2.17 (t, *J* = 11.2 Hz, 1H), 2.06 (td, *J* = 11.8, 3.2 Hz, 1H).

¹³C NMR (126 MHz, CDCl₃) δ 162.85, 161.62, 151.77, 147.70, 135.74, 133.96, 131.47, 130.01, 129.90, 129.49, 129.40, 129.22, 128.89, 128.54, 127.79, 127.64, 126.92, 126.86, 126.26, 125.14, 123.53, 60.03, 57.24, 56.57, 51.30, 48.13, 47.23, 41.08.

Mass 451.2

### 21. Preparation Example 21: Formula N312

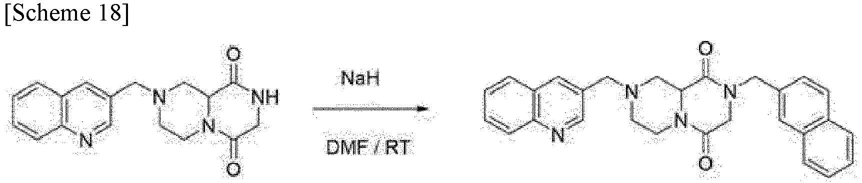

0.2 g (0.64 mmol) of 2-(quinolin-3-ylmethyl)hexahydro-2*H*-pyrazino[1,2-*a*]pyrazine-6,9-dione, 0.039 g (0.97 mmol) of sodium hydride, and dimethylformamide were input and stirred at room temperature for 30 minutes, and 0.171 g (0.77 mmol) of 2-(bromomethyl)naphthalene was added thereto, followed by stirring at room temperature overnight. 10 mL of an ammonium chloride aqueous solution and 20 mL of ethyl acetate were added and stirred, and then an organic layer was extracted and washed with 10 mL of a sodium chloride aqueous solution. An organic layer was dried over anhydrous magnesium sulfate and filtered. The reaction solution was concentrated and purified by silica chromatography, thereby 8-(naphthalen-2-yl)-2-(quinolin-3-ylmethyl)hexahydro-2*H*-pyrazino[1,2-*a*] pyrazine-6,9-dione (0.181 g, yield: 62%).

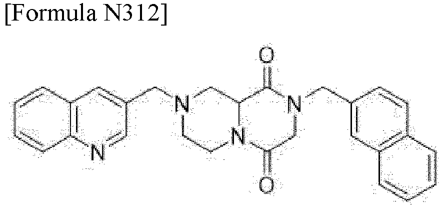

### 8-(naphthalen-2-yl)-2-(quinolin-3-ylmethyl)hexahydro-2H-pyrazino[1,2-a]pyrazine-6,9-dione

¹H NMR (500 MHz, CDCl₃) δ 8.91 (d, *J=* 2.1 Hz, 1H), 8.12 (d, *J=* 8.5 Hz, 1H), 8.05 (d, *J* = 1.5 Hz, 1H), 7.85 - 7.77 (m. 4H), 7.73 - 7.67 (m, 2H), 7.55 (ddd, *J* = 8.1, 6.9, 1.1 Hz, 1H), 7.52 - 7.45 (m, 2H), 7.36 (dd, *J* = 8.4, 1.7 Hz, 1H), 4.76 - 4.66 (m, 2H), 4.47 (ddd, *J* = 13.2, 3.0, 1.7 Hz, 1H), 4.21 - 4.16 (m, 1H), 3.90 (s, 2H), 3.77 (dd, *J* = 56.3, 13.5 Hz, 2H), 3.59 (ddd, *J* = 11.3, 3.2, 1.7 Hz, 1H), 2.88 - 2.83 (m, 1H), 2.79 (td, *J* = 12.8, 3.3 Hz, 1H), 2.19 (t, *J* = 11.2 Hz, 1H), 2.10 (td, *J* = 11.7, 3.2 Hz, 1H).

¹³C NMR (126 MHz, CDCl₃) δ 163.43, 161.78, 151.88, 147.80, 135.84, 133.29, 133.11, 132.34, 129.98, 129.50, 129.32, 129.12, 127.89, 127.84, 127.80, 127.74, 126.96, 126.63, 126.46, 126.08, 60.13, 57.31, 56.67, 51.46, 49.63, 48.64, 41.26.

Mass 451.2

### 22. Preparation Example 22: Formula N313

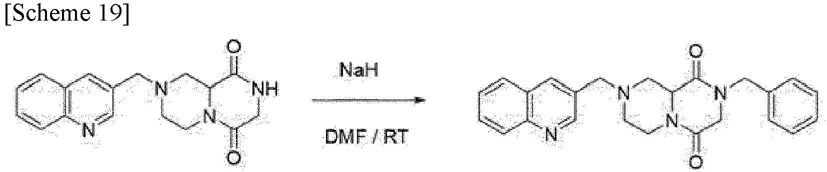

0.2 g (0.64 mmol) of 2-(quinolin-3-ylmethyl)hexahydro-2*H*-pyrazino[1,2-*a*]pyrazine-6,9-dione, 0.039 g (0.97 mmol) of sodium hydride, and dimethylformamide were input and stirred at room temperature for 30 minutes, and then 0.08 mL (0.71 mmol) of benzyl bromide was added thereto, followed by stirring at room temperature overnight. 10 mL of an ammonium chloride aqueous solution and 20 mL of ethyl acetate were added and stirred, and then an organic layer was extracted and washed with 10 mL of a sodium chloride aqueous solution. An organic layer was dried over anhydrous magnesium sulfate and filtered. The reaction solution was concentrated and purified by silica chromatography, thereby obtaining 8-benzyl-2-(quinolin-3-ylmethyl)hexahydro-2*H*-pyrazino[1,2-*a*]pyrazine-6,9-dione (0.106 g, yield: 41%).

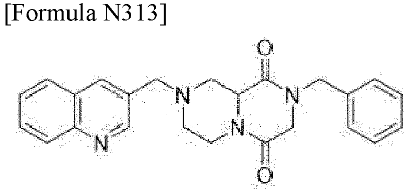

### 8-benzyl-2-(quinolin-3-ylmethyl)hexahydro-2H-pyrazino[1,2-a]pyrazine-6,9-dione

¹H NMR (500 MHz, CDCl₃) δ 8.91 (d, *J* = 2.2 Hz, 1H), 8.11 (d, *J* = 8.4 Hz, 1H), 8.06 (d, *J=* 1.5 Hz, 1H), 7.83 - 7.79 (m, 1H), 7.71 (m, 1H), 7.56 (m, 1H), 7.37 - 7.28 (m, 3H), 7.27 - 7.23 (m, 2H), 4.57 (s, 2H), 4.49 (ddd, *J* = 13.2, 3.2, 1.8 Hz, 1H), 4.20 - 4.15 (m, 1H), 3.90 - 3.82 (m, 3H), 3.73 (d, *J=* 13.5 Hz, 1H), 3.58 (m, 1H), 2.91 - 2.85 (m, 1H), 2.80 (td, *J=* 12.7, 3.4 Hz, 1H), 2.19 (t, *J=* 11.2 Hz, 1H), 2.12 (td, *J=* 11.7, 3.3 Hz, 1H).

¹³C NMR (126 MHz, CDCl₃) δ 163.26, 161.73, 151.80, 147.73, 135.76, 134.85, 129.89, 129.41, 129.25, 128.97, 128.52, 128.24, 127.80, 127.65, 126.87, 60.07, 57.20, 56.59, 51.39, 49.35, 48.53, 41.19.

Mass 401.2

### 23. Preparation Example 23: Formula N322

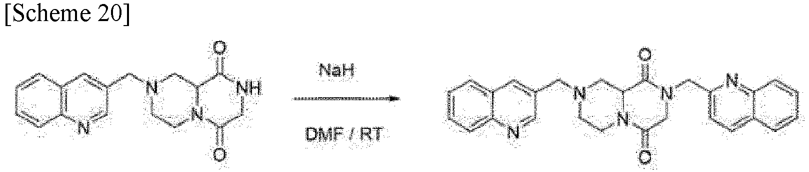

0.2 g (0.64 mmol) of 2-(quinolin-3-ylmethyl)hexahydro-2*H*-pyrazino[1,2-*a*]pyrazine-6,9-dione, 0.077 g (1.93 mmol) of sodium hydride, and dimethylformamide were input and stirred at room temperature for 30 minutes, and then 0.166 g (0.77 mmol) of 2-(chloromethyl)quinoline hydrochloride was added thereto, followed by stirring at room temperature overnight. 10 mL of an ammonium chloride aqueous solution and 20 mL of ethyl acetate were added to the resulting mixture and then stirred, and an organic layer was extracted and washed with 10 mL of a sodium chloride solution. An organic layer was dried over anhydrous magnesium sulfate and filtered. The reaction solution was concentrated and purified by silica chromatography, thereby obtaining 8-(quinolin-2-ylmethyl)-2-(quinolin-3-ylmethyl)hexahydro-2*H*-pyrazino[1,2-*a*]pyrazine-6,9-dione (0.158 g, yield: 54%).

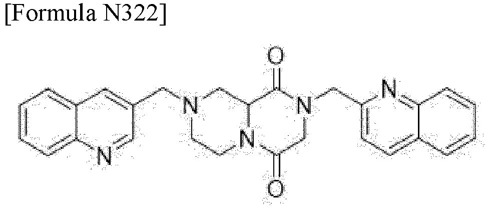

### 8-(quinolin-2-ylmethyl)-2-(quinolin-3-ylmethyl)hexahydro-2H-pyrazino[1,2-a]pyrazine-6,9-dione

¹H NMR (500 MHz, CDCl₃) δ 8.91 (d, *J* = 2.2 Hz, 1H), 8.16 - 8.00 (m, 4H), 7.81 - 7.79 (m, 2H), 7.74 - 7.67 (m, 2H), 7.54 (m, 2H), 7.37 (d, *J* = 8.4 Hz, 1H), 4.85 (s, 2H), 4.53 (ddd, *J=* 13.2, 3.1, 1.7 Hz, 1H), 4.25 - 4.18 (m, 3H), 3.79 (dd, *J* = 51.5, 13.4 Hz, 2H), 3.57 (m, 1H), 2.92 - 2.79 (m, 2H), 2.27 - 2.11 (m, 2H).

¹³C NMR (126 MHz, CDCl₃) δ 163.68, 161.94, 155.18, 151.80, 147.70, 147.66, 137.32, 135.77, 129.89, 129.85, 129.40, 129.22, 127.79, 127.65, 127.59, 127.44, 126.86, 126.73, 120.11, 77.32, 77.06, 76.81, 60.07, 57.20, 56.56, 51.55, 51.43, 49.86, 41.24.

Mass 452.2

### 24. Preparation Example 24: Formula N323

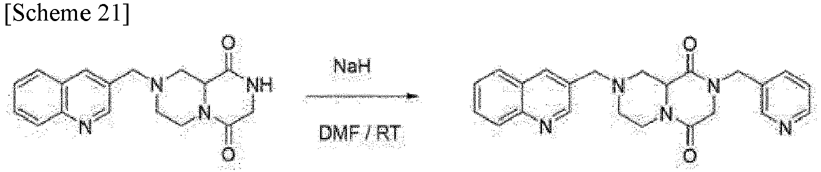

0.2 g (0.64 mmol) of 2-(quinolin-3-ylmethyl)hexahydro-2*H*-pyrazino[1,2-*a*]pyrazine-6,9-dione, 0.077 g (1.93 mmol) of sodium hydride, and dimethylformamide were input and stirred at room temperature for 30 minutes, and 0.127 g (0.77 mmol) of 3-(chloromethyl)pyridine hydrochloride was added thereto, followed by stirring at room temperature overnight. 10 mL of an ammonium chloride aqueous solution and 20 mL of ethyl acetate were added and stirred, and an organic layer was extracted and washed with 10 mL of a sodium chloride aqueous solution. An organic layer was dried over anhydrous magnesium sulfate and filtered. The reaction solution was concentrated and purified by silica chromatography, thereby obtaining 8-(pyridin-3-ylmethyl)-2-(quinolin-3-ylmethyl)hexahydro-2*H*-pyrazino[1,2-*a*]pyrazine-6,9-dione (0.05 g, yield: 19%).

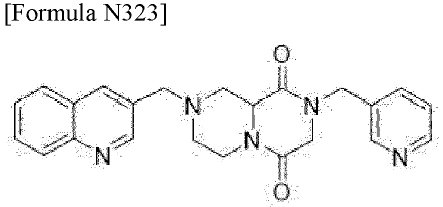

### 8-(pyridin-3-ylmethyl)-2-(quinolin-3-ylmethyl)hexahydro-2H-pyrazino[1,2-a]pyrazine-6,9-dione

¹H NMR (500 MHz, CDCl₃) δ 8.91 (d, *J* = 2.2 Hz, 1H), 8.57 (dd, *J* = 4.8, 1.6 Hz, 1H), 8.53 (d, *J* = 1.8 Hz, 1H), 8.11 (d, *J* = 8.5 Hz, 1H), 8.06 (d, *J=* 1.6 Hz, 1H), 7.81 (dd, *J* = 8.3, 1.0 Hz, 1H), 7.71 (ddd, *J* = 8.4, 6.9, 1.4 Hz, 1H), 7.64 - 7.60 (m, 1H), 7.58 - 7.54 (m, 1H), 7.32 - 7.26 (m, 1H), 4.61 - 4.54 (m, 2H), 4.49 (ddd, *J=* 13.2, 3.1, 1.7 Hz, 1H), 4.21 - 4.16 (m, 1H), 3.96 - 3.86 (m, 2H), 3.86 - 3.70 (m, 2H), 3.55 (m, 1H), 2.92 - 2.86 (m, 1H), 2.81 (td, *J=* 12.7, 3.3 Hz, 1H), 2.22 - 2.09 (m, 2H).

¹³C NMR (126 MHz, CDCl₃) δ 163.54, 161.31, 151.74, 149.68, 147.67, 136.23, 135.80, 130.76, 129.82, 129.45, 129.18, 127.78, 127.65, 126.90, 123.87, 77.37, 77.12, 76.86, 60.02, 57.11, 56.46, 51.36, 48.72, 47.02, 41.24.

Mass 402.2

### 25. Preparation Example 25: Formula N324

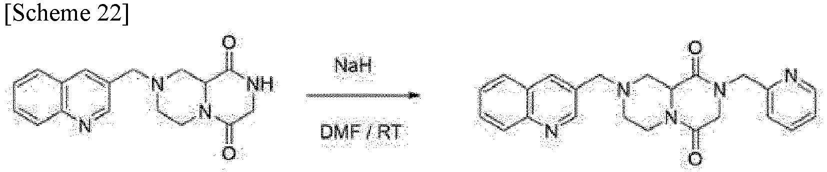

0.2 g (0.64 mmol) of 2-(quinolin-3-ylmethyl)hexahydro-2H-pyrazino[1,2-a]pyrazine-6,9-dione, 0.077 g (1.93 mmol) of sodium hydride, and dimethylformamide were input and stirred at room temperature for 30 minutes, and 0.196 g (0.77 mmol) of 2-(bromomethyl)pyridine hydrobromide was added thereto, followed by stirring at room temperature overnight. 10 mL of an ammonium chloride aqueous solution and 20 mL of ethyl acetate were added and stirred, and an organic layer was extracted and washed with 10 mL of a sodium chloride aqueous solution. An organic layer was dried over anhydrous magnesium sulfate and filtered. The reaction solution was concentrated and purified by silica chromatography, thereby obtaining 8-(pyridin-2-ylmethyl)-2-(quinolin-3-ylmethyl)hexahydro-2*H*-pyrazino[1,2-*a*]pyrazine-6,9-dione (0.068 g, yield: 26%).

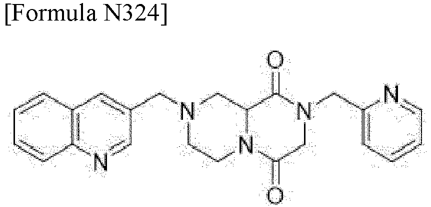

### 8-(pyridin-2-ylmethyl)-2-(quinolin-3-ylmethyl)hexahydro-2H-pyrazino[1,2-a]pyrazine-6,9-dione

¹H NMR (500 MHz, CDCl₃) δ 8.90 (d, *J=* 2.1 Hz, 1H), 8.53 (ddd, *J* = 4.9, 1.7, 0.9 Hz, 1H), 8.11 (d, *J* = 8.5 Hz, 1H), 8.05 (d, *J* = 1.5 Hz, 1H), 7.80 (dd, *J* = 8.2, 1.0 Hz, 1H), 7.73 - 7.63 (m, 2H), 7.55 (m, 1H), 7.26 (d, *J* = 7.8 Hz, 1H), 7.21 (m, 1H), 4.67 (dd, *J* = 43.5, 14.9 Hz, 2H), 4.51 (m, 1H), 4.22 - 4.06 (m, 3H), 3.77 (dd, *J=* 47.4, 13.5 Hz, 2H), 3.53 (m, 1H), 2.91 - 2.86 (m, 1H), 2.82 (td, *J* = 12.6, 3.3 Hz, 1H), 2.20 (t, *J* = 11.2 Hz, 1H), 2.14 (td, *J* = 11.7, 3.2 Hz, 1H).

¹³C NMR (126 MHz, CDCl₃) δ 163.55, 161.89, 154.98, 151.75, 149.57, 147.63, 137.01, 135.76, 129.89, 129.38, 129.15, 127.77, 127.63, 126.84, 122.86, 122.59, 60.00, 57.09, 56.43, 51.39, 50.97, 49.78, 41.18.

Mass 402.2

### 26. Preparation Example 26: Formula N401

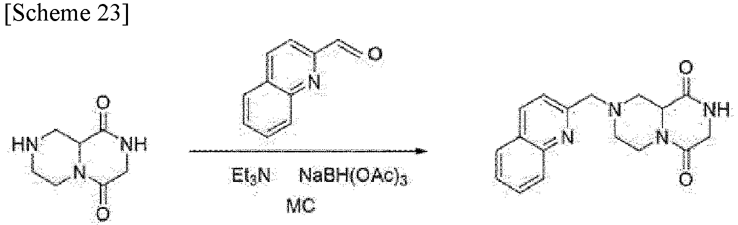

8.1 g (28.6 mmol) of tetrahydro-2H-pyrazino[1,2-a]pyrazine-1,4(3H,6H)-dione trifluoroacetate, 81 mL of dichloromethane, 8.1 mL of triethylamine, and 4.5 g (28.6 mmol) of 2-quinolinecarboxaldehyde were input at room temperature. 12.1 g (57.3 mmol) of sodium triacetoxyborohydride was added and the resulting mixture was stirred at room temperature overnight. The reaction solution was washed with 65 mL of distilled water. The reaction solution was washed with 65 mL of a sodium bicarbonate aqueous solution and 65 mL of distilled water. An organic layer was dried over anhydrous magnesium sulfate and filtered. The reaction solution was concentrated and purified by silica chromatography, thereby obtaining 2-(quinolin-2-ylmethyl)hexahydro-2*H*-pyrazino[1,2-*a*]pyrazine-6,9-dione (5.8 g, yield: 65%).

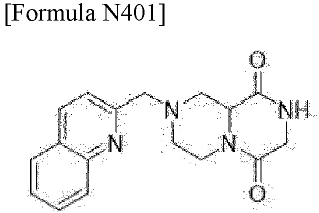

### 2-(quinolin-2-ylmethyl)hexahydro-2H-pyrazino[1,2-a]pyrazine-6,9-dione

¹H NMR (400 MHz, MeOD) δ 8.30 (d, *J=* 8.5 Hz, 1H), 7.98 (d, *J=* 8.5 Hz, 1H), 7.89 (d, *J* = 8.1 Hz,0 1H), 7.75 - 7.67 (m, 2H), 7.57 - 7.53 (m, 1H), 5.45 (d, *J=* 1.9 Hz, 1H), 4.48 - 4.38 (m, 1H), 4.14 - 4.10 (m, 1H), 4.00 - 3.80 (m, 4H), 3.35 - 3.23 (m, 1H), 2.96 - 2.79 (m, 2H), 2.28 - 2.14 (m, 2H).

¹³C NMR (101 MHz, MeOD) δ 167.20, 164.67, 160.14, 148.34, 138.76, 131.12, 129.05, 129.01, 128.87, 127.85, 122.56, 64.91, 58.03, 57.02, 53.14, 45.10, 42.43.

Mass 311.2

### 27. Preparation Example 27: Formula N411

A compound of Formula N411 below was prepared using the compound of Formula N401 in Preparation Example 26.

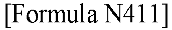

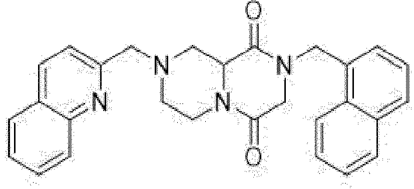

¹H NMR (500 MHz, CDCl₃) δ 8.16 (d, *J* = 8.4 Hz, 1H), 8.08 (d, *J=* 8.4 Hz, 1H), 8.05 - 8.00 (m, 1H), 7.88 - 7.80 (m, 3H), 7.73 - 7.69 (m,1H), 7.62 (d, *J=* 8.4 Hz, 1H), 7.56 - 7.49 (m, 3H), 7.45 - 7.35 (m, 2H), 5.06 (dd, *J=* 54.7, 14.5 Hz, 2H), 4.48 - 4.40 (m, 1H), 4.27 - 4.19 (m, 1H), 3.93 (dd, *J* = 42.9, 13.8 Hz, 2H), 3.73 (s, 2H), 3.64 (m, 1H), 2.87 (m, 1H), 2.80 (m, 1H), 2.27 (t, *J* = 11.3 Hz, 1H), 2.19 (td, *J* = 11.8, 3.2 Hz, 1H).

¹³C NMR (126 MHz, CDCl₃) δ 162.98, 161.63, 158.35, 147.78, 136.82, 134.05, 131.58, 130.12, 129.66, 129.56, 129.21, 128.96, 128.63, 127.65, 127.56, 127.03, 126.49, 126.35, 125.22, 123.63, 120.99, 64.80, 57.48, 56.96, 51.81, 48.24, 47.31, 41.21.

Mass 451.21

### 28. Preparation Example 28: Formula N412

A compound of Formula N412 below was prepared using the compound of Formula N401 in Preparation Example 26.

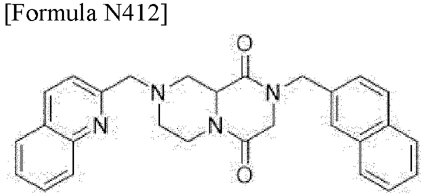

¹H NMR (400 MHz, CDCl₃) δ 8.18 (d, *J=* 8.5 Hz, 1H), 8.08 (d, *J=* 8.5 Hz, 1H), 7.84 - 7.80 (m, 4H), 7.74 - 7.71 (m, 2H), 7.64 (d, *J=* 8.5 Hz, 1H), 7.58 - 7.47 (m, 3H), 7.37 (dd, *J=* 8.4, 1.5 Hz, 1H), 4.73 (s, 2H), 4.52 - 4.48 (m, 1H), 4.27 - 4.24 (m, 1H), 4.03 - 3.87 (m, 4H), 3.64 - 3.56 (m, 1H), 2.95 - 2.80 (m, 2H), 2.33 - 2.20 (m, 2H).

¹³C NMR (126 MHz, CDCl₃) δ 163.44, 161.67, 158.33, 147.77, 136.80, 133.28, 133.09, 132.36, 129.65, 129.20, 129.10, 127.87, 127.82, 127.79, 127.64, 127.55, 126.60, 126.48, 126.42, 126.08, 121.00, 64.81, 57.44, 56.98, 51.86, 49.59, 48.63, 41.29.

Mass 451.21

### 29. Preparation Example 29: Formula N413

A compound of Formula N413 below was prepared using the compound of Formula N401 in Preparation Example 26.

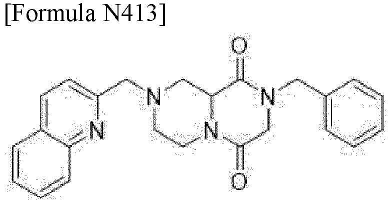

¹H NMR (400 MHz, CDCl₃) δ 8.17 (d, *J=* 8.5 Hz, 1H), 8.08 (d, *J=* 8.5 Hz, 1H), 7.82 (d, *J* = 8.1 Hz, 1H), 7.72 (t, *J* = 7.7 Hz, 1H), 7.63 (d, *J* = 8.5 Hz, 1H), 7.56 - 7.53 (m, 1H), 7.39 - 7.30 (m, 3H), 7.27 - 7.24 (m, 2H), 4.63 - 4.46 (m, 3H), 4.26 - 4.19 (m, 1H), 4.00 - 3.88 (m, 4H), 3.58 (d, *J* = 11.3 Hz, 1H), 2.95 - 2.81 (m, 2H), 2.33 - 2.18 (m, 2H).

¹³C NMR (101 MHz, CDCl₃) δ 163.28, 161.67, 158.24, 147.60, 136.86, 134.83, 129.66, 128.98, 128.52, 128.25, 127.59, 127.49, 126.47, 120.95, 64.64, 57.32, 56.86, 51.78, 49.33, 48.51, 41.22.

Mass 401.19

### 30. Preparation Example 30: Formula N414

A compound of Formula N414 below was prepared using the compound of Formula N401 in Preparation Example 26.

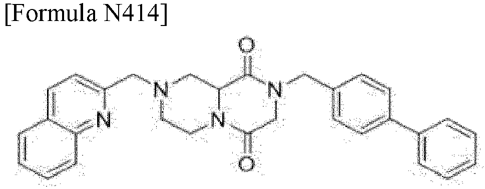

¹H NMR (400 MHz, CDCl₃) δ 8.17 (d, *J=* 8.5 Hz, 1H), 8.08 (d, *J=* 8.4 Hz, 1H), 7.83 - 7.81 (m, 1H), 7.74 - 7.70 (m, 1H), 7.63 (d, *J=* 8.5 Hz, 1H), 7.59 - 7.51 (m, 5H), 7.46 - 7.43 (m, 2H), 7.38 - 7.30 (m, 3H), 4.65 - 4.56 (m, 2H), 4.55 - 4.48 (m, 1H), 4.27 - 4.20 (m, 1H), 4.02 - 3.87 (m, 4H), 3.63 - 3.56 (m, 1H), 2.96 - 2.80 (m, 2H), 2.32 - 2.21 (m, 2H).

¹³C NMR (101 MHz, CDCl₃) δ 163.32, 161.66, 158.23, 147.63, 141.26, 140.47, 136.83, 133.81, 129.65, 129.02, 128.83, 127.71, 127.58, 127.51, 127.49, 127.10, 126.46, 120.95, 77.36, 77.05, 76.73, 64.69, 57.35, 56.88, 51.79, 49.08, 48.58, 41.24.

Mass 477.22

### 31. Preparation Example 31: Formula N422

A compound of Formula N422 below was prepared using the compound of Formula N401 in Preparation Example 26.

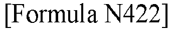

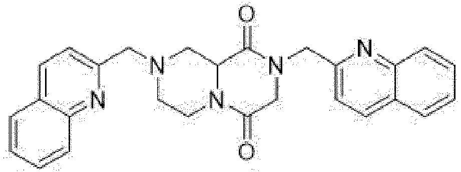

¹H NMR (400 MHz, CDCl₃) δ 8.16 (t, *J=* 7.8 Hz, 2H), 8.09 - 8.02 (m, 2H), 7.83 - 7.80 (m, 2H), 7.75 - 7.68 (m, 2H), 7.63 (d, *J* = 8.5 Hz, 1H), 7.58 - 7.51 (m, 2H), 7.38 (d, *J* = 8.4 Hz, 1H), 4.90 - 4.80 (m, 2H), 4.57 - 4.52 (m, 1H), 4.31 - 4.13 (m, 3H), 3.95 (dd, *J=* 30.5, 13.8 Hz, 2H), 3.62 - 3.53 (m, 1H), 2.97 - 2.82 (m, 2H), 2.38 - 2.21 (m, 2H).

¹³C NMR (101 MHz, CDCl₃) δ 163.72, 161.87, 158.27, 155.21, 147.65, 137.39, 136.82, 129.89, 129.64, 129.21, 129.05, 127.60, 127.58, 127.49, 127.46, 126.75, 126.45, 120.95, 120.11, 64.72, 57.32, 56.89, 51.83, 51.57, 49.83, 41.28.

Mass 452.2

### 32. Preparation Example 32: Formula N423

A compound of Formula N423 below was prepared using the compound of Formula N401 in Preparation Example 26.

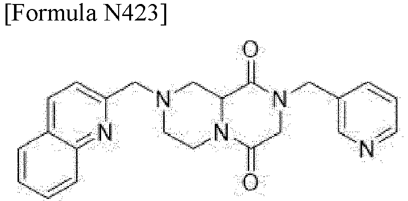

¹H NMR (400 MHz, CDCl₃) δ 8.61 - 8.51 (m, 2H), 8.17 (d, *J=* 8.5 Hz, 1H), 8.08 (d, *J* = 8.5 Hz, 1H), 7.82 (d, *J=* 8.1 Hz, 1H), 7.72 (t, *J=* 7.7 Hz, 1H), 7.63 - 7.61 (m, 2H), 7.56 - 7.53 (m, 1H), 7.32 - 7.29 (m, 1H), 4.58 (s, 2H), 4.52 - 4.49 (m, 1H), 4.25 - 4.21 (m, 1H), 4.01 - 3.86 (m, 4H), 3.60 - 3.53 (m, 1H), 2.94 - 2.82 (m, 2H), 2.30 - 2.21 (m, 2H).

¹³C NMR (101 MHz, CDCl₃) δ 163.57, 161.23, 158.13, 149.73, 149.70, 147.62, 136.86, 136.27, 130.77, 129.67, 129.02, 127.59, 127.48, 126.49, 123.92, 120.94, 64.65, 57.24, 56.79, 51.74, 48.72, 47.02, 41.28.

Mass 402.19

### 33. Preparation Example 33: Formula N424

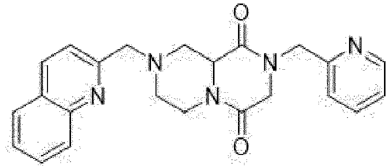

¹H NMR (500 MHz, CDCl₃) δ 8.55 - 8.53 (m, 1H), 8.15 (d, *J* = 8.4 Hz, 1H), 8.07 (d, *J* = 8.4 Hz, 1H), 7.81 (dd, *J* = 8.1, 1.1 Hz, 1H), 7.74 - 7.64 (m, 2H), 7.61 (d, *J* = 8.4 Hz, 1H), 7.55 - 7.52 (m, 1H), 7.29 - 7.24 (m, 1H), 7.21 (m, 1H), 4.71 - 4.63 (m, 2H), 4.55 - 4.51 (m, 1H), 4.27 - 4.20 (m, 1H), 4.18 - 4.06 (m, 2H), 3.93 (m, 2H), 3.55 (ddd, *J* = 11.4, 3.2, 1.7 Hz, 1H), 2.96 - 2.81 (m, 2H), 2.33 - 2.21 (m, 2H).

¹³C NMR (126 MHz, CDCl₃) δ 163.60, 161.84, 158.27, 155.08, 149.62, 147.68, 137.02, 136.74, 129.59, 129.10, 127.57, 127.48, 126.41, 122.87, 122.59, 120.94, 64.72, 57.26, 56.81, 51.81, 51.05, 49.81, 41.26.

Mass 402.19

### 34. Preparation Example 34: Formula N502

The following N502 compound was prepared using the N501 compound of Preparation Example 2.

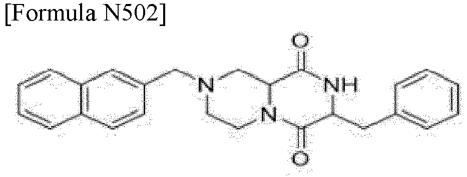

¹H NMR (500 MHz, CDCl₃) δ 7.86 - 7.76 (m, 3H), 7.64 (d, *J* = 17.1 Hz, 1H), 7.51 - 7.43 (m, 2H), 7.43 - 7.36 (m, 1H), 7.33 - 7.29 (m, 2.5H), 7.22 - 7.14 (m, 2.5H), 7.03 (s, 0.5H), 6.90 (s, 0.5H), 4.52 - 4.40 (m, 1H), 4.38 - 4.26 (m, 1H), 3.92 (dd, *J* = 11.1, 2.8 Hz, 0.5H), 3.73 - 3.43 (m, 2H), 3.29 - 3.24 (m, 1H), 3.19 (dd, *J* = 13.6, 5.6 Hz, 0.5H), 3.08 - 3.01 (m, 2H), 2.82 - 2.79 (m, 0.5H), 2.72 - 2.63 (m, 1H), 2.52 (td, *J* = 12.8, 3.4 Hz, 0.5H), 2.05 - 1.93 (m, 1H), 1.74 (td, *J* = 11.4, 3.0 Hz, 0.5H), 0.78 (t, *J* = 11.2 Hz, 0.5H).

¹³C NMR (176 MHz, CDCl₃) δ 165.06, 162.44, 135.04, 134.68, 133.28, 132.86, 130.78, 129.93, 129.01, 128.93, 128.63, 128.33, 128.05, 127.68, 127.63, 127.11, 126.05, 125.77, 62.15, 58.54, 55.07, 50.82, 46.35, 41.29, 35.98.

Mass 400.2

### 35. Preparation Example 35: Formula N503

A N503 compound was prepared by further performing the following reaction using the N502 compound of Preparation Example 34 as a starting material.

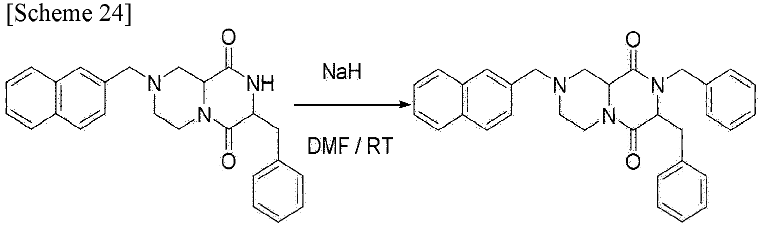

0.3 g (0.75 mmol) of 3-benzyl-8-(naphthalen-2-ylmethyl)hexahydro-1*H*-pyrazino[1,2-*a*]pyrazine-1,4(6*H*)-dione, 0.06 g (1.5 mmol) of sodium hydride, and 6 mL of dimethylformamide were input and stirred for 1 hour, and 0.13 mL (1.1 mmol) of benzyl bromide was added thereto, followed by stirring at room temperature overnight. The reaction was terminated with 10 mL of an ammonium chloride aqueous solution and 15 mL of ethyl acetate. An organic layer was extracted and washed with 20 mL of a sodium chloride aqueous solution. The organic layer was dried over anhydrous magnesium sulfate and filtered. The reaction solution was concentrated and purified by silica chromatography, thereby obtaining 2,3-dibenzyl-8-(naphthalen-2-ylmethyl)hexahydro-1*H*-pyrazino[1,2-*a*]pyrazine-1,4(6*H*)-dione (0.28 g, yield: 76%).

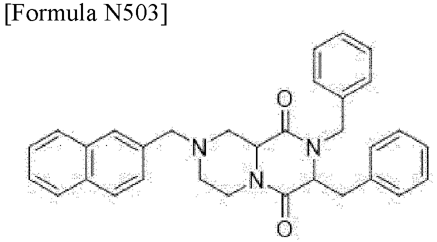

¹H NMR (500 MHz, CDCl₃) δ 7.87 - 7.74 (m, 3H), 7.59 (s, 1H), 7.52 - 7.43 (m, 2H), 7.38 - 7.28 (m, 8H), 7.17 (t, *J* = 7.4 Hz, 1H), 7.09 (d, *J* = 7.3 Hz, 2H), 5.70 (d, *J* = 14.6 Hz, 1H), 4.47 - 4.36 (m, 1H), 4.20 - 4.16 (m, 1H), 4.05 - 3.89 (m, 2H), 3.39 (dd, *J* = 45.5, 13.0 Hz, 2H), 3.25 (ddd, *J* = 18.2, 14.0, 3.7 Hz, 2H), 2.91 - 2.84 (m, 1H), 2.65 - 2.56 (m, 2H).

¹³C NMR (126 MHz, CDCl₃) δ 165.30, 162.67, 135.60, 134.88, 134.70, 133.47, 133.09, 131.03, 130.18, 129.27, 129.19, 128.99, 128.89, 128.58, 128.40, 128.30, 128.23, 128.04, 127.97, 127.94, 127.88, 127.41, 126.29, 126.02, 62.91, 59.49, 56.67, 55.31, 51.07, 46.94, 41.09, 37.17.

Mass 490.24

### 36. Preparation Example 36: Formula N504

The following N504 compound was prepared using the N501 compound of Preparation Example 2.

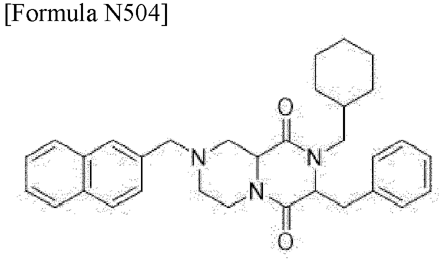

¹H NMR (500 MHz, CDCl₃) δ 7.82 - 7.74 (m, 3H), 7.64 - 7.56 (m, 1H), 7.50 - 7.42 (m, 2H), 7.39 - 7.23 (m, 4H), 7.06 - 6.99 (m, 2H), 4.46 - 4.40 (m, 0.5H), 4.36 - 4.27 (m, 1H), 4.22 (t, *J* = 3.7 Hz, 0.5H), 4.17 - 4.05 (m, 1.5H), 3.88 (dd, *J* = 11.1, 3.1 Hz, 0.5H), 3.60 (dd, *J* = 107.4, 13.1 Hz, 1H), 3.45 - 3.20 (m, 2H), 3.16 - 3.06 (m, 1H), 2.84 - 2.79 (m, 0.5H), 2.76 - 2.67 (m, 1H), 2.65 - 2.56 (m, 1.5H), 2.40 - 2.33 (m, 0.5H), 2.31 - 2.25 (m, 0.5H), 1.95 - 1.81 (m, 1H), 1.79 - 1.56 (m, 6H), 1.31 - 1.12 (m, 2H), 1.07 - 0.87 (m, 2H).

¹³C NMR (126 MHz, CDCl₃) δ 165.55, 162.82, 134.88, 134.57, 133.44, 133.07, 131.13, 130.23, 129.11, 128.90, 128.27, 128.17, 127.94, 127.84, 127.70, 127.38, 126.25, 125.98, 62.85, 61.20, 51.04, 50.12, 37.57, 35.92, 32.16, 31.38, 30.77, 29.93, 29.59, 26.55, 26.04, 25.86.

Mass 496.29

### 37. Preparation Example 37: Formula N511

The following N511 compound was prepared using the N501 compound of Preparation Example 2.

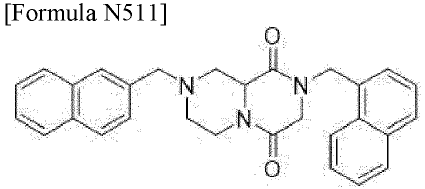

¹H NMR (500 MHz, CDCl₃) δ 8.06 - 8.01 (m, 1H), 7.90 - 7.79 (m, 5H), 7.72 (s, 1H), 7.57 - 7.34 (m, 7H), 5.05 (dd, *J* = 34.8, 14.5 Hz, 2H), 4.42 - 4.39 (m, 1H), 4.20 - 4.17 (m, 1H), 3.82 (d, *J* = 13.1 Hz, 1H), 3.71 - 3.62 (m, 4H), 2.84 - 2.81 (m, 1H), 2.74 (td, *J* = 12.7, 3.3 Hz, 1H), 2.14 (t, *J* = 11.2 Hz, 1H), 2.01 (td, *J* = 11.8, 3.1 Hz, 1H).

¹³C NMR (126 MHz, CDCl₃) δ 163.27, 161.81, 134.87, 134.23, 133.53, 133.18, 131.76, 130.36, 129.72, 129.14, 128.81, 128.45, 128.07, 128.00, 127.94, 127.39, 127.17, 126.51, 126.38, 126.10, 125.40, 123.86, 62.95, 57.65, 57.01, 51.50, 48.42, 47.47, 41.45.

Mass 450.21

### 38. Preparation Example 38: Formula N512

The following N512 compound was prepared using the N501 compound of Preparation Example 2.

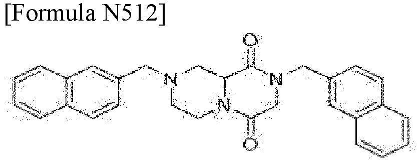

¹H NMR (500 MHz, CDCl₃) δ 7.90 - 7.77 (m, 6H), 7.70 (d, *J* = 17.1 Hz, 2H), 7.55 - 7.43 (m, 5H), 7.37 - 7.34 (m, 1H), 4.80 - 4.66 (m, 2H), 4.47 - 4.44 (m, 1H), 4.20 - 4.17 (m, 1H), 3.88 (s, 2H), 3.83 - 3.65 (m, 2H), 3.62 - 3.59 (m, 1H), 2.87 - 2.74 (m, 2H), 2.17 (t, *J* = 11.2 Hz, 1H), 2.05 (td, *J* = 11.8, 3.2 Hz, 1H).

¹³C NMR (126 MHz, CDCl₃) δ 163.75, 161.88, 134.86, 133.53, 133.46, 133.27, 133.18, 132.58, 129.26, 128.45, 128.08, 128.05, 128.01, 128.00, 127.98, 127.93, 127.40, 126.78, 126.60, 126.37, 126.29, 126.10, 62.96, 57.61, 56.99, 51.58, 49.76, 48.82, 41.53.

Mass 450.21

### 39. Preparation Example 39: Formula N514

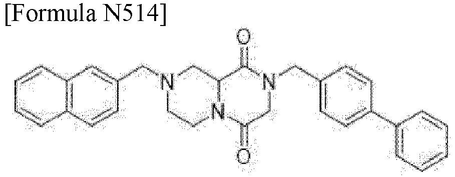

¹H NMR (500 MHz, CDCl₃) δ 7.85 - 7.80 (m, 3H), 7.73 (s, 1H), 7.57 - 7.54 (m, 4H), 7.51 - 7.42 (m, 7H), 7.37 - 7.31 (m, 3H), 4.64 - 4.57 (m, 2H), 4.50 - 4.46 (m, 1H), 4.21 - 4.17 (m, 1H), 3.96 - 3.87 (m, 2H), 3.85 - 3.67 (m,2H), 3.60 (ddd, *J* = 11.4, 3.1, 1.7 Hz, 1H), 2.92 - 2.86 (m, 1H), 2.80 (td, *J* = 12.7, 3.3 Hz, 1H), 2.18 (t, *J* = 11.2 Hz, 1H), 2.09 (td, *J* = 11.8, 3.2 Hz, 1H).

¹³C NMR (126 MHz, CDCl₃) δ 163.70, 161.91, 141.45, 140.72, 134.86, 134.15, 133.53, 133.17, 129.26, 129.08, 128.44, 128.07, 127.99, 127.93, 127.76, 127.39, 127.34, 126.36, 126.09, 62.97, 57.60, 56.98, 51.58, 49.29, 48.86, 41.55.

Mass 476.23

### 40. Preparation Example 40: Formula N522

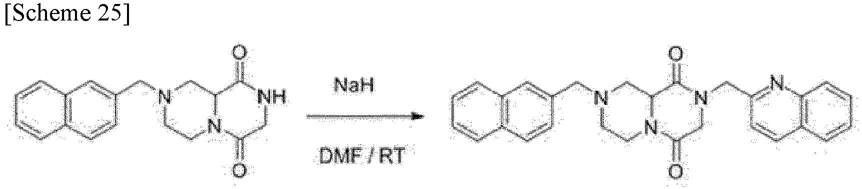

0.25 g (0.81 mmol) of 8-[(naphthalen-2-yl)methyl]tetrahydro-2*H*-pyrazino[1,2-*a*]pyrazine-1,4(3*H*,6*H*)-dione, 0.081 g (2.02 mmol) of sodium hydride, and dimethylformamide were input and stirred for 1 hour, and 0.208 g (0.97 mmol) of 2-(chloromethyl)quinoline hydrochloride was added thereto, followed by stirring at room temperature overnight. The reaction was terminated with 10 mL of an ammonium chloride aqueous solution and 10 mL of ethyl acetate. An organic layer was extracted and washed with 10 mL of a sodium chloride aqueous solution. An organic layer was dried over anhydrous magnesium sulfate and filtered. The reaction solution was concentrated and purified by silica chromatography, thereby obtaining 2-(naphthalen-2-ylmethyl)-8-(quinolin-2-ylmethyl)hexahydro-2*H*-pyrazino[1,2-*a*]pyrazine-6,9-dione (0.195g, yield: 53%).

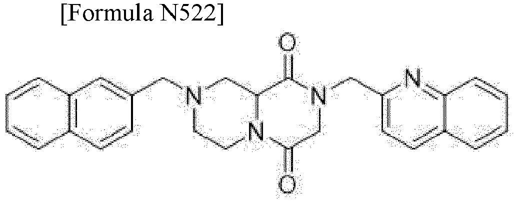

### 2-(naphthalen-2-ylmethyl)-8-(quinolin-2-ylmethyl)hexahydro-2H pyrazino[1,2-a]pyrazine-6,9-dione

¹H NMR (400 MHz, CDCl₃) δ 8.13 (d, *J* = 8.4 Hz, 1H), 8.02 (d, *J* = 8.4 Hz, 1H), 7.85 - 7.77 (m, 4H), 7.75 - 7.68 (m, 2H), 7.57 - 7.43 (m, 4H), 7.37 (d, *J* = 8.4 Hz, 1H), 4.85 (s, 2H), 4.55 - 4.45 (m, 1H), 4.27 - 4.15 (m, 3H), 3.76 (dd, *J* = 57.1, 13.1 Hz, 2H), 3.60 - 3.55 (m, 1H), 2.92 - 2.78 (m, 2H), 2.21 (t, *J* = 11.2 Hz, 1H), 2.10 (td, *J* = 11.6, 3.2 Hz, 1H).

¹³C NMR (126 MHz, CDCl₃) δ 163.83, 161.87, 155.25, 147.65, 137.28, 134.57, 133.25, 132.90, 129.82, 129.21, 128.16, 127.82, 127.72, 127.65, 127.57, 127.42, 127.13, 126.69, 126.08, 125.81, 120.10, 62.71, 57.30, 56.69, 51.54, 51.34, 49.83, 41.32.

Mass 451.4

### 41. Preparation Example 41: Formula N523

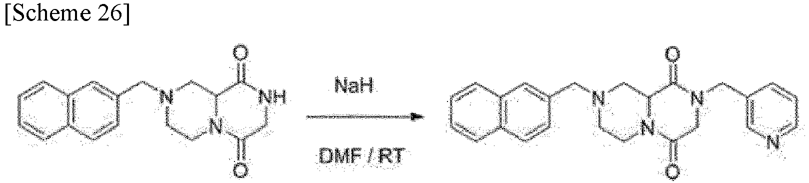

0.25 g (0.81 mmol) of 8-[(naphthalen-2-yl)methyl]tetrahydro-2*H*-pyrazino[1,2-a]pyrazine-1,4(*3H*, *6H*)-dione, 0.081 g (2.02 mmol) of sodium hydride, and dimethylformamide were input and stirred for 1 hour, and 0.159 g (0.97 mmol) of 3-(chloromethyl)pyridine hydrochloride was added, followed by stirring at room temperature overnight. The reaction was terminated with 10 mL of an ammonium chloride aqueous solution and 10 mL of ethyl acetate. An organic layer was extracted and washed with 10 mL of a sodium chloride aqueous solution. An organic layer was dried over anhydrous magnesium sulfate and filtered. The reaction solution was concentrated and purified by silica chromatography, thereby obtaining 2-(naphthalen-2-ylmethyl)-8-(pyridin-3-ylmethyl)hexahydro-2*H*-pyrazino[1,2-*a*]pyrazine-6,9-dione (0.201 g, yield: 62%).

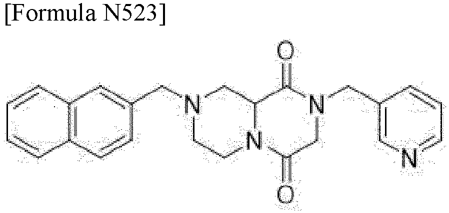

### 2-(naphthalen-2-ylmethyl)-8-(pyridin-3-ylmethyl)hexahydro-2H-pyrazino[1,2-a]pyrazine-6,9-dione

¹H NMR (500 MHz, CDCl₃) δ 8.55 (dd, *J* = 4.8, 1.6 Hz, 1H), 8.51 (d, *J* = 1.9 Hz, 1H), 7.84 - 7.76 (m, 4H), 7.71 (s, 1H), 7.60 - 7.58 (m, 1H), 7.50 - 7.41 (m, 4H), 7.29 - 7.24 (m, 1H), 4.58 - 4.41 (m, 3H), 4.22 - 4.19 (m, 1H), 3.90 - 3.78 (m, 3H), 3.73 - 3.67 (m, 1H), 3.59 - 3.54 (m, 1H), 2.95 - 2.78 (m, 2H), 2.21 - 2.04 (m, 2H).

¹³C NMR (126 MHz, CDCl₃) δ 163.70, 161.42, 149.74, 136.34, 133.34, 133.06, 130.89, 128.37, 128.16, 128.11, 127.84, 127.76, 127.23, 127.21, 126.27, 126.05, 123.94, 62.70, 57.12, 56.51, 51.31, 48.82, 47.10, 41.25.

Mass 401.2

### 42. Preparation Example 42: Formula N524

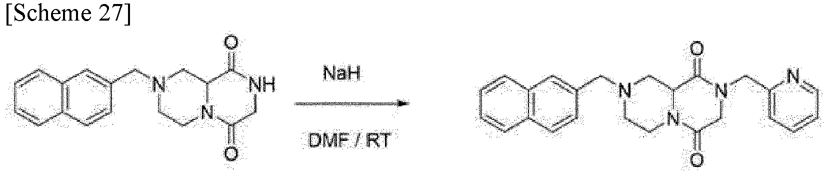

0.25 g (0.81 mmol) of 8-[(naphthalen-2-yl)methyl]tetrahydro-2*H*-pyrazino[1,2-a]pyrazine-1,4(*3H*, *6H*)-dione, 0.081 g (2.02 mmol) of sodium hydride, and dimethylformamide were input and stirred for 1 hour, and 0.245 g (0.97 mmol) of 2-(bromomethyl)pyridine hydrobromide was added thereto, followed by stirring at room temperature overnight. The reaction was terminated with 10 mL of an ammonium chloride aqueous solution and 10 mL of ethyl acetate. An organic layer was extracted and washed with 10 mL of a sodium chloride aqueous solution. An organic layer was dried over anhydrous magnesium sulfate and filtered. The reaction solution was concentrated and purified by silica chromatography, thereby obtaining 2-(naphthalen-2-ylmethyl)-8-(pyridin-2-ylmethyl)hexahydro-2*H*-pyrazino[1,2-*a*]pyrazine-6,9-dione (0.033g, yield: 10%).

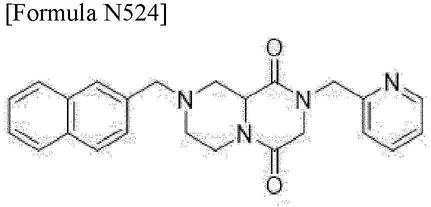

### 2-(naphthalen-2-ylmethyl)-8-(pyridin-2-ylmethyl)hexahydro-2H-pyrazino[1,2-a]pyrazine-6,9-dione

¹H NMR (500 MHz, CDCl₃) δ 8.55 - 8.52 (m, 1H), 7.84 - 7.78 (m, 3H), 7.71 (s, 1H), 7.66 (m, 1H), 7.50 - 7.43 (m, 3H), 7.28 - 7.24 (m, 1H), 7.20 (m, 1H), 4.75 - 4.59 (m, 2H), 4.52 - 4.47 (m, 1H), 4.21 - 4.17 (m, 1H), 4.17 - 4.05 (m, 2H), 3.84 - 3.65 (m, 2H), 3.57 - 3.53 (m, 1H), 2.91 - 2.85 (m, 1H), 2.81 (td, *J* = 12.6, 3.3 Hz, 1H), 2.17 (t, *J* = 11.2 Hz, 1H), 2.09 (td, *J* = 11.8, 3.2 Hz, 1H).

¹³C NMR (126 MHz, CDCl₃) δ 163.72, 161.84, 155.09, 149.60, 137.00, 134.59, 133.26, 132.91, 128.16, 127.82, 127.73, 127.65, 127.13, 126.09, 125.81, 122.85, 122.58, 62.71, 57.25, 56.63, 51.34, 51.02, 49.79, 41.31.

Mass 401.2

### 43. Preparation Example 43: Formula N601

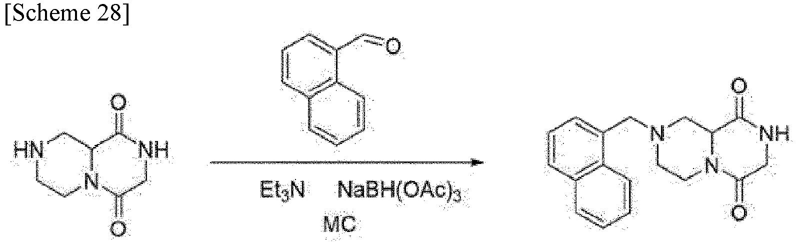

1.5 g (5.3 mmol) of tetrahydro-2*H*-pyrazino[1,2-*a*]pyrazine-1,4(*3H*,*6H*)-dione trifluoroacetate, 15 mL of dichloromethane, 1.5 mL of triethylamine, and 0.83 g (5.3 mmol) of 1-naphthaldehyde were input at room temperature. 2.25 g (10.59 mmol) of sodium triacetoxyborohydride was added, and the resulting mixture was stirred at room temperature overnight. The reaction solution was washed with 10 mL of distilled water. The reaction solution was washed with a sodium bicarbonate aqueous solution and 10 mL of distilled water in this order. An organic layer was dried over anhydrous magnesium sulfate and filtered. The reaction solution was concentrated and purified by silica chromatography, thereby obtaining 2-(naphthalen-1-ylmethyl)hexahydro-2*H*-pyrazino[1,2-*a*]pyrazine-6,9-dione (1.526g, yield: 93%).

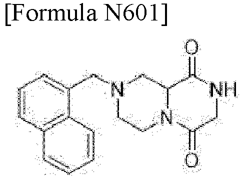

### 2-(naphthalen-1-ylmethyl)hexahydro-2H-pyrazino[1,2-a]pyrazine-6,9-dione

¹H NMR (500 MHz, CDCl₃) δ 8.24 (d, *J* = 7.8 Hz, 1H), 7.86 - 7.77 (m, 2H), 7.53 - 7.47 (m, 2H), 7.45 - 7.35 (m, 3H), 4.48 (d, *J* = 13.1 Hz, 1H), 4.08 - 4.02 (m, 2H), 3.98 (s, 2H), 3.89 - 3.83 (m, 1H), 3.52 - 3.46 (m, 1H), 2.91 - 2.84 (m, 1H), 2.72 (td, *J* = 12.8, 3.2 Hz, 1H), 2.18 (t, *J* = 11.2 Hz, 1H), 2.10 (td, *J* = 11.8, 3.1 Hz, 1H).

¹³C NMR (126 MHz, CDCl₃) δ 166.11, 161.73, 133.90, 132.67, 132.38, 128.50, 128.48, 127.77, 125.89, 125.74, 125.09, 124.62, 60.93, 56.88, 56.37, 51.57, 44.48, 41.47.

Mass 310.15

### 44. Preparation Example 44: Formula N611

The following N611 compound was prepared using the N601 compound of Preparation Example 43.

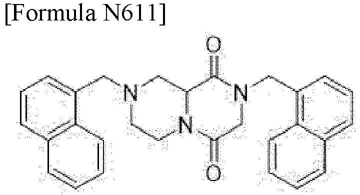

¹H NMR (500 MHz, CDCl₃) δ 8.27 (d, *J* = 8.1 Hz, 1H), 8.06 - 8.01 (m, 1H), 7.90 - 7.77 (m, 4H), 7.56 - 7.47 (m, 4H), 7.44 - 7.36 (m, 4H), 5.11 - 5.01 (m, 2H), 4.41 - 4.36 (m, 1H), 4.18 - 4.13 (m, 1H), 3.97 (dd, *J* = 121.6, 12.9 Hz, 2H), 3.73 - 3.66 (m, 3H), 2.82 (d, *J* = 11.6 Hz, 1H), 2.67 (td, *J* = 12.7, 3.2 Hz, 1H), 2.19 (t, *J* = 11.2 Hz, 1H), 2.03 (td, *J* = 11.9, 3.1 Hz, 1H).

¹³C NMR (126 MHz, CDCl₃) δ 163.04, 161.49, 133.95, 133.93, 132.75, 132.41, 131.47, 130.07, 129.45, 128.86, 128.53, 128.48, 127.76, 126.90, 126.23, 125.88, 125.73, 125.12, 125.10, 124.67, 123.56, 60.92, 57.40, 56.95, 51.31, 48.14, 47.22, 41.20.

Mass 450.21

### 45. Preparation Example 45 Formula N612

The following N612 compound was prepared using the N601 compound of Preparation Example 43.

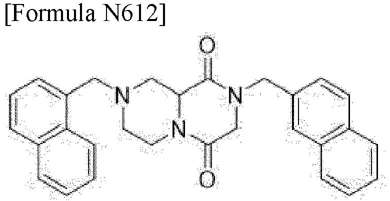

¹H NMR (500 MHz, CDCl₃) δ 8.27 (d, *J* = 8.1 Hz, 1H), 7.88 - 7.78 (m, 5H), 7.69 (s, 1H), 7.55 - 7.45 (m, 4H), 7.42 - 7.35 (m, 3H), 4.78 - 4.67 (m, 2H), 4.46 - 4.40 (m, 1H), 4.18 - 4.14 (m, 1H), 4.09 (d, *J* = 12.7 Hz, 1H), 3.89 (s, 2H), 3.86 (d, *J* = 12.9 Hz, 1H), 3.69 - 3.64 (m, 1H), 2.87 - 2.83 (m, 1H), 2.71 (td, *J* = 12.7, 3.3 Hz, 1H), 2.21 (t, *J* = 11.2 Hz, 1H), 2.08 (td, *J* = 11.8, 3.2 Hz, 1H).

¹³C NMR (126 MHz, CDCl₃) δ 163.53, 161.57, 133.93, 133.18, 132.99, 132.75, 132.41, 132.30, 129.00, 128.48, 127.77, 127.76, 127.73, 127.69, 126.51, 126.33, 125.98, 125.88, 125.73, 125.10, 124.67, 60.94, 57.36, 56.94, 51.38, 49.49, 48.54, 41.29.

Mass 450.21

### 46. Preparation Example 46 Formula N613

The following N613 compound was prepared using the N601 compound of Preparation Example 43.

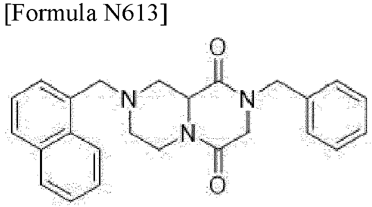

¹H NMR (400 MHz, CDCl₃) δ 8.30 - 8.24 (m, 1H), 7.89 - 7.78 (m, 2H), 7.57 - 7.47 (m, 2H), 7.44 - 7.28 (m, 6H), 7.28 - 7.22 (m, 2H), 4.66 - 4.51 (m, 2H), 4.45 (ddd, *J* = 13.1, 2.9, 1.8 Hz, 1H), 4.18 - 4.06 (m, 2H), 3.92 - 3.82 (m, 3H), 3.68 - 3.61 (m, 1H), 2.91 - 2.82 (m, 1H), 2.72 (td, *J* = 12.7, 3.3 Hz, 1H), 2.20 (t, *J* = 11.2 Hz, 1H), 2.09 (td, *J* = 11.8, 3.2 Hz, 1H).

¹³C NMR (126 MHz, CDCl₃) δ 163.46, 161.62, 134.92, 133.94, 132.76, 132.42, 128.94, 128.49, 128.19, 127.78, 125.88, 125.74, 125.10, 124.68, 60.93, 57.32, 56.93, 51.38, 49.29, 48.52, 41.29.

Mass 400.2

### 47. Preparation Example 47 Formula N614

The following N614 compound was prepared using the N601 compound of Preparation Example 43.

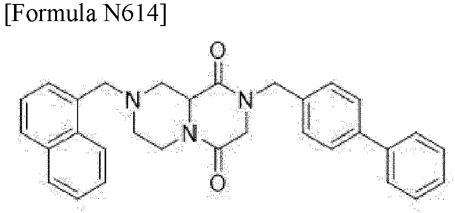

¹H NMR (400 MHz, CDCl₃) δ 8.27 (d, *J* = 8.0 Hz, 1H), 7.87 - 7.84 (m, 1H), 7.82 - 7.78 (m, 1H), 7.58 - 7.47 (m, 6H), 7.46 - 7.38 (m, 4H), 7.37 - 7.30 (m, 3H), 4.65 - 4.56 (m, 2H), 4.48 - 4.42 (m, 1H), 4.18 - 4.12 (m, 1H), 4.11 - 4.07 (m, 1H), 3.91 (s, 2H), 3.89 - 3.84 (m, 1H), 3.67 - 3.62 (m, 1H), 2.86 (d, *J* = 11.8 Hz, 1H), 2.72 (td, *J* = 12.7, 3.2 Hz, 1H), 2.21 (t, *J* = 11.2 Hz, 1H), 2.09 (td, *J* = 11.8, 3.1 Hz, 1H).

¹³C NMR (126 MHz, CDCl₃) δ 163.49, 161.60, 141.20, 140.45, 133.93, 133.88, 132.74, 132.41, 128.96, 128.81, 128.49, 127.77, 127.67, 127.48, 127.07, 125.88, 125.73, 125.10, 124.67, 60.94, 57.35, 56.93, 51.39, 49.03, 48.59, 41.31.

Mass 476.23

### 48. Preparation Example 48 Formula N622

The following N622 compound was prepared using the N601 compound of Preparation Example 43.

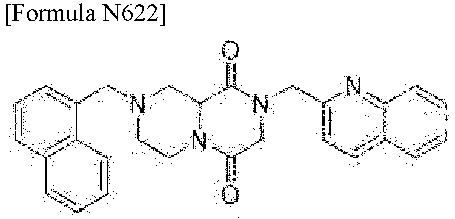

¹H NMR (400 MHz, CDCl₃) δ 8.30 - 8.24 (m, 1H), 8.15 (d, *J* = 8.5 Hz, 1H), 8.03 (d, *J* = 8.5 Hz, 1H), 7.89 - 7.69 (m, 4H), 7.58 - 7.47 (m, 3H), 7.43 - 7.36 (m, 3H), 4.86 (s, 2H), 4.53 - 4.44 (m, 1H), 4.23 - 4.16 (m, 3H), 3.99 (dd, *J* = 88.3, 12.9 Hz, 2H), 3.66 - 3.60 (m, 1H), 2.93 - 2.83 (m, 1H), 2.75 (td, *J* = 12.7, 3.2 Hz, 1H), 2.26 (t, *J* = 11.4 Hz, 1H), 2.12 (td, *J* = 11.8, 3.1 Hz, 1H).

¹³C NMR (126 MHz, CDCl₃) δ 163.85, 161.81, 155.25, 147.61, 137.24, 133.90, 132.74, 132.38, 129.79, 129.17, 128.45, 127.75, 127.54, 127.38, 126.65, 125.85, 125.70, 125.08, 124.65, 120.04, 77.35, 77.10, 76.84, 60.88, 57.26, 56.85, 51.48, 51.40, 49.80, 41.29, 0.00.

Mass 451.21

### 49. Preparation Example 49 Formula N641

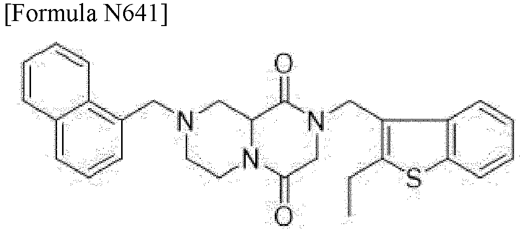

¹H NMR (400 MHz, CDCl₃) δ 8.28 - 8.24 (m, 1H), 7.89 - 7.83 (m, 1H), 7.83 - 7.72 (m, 2H), 7.71 - 7.67 (m, 1H), 7.56 - 7.46 (m, 2H), 7.43 - 7.36 (m, 2H), 7.36 - 7.26 (m, 2H), 4.90 - 4.80 (m, 2H), 4.38 (ddd, *J* = 13.2, 3.0, 1.8 Hz, 1H), 4.16 - 4.05 (m, 2H), 3.86 - 3.82 (m, 1H), 3.73 - 3.70 (m, 2H), 3.69 - 3.64 (m, 1H), 2.99 - 2.92 (m, 2H), 2.85 - 2.78 (m, 1H), 2.66 (td, *J* = 12.7, 3.3 Hz, 1H), 2.15 (t, *J* = 11.2 Hz, 1H), 2.07 - 1.97 (m, 1H), 1.35 (t, *J* = 7.5 Hz, 3H).

¹³C NMR (126 MHz, CDCl₃) δ 163.23, 161.46, 148.25, 139.29, 138.05, 133.98, 132.78, 132.46, 128.53, 127.80, 125.93, 125.78, 125.14, 124.69, 124.64, 124.23, 123.31, 122.39, 121.35, 60.94, 57.38, 56.97, 51.36, 47.65, 45.47, 41.25, 39.39, 21.97, 16.18.

Mass 484.4

### 50. Preparation Example 50: Formula N701

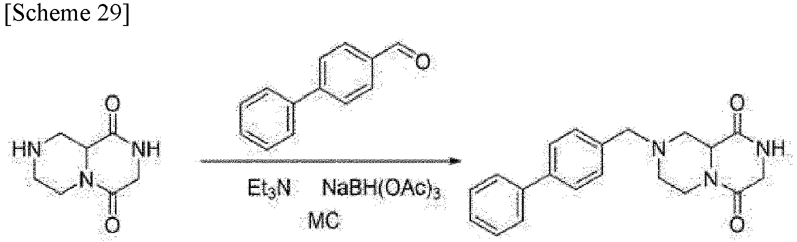

0.3 g (1.06 mmol) of tetrahydro-2*H*-pyrazino[1,2-*a*]pyrazine-1,4(3*H*,6*H*)-dione trifluoroacetate, 3 mL of dichloromethane, 0.3 mL of triethylamine, and 0.193 g (1.06 mmol) of biphenyl-4-carboxaldehyde were input at room temperature. 0.449 g (2.12 mmol) of sodium triacetoxyborohydride was added, and the resulting mixture was stirred at room temperature for 3 hours. The reaction solution was washed with 24 mL of distilled water. The reaction solution was washed with 24 mL of a sodium bicarbonate aqueous solution and then with 24 mL of distilled water. An organic layer was dried over anhydrous magnesium sulfate and filtered. The reaction solution was concentrated and purified by silica chromatography, thereby obtaining 2-([1,1'-biphenyl]-4-ylmethyl)hexahydro-2*H*-pyrazino[1,2-*a*]pyrazine-6,9-dione (0.249g, yield: 70%).

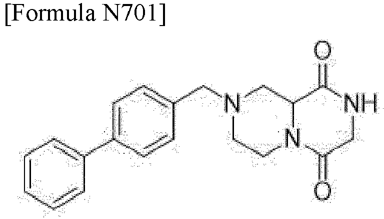

### 2-([1,1'-biphenyl]-4-ylmethyl)hexahydro-2H-pyrazino[1,2-a]pyrazine-6,9-dione

¹H NMR (400 MHz, CDCl₃) δ 7.64 - 7.52 (m, 4H), 7.50 - 7.41 (m, 2H), 7.41 - 7.31 (m, 3H), 7.15 (s, 1H), 4.59 - 4.47 (m, 1H), 4.18 - 4.08 (m, 1H), 4.07 - 3.99 (m, 2H), 3.63 (dd, *J* = 39.7, 13.2 Hz, 2H), 3.50 - 3.42 (m, 1H), 2.98 - 2.72 (m, 2H), 2.21 - 2.00 (m, 2H).

¹³C NMR (101 MHz, CDCl₃) δ 165.95, 161.71, 140.78, 140.42, 135.98, 129.50, 128.77, 127.28, 127.19, 127.06, 62.23, 56.88, 56.08, 51.48, 44.57, 41.48.

Mass 336.2

### 51. Preparation Example 51: Formula N711

A compound of Formula N711 was obtained by further performing the step of Scheme 30 below using the compound of Formula N701 obtained in Preparation Example 50 as a starting material.

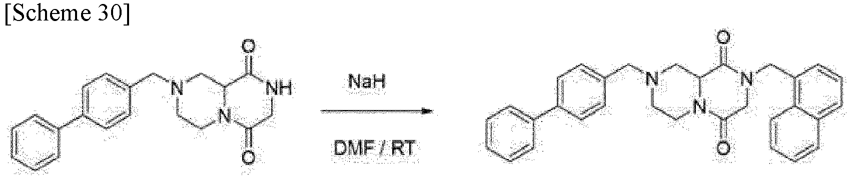

0.2 g (0.60 mmol) of 2-([1,1'-biphenyl]-4-ylmethyl)hexahydro-2*H*-pyrazino[1,2-*a*]pyrazine-6,9-dione, 0.036 g (0.89 mmol) of sodium hydride, and dimethylformamide were input and stirred for 1 hour, and then 0.158 g (0.72 mmol) of 1-(bromomethyl)naphthalene was added, followed by stirring at room temperature overnight. The reaction was terminated with 10 mL of an ammonium chloride aqueous solution and 10 mL of ethyl acetate. An organic layer was extracted and washed with 10 mL of an aqueous sodium chloride solution. An organic layer was dried over anhydrous magnesium sulfate and filtered. The reaction solution was concentrated and purified by silica chromatography, thereby obtaining 2-([1,1'-biphenyl]-4-ylmethyl)hexahydro-2*H*-pyrazino[1,2-*a*]pyrazine-6,9-dione (0.233g, yield: 81%).

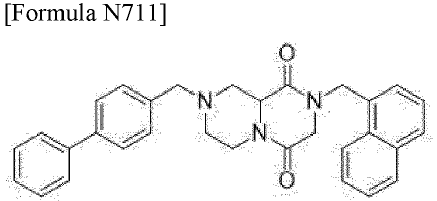

### 2-([1,1'-biphenyl]-4-ylmethyl)-8-(naphthalen-1-ylmethyl)hexahydro-2H-pyrazino[1,2-a]pyrazine-6,9-dione

¹H NMR (400 MHz, CDCl₃) δ 8.06 - 8.02 (m, 1H), 7.92 - 7.81 (m, 2H), 7.63 - 7.48 (m, 6H), 7.48 - 7.30 (m, 7H), 5.13 - 5.02 (m, 2H), 4.46 - 4.40 (m, 1H), 4.22 - 4.16 (m, 1H), 3.77 - 3.51 (m, 5H), 2.89 - 2.70 (m, 2H), 2.16 - 1.95 (m, 2H).

¹³C NMR (126 MHz, CDCl₃) δ 163.04, 161.54, 140.85, 140.43, 136.08, 133.97, 131.49, 130.07, 129.49, 128.88, 128.75, 128.56, 127.24, 127.19, 127.07, 126.91, 126.25, 125.14, 123.58, 62.21, 57.39, 56.64, 51.27, 48.15, 47.22, 41.19.

Mass 476.4

### 52. Preparation Example 52: Formula N712

A compound of Formula N712 was obtained by further performing the step of Scheme 31 below using the compound of Formula N701 obtained in Preparation Example 50 as a starting material.

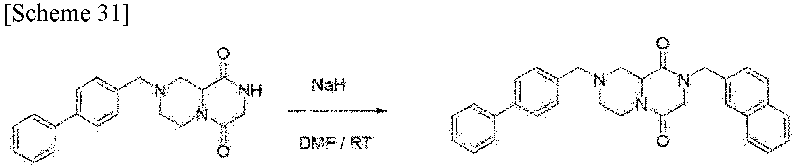

0.2 g (0.60 mmol) of 2-([1,1'-biphenyl]-4-ylmethyl)hexahydro-2H-pyrazino[1,2-*a*]pyrazine-6,9-dione, 0.036 g (0.89 mmol) of sodium hydride, and dimethylformamide were input and stirred for 1 hour, and 0.158 g (0.72 mmol) of 2-(bromomethyl)naphthalene was added thereto, followed by stirring at room temperature overnight. The reaction was terminated with 10 mL of an ammonium chloride aqueous solution and 10 mL of ethyl acetate. An organic layer was extracted and washed with 10 mL of a sodium chloride aqueous solution. An organic layer was dried over anhydrous magnesium sulfate and filtered. The reaction solution was concentrated and purified by silica chromatography, thereby obtaining 2-([1,1'-biphenyl]-4-ylmethyl)-8-(naphthalen-2-ylmethyl)hexahydro-2*H*-pyrazino[1,2-*a*]pyrazine-6,9-dione (0.250g, yield: 87%).

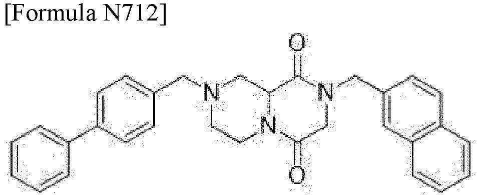

### 2-([1,1'-biphenyl]-4-ylmethyl)-8-(naphthalen-2-ylmethyl)hexahydro-2H-pyrazino[1,2-a]pyrazine-6,9-dione

¹H NMR (400 MHz, CDCl₃) δ 7.87 - 7.78 (m, 3H), 7.70 (s, 1H), 7.63 - 7.54 (m, 4H), 7.53 - 7.31 (m, 8H), 4.79 - 4.67 (m, 2H), 4.53 - 4.43 (m, 1H), 4.24 - 4.14 (m, 1H), 3.90 (s, 2H), 3.76 - 3.52 (m, 3H), 2.96 - 2.73 (m, 2H), 2.20 - 1.98 (m, 2H).

¹³C NMR (126 MHz, CDCl₃) δ 163.62, 161.72, 140.96, 140.54, 136.18, 133.31, 133.12, 132.42, 129.61, 129.11, 128.86, 127.90, 127.85, 127.82, 127.35, 127.30, 127.18, 126.63, 126.45, 126.12, 62.33, 57.46, 56.73, 51.46, 49.62, 48.67, 41.38.

Mass 476.23

### 53. Preparation Example 53: Formula N713

A compound of Formula N713 was obtained by further performing the step of Scheme 32 below using the compound of Formula N701 obtained in Preparation Example 50 as a starting material.

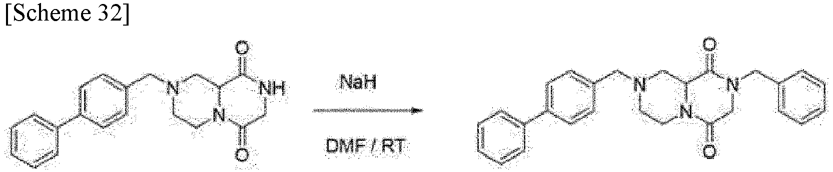

0.2 g (0.60 mmol) of 2-([1,1'-biphenyl]-4-ylmethyl)hexahydro-2H-pyrazino[1,2-*a*]pyrazine-6,9-dione, 0.036 g (0.89 mmol) of sodium hydride, and dimethylformamide were input and stirred for 1 hour, 0.08 mL (0.72 mmol) of benzyl bromide was added thereto, followed by stirring at room temperature overnight. The reaction was terminated with 10 mL of an ammonium chloride aqueous solution and 10 mL of ethyl acetate. An organic layer was extracted and washed with 10 mL of a sodium chloride aqueous solution. An organic layer was dried over anhydrous magnesium sulfate and filtered. The reaction solution was concentrated and purified by silica chromatography, thereby obtaining 2-([1,1'-biphenyl]-4-ylmethyl)-8-benzylhexahydro-2*H*-pyrazino[1,2-*a*]pyrazine-6,9-dione (0.228g, yield: 89%).

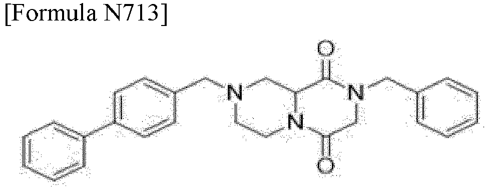

### 2-([1,1'-biphenyl]-4-ylmethyl)-8-benzylhexahydro-2H-pyrazino[1,2-a]pyrazine-6,9-dione

¹H NMR (400 MHz, CDCl₃) δ 7.65 - 7.53 (m, 4H), 7.48 - 7.30 (m, 8H), 7.28 -7.24 (m, 2H), 4.64 - 4.45 (m, 3H), 4.22 - 4.13 (m, 1H), 3.87 (s, 2H), 3.74 - 3.54 (m, 3H), 2.93 - 2.86 (m, 2H), 2.17 - 2.02 (m, 2H).

¹³C NMR (126 MHz, CDCl₃) δ 163.52, 161.76, 140.94, 140.52, 136.15, 135.00, 129.60, 129.04, 128.84, 128.61, 128.30, 127.33, 127.28, 127.16, 62.31, 57.41, 56.69, 51.44, 49.41, 48.63, 41.37.

Mass 426.21

### 54. Preparation Example 54: Formula N714

A compound of Formula N714 was obtained by further performing the step of Scheme 33 below using the compound of Formula N701 obtained in Preparation Example 50 as a starting material.

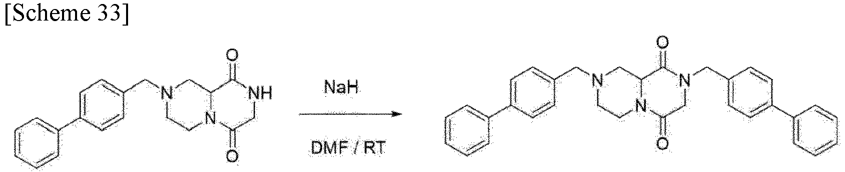

0.2 g (0.60 mmol) of 2-([1,1'-biphenyl]-4-ylmethyl)hexahydro-2H-pyrazino[1,2-*a*]pyrazine-6,9-dione, 0.036 g (0.89 mmol) of sodium hydride, and dimethylformamide were input and stirred for 1 hour, and 0.177m g (0.72 mmol) of 4-(bromomethyl)biphenyl was added thereto, followed by stirring at room temperature overnight. The reaction was terminated with 10 mL of an ammonium chloride aqueous solution and 10 mL of ethyl acetate. An organic layer was extracted and washed with 10 mL of a sodium chloride aqueous solution. An organic layer was dried over anhydrous magnesium sulfate and filtered. The reaction solution was concentrated and purified by silica chromatography, thereby obtaining 2,8-bis([1,1'-biphenyl]-4-ylmethyl)hexahydro-2*H*-pyrazino[1,2-*a*]pyrazine-6,9-dione (0.261g, yield: 86%).

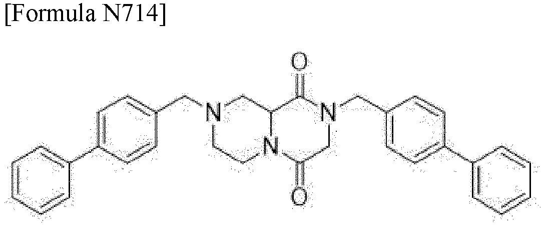

### 2,8-bis([1,1'-biphenyl]-4-ylmethyl)hexahydro-2H-pyrazino[1,2-a]pyrazine-6,9-dione

¹H NMR (400 MHz, CDCl₃) δ 7.63 - 7.52 (m, 8H), 7.48 - 7.30 (m, 10H), 4.68 - 4.55 (m, 2H), 4.54 - 4.46 (m, 1H), 4.22 - 4.16 (m, 1H), 3.92 (s, 2H), 3.73 - 3.55 (m, 3H), 2.93 - 2.75 (m, 2H), 2.21 - 2.01 (m, 2H).

¹³C NMR (126 MHz, CDCl₃) δ 163.54, 161.72, 141.28, 140.92, 140.53, 140.49, 136.16, 133.98, 129.58, 129.08, 128.90, 128.83, 127.75, 127.57, 127.32, 127.26, 127.15, 62.29, 57.41, 56.68, 51.43, 49.12, 48.68, 41.36.

Mass 502.4

### 55. Preparation Example 55: Formula N722

A compound of Formula N722 was obtained by further performing the step of Scheme 34 below using the compound of Formula N701 obtained in Preparation Example 50 as a starting material.

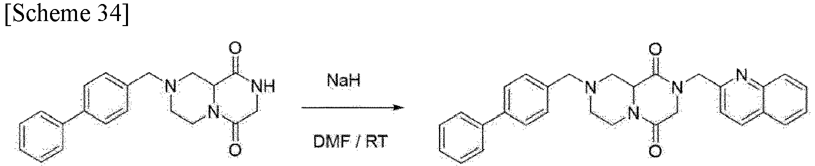

0.25 g (0.75 mmol) of 2-([1,1'-biphenyl]-4-ylmethyl)hexahydro-2*H*-pyrazino[1,2-*a*]pyrazine-6,9-dione, 0.097 g (2.42 mmol) of sodium hydride, and dimethylformamide were input and stirred for 1 hour, and 0.208m g (0.97 mmol) of 2-(chloromethyl)quinoline hydrochloride was added thereto, followed by stirring at room temperature overnight. The reaction was terminated with 10 mL of an ammonium chloride aqueous solution and 10 mL of ethyl acetate. An organic layer was extracted and washed with 10 mL of a sodium chloride aqueous solution. An organic layer was dried over anhydrous magnesium sulfate and filtered. The reaction solution was concentrated and purified by silica chromatography, thereby obtaining 2-([1,1'-biphenyl]-4-ylmethyl)-8-(quinolin-2-ylmethyl)hexahydro-2*H*-pyrazino[1,2-*a*]pyrazine-6,9-dione (0.301g, yield: 84%).

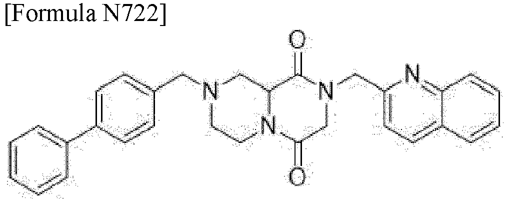

### 2-([1,1'-biphenyl]-4-ylmethyl)-8-(quinolin-2-ylmethyl)hexahydro-2H-pyrazino[1,2-a]pyrazine-6,9-dione

¹H NMR (400 MHz, CDCl₃) δ 8.15 (d, *J* = 8.5 Hz, 1H), 8.02 (d, *J* = 8.5 Hz, 1H), 7.80 (d, *J* = 8.1 Hz, 1H), 7.75 - 7.69 (m, 1H), 7.62 - 7.51 (m, 5H), 7.47 - 7.32 (m, 6H), 4.86 (s, 2H), 4.58 - 4.47 (m, 1H), 4.28 - 4.12 (m, 3H), 3.72 - 3.53 (m, 3H), 2.97 - 2.77 (m, 2H), 2.23 - 2.04 (m, 2H).

¹³C NMR (126 MHz, CDCl₃) δ 163.95, 161.97, 155.37, 147.78, 140.96, 140.52, 137.37, 136.17, 129.91, 129.61, 129.34, 128.85, 127.67, 127.53, 127.34, 127.28, 127.17, 126.79, 120.20, 62.33, 57.42, 56.68, 51.67, 51.50, 49.97, 41.43.

Mass 477.4

### 56. Preparation Example 56: Formula N723

A compound of Formula N723 was obtained by further performing the step of Scheme 35 below using the compound of Formula N701 obtained in Preparation Example 50 as a starting material.

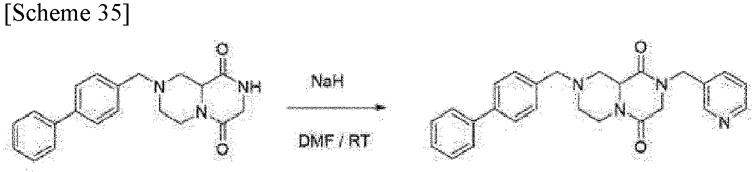

0.25 g (0.75 mmol) of 2-([1,1'-biphenyl]-4-ylmethyl)hexahydro-2*H*-pyrazino[1,2-*a*]pyrazine-6,9-dione, 0.097 g (2.42 mmol) of sodium hydride, and dimethylformamide were input and stirred for 1 hour, and 0.159 mg (0.97 mmol) of 3-(chloromethyl)pyridine hydrochloride was added thereto, followed by stirring at room temperature overnight. The reaction was terminated with 10 mL of an ammonium chloride aqueous solution and 10 mL of ethyl acetate. An organic layer was extracted and washed with 10 mL of a sodium chloride aqueous solution. An organic layer was dried over anhydrous magnesium sulfate and filtered. The reaction solution was concentrated and purified by silica chromatography, thereby obtaining 2-([1,1'-biphenyl]-4-ylmethyl)-8-(pyridin-3-ylmethyl)hexahydro-2*H*-pyrazino[1,2-*a*]pyrazine-6,9-dione (0.161g, yield: 50%).

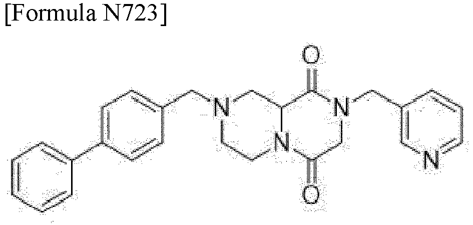

### 2-([1,1'-biphenyl]-4-ylmethyl)-8-(pyridin-3-ylmethyl)hexahydro-2H-pyrazino[1,2-a]pyrazine-6,9-dione

¹H NMR (500 MHz, CDCl₃) δ 8.58 (dd, *J* = 4.8, 1.6 Hz, 1H), 8.55 (d, *J* = 1.7 Hz, 1H), 7.64 - 7.54 (m, 5H), 7.47 - 7.41 (m, 2H), 7.41 - 7.32 (m, 3H), 7.31 - 7.27 (m, 1H), 4.62 - 4.54 (m, 2H), 4.50 (ddd, *J* = 13.2, 3.1, 1.8 Hz, 1H), 4.23 - 4.15 (m, 1H), 3.96 - 3.85 (m, 2H), 3.73 - 3.53 (m, 3H), 2.93 - 2.87 (m, 1H), 2.81 (td, *J* = 12.7, 3.4 Hz, 1H), 2.16 - 2.03 (m, 2H).

¹³C NMR (126 MHz, CDCl₃) δ 163.74, 161.25, 149.76, 140.84, 140.49, 136.21, 135.99, 130.79, 129.51, 128.77, 127.27, 127.22, 127.08, 123.85, 62.23, 57.28, 56.55, 51.34, 48.75, 47.04, 41.37.

Mass 427.2

### 57. Preparation Example 57: Formula N724

A compound of Formula N724 was obtained by further performing the step of Scheme 36 below using the compound of Formula N701 obtained in Preparation Example 50 as a starting material.

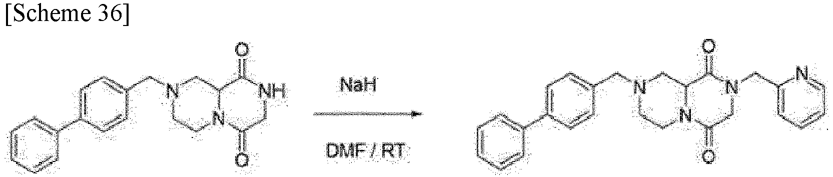

0.25 g (0.75 mmol) of 2-([1,1'-biphenyl]-4-ylmethyl)hexahydro-2*H*-pyrazino[1,2-*a*]pyrazine-6,9-dione, 0.097 g (2.42 mmol) of sodium hydride, and dimethylformamide were input and stirred for 1 hour, and 0.245m g (0.97 mmol) of 2-(bromomethyl)pyridine hydrobromide was added thereto, followed by stirring at room temperature overnight. The reaction was terminated with 10 mL of an ammonium chloride aqueous solution and 10 mL of ethyl acetate. An organic layer was extracted and washed with 10 mL of a sodium chloride aqueous solution. An organic layer was dried over anhydrous magnesium sulfate and filtered. The reaction solution was concentrated and purified by silica chromatography, thereby obtaining 2-([1,1'-biphenyl]-4-ylmethyl)-8-(pyridin-2-ylmethyl)hexahydro-2*H*-pyrazino[1,2-*a*]pyrazine-6,9-dione (0.172g, yield: 53%).

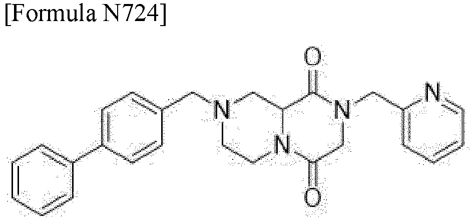

### 2-([1,1'-biphenyl]-4-ylmethyl)-8-(pyridin-2-ylmethyl)hexahydro-2H-pyrazino[1,2-a]pyrazine-6,9-dione

¹H NMR (500 MHz, CDCl₃) δ 8.54 (ddd, *J* = 4.9, 1.7, 0.9 Hz, 1H), 7.68 - 7.63 (m, 1H), 7.60 - 7.53 (m, 4H), 7.46 - 7.41 (m, 2H), 7.40 - 7.31 (m, 3H), 7.28 - 7.25 (m, 1H), 7.20 (ddd, *J* = 7.6, 4.9, 1.1 Hz, 1H), 4.67 (dd, *J* = 50.2, 14.9 Hz, 2H), 4.52 (ddd, *J* = 13.1, 3.1, 1.8 Hz, 1H), 4.21 - 4.05 (m, 3H), 3.72 - 3.51 (m, 3H), 2.93 - 2.86 (m, 1H), 2.82 (td, *J* = 12.6, 3.3 Hz, 1H), 2.17 - 2.05 (m, 2H).

¹³C NMR (126 MHz, CDCl₃) δ 163.73, 161.83, 155.12, 149.61, 140.86, 140.41, 136.97, 136.09, 129.50, 128.74, 127.23, 127.17, 127.06, 122.83, 122.56, 77.32, 77.06, 76.81, 62.22, 57.25, 56.51, 51.38, 51.04, 49.81, 41.30, 0.00.

Mass 427.2

### 58. Preparation Example 58: Formula S2115

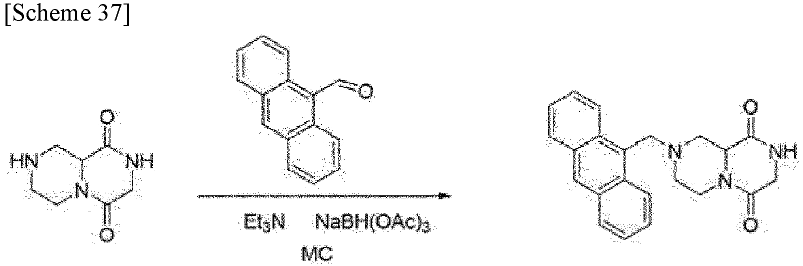

0.5 g (1.77 mmol) of tetrahydro-2*H*-pyrazino[1,2-*a*]pyrazine-1,4(3*H*,6*H*)-dione trifluoroacetate, 5 mL of dichloromethane, 0.5 mL of triethylamine, and 0.36 g (1.77 mmol) of 9-Anthracenecarboxaldehyde were input at room temperature. 0.748 g (3.53 mmol) of sodium triacetoxyborohydride was added, and stirred at room temperature for 1.5 hours. The reaction solution was washed with 10 mL of distilled water. The reaction solution was washed with a sodium bicarbonate aqueous solution and 10 mL of distilled water in this order. An organic layer was dried over anhydrous magnesium sulfate and filtered. The reaction solution was concentrated and purified by silica chromatography, thereby obtaining 2-(anthracen-9-ylmethyl)hexahydro-2*H*-pyrazino[1,2-*a*]pyrazine-6,9-dione (0.497g, yield: 78%).

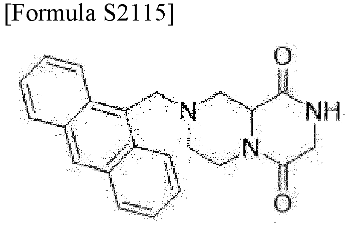

### 2-(anthracen-9-ylmethyl)hexahydro-2H-pyrazino[1,2-a]pyrazine-6,9-dione

¹H NMR (500 MHz, CDCl₃) δ 8.44 (s, 1H), 8.40 (d, *J* = 8.7 Hz, 2H), 8.01 (d, *J* = 8.2 Hz, 2H), 7.58 - 7.41 (m, 4H), 6.65 (s, 1H), 4.62 - 4.39 (m, 3H), 4.13 - 3.97 (m, 3H), 3.62 - 3.54 (m, 1H), 2.85 - 2.75 (m, 1H), 2.64 (td, *J* = 12.8, 3.2 Hz, 1H), 2.43 (t, *J* = 11.2 Hz, 1H), 2.32 (td, *J* = 11.8, 3.1 Hz, 1H).

¹³C NMR (126 MHz, CDCl₃) δ 165.78, 161.49, 131.40, 129.13, 128.05, 127.97, 125.97, 124.93, 124.62, 57.17, 56.58, 53.85, 51.36, 44.63, 41.65.

Mass 360.17

### 59. Preparation Example 59: Formula S2120

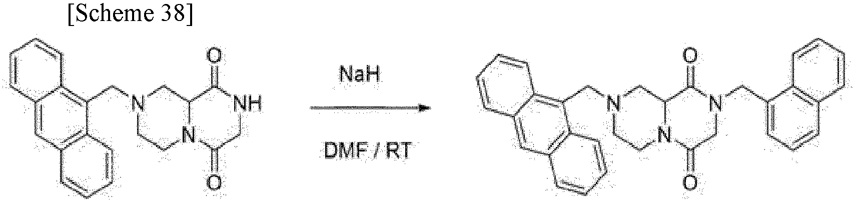

0.1 g (0.25 mmol) of 2-(anthracen-9-ylmethyl)hexahydro-2H-pyrazino[1,2-a]pyrazine-6,9-dione, 0.015 g (0.38 mmol) of sodium hydride, and dimethylformamide were input and stirred at room temperature for 1 hour, and 0.067 g (0.30 mmol) of 1-(bromomethyl)naphthalene was added, followed by stirring at room temperature overnight. 10 mL of an ammonium chloride aqueous solution and 20 mL of ethyl acetate were added and stirred, and an organic layer was extracted and washed with 10 mL of a sodium chloride aqueous solution. An organic layer was dried over anhydrous magnesium sulfate and filtered. The reaction solution was concentrated and purified by silica chromatography, thereby obtaining 2-(anthracen-9-ylmethyl)-8-(naphthalen-1-ylmethyl)hexahydro-2H-pyrazino[1,2-a]pyrazine-6,9-dione (0.118g, yield: 93%).

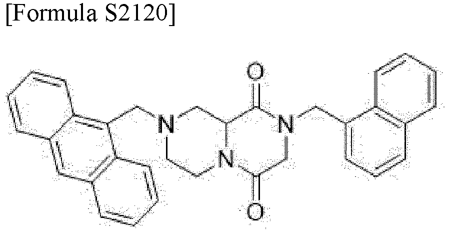

### 2-(anthracen-9-ylmethyl)-8-(naphthalen-1-ylmethyl)hexahydro-2H-pyrazino[1,2-a]pyrazine-6,9-dione

¹H NMR (400 MHz, CDCl₃) δ 8.50 - 8.37 (m, 3H), 8.06 - 7.99 (m, 3H), 7.93 - 7.82 (m, 2H), 7.59 - 7.36 (m, 8H), 5.07 (dd, *J* = 40.3, 14.4 Hz, 2H), 4.53 (dd, *J* = 40.2, 13.0 Hz, 2H), 4.38 - 4.28 (m, 1H), 4.19 - 4.13 (m, 1H), 3.83 - 3.63 (m, 3H), 2.83 - 2.70 (m, 1H), 2.58 (td, *J* = 12.7, 3.2 Hz, 1H), 2.40 (t, *J* = 11.2 Hz, 1H), 2.24 (td, *J* = 11.7, 3.2 Hz, 1H).

¹³C NMR (126 MHz, CDCl₃) δ 163.20, 161.60, 134.14, 131.64, 131.55, 130.26, 129.66, 129.28, 129.05, 128.78, 128.18, 127.10, 126.42, 126.09, 125.29, 125.07, 124.80, 123.77, 57.82, 57.22, 53.98, 51.30, 48.30, 47.44, 41.48.

Mass 500.23

### 60. Preparation Example 60: Formula S2121

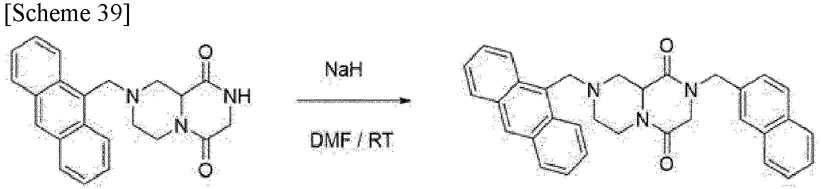

0.1 g (0.25 mmol) of 2-(anthracen-9-ylmethyl)hexahydro-2*H*-pyrazino[1,2-*a*]pyrazine-6,9-dione, 0.015 g (0.38 mmol) of sodium hydride, and dimethylformamide were input and stirred at room temperature for 1 hour, and 0.067 g (0.30 mmol) of 2-(bromomethyl)naphthalene was added, followed by stirring at room temperature overnight. 10 mL of an ammonium chloride aqueous solution and 20 mL of ethyl acetate were added and stirred, and an organic layer was extracted and washed with 10 mL of a sodium chloride aqueous solution. An organic layer was dried over anhydrous magnesium sulfate and filtered. The reaction solution was concentrated and purified by silica chromatography, thereby obtaining 2-(anthracen-9-ylmethyl)-8-(naphthalen-2-ylmethyl)hexahydro-2*H*-pyrazino[1,2-*a*]pyrazine-6,9-dione (0.088g, yield: 69%).

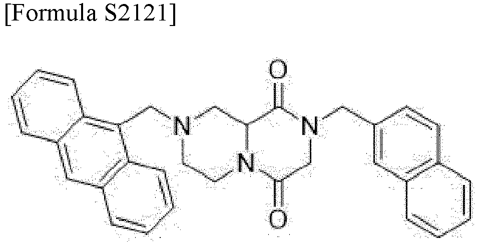

### 2-(anthracen-9-ylmethyl)-8-(naphthalen-2-ylmethyl)hexahydro-2H-pyrazino[1,2-a]pyrazine-6,9-dione

¹H NMR (400 MHz, CDCl₃) δ 8.48 - 8.39 (m, 3H), 8.07 - 7.98 (m, 2H), 7.88 - 7.77 (m, 3H), 7.71 (s, 1H), 7.58 - 7.45 (m, 6H), 7.37 (dd, *J* = 8.4, 1.6 Hz, 1H), 4.74 (dd, *J* = 40.8, 14.3 Hz, 2H), 4.55 (dd, *J* = 43.0, 13.0 Hz, 2H), 4.41 - 4.32 (m, 1H), 4.21 - 4.15 (m, 1H), 3.91 (s, 2H), 3.79 - 3.72 (m, 1H), 2.79 - 2.72 (m, 1H), 2.61 (td, *J* = 12.7, 3.2 Hz, 1H), 2.46 (t, *J* = 11.2 Hz, 1H), 2.28 (td, *J* = 11.7, 3.2 Hz, 1H).

¹³C NMR (126 MHz, CDCl₃) δ 163.71, 161.70, 133.34, 133.15, 132.44, 131.51, 129.24, 129.15, 128.18, 128.14, 127.90, 127.89, 127.85, 126.66, 126.48, 126.12, 126.07, 125.05, 124.78, 57.70, 57.26, 53.93, 51.19, 49.63, 48.68, 41.53.

Mass 500.4

### 61. Preparation Example 61: Formula S2124

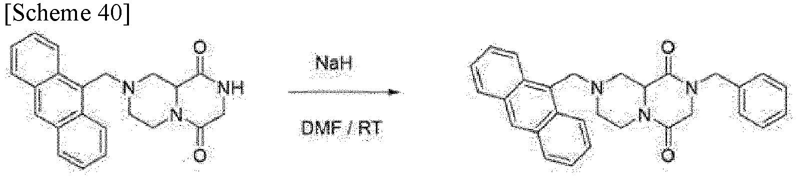

0.1 g (0.25 mmol) of 2-(anthracen-9-ylmethyl)hexahydro-2*H*-pyrazino[1,2-*a*]pyrazine-6,9-dione, 0.015 g (0.38 mmol) of sodium hydride, and dimethylformamide were input and stirred at room temperature for 1 hour, and 0.036mL (0.30 mmol) of benzyl bromide was added thereto, followed by stirring at room temperature overnight. 10 mL of an ammonium chloride aqueous solution and 20 mL of ethyl acetate were added and stirred, and an organic layer was extracted and washed with 10 mL of a sodium chloride aqueous solution. An organic layer was dried over anhydrous magnesium sulfate and filtered. The reaction solution was concentrated and purified by silica chromatography, thereby obtaining 2-(anthracen-9-ylmethyl)-8-benzylhexahydro-2*H*-pyrazino[1,2-*a*]pyrazine-6,9-dione (0.1g, yield: 87%).

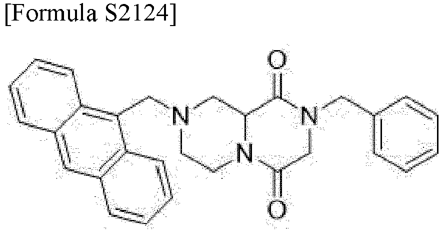

### 2-(anthracen-9-ylmethyl)-8-benzylhexahydro-2H-pyrazino[1,2-a]pyrazine-6,9-dione

¹H NMR (400 MHz, CDCl₃) δ 8.49 - 8.38 (m, 3H), 8.06 - 7.98 (m, 2H), 7.57 - 7.44 (m, 4H), 7.39 - 7.23 (m, 6H), 4.65 - 4.46 (m, 4H), 4.40 - 4.35 (m, 1H), 4.20 - 4.12 (m, 1H), 3.87 (s, 2H), 3.76 - 3.70 (m, 1H), 2.80 - 2.71 (m, 1H), 2.61 (td, *J* = 12.7, 3.2 Hz, 1H), 2.44 (t, *J* = 11.2 Hz, 1H), 2.28 (td, *J* = 11.7, 3.1 Hz, 1H).

¹³C NMR (126 MHz, CDCl₃) δ 163.48, 161.61, 134.91, 134.09, 131.38, 131.37, 129.10, 128.96, 128.49, 128.21, 128.04, 128.01, 125.93, 124.91, 124.64, 57.52, 57.12, 53.78, 51.04, 49.30, 48.51, 41.40.

Mass 450.2

### 62. Preparation Example 62: Formula S2125

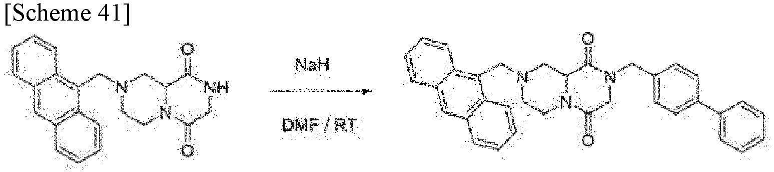

0.1 g (0.25 mmol) of 2-(anthracen-9-ylmethyl)hexahydro-2*H*-pyrazino[1,2-*a*]pyrazine-6,9-dione, 0.015 g (0.38 mmol) of sodium hydride, and dimethylformamide were input and stirred at room temperature for 1 hour, 0.075 mg (0.30 mmol) of 4-bromomethyl biphenyl was added thereto, followed by stirring at room temperature overnight. 10 mL of an ammonium chloride aqueous solution and 20 mL of ethyl acetate were added and stirred, and an organic layer was extracted and washed with 10 mL of a sodium chloride aqueous solution. An organic layer was dried over anhydrous magnesium sulfate and filtered. The reaction solution was concentrated and purified by silica chromatography, thereby obtaining 8-([1,1'-biphenyl]-4-ylmethyl)-2-(anthracen-9-ylmethyl)hexahydro-2*H*-pyrazino[1,2-*a*]pyrazine-6,9-dione (0.096g, yield: 72%).

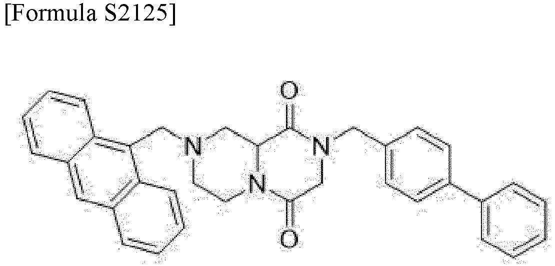

### 8-([1,1'-biphenyl]-4-ylmethyl)-2-(anthracen-9-ylmethyl)hexahydro-2H-pyrazino[1,2-a]pyrazine-6,9-dione

¹H NMR (400 MHz, CDCl₃) δ 8.50 - 8.36 (m, 3H), 8.07 - 7.98 (m, 2H), 7.62 - 7.41 (m, 10H), 7.39 - 7.29 (m, 3H), 4.70 - 4.46 (m, 4H), 4.42 - 4.34 (m, 1H), 4.20 - 4.13 (m, 1H), 3.92 (s, 2H), 3.78 - 3.70 (m, 1H), 2.76 (d, *J* = 11.5 Hz, 1H), 2.61 (td, *J* = 12.7, 3.2 Hz, 1H), 2.46 (t, *J* = 11.2 Hz, 1H), 2.29 (td, *J* = 11.7, 3.1 Hz, 1H).

¹³C NMR (126 MHz, CDCl₃) δ 149.41, 147.48, 127.13, 126.36, 119.76, 117.26, 114.99, 114.86, 114.71, 113.92, 113.88, 113.57, 113.39, 112.97, 111.82, 110.80, 110.53, 43.44, 43.02, 39.68, 36.93, 34.93, 34.48, 27.30.

Mass 526.4

### 63. Preparation Example 63: Formula S2139

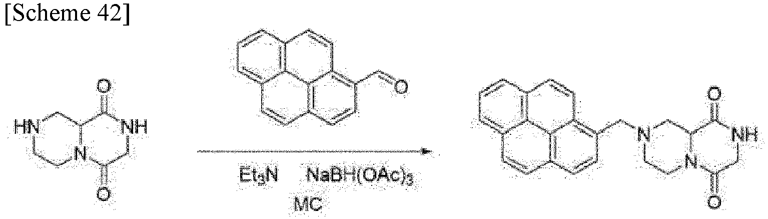

1.5 g (5.21 mmol) of tetrahydro-2H-pyrazino[1,2-a]pyrazine-1,4(3H,6*H*)-dione trifluoroacetate, 1.5 mL of dichloromethane, 1.5 mL of triethylamine, and 1.2 g (5.21 mmol) of 1-pyrenecarboxaldehyde were input at room temperature. 2.2 g (10.4 mmol) of sodium triacetoxyborohydride was added, and stirred at room temperature overnight. The reaction solution was washed with 10 mL of distilled water. The reaction solution was washed with a sodium bicarbonate aqueous solution and 10 mL of distilled water in this order. An organic layer was dried over anhydrous magnesium sulfate and filtered. The reaction solution was concentrated. 2 mL of dichloromethane was added to the concentrated residue, and the reaction solution was dissolved. 6 mL of n-heptane was slowly added dropwise to generate crystals, and the reaction product was filtered. The solid was dried, thereby obtaining 2-(pyren-1-ylmethyl)hexahydro-2*H*-pyrazino[1,2-a]pyrazine-6,9-dione.

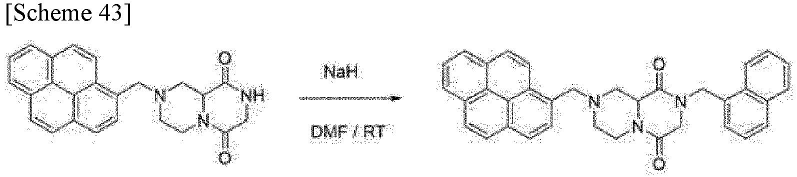

0.25 g (0.65 mmol) of the 2-(pyren-1-ylmethyl)hexahydro-2*H*-pyrazino[1,2-a]pyrazine-6,9-dione, 0.039 g (0.97 mmol) of sodium hydride, and dimethylformamide were input and stirred at room temperature for 30 minutes, and 0.172 g (0.78 mmol) of 1-(bromomethyl)naphthalene was added thereto, followed by stirring at room temperature overnight. 10 mL of an ammonium chloride aqueous solution and 20 mL of ethyl acetate were added and stirred, and an organic layer was extracted and washed with 10 mL of a sodium chloride aqueous solution. An organic layer was dried over anhydrous magnesium sulfate and filtered. The reaction solution was concentrated and purified by silica chromatography, thereby obtaining 8-(naphthalen-1-yl)-2-(pyren-1-ylmethyl)hexahydro-2*H*-pyrazino[1,2-a]pyrazine-6,9-dione (0.344g, yield: 99%).

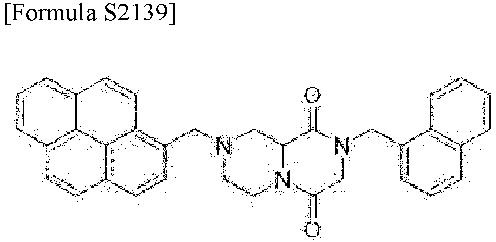

### 8-(naphthalen-1-yl)-2-(pyren-1-ylmethyl)hexahydro-2H-pyrazino[1,2-a]pyrazine-6,9-dione

¹H NMR (400 MHz, CDCl₃) δ 8.49 (d, *J* = 9.2 Hz, 1H), 8.24 - 8.10 (m, 4H), 8.09 - 7.98 (m, 4H), 7.94 - 7.82 (m, 3H), 7.57 - 7.49 (m, 2H), 7.45 - 7.34 (m, 2H), 5.13 - 4.99 (m, 2H), 4.41 - 4.34 (m, 2H), 4.23 - 4.16 (m, 1H), 4.10 (d, *J* = 12.8 Hz, 1H), 3.80 - 3.69 (m, 3H), 2.85 - 2.77 (m, 1H), 2.66 (td, *J* = 12.7, 3.1 Hz, 1H), 2.27 (t, *J* = 11.2 Hz, 1H), 2.09 (td, *J* = 11.8, 3.1 Hz, 1H).

¹³C NMR (126 MHz, CDCl₃) δ 163.14, 161.63, 134.08, 131.60, 131.37, 131.25, 130.92, 130.58, 130.20, 130.12, 129.58, 128.99, 128.65, 128.40, 127.49, 127.03, 126.36, 126.08, 125.28, 125.24, 125.21, 124.81, 124.52, 124.06, 123.70, 60.96, 57.55, 57.15, 51.37, 48.26, 47.33, 41.33.

Mass 524.4

### 64. Preparation Example 64: Formula S2140

A compound of Formula N2140 was obtained by further performing the step of Scheme 44 below using the compound obtained through Scheme 42 in Preparation Example 63 as a starting material.

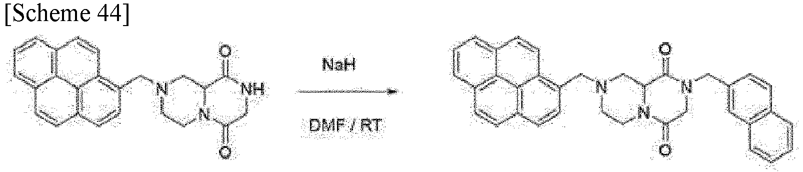

0.25 g (0.65 mmol) of 2-(pyren-1-ylmethyl)hexahydro-2*H*-pyrazino[1,2-a]pyrazine-6,9-dione, 0.039 g (0.97 mmol) of sodium hydride, and dimethylformamide were input and stirred at room temperature for 30 minutes, and 0.172 g (0.78 mmol) of 2-(bromomethyl)naphthalene was added, followed by stirred at room temperature overnight. 10 mL of an ammonium chloride aqueous solution and 20 mL of ethyl acetate were added and stirred, and an organic layer was extracted and washed with 10 mL of a sodium chloride aqueous solution. An organic layer was dried over anhydrous magnesium sulfate and filtered. The reaction solution was concentrated and purified by silica chromatography, thereby obtaining 8-(naphthalen-2-yl)-2-(pyren-1-ylmethyl)hexahydro-2*H*-pyrazino[1,2-a]pyrazine-6,9-dione (0.321g, yield: 94%).

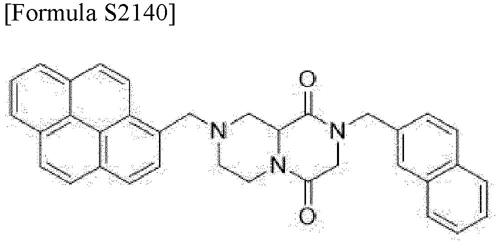

### 8-(naphthalen-2-yl)-2-(pyren-1-ylmethyl)hexahydro-2H-pyrazino[1,2-a]pyrazine-6,9-dione

¹H NMR (400 MHz, CDCl₃) δ 8.49 (d, *J* = 9.2 Hz, 1H), 8.24 - 8.10 (m, 4H), 8.08 - 7.97 (m, 3H), 7.95 - 7.89 (m, 1H), 7.85 - 7.77 (m, 3H), 7.69 (s, 1H), 7.53 - 7.45 (m, 2H), 7.36 (dd, *J* = 8.4, 1.6 Hz, 1H), 4.77 - 4.67 (m, 2H), 4.46 - 4.34 (m, 2H), 4.22 - 4.16 (m, 1H), 4.10 (d, *J* = 12.8 Hz, 1H), 3.96 - 3.83 (m, 2H), 3.77 - 3.68 (m, 1H), 2.88 - 2.81 (m, 1H), 2.69 (td, *J* = 12.8, 3.2 Hz, 1H), 2.34 - 2.26 (m, 1H), 2.13 (td, *J* = 11.8, 3.1 Hz, 1H).

¹³C NMR (126 MHz, CDCl₃) δ 163.63, 161.71, 133.32, 133.13, 132.43, 131.37, 131.25, 130.92, 130.59, 130.11, 129.12, 128.39, 127.89, 127.86, 127.83, 127.49, 126.64, 126.45, 126.11, 126.08, 125.29, 125.21, 124.81, 124.52, 124.06, 60.98, 57.50, 57.16, 51.42, 49.61, 48.66, 41.41.

Mass 524.23

### 65. Preparation Example 65: Formula S2141

A compound of Formula N2141 was obtained by further performing the step of Scheme 45 below using the compound obtained through Scheme 42 in Preparation Example 63 as a starting material.

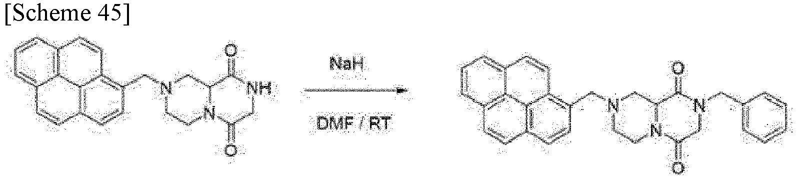

0.25 g (0.65 mmol) of 2-(pyren-1-ylmethyl)hexahydro-2H-pyrazino[1,2-a]pyrazine-6,9-dione, 0.039 g (0.97 mmol) of sodium hydride, and dimethylformamide were input and stirred at room temperature for 30 minutes, and 0.09 mL (0.78 mmol) of benzyl bromide was added thereto, followed by stirring at room temperature overnight. 10 mL of an ammonium chloride aqueous solution and 20 mL of ethyl acetate were added and stirred, and an organic layer was extracted and washed with 10 mL of a sodium chloride aqueous solution. An organic layer was dried over anhydrous magnesium sulfate and filtered. The reaction solution was concentrated and purified by silica chromatography, thereby obtaining 8-benzyl-2-(pyren-1-ylmethyl)hexahydro-2*H*-pyrazino[1,2-*a*]pyrazine-6,9-dione (0.323g, yield: 99%).

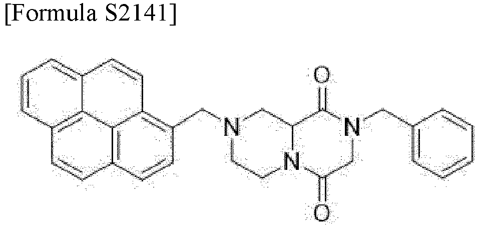

### 8-benzyl-2-(pyren-1-ylmethyl)hexahydro-2H-pyrazino[1,2-a]pyrazine-6,9-dione

¹H NMR (400 MHz, CDCl₃) δ 8.48 (d, *J* = 9.2 Hz, 1H), 8.19 (t, *J* = 7.8 Hz, 2H), 8.12 (dd, *J* = 8.5, 3.0 Hz, 2H), 8.08 - 7.98 (m, 3H), 7.94 - 7.88 (m, 1H), 7.39 - 7.28 (m, 3H), 7.28 - 7.21 (m, 2H), 4.56 (s, 2H), 4.46 - 4.35 (m, 2H), 4.22 - 4.05 (m, 2H), 3.93 - 3.79 (m, 2H), 3.73 - 3.68 (m, 1H), 2.88 - 2.82 (m, 2H), 2.70 (td, *J* = 12.7, 3.2 Hz, 1H), 2.29 (t, *J* = 11.2 Hz, 1H), 2.14 (td, *J* = 11.7, 3.0 Hz, 1H).

¹³C NMR (126 MHz, CDCl₃) δ 163.54, 161.75, 135.02, 131.36, 131.23, 130.91, 130.57, 130.10, 129.05, 128.60, 128.39, 128.31, 127.48, 126.07, 125.28, 125.19, 124.79, 124.50, 124.05, 60.95, 57.45, 57.14, 51.41, 49.40, 48.62, 41.40.

Mass 474.4

### 66. Preparation Example 66: Formula S2142

A compound of Formula N2142 was obtained by further performing the step of Scheme 46 below using the compound obtained through Scheme 42 in Preparation Example 63 as a starting material.

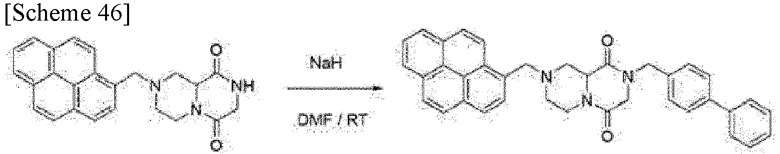

0.25 g (0.65 mmol) of 2-(pyren-1-ylmethyl)hexahydro-2*H*-pyrazino[1,2-a]pyrazine-6,9-dione, 0.039 g (0.97 mmol) of sodium hydride, and dimethylformamide were input and stirred at room temperature for 30 minutes, and 0.192 g (0.78 mmol) of 4-bromomethyl biphenyl was added thereto, followed by stirring at room temperature overnight. 10 mL of an ammonium chloride aqueous solution and 20 mL of ethyl acetate were added and stirred, and an organic layer was extracted and washed with 10 mL of a sodium chloride aqueous solution. An organic layer was dried over anhydrous magnesium sulfate and filtered. The reaction solution was concentrated and purified by silica chromatography, thereby obtaining 8-([1,1'-biphenyl]-4-ylmethyl)-2-(pyren-1-ylmethyl)hexahydro-2*H*-pyrazino[1,2-a]pyrazine-6,9-dione (0.314g, yield: 87%).

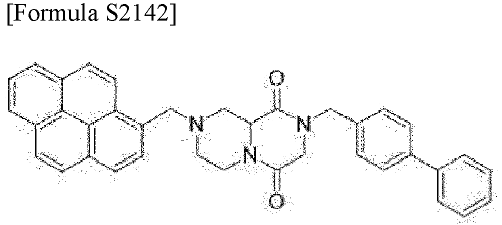

¹H NMR (500 MHz, CDCl₃) δ 8.47 (d, *J* = 9.2 Hz, 1H), 8.20 - 8.15 (m, 2H), 8.11 (dd, *J* = 8.5, 4.2 Hz, 2H), 8.06 - 7.97 (m, 3H), 7.90 (d, *J* = 7.8 Hz, 1H), 7.59 - 7.51 (m, 4H), 7.45 - 7.40 (m, 2H), 7.37 - 7.28 (m, 3H), 4.58 (s, 2H), 4.46 - 4.40 (m, 1H), 4.36 (d, *J* = 12.9 Hz, 1H), 4.20 - 4.14 (m, 1H), 4.10 (d, *J* = 12.9 Hz, 1H), 3.89 (s, 2H), 3.73 - 3.68 (m, 1H), 2.87 - 2.81 (m, 1H), 2.69 (td, *J* = 12.8, 3.3 Hz, 1H), 2.28 (t, *J* = 11.2 Hz, 1H), 2.13 (td, *J* = 11.8, 3.2 Hz, 1H).

¹³C NMR (126 MHz, CDCl₃) δ 163.47, 161.62, 141.20, 140.46, 133.89, 131.25, 131.12, 130.80, 130.47, 129.99, 128.97, 128.81, 128.27, 127.66, 127.49, 127.37, 127.07, 125.96, 125.16, 125.09, 124.69, 124.39, 123.94, 60.86, 57.37, 57.04, 51.31, 49.03, 48.59, 41.31.

Mass 550.4

### 67. Preparation Example 67: Formula S2217

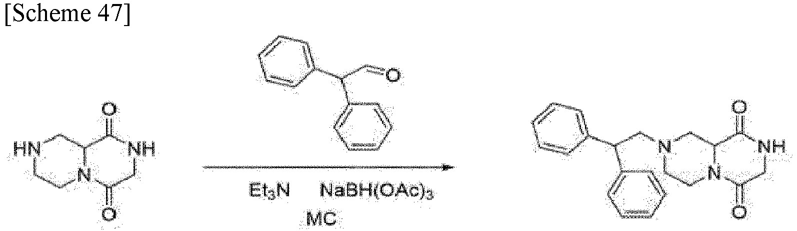

0.5 g (1.77 mmol) of tetrahydro-2*H*-pyrazino[1,2-*a*]pyrazine-1,4(3*H*,6*H*)-dione trifluoroacetate, 5mL of dichloromethane, 0.5 mL of triethylamine, and 0.31mL (1.77 mmol) of diphenylacetaldehyde were input at room temperature. 0.748 g (3.53 mmol) of sodium triacetoxyborohydride was added, and stirred at room temperature overnight. The reaction solution was washed with 10 mL of distilled water. The reaction solution was washed with a sodium bicarbonate aqueous solution and 10 mL of distilled water in this order. An organic layer was dried over anhydrous magnesium sulfate and filtered. The reaction solution was concentrated and purified by silica chromatography, thereby obtaining 2-(2,2-diphenylethyl)hexahydro-2*H*-pyrazino[1,2-*a*]pyrazine-6,9-dione (0.748g).

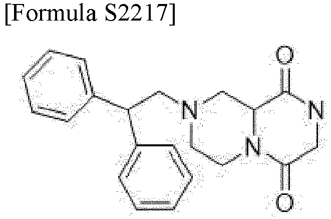

### 2-(2,2-diphenylethyl)hexahydro-2H-pyrazino[1,2-a]pyrazine-6,9-dione

¹H NMR (500 MHz, CDCl₃) δ 7.31 - 7.16 (m, 10H), 6.95 (s, 1H), 4.44 (ddd, *J* = 13.1, 2.9, 1.9 Hz, 1H), 4.24 (t, *J=* 7.8 Hz, 1H), 4.00 (s, 2H), 3.98 - 3.93 (m, 1H), 3.48 (ddd, *J=* 11.3, 3.1, 1.6 Hz, 1H), 3.10 - 2.98 (m, 2H), 2.93 - 2.88 (m, 1H), 2.66 (td, *J* = 12.7, 3.3 Hz, 1H), 2.18 - 2.08 (m, 2H).

¹³C NMR (126 MHz, CDCl₃) δ 166.03, 161.75, 143.29, 143.18, 128.55, 128.25, 128.24, 126.53, 63.08, 56.83, 56.34, 52.05, 48.53, 44.68, 41.50.

Mass 350.2

### 68. Preparation Example 68: Formula S2126

A compound of Formula N2126 was obtained by further performing the step of Scheme 48 below using the compound S2217 obtained through Scheme 47 in Preparation Example 67 as a starting material.

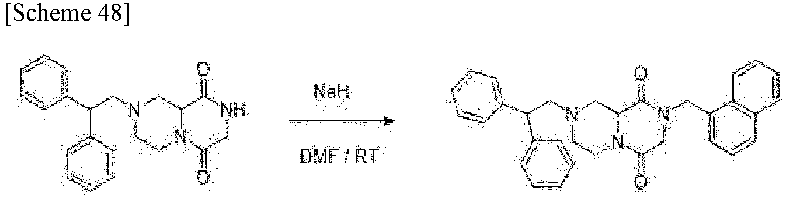

0.1 g (0.29 mmol) of 2-(2,2-diphenylethyl)hexahydro-2H-pyrazino[1,2-a]pyrazine-6,9-dione, 0.017 g (0.43 mmol) of sodium hydride, and dimethylformamide were input and stirred at room temperature for 30 minutes, and 0.075m g (0.34 mmol) of 1-(bromomethyl)naphthalene was added thereto, followed by stirring at room temperature overnight. 10 mL of an ammonium chloride aqueous solution and 20 mL of ethyl acetate were added and stirred, and an organic layer was extracted and washed with 10 mL of a sodium chloride aqueous solution. An organic layer was dried over anhydrous magnesium sulfate and filtered. The reaction solution was concentrated and purified by silica chromatography, thereby obtaining 2-(2,2-diphenylethyl)-8-(naphthalen-1-ylmethyl)hexahydro-2*H*-pyrazino[1,2-*a*]pyrazine-6,9-dione (0.096g, yield: 68%).

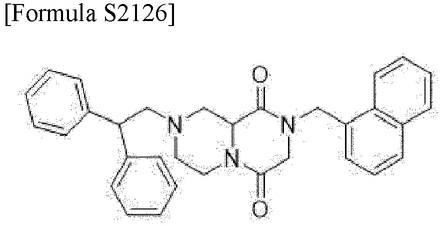

### 2-(2,2-diphenylethyl)-8-(naphthalen-1-ylmethyl)hexahydro-2H-pyrazino[1,2-a]pyrazine-6,9-dione

¹H NMR (400 MHz, CDCl₃) δ 8.05 - 8.00 (m, 1H), 7.91 - 7.82 (m, 2H), 7.57 - 7.49 (m, 2H), 7.45 - 7.36 (m, 2H), 7.33 - 7.16 (m, 10H), 5.08 (dd, *J* = 43.9, 14.5 Hz, 2H), 4.36 (d, *J* = 13.1 Hz, 1H), 4.27 (t, *J* = 7.8 Hz, 1H), 4.07 - 4.01 (m, 1H), 3.74 - 3.61 (m, 3H), 3.12 - 2.98 (m, 2H), 2.90 - 2.83 (m, 1H), 2.61 (td, *J* = 12.6, 3.2 Hz, 1H), 2.17 - 1.99 (m, 2H).

Mass 490.4

### 69. Preparation Example 69: Formula S2127

A compound of Formula N2127 was obtained by further performing the step of Scheme 49 below using the compound S2217 obtained through Scheme 47 in Preparation Example 67 as a starting material.

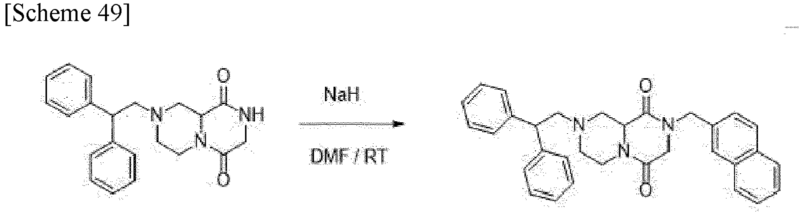

0.1 g (0.29 mmol) of 2-(2,2-diphenylethyl)hexahydro-2*H*-pyrazino[1,2-a]pyrazine-6,9-dione, 0.017 g (0.43 mmol) of sodium hydride, and dimethylformamide were input and stirred at room temperature for 30 minutes, and 0.075m g (0.34 mmol) of 2-(bromomethyl)naphthalene was added thereto, followed by stirring at room temperature overnight. 10 mL of an ammonium chloride aqueous solution and 20 mL of ethyl acetate were added and stirred, and an organic layer was extracted and washed with 10 mL of a sodium chloride aqueous solution. An organic layer was dried over anhydrous magnesium sulfate and filtered. The reaction solution was concentrated and purified by silica chromatography, thereby obtaining 2-(2,2-diphenylethyl)-8-(naphthalen-2-ylmethyl)hexahydro-2H-pyrazino[1,2-a]pyrazine-6,9-dione (0.077g, yield: 55%).

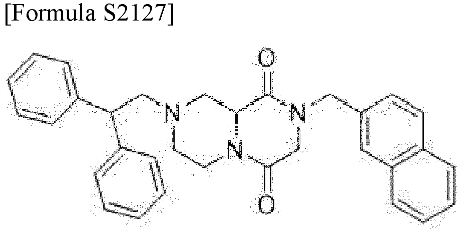

### 2-(2,2-diphenylethyl)-8-(naphthalen-2-ylmethyl)hexahydro-2H-pyrazino[1,2-a]pyrazine-6,9-dione

¹H NMR (400 MHz, CDCl₃) δ 7.87 - 7.77 (m, 3H), 7.70 (s, 1H), 7.54 - 7.46 (m, 2H), 7.39 - 7.17 (m, 11H), 4.80 - 4.68 (m, 2H), 4.44 - 4.38 (m, 1H), 4.27 (t, *J* = 7.8 Hz, 1H), 4.07 - 4.01 (m, 1H), 3.88 (s, 2H), 3.67 - 3.57 (m, 1H), 3.12 - 2.99 (m, 2H), 2.92 - 2.86 (m, 1H), 2.65 (td, *J* = 12.6, 3.2 Hz, 1H), 2.20 - 2.05 (m, 2H).

Mass 490.4

### 70. Preparation Example 70: Formula S2130

A compound of Formula N2130 was obtained by further performing the step of Scheme 50 below using the compound S2217 obtained through Scheme 47 in Preparation Example 67 as a starting material.

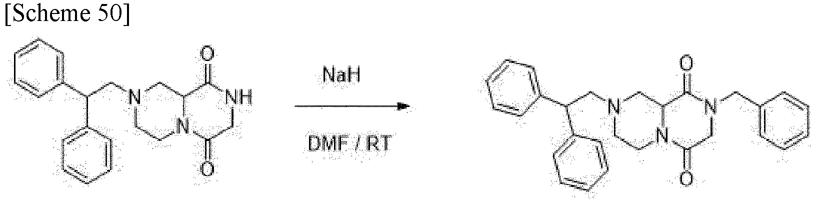

0.168g (0.481 mmol) of 2-(2,2-diphenylethyl)hexahydro-2*H*-pyrazino[1,2-a]pyrazine-6,9-dione, 0.029 g (0.73 mmol) of sodium hydride, and dimethylformamide were input and stirred at room temperature for 30 minutes, and 0.069 mL (0.58 mmol) of benzyl bromide was added thereto, followed by stirring at room temperature overnight. 10 mL of an ammonium chloride aqueous solution and 20 mL of ethyl acetate were added and stirred, and an organic layer was extracted and washed with 10 mL of a sodium chloride aqueous solution. An organic layer was dried over anhydrous magnesium sulfate and filtered. The reaction solution was concentrated and purified by silica chromatography, thereby obtaining 8-benzyl-2-(2,2-diphenylethyl)hexahydro-2H-pyrazino[1,2-a]pyrazine-6,9-dione (0.058g, yield: 27%).

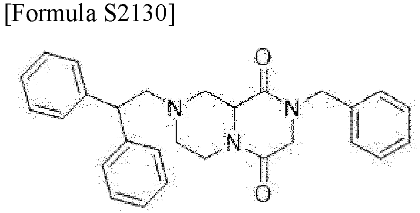

### 8-benzyl-2-(2,2-diphenylethyl)hexahydro-2H-pyrazino[1,2-a]pyrazine-6,9-dione

¹H NMR (400 MHz, CDCl₃) δ 7.39 - 7.15 (m, 15H), 4.57 (s, 2H), 4.45 - 4.37 (m, 1H), 4.26 (t, *J* = 7.8 Hz, 1H), 4.06 - 3.97 (m, 1H), 3.84 (s, 2H), 3.63 - 3.57 (m, 1H), 3.10 - 3.00 (m, 2H), 2.93 - 2.85 (m, 1H), 2.65 (td, *J* = 12.6, 3.3 Hz, 1H), 2.17 - 2.06 (m, 2H).

Mass 440.2

### 71. Preparation Example 71: Formula S2131

A compound of Formula N2131 was obtained by further performing the step of Scheme 51 below using the compound S2217 obtained through Scheme 47 in Preparation Example 67 as a starting material.

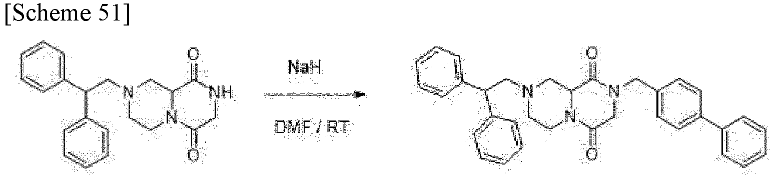

0.1g (0.29 mmol) of 2-(2,2-diphenylethyl)hexahydro-2*H*-pyrazino[1,2-a]pyrazine-6,9-dione, 0.017 g (0.43 mmol) of sodium hydride, and dimethylformamide were input and stirred at room temperature for 30 minutes, and 0.085 mL (0.34 mmol) of 4-bromomethyl biphenyl was added thereto, followed by stirring at room temperature overnight. 10 mL of an ammonium chloride aqueous solution and 20 mL of ethyl acetate were added and stirred, and an organic layer was extracted and washed with 10 mL of a sodium chloride aqueous solution. An organic layer was dried over anhydrous magnesium sulfate and filtered. The reaction solution was concentrated and purified by silica chromatography, thereby obtaining 8-([1,1'-biphenyl]-4-ylmethyl)-2-(2,2-diphenylethyl)hexahydro-2H-pyrazino[1,2-a]pyrazine-6,9-dione (0.102g, yield: 68%).

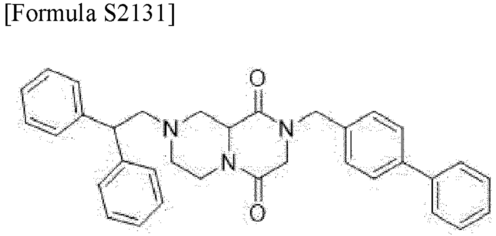

### 8-([1,1'-biphenyl]-4-ylmethyl)-2-(2,2-diphenylethyl)hexahydro-2H-pyrazino[1,2-a]pyrazine-6,9-dione

¹H NMR (400 MHz, CDCl₃) δ 7.60 - 7.54 (m, 4H), 7.47 - 7.41 (m, 2H), 7.39 - 7.16 (m, 13H), 4.69 - 4.54 (m, 2H), 4.42 (d, *J* = 13.1 Hz, 1H), 4.26 (t, *J* = 7.8 Hz, 1H), 4.09 - 3.98 (m, 1H), 3.89 (s, 2H), 3.67 - 3.56 (m, 1H), 3.14 - 2.97 (m, 2H), 2.90 (d, *J* = 11.5 Hz, 1H), 2.66 (td, *J* = 12.6, 3.2 Hz, 1H), 2.19 - 2.06 (m, 2H).

Mass 516.4

### 72. Preparation Example 72: Formula S2204

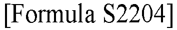

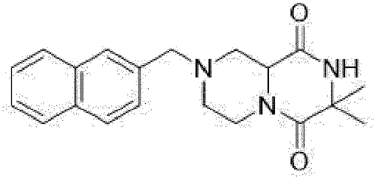

¹H NMR (500 MHz, CDCl₃) δ 7.84 - 7.78 (m, 3H), 7.71 (s, 1H), 7.50 - 7.43 (m, 3H), 7.34 (s, 1H), 4.50 (ddd, *J* = 13.2, 3.0, 1.9 Hz, 1H), 4.11 (dd, *J* = 11.0, 3.3 Hz, 1H), 3.72 (dd, *J* = 63.4, 13.1 Hz, 2H), 3.47 (ddd, *J* = 11.3, 3.2, 1.7 Hz, 1H), 2.92 - 2.85 (m, 1H), 2.82 - 2.75 (m, 1H), 2.15 - 2.03 (m, 2H), 1.48 (s, 3H), 1.45 (s, 3H).

¹³C NMR (126 MHz, CDCl₃) δ 168.25, 165.53, 134.68, 133.26, 132.89, 128.15, 127.75, 127.71, 127.65, 127.11, 126.10, 125.81, 62.71, 57.45, 56.43, 56.03, 51.70, 41.70, 29.16, 28.95.

Mass 338.18

### 73. Preparation Example 73: Formula S2211

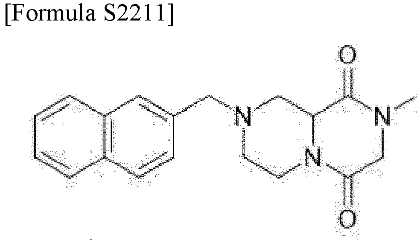

¹H NMR (500 MHz, CDCl₃) δ 7.83 - 7.77 (m, 3H), 7.69 (s, 1H), 7.49 - 7.42 (m, 3H), 4.47 (ddd, *J* = 13.1, 3.2, 1.8 Hz, 1H), 3.92 (s, 2H), 3.96 - 3.88 (m, 2H), 3.83 - 3.59 (m, 1H), 3.50 (ddd, *J* = 11.4, 3.2, 1.8 Hz, 1H), 2.90 (s, 3H), 2.88 - 2.82 (m, 1H), 2.81 - 2.71 (m, 1H), 2.13 - 1.99 (m, 2H).

¹³C NMR (126 MHz, CDCl₃) δ 163.42, 161.37, 134.45, 133.19, 132.83, 128.08, 127.79, 127.66, 127.59, 127.12, 126.03, 125.76, 62.60, 57.12, 56.57, 51.23, 51.10, 41.18, 33.25.

Mass 324.17

### 74. Preparation Example 74: Formula S2212

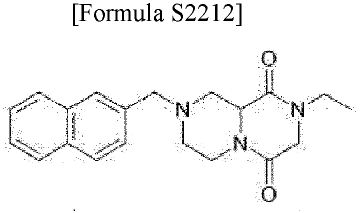

¹H NMR (500 MHz, CDCl₃) δ 7.83 - 7.77 (m, 3H), 7.70 (s, 1H), 7.50 - 7.42 (m, 3H), 4.48 (ddd, *J* = 13.2, 3.1, 1.8 Hz, 1H), 4.11 - 4.04 (m, 1H), 4.00 - 3.88 (m, 2H), 3.72 (dd, *J* = 77.0, 13.1 Hz, 2H), 3.51 (ddd, *J* = 11.4, 3.2, 1.8 Hz, 1H), 3.46 - 3.32 (m, 2H), 2.89 - 2.83 (m, 1H), 2.78 (td, *J* = 12.6, 3.3 Hz, 1H), 2.14 - 2.01 (m, 2H), 1.13 (t, *J* = 7.2 Hz, 3H).

¹³C NMR (126 MHz, CDCl₃) δ 162.98, 161.87, 134.61, 133.29, 132.93, 128.17, 127.86, 127.76, 127.68, 127.20, 126.12, 125.84, 62.72, 57.29, 56.67, 51.33, 48.59, 41.30, 40.89, 11.60.

Mass 338.18

### 75. Preparation Example 75: Formula S2213

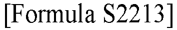

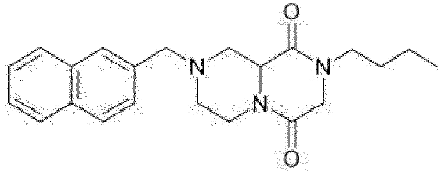

¹H NMR (500 MHz, CDCl₃) δ 7.83 - 7.78 (m, 3H), 7.71 (s, 1H), 7.50 - 7.43 (m, 3H), 4.49 (ddd, *J* = 13.2, 3.2, 1.8 Hz, 1H), 4.12 - 4.07 (m, 1H), 4.00 - 3.90 (m, 2H), 3.73 (dd, *J* = 77.3, 13.1 Hz, 2H), 3.52 (ddd, *J* = 11.4, 3.3, 1.8 Hz, 1H), 3.42 - 3.29 (m, 2H), 2.90 - 2.84 (m, 1H), 2.79 (td, *J* = 12.6, 3.4 Hz, 1H), 2.14 - 2.03 (m, 2H), 1.57 - 1.48 (m, 2H), 1.35 - 1.25 (m, 2H), 0.93 (t, *J* = 7.4 Hz, 3H).

¹³C NMR (126 MHz, CDCl₃) δ 163.30, 161.97, 134.69, 133.36, 133.00, 128.24, 127.91, 127.82, 127.74, 127.24, 126.17, 125.90, 62.80, 57.36, 56.82, 51.41, 49.23, 45.86, 41.38, 28.51, 20.01, 13.80.

Mass 366.21

### 76. Preparation Example 76: Formula S2214

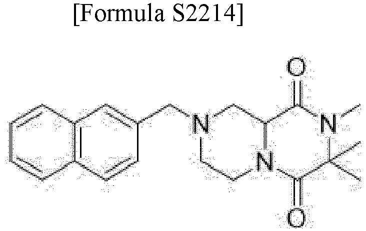

¹H NMR (500 MHz, CDCl₃) δ 7.85 - 7.78 (m, 3H), 7.71 (s, 1H), 7.51 - 7.43 (m, 3H), 4.46 (brs, 1H), 4.14 - 4.07 (m, 1H), 3.80 (dd, *J* = 80.7, 13.1 Hz, 2H), 3.59 (dd, *J* = 11.9, 2.3 Hz, 1H), 3.18 (ddd, *J* = 13.5, 12.9, 4.2 Hz, 1H), 3.01 - 2.92 (m, 4H), 2.36 (d, *J* = 11.9 Hz, 1H), 2.17 (ddd, *J* = 12.7, 11.8, 3.9 Hz, 1H), 1.59 (s, 3H), 1.53 (s, 3H).

¹³C NMR (126 MHz, CDCl₃) δ 171.49, 163.97, 134.07, 133.28, 132.99, 128.41, 128.05, 127.75, 127.70, 126.94, 126.26, 126.01, 79.55, 62.28, 60.94, 60.45, 50.98, 38.55, 28.29, 26.08, 25.57.

Mass 350.18

### 77. Preparation Example 77: Formula S2215

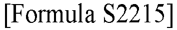

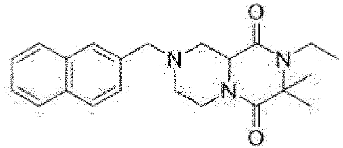

¹H NMR (500 MHz, CDCl₃) δ 7.86 - 7.78 (m, 3H), 7.71 (s, 1H), 7.51 - 7.42 (m, 3H), 4.44 (s, 1H), 4.13 - 4.04 (m, 1H), 3.89 - 3.51 (m, 3H), 3.46 - 3.31 (m, 1H), 3.25 - 3.12 (m, 1H), 3.01 - 2.93 (m, 1H), 2.35 (d, *J* = 11.9 Hz, 1H), 2.22 - 2.15 (m, 1H), 2.11 - 2.00 (m, 1H), 1.62 - 1.50 (m, 6H), 1.24 - 119 (m, 3H).

¹³C NMR (126 MHz, CDCl₃) δ 171.81, 163.70, 134.24, 133.40, 133.11, 128.53, 128.17, 127.87, 127.81, 127.06, 126.36, 126.12, 79.51, 62.42, 61.55, 60.46, 51.15, 38.69, 38.26, 27.23, 25.87, 14.57.

Mass 366.21

### 78. Preparation Example 78: Formula S2216

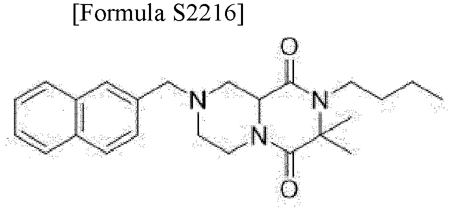

¹H NMR (500 MHz, CDCl₃) δ 7.84 - 7.77 (m, 3H), 7.70 (s, 1H), 7.50 - 7.40 (m, 3H), 4.51 - 4.45 (m, 1H), 4.08 - 4.04 (m, 1H), 3.81 - 3.61 (m, 2H), 3.52 (ddd, *J* = 11.3, 3.2, 1.8 Hz, 1H), 3.39 - 3.17 (m, 2H), 2.90 - 2.70 (m, 2H), 2.11 - 2.00 (m, 2H), 1.60 - 1.52 (m, 5H), 1.48 (s, 3H), 1.37 - 1.28 (m, 2H), 0.95 - 0.90 (m, 3H).

¹³C NMR (126 MHz, CDCl₃) δ 168.18, 163.81, 134.76, 133.33, 132.95, 128.18, 127.83, 127.78, 127.69, 127.18, 126.09, 125.81, 77.41, 77.16, 76.90, 62.77, 61.15, 57.02, 56.88, 51.61, 43.08, 41.76, 31.36, 26.62, 26.28, 20.51, 13.80.

Mass 394.24

### 79. Preparation Example 79: Formula N502

For the following derivative synthesis, benzyl 4-(naphthalen-2-ylmethyl)piperazine-2-carboxylate was also used as above, and the following synthesis method was used.

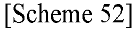

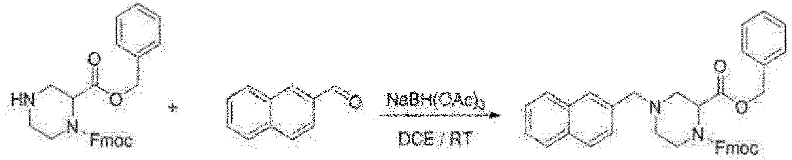

1.0 g (2.3 mmol) of 2-naphthaldehyde 0.4 g (2.3 mmol), 1-((9H-fluoren-9-yl)methyl) 2-benzyl piperazine-1,2-dicarboxylate, 20 mL of 1,2-dichloroethane, 1.4 g (6.8 mmol) of sodium triacetoxyborohydride, and 3.5 g of molecular sieve 4Å were input, and stirred at room temperature overnight. 25 mL of distilled water was added to the reaction mixture, followed by vigorous stirring for 20 minutes. An organic layer was extracted and washed with a sodium chloride aqueous solution. The organic layer was dried over anhydrous sodium sulfate and concentrated under reduced pressure, thereby obtaining a compound as a residue, and then the compound (1.4 g) was used in the following step.

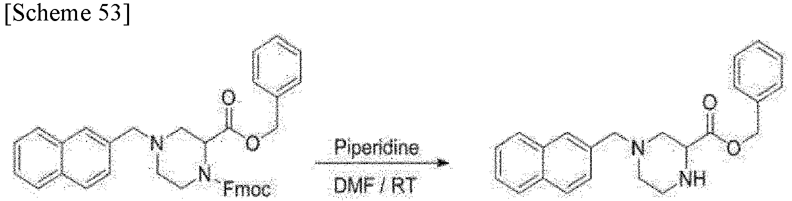

1.4 g of 1-((9H-fluoren-9-yl)methyl) 2-benzyl 4-(naphthalen-2-ylmethyl)piperazine-1,2-dicarboxylate, 7 ml of dimethylformamide, and 3.2 ml of piperidine were input and stirred at room temperature for 1 hour. 80 ml of an ammonium chloride aqueous solution and dichloromethane were added to the reaction mixture and stirred, and then an organic layer was extracted. The organic layer was dried over anhydrous magnesium sulfate, and then concentrated under reduced pressure. The concentrated residue was purified by silica chromatography, thereby obtaining benzyl 4-(naphthalen-2-ylmethyl)piperazine-2-carboxylate (0.47 g, yield: 54%).

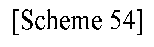

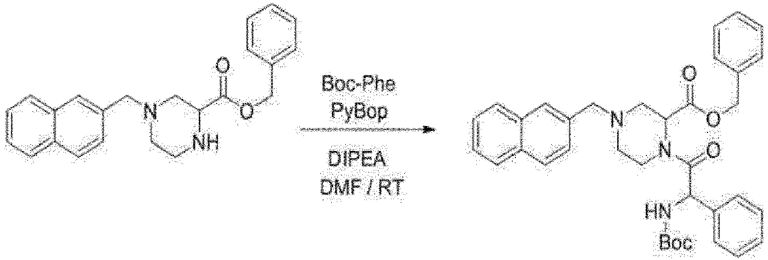

1.9 g (5.3 mmol) of the benzyl 4-(naphthalen-2-ylmethyl)piperazine-2-carboxylate, 1.5 g (5.5 mmol) of N-(*tert*-butoxycarbonyl)-*L*-phenylalanine, 15 mL of dimethylformamide, 2.9 g (5.5 mmol) of (benzotriazol-1-yloxy)tripyrrolidinophosphonium hexafluorophosphate, and 1.0 g (7.9 mmol) of N,N-diisopropylethylamine were input and stirred at room temperature overnight. 60 mL of distilled water and 30 mL of ethyl acetate were added to the reaction mixture and stirred. An organic layer was extracted and washed with a sodium chloride aqueous solution. The organic layer was dried over anhydrous magnesium sulfate and concentrated under reduced pressure. The concentrated residue was purified by silica chromatography, thereby obtaining benzyl 1-(2-((*tert*-butoxycarbonyl)amino)-2-phenylacetyl)-4-(naphthalen-2-ylmethyl)piperazine-2-carboxylate (2.6 g, yield: 81%).

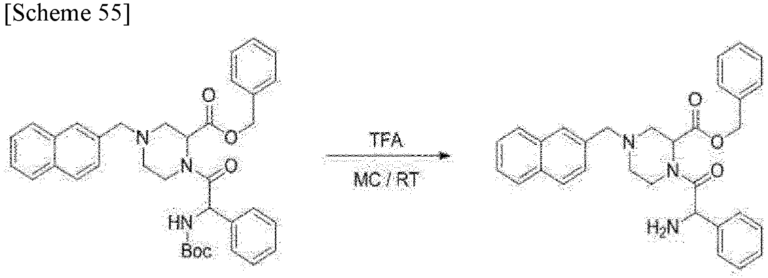

2.6 g (4.3 mmol) of the benzyl 1-(2-((*tert*-butoxycarbonyl)amino)-2-phenylacetyl)-4-(naphthalen-2-ylmethyl)piperazine-2-carboxylate, 26 mL of dichloromethane, and 6.5 mL of trifluoroacetate were input and stirred at room temperature for 30 minutes. The reaction solution was neutralized with 80 mL of a sodium carbonate aqueous solution, and an organic layer was extracted. The organic layer was dried over anhydrous magnesium sulfate and concentrated under reduced pressure, thereby obtaining benzyl 1-(2-amino-2-phenylacetyl)-4-(naphthalen-2-ylmethyl)piperazine-2-carboxylate as a residue (1.9 g), which was used in the following step.

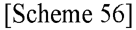

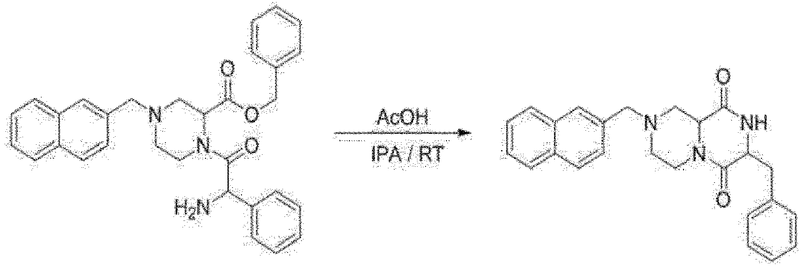

1.9 g of the benzyl 1-(2-amino-2-phenylacetyl)-4-(naphthalen-2-ylmethyl)piperazine-2-carboxylate residue, 10 mL of isopropanol, and 3 mL of acetic acid were input and stirred at room temperature overnight. 110 mL of a sodium bicarbonate aqueous solution, and 30 mL of ethyl acetate were added to the reaction mixture and stirred. An organic layer was extracted and washed with 30 mL of a sodium chloride aqueous solution. The organic layer was dried over anhydrous magnesium sulfate and concentrated under reduced pressure. The concentrated residue was purified by silica chromatography, thereby obtaining 3-benzyl-8-(naphthalen-2-ylmethyl)hexahydro-1*H*-pyrazino[1,2-*a*]pyrazine-1,4(6*H*)-dione (N502) (1.5 g, two-step yield: 88%).

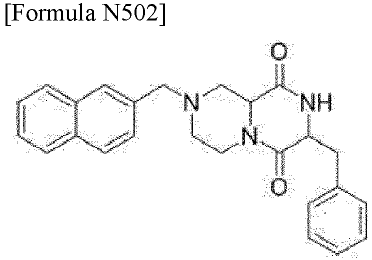

### 3-benzyl-8-(naphthalen-2-ylmethyl)hexahydro-1H-pyrazino[1,2-a]pyrazine-1,4(6H)-dione

¹H NMR (500 MHz, CDCl₃) δ 7.86 - 7.76 (m, 3H), 7.64 (d, *J* = 17.1 Hz, 1H), 7.51 - 7.43 (m, 2H), 7.43 - 7.36 (m, 1H), 7.33 - 7.29 (m, 2.5H), 7.22 - 7.14 (m, 2.5H), 7.03 (s, 0.5H), 6.90 (s, 0.5H), 4.52 - 4.40 (m, 1H), 4.38 - 4.26 (m, 1H), 3.92 (dd, *J* = 11.1, 2.8 Hz, 0.5H), 3.73 - 3.43 (m, 2H), 3.29 - 3.24 (m, 1H), 3.19 (dd, *J* = 13.6, 5.6 Hz, 0.5H), 3.08 - 3.01 (m, 2H), 2.82 - 2.79 (m, 0.5H), 2.72 - 2.63 (m, 1H), 2.52 (td, *J* = 12.8, 3.4 Hz, 0.5H), 2.05 - 1.93 (m, 1H), 1.74 (td, *J* = 11.4, 3.0 Hz, 0.5H), 0.78 (t, *J* = 11.2 Hz, 0.5H).

Mass 400.2

### <Experimental Examples>

### Anti-cancer therapeutic activity-enhancing effect of compound of Preparation Example

### 1. Preparation of SKOV3 and SKOV3-TR

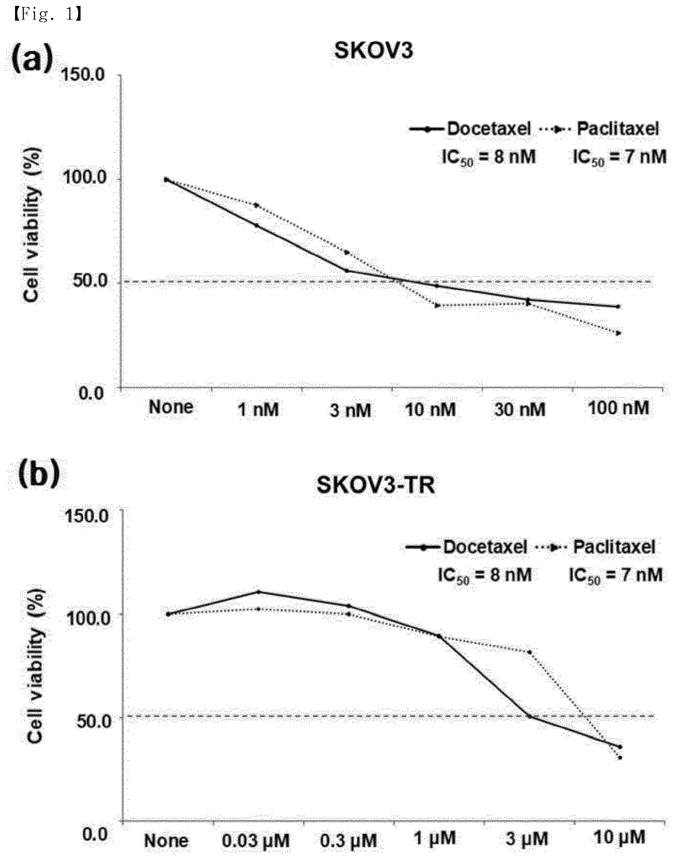

The epithelial ovarian cancer cell line SKOV3 and the resistant cancer cell line SKOV3-TR, which is derived therefrom and resistant to the anticancer agent paclitaxel, were subjected to a cell experiment, and FIG. 1(a) shows the IC50 values of the anticancer agents, paclitaxel and docetaxel, in SKOV3, and FIG. 1(b) shows the anticancer agent IC50 values of the anticancer agents, paclitaxel and docetaxel, in SKOV3-TR.

### 2. WST-1 assay by paclitaxel treatment

According to the results of WST-1 assays, which measure cell proliferation or survival, a reduction in metabolism in SKOV3 and SKOV3-TR was induced by paclitaxel treatment. The WST-1 assays were performed after SKOV3 and SKOV3-TR were co-treated with 79 types of compounds and paclitaxel.

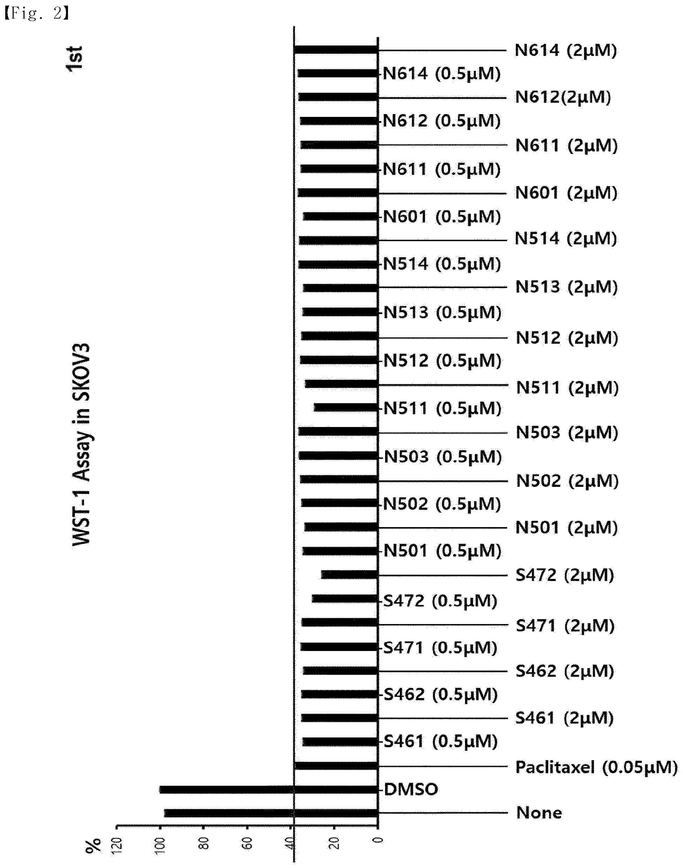

Specifically, the two cancer cell lines grown in 96-well plates were treated with the 79 types of compounds at a low concentration (0.5 µM) or a high concentration (2 µM) and paclitaxel, and the paclitaxel concentration used herein was 3 µM or 4 µM for the SKOV3-TR cell line, and 2 nM or 0.05 µM for the SKOV3 cell line. For cytotoxicity analysis, three days after co-treatment of DMSO (paclitaxel, compound solvent), paclitaxel and the 79 types of compounds, the WST-1 assays were performed. The results are shown in FIGS. 2 to 13.

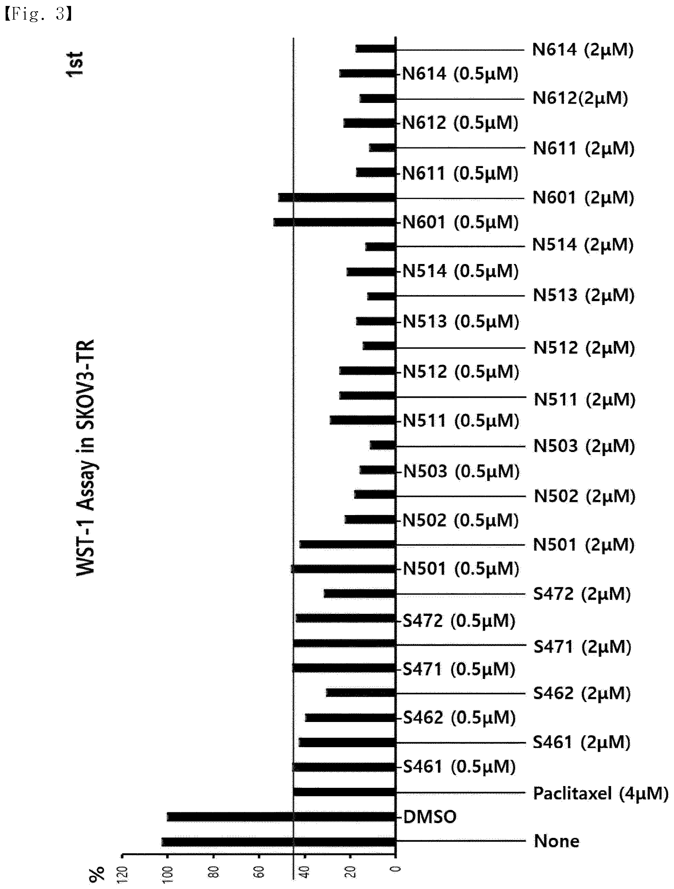

FIGS. 2 and 3 show None, DMSO, paclitaxel, S461 (0.5µM), S461 (2 µM), S462 (0.5 µM), S462 (2 µM), S471 (0.5 µM), S471 (2 µM), S472 (0.5 µM), S472 (2 µM), N501 (0.5 µM), N501 (2 µM), N502 (0.5 µM), N502 (2 µM), N503 (0.5 µM), N503 (2 µM), N511 (0.5 µM), N511 (2 µM), N512 (0.5 µM), N512 (2 µM), N513 (0.5 µM), N513 (2 µM), N514 (0.5 µM), N514 (2 µM), N601 (0.5 µM), N601 (2 µM), N611 (0.5 µM), N611 (2 µM),N612 (0.5 µM), N612 (2 µM), N614 (0.5 µM), and N614 (2 µM) in this order.

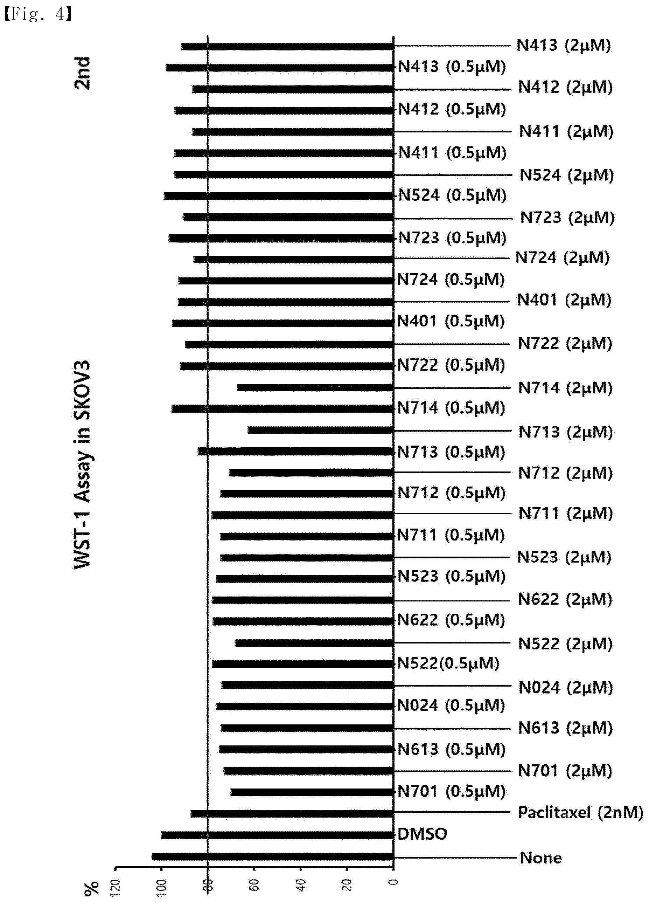

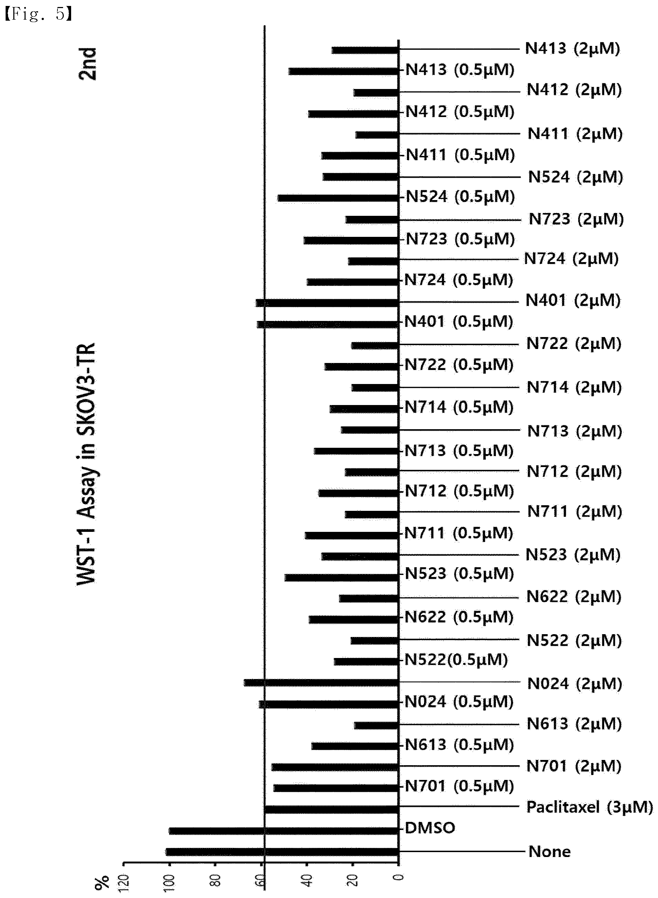

FIGS. 4 and 5 show None, DMSO, paclitaxel, N701 (0.5 µM), N701 (2 µM), N613 (0.5 µM), N613 (2 µM), N024 (0.5 µM), N024 (2 µM), N522 (0.5 µM), N522 (2 µM), N622 (0.5 µM), N622 (2 µM), N523 (0.5 µM), N523 (2 µM), N711 (0.5 µM), N711 (2 µM), N712 (0.5 µM), N712 (2 µM), N713 (0.5 µM), N713 (2 µM), N714 (0.5 µM), N714 (2 µM), N722 (0.5 µM), N722 (2 µM), N401 (0.5 µM), N401 (2 µM), N724 (0.5 µM), N724 (2 µM),N 723 (0.5 µM), N723 (2 µM), N524 (0.5 µM), N524 (2 µM), N411 (0.5 µM), N411 (2 µM),N412 (0.5 µM), N412 (2 µM), N413 (0.5 µM), and N413 (2 µM) in this order.

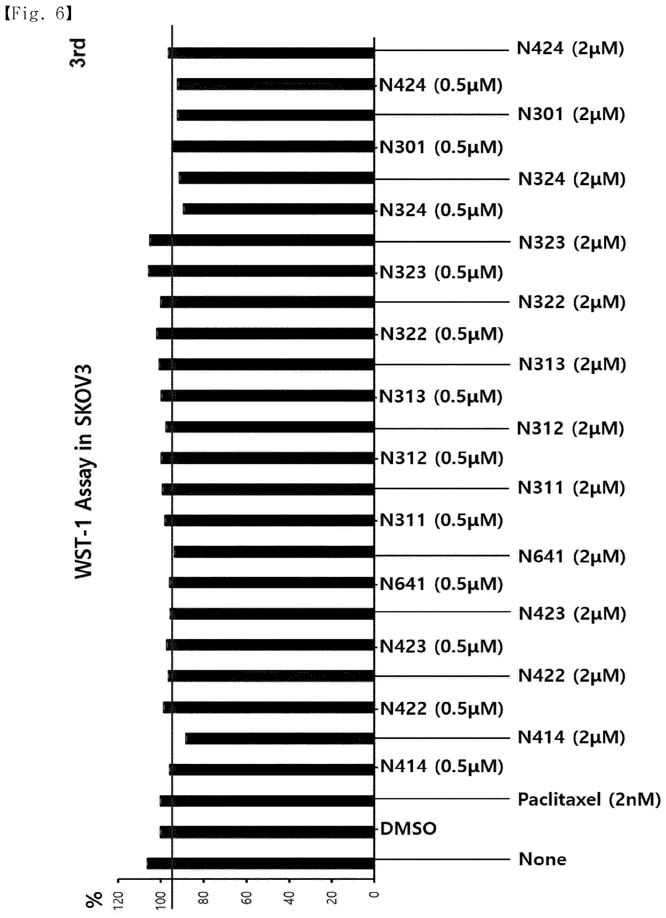

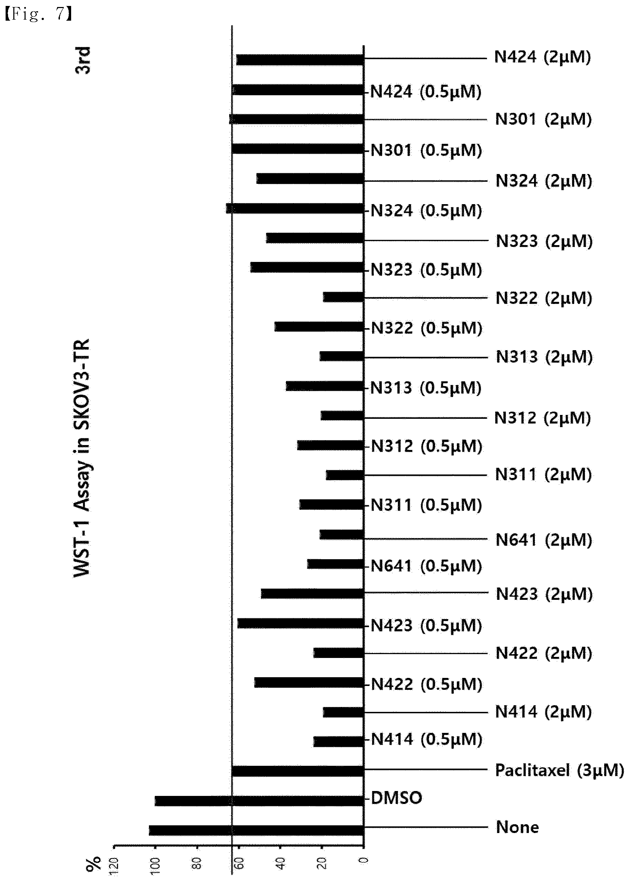

FIGS. 6 and 7 show None, DMSO, paclitaxel, N414 (0.5 µM), N414 (2 µM), N422 (0.5 µM), N422 (2 µM), N423 (0.5 µM), N423 (2 µM), N641 (0.5 µM), N641 (2 µM), N311 (0.5 µM), N311 (2 µM), N312 (0.5 µM), N312 (2 µM), N313 (0.5 µM), N313 (2 µM), N322 (0.5 µM), N322 (2 µM), N323 (0.5 µM), N323 (2 µM), N324 (0.5 µM), N324 (2 µM), N301 (0.5 µM), N301 (2 µM), N424 (0.5 µM), and N424 (2 µM) in this order.

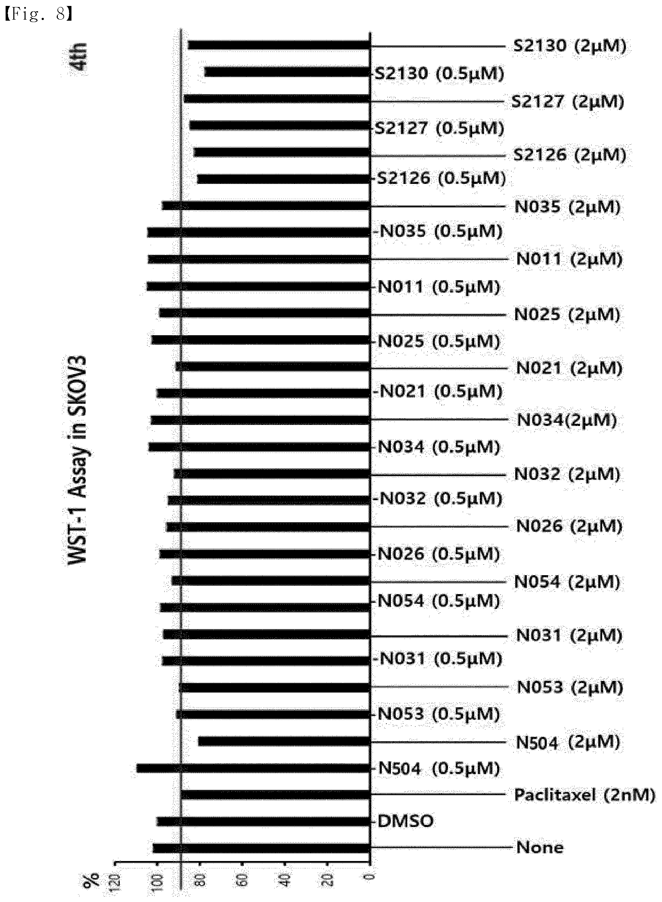

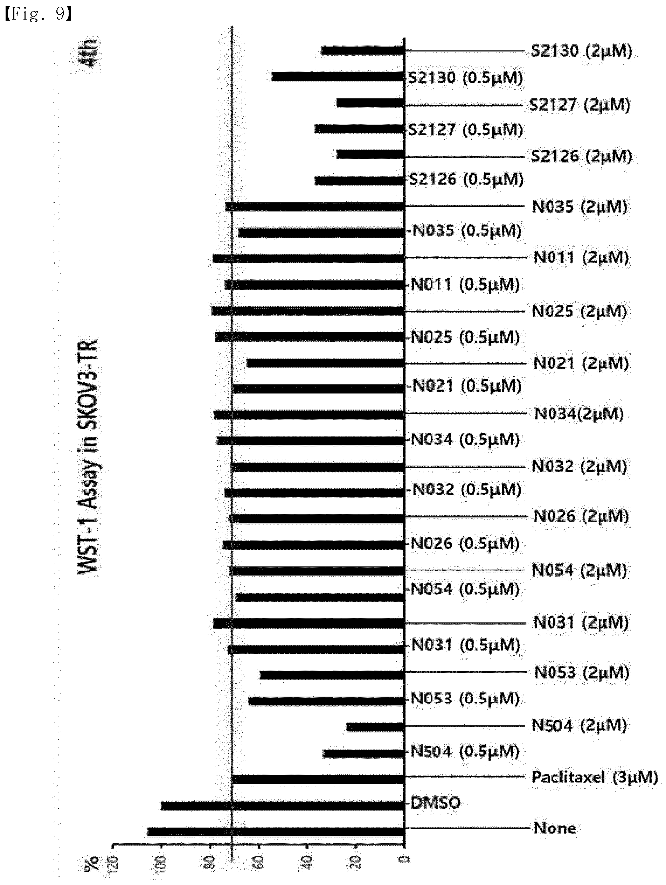

FIGS. 8 and 9 show None, DMSO, paclitaxel, N504 (0.5 µM), N504 (2 µM), N053 (0.5 µM), N053 (2 µM), N031 (0.5 µM), N031 (2 µM), N054 (0.5 µM), N054 (2 µM), N026 (0.5 µM), N026 (2 µM), N032 (0.5 µM), N032 (2 µM), N034 (0.5 µM), N034 (2 µM), N021 (0.5 µM), N021 (2 µM), N025 (0.5 µM), N025 (2 µM), N011 (0.5 µM), N011 (2 µM), N035 (0.5 µM), N035 (2 µM), S2126 (0.5 µM), S2126 (2 µM), S2127 (0.5 µM), S2127 (2 µM), S2130 (0.5 µM), and S2130 (2 µM) in this order.

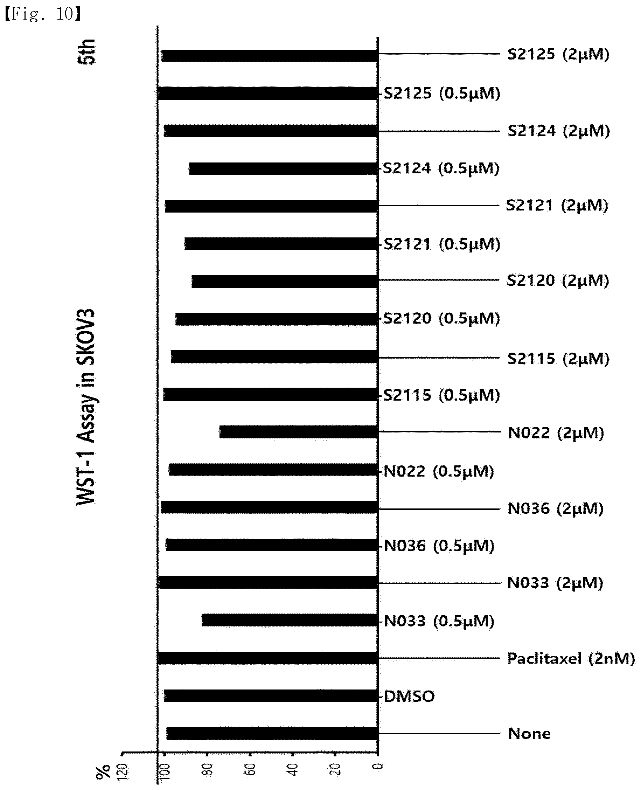

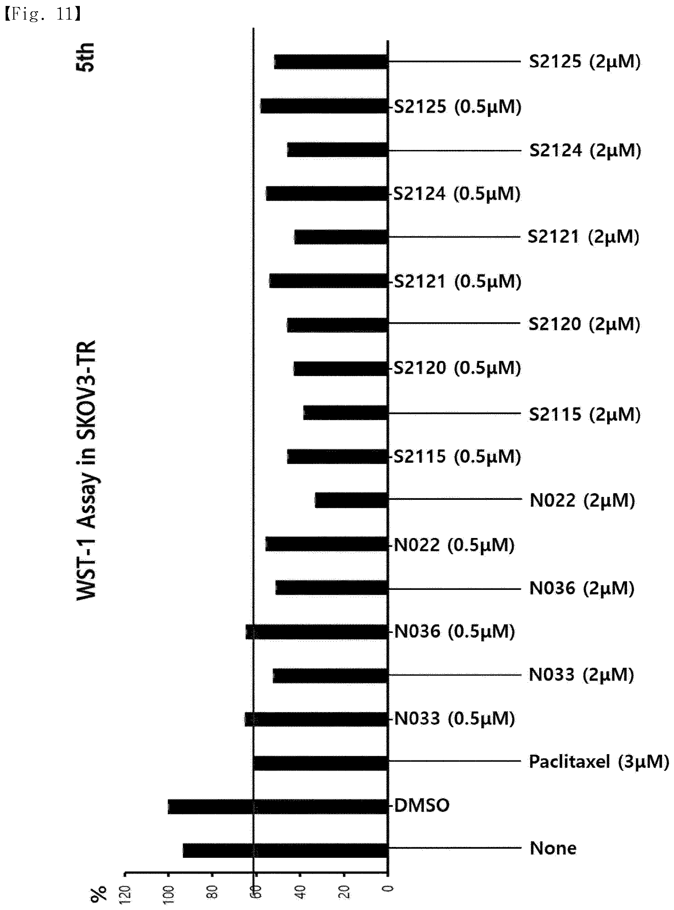

FIGS. 10 and 11 show None, DMSO, paclitaxel, N033 (0.5 µM), N033 (2 µM), N036 (0.5 µM), N036 (2 µM), N022 (0.5 µM), N022 (2 µM), S2115 (0.5 µM), S2115 (2 µM), S2120 (0.5 µM), S2120 (2 µM), S2121 (0.5 µM), S2121 (2 µM), S2124 (0.5 µM), S2124 (2 µM), S2125 (0.5 µM), and S2125 (2 µM) in this order.

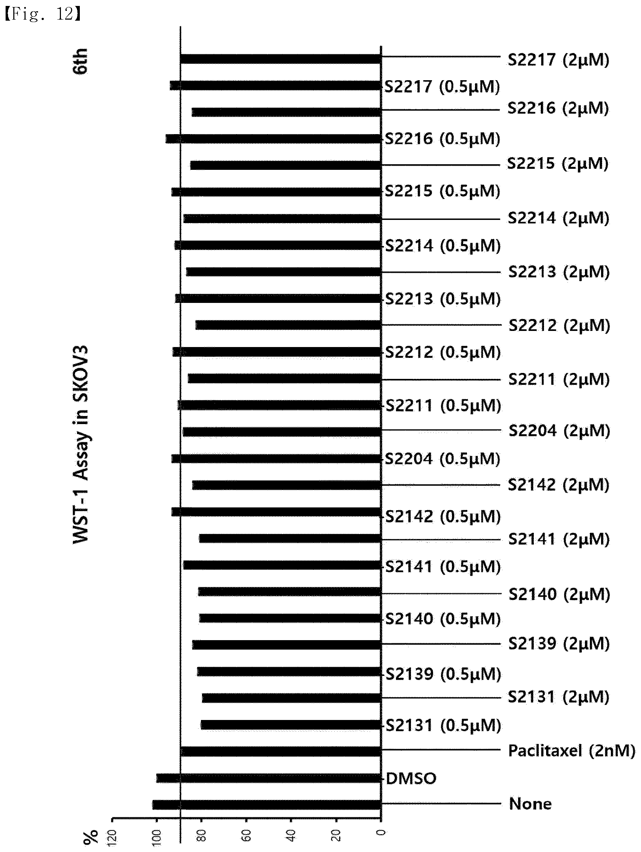

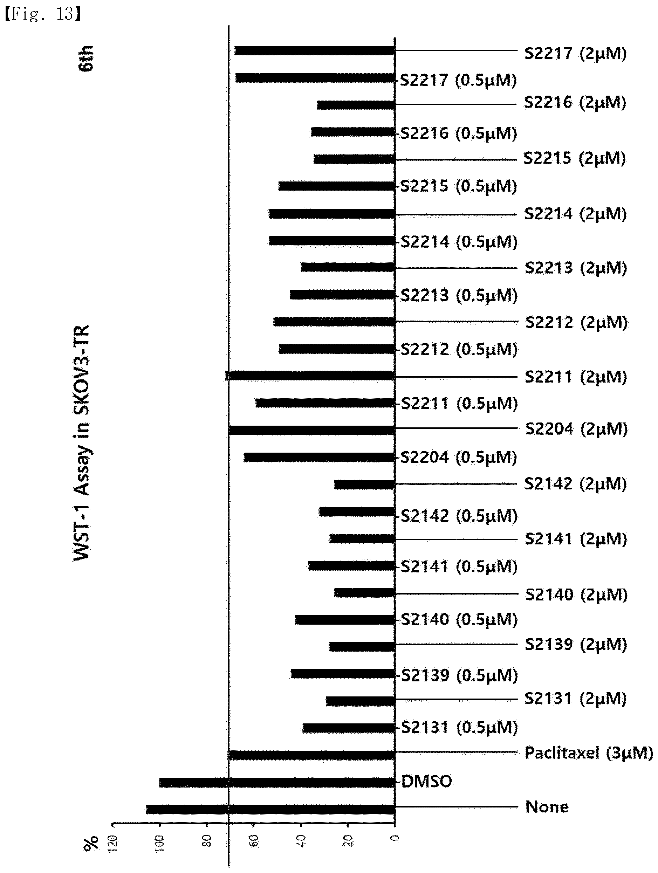

FIGS. 12 and 13 show None, DMSO, paclitaxel, S2131 (0.5 µM), S2131 (2 µM), S2139 (0.5 µM), S2139 (2 µM), S2140 (0.5 µM), S2140 (2 µM), S2141 (0.5 µM), S2141 (2 µM), S2142 (0.5 µM), S2142 (2 µM), S2204 (0.5 µM), S2204 (2 µM), S2211 (0.5 µM), S2211 (2 µM), S2212 (0.5 µM), S2212 (2 µM), S2213 (0.5 µM), S2213 (2 µM), S2214 (0.5 µM), S2214 (2 µM), S2215 (0.5 µM), S2215 (2 µM), S2216 (0.5 µM), S2216 (2 µM), S2217 (0.5 µM), and S2217 (2 µM) in this order.

That is, in the paclitaxel-treated group, due to the phenomena such as the induction of cell death and the inhibition of cell growth in SKOV3 and SKOV3-TR, the absorbance at 450 nm was reduced compared to the DMSO-treated group. When the compounds were treated in combination with paclitaxel, in the resistant cancer cell line SKOV3-TR, the anticancer efficacy of paclitaxel was enhanced and showed a lower absorbance as compared to the paclitaxel-treated group, whereas, in SKOV3, the anticancer efficacy of paclitaxel was not enhanced. In addition, in SKOV3-TR, the paclitaxel-induced reduction in metabolism is further promoted in the presence of the 2 µM compound compared to the 0.5 µM compound, showing that a dose-response relationship is established. A baseline was applied at the absorbance of the paclitaxel-treated group, which is a comparative example. For the enhanced anticancer efficacy of paclitaxel, the absorbances (OD values at 450 nm) were significantly reduced in the WST-1 assay in the paclitaxel+compound treatment, compared to the paclitaxel-treated group.

### 3. WST-1 assay by docetaxel treatment

According to the results of WST-1 assays, which measure cell proliferation or survival, a reduction in metabolism in SKOV3 and SKOV3-TR was induced by docetaxel treatment.

After SKOV3 and SKOV3-TR were co-treated with 79 types of compounds and docetaxel, the WST-1 assays were performed.

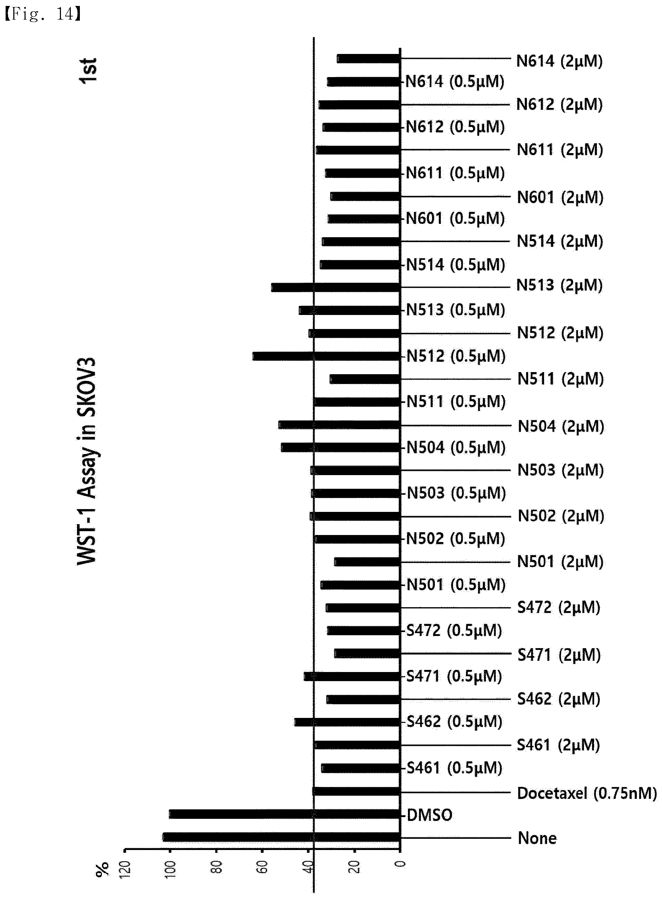

Specifically, the two cancer cell lines grown in 96-well plates were treated with the 79 types of compounds at a low concentration (0.5 µM) or a high concentration (2 µM) and docetaxel, and the docetaxel concentration used herein were 1 µM for the SKOV3-TR cell line and 0.75 nM for the SKOV3 cell line. For cytotoxicity analysis, three days after the co-treatment of DMSO (docetaxel, compound solvent), docetaxel and the 79 types of compounds, the WST-1 assays were performed. The results are shown in FIGS. 14 to 25.

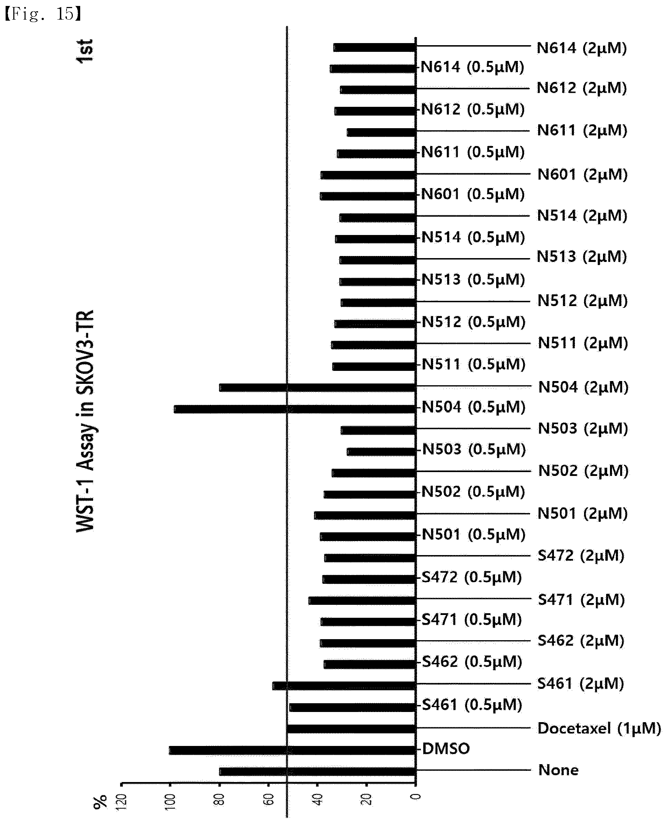

FIGS. 14 and 15 show None, DMSO, docetaxel, S461 (0.5 µM), S461 (2 µM), S462 (0.5 µM), S462 (2 µM), S471 (0.5 µM), S471 (2 µM), S472 (0.5 µM), S472 (2 µM), N501 (0.5 µM), N501 (2 µM), N502 (0.5 µM), N502 (2 µM), N503 (0.5 µM), N503 (2 µM), N504 (0.5 µM), N504 (2 µM), N511 (0.5 µM), N511 (2 µM), N512 (0.5 µM), N512 (2 µM), N513 (0.5 µM), N513 (2 µM), N514 (0.5 µM), N514 (2 µM), N601 (0.5 µM), N601 (2 µM), N611 (0.5 µM), N611 (2 µM), N612 (0.5 µM), N612 (2 µM), N614 (0.5 µM), and N614 (2 µM) in this order.

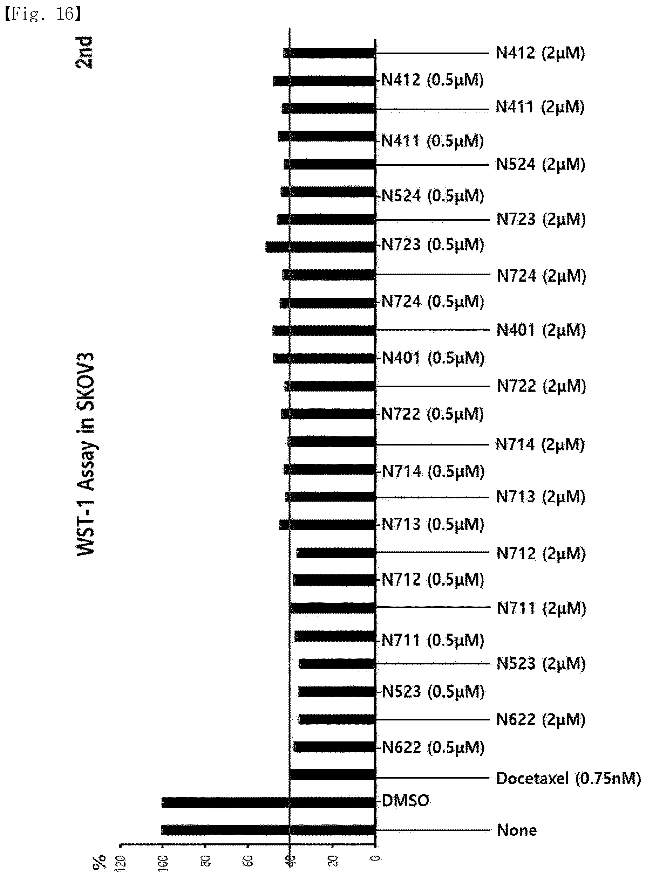

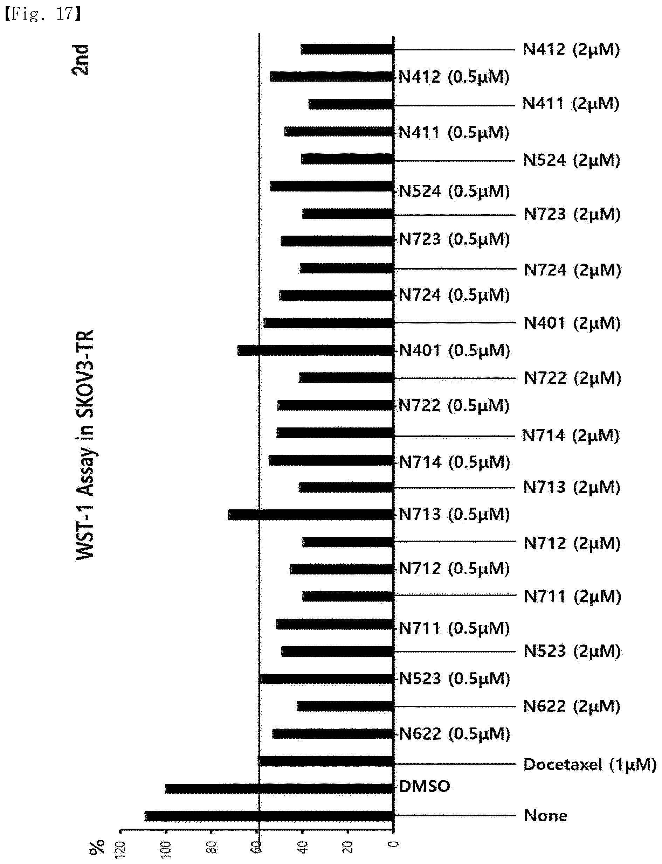

FIGS. 16 and 17 show None, DMSO, docetaxel, N622(0.5µM), N622(2µM), N523(0.5µM), N523(2µM), N711(0.5µM), N711(2µM), N712(0.5µM), N712(2µM), N713(0.5µM), N713(2µM), N714(0.5µM), N714(2µM), N722(0.5µM), N722(2µM), N401(0.5µM), N401(2µM), N724(0.5µM), N724(2µM), N723(0.5µM), N723(2µM), N524(0.5µM), N524(2µM), N411(0.5µM), N411(2µM), N412(0.5µM) and N412(2µM) in this order.

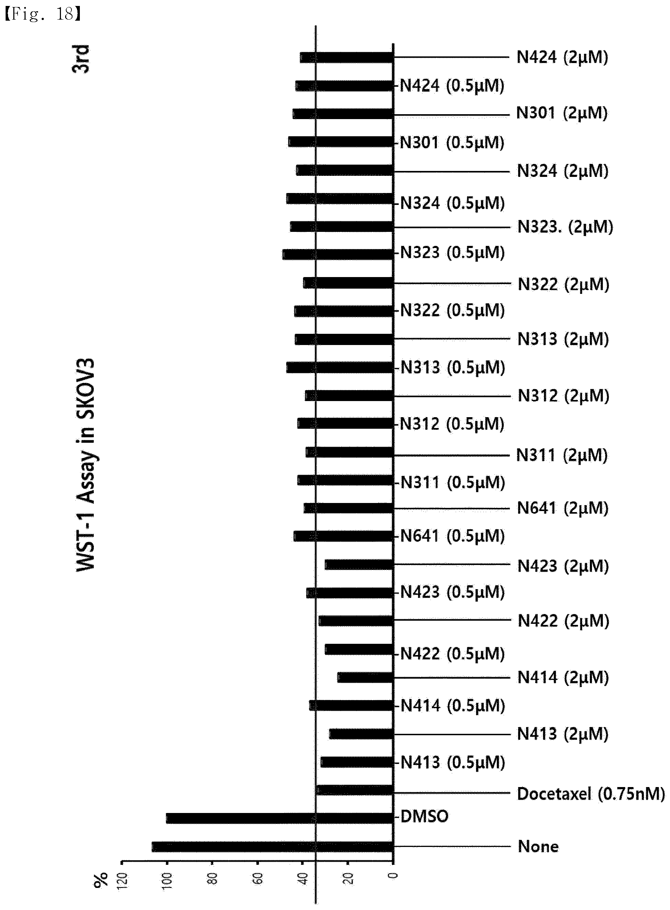

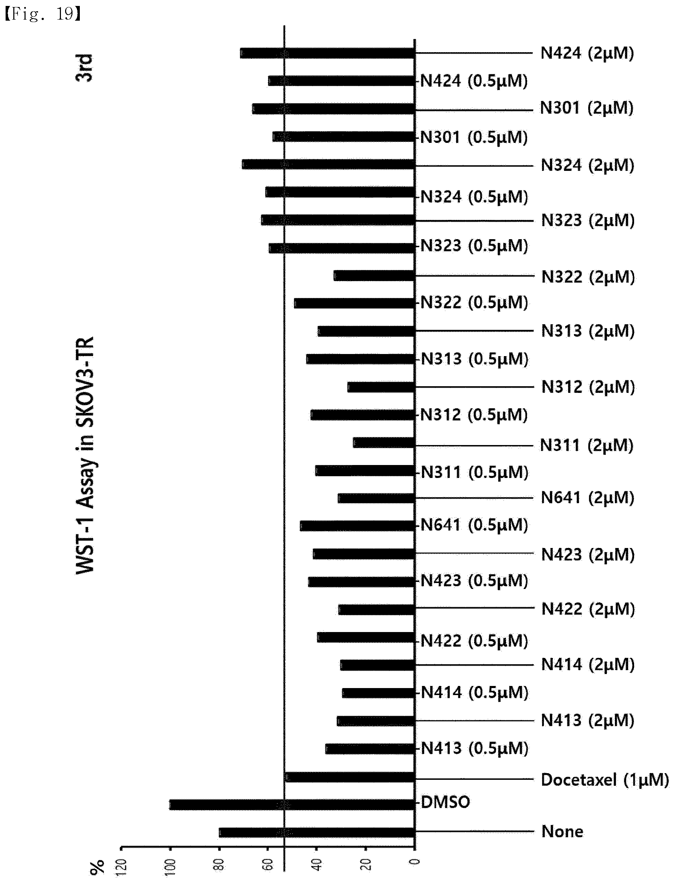

FIGS. 18 and 19 show None, DMSO, docetaxel, N413(0.5µM), N413(2µM), N414(0.5µM), N414(2µM), N422(0.5µM), N422(2µM), N423(0.5µM), N423(2µM), N641(0.5µM), N641(2µM), N311(0.5µM), N311(2µM), N312(0.5µM), N312(2µM), N313(0.5µM), N313(2µM), N322(0.5µM), N322(2µM), N323(0.5µM), N323(2µM), N324(0.5pM), N324(2µM), N301(0.5µM), N301(2µM), N424(0.5µM) and N424(2µM) in this order.

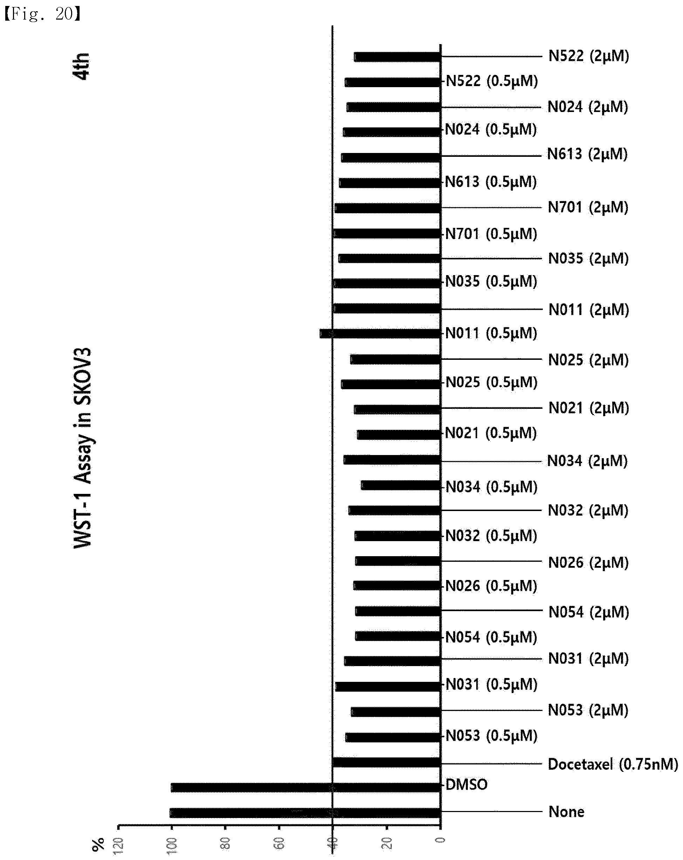

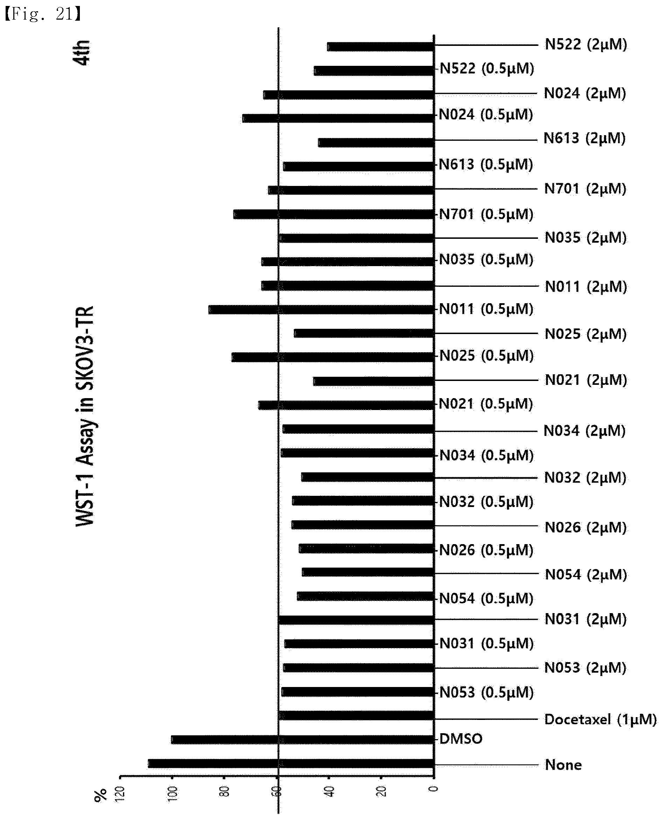

FIGS. 20 and 21 show None, DMSO, docetaxel, N053(0.5µM), N053(2µM), N031(0.5µM), N031(2µM), N054(0.5µM), N054(2µM), N026(0.5µM), N026(2µM), N032(0.5µM), N032(2µM), N034(0.5µM), N034(2µM), N021(0.5µM), N021(2µM), N025(0.5µM), N025(2µM), N011(0.5µM), N011(2µM), N035(0.5µM), N035(2µM), N701(0.5µM), N701(2µM), N613(0.5µM), N613(2µM), N024(0.5µM), N024(2µM), N522(0.5µM) and N522(2µM) in this order.

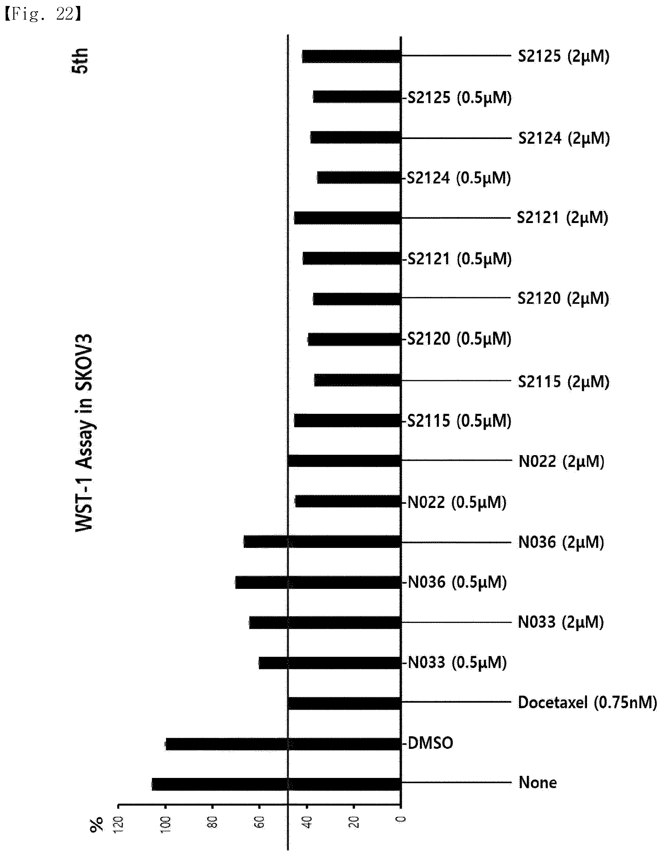

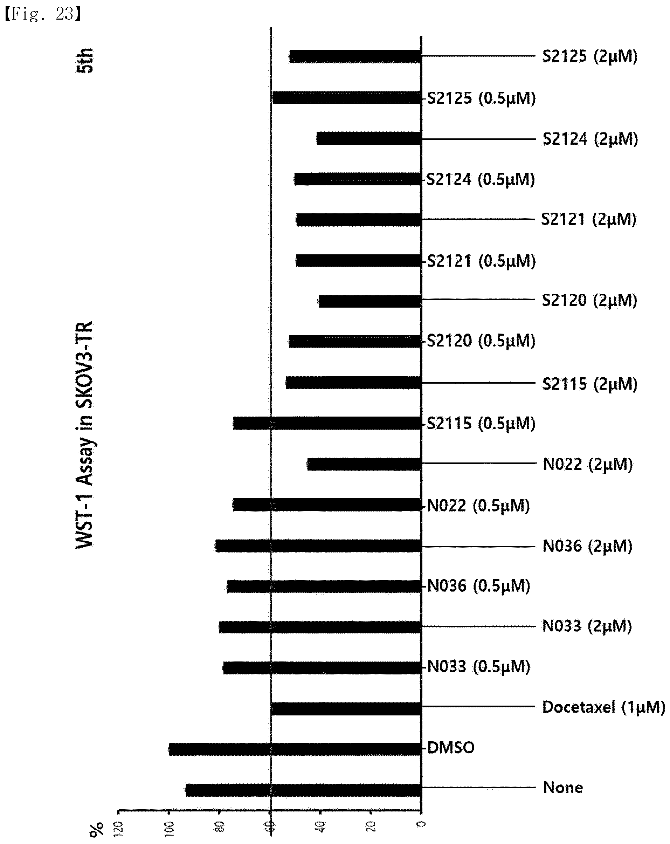

FIGS. 22 and 23 show None, DMSO, docetaxel, N033(0.5µM), N033(2µM), N036(0.5µM), N036(2µM), N022(0.5µM), N022(2µM), S2115(0.5µM), S2115(2µM), S2120(0.5µM), S2120(2µM), S2121(0.5pM), S2121(2µM), S2124(0.5pM), S2124(2µM), S2125(0.5µM) and S2125(2µM) in this order.

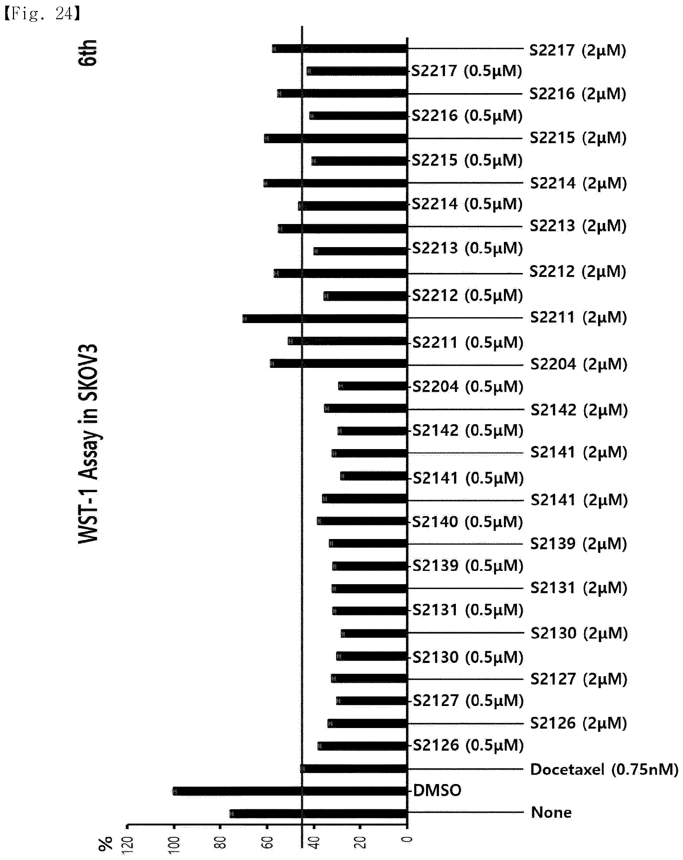

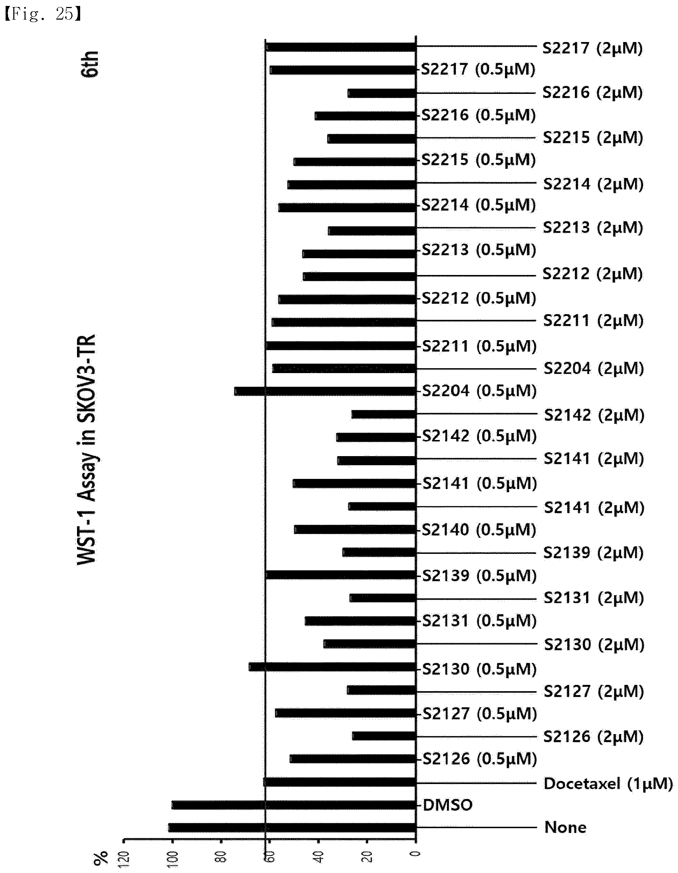

FIGS. 24 and 25 show None, DMSO, docetaxel, S2126(0.5µM), S2126(2µM), S2127(0.5µM), S2127(2µM), S2130(0.5µM), S2130(2µM), S2131(0.5µM), 82131(2µM), S2139(0.5µM), S2139(2µM), S2140(0.5µM), S2140(2µM), S2141(0.5µM), S2141(2µM), S2142(0.5µM), S2142(2µM), S2204(0.5µM), S2204(2µM), 82211(0.5µM), S2211(2µM), S2212(0.5µM), S2212(2µM), S2213(0.5µM), S2213(2µM), S2214(0.5µM), S2214(2µM), S2215(0.5pM), S2215(2µM), S2216(0.5µM), S2216(2µM), S2217(0.5µM) and S2217(2µM) in this order.

That is, in the docetaxel-treated group, due to phenomena such as the induction of cell death and the inhibition of cell growth in SKOV3 and SKOV3-TR, the absorbance at 450 nm was reduced compared to the DMSO-treated group. When the compounds were co-treated with docetaxel, in the resistant cancer cell line SKOV3-TR, the anticancer efficacy of docetaxel was enhanced, thereby exhibiting a lower absorbance than the docetaxel-treated group, in SKOV3, the anticancer efficacy of docetaxel was not enhanced. In addition, in SKOV3-TR, the docetaxel-induced reduction in metabolism is further promoted in the presence of the 2 µM compound compared to the 0.5 µM compound, showing that a dose-response relationship is established. A baseline was applied at the absorbance of the docetaxel -treated group, which is a comparative example. For the enhanced anticancer efficacy of docetaxel, the absorbances (OD values at 450 nm) were significantly reduced in the WST-1 assay by the docetaxel+compound treatment, compared to the docetaxel-treated group.

It should be understood by those of ordinary skill in the art that the above description of the present application is exemplary, and the exemplary embodiments disclosed herein can be easily modified into other specific forms without departing from the technical spirit or essential features of the present application. Therefore, it should be interpreted that the exemplary embodiments described above are exemplary in all aspects, and are not restrictive. The scope of the present application is defined by the appended claims and encompasses all modifications and alterations derived from meanings, the scope and equivalents of the appended claims.

## Claims

1. A compound represented by Formula 1 below or a pharmaceutically acceptable salt thereof: 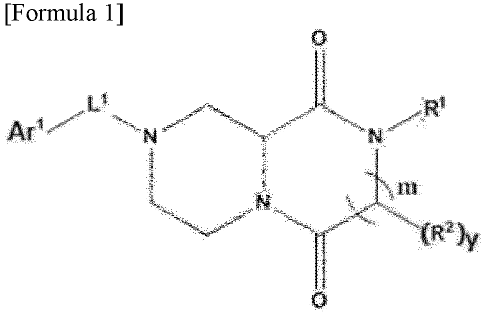 In Formula 1,
L¹ is a direct bond or C₁₋₁₀ alkylene;
Ar¹ is C₁₋₁₂ alkyl, or 5- to 16-membered monocyclic, bicyclic, tricyclic, or tetracyclic ring which contains 0 to 3 heteroatoms independently selected from O, N or S, wherein the ring is un substituted or substituted with groups independently selected from C₁₋₆ haloalkyl, halogen, oxo, - OCHF₂, -CN, nitro, -C(=O)NZ^{a}Z^{a}, -C(=O)Z^{b}, -C(=O)OZ^{b}, -C(=NZ^{a})NZ^{a}Z^{a}, -OZ^{a}, -OC(=O)Z^{b}, - OC(=O)NZ^{a}Z^{a}, -O-C₁₋₆ alkyl N(Z^{a})C(=O)OZ^{b}, -OC(=O)N(Z^{a})S(=O)₂Z^{b}, -OC₂₋₆ alkyl NZ^{a}Z^{a}, - OC₂₋₆ alkyl OZ^{a}, -SZ^{a}, -S(=O)Z^{b}, -S(=O)₂Z^{b}, -S(=O)₂NZ^{a}Z^{a}, -S(=O)₂N(Z^{a})C(=O)Z^{b}, - S(=O)₂N(Z^{a})C(=O)OZ^{b}, -S(=O)₂N(Z^{a})C(=O)NZ^{a}Z^{a}, -NZ^{a}Z^{a}, -NZ^{c}Z^{c}, -N(Z^{a})C(=O)Z^{b}, - N(Z^{a})C(=O)OZ^{b}, -N(Z^{a})C(=O)NZ^{a}Z^{a}, -N(Z^{a})C(=NZ^{a})NZ^{a}Z^{a}, -N(Z^{a})S(=O)₂Z^{b}, - N(Z^{a})S(=O)₂NZ^{a}Z^{a}, -NZ^{a}C₂₋₆ alkyl NZ^{a}Z^{a}, -NZ^{a}C₂₋₆ alkyl OZ^{a}, C₆₋₁₀ aryl, C₅₋₁₀ heteroaryl, -C₁₋₆ alkyl, -C₃₋₁₀ cycloalkyl, -C₂₋₆ alkenyl and -C₂₋₆ alkynyl (here, -C₁₋₆ alkyl, -C₃₋₁₀ cycloalkyl, -C₂₋₆ alkenyl or -C₂₋₆ alkynyl is substituted with 0 to 3 substituents independently selected from C₁₋₆ haloalkyl, halogen, cyano, nitro, C₆₋₁₂ aryl or C₅₋₁₂ heteroaryl);
Z^{a} is each independently hydrogen or Z^{b};
Z^{b} is each independently phenyl, benzyl, C₁₋₆ alkyl, C₄₋₈ heterocycloalkyl, or C₃₋₈ cycloalkyl, wherein the phenyl, benzyl, C₁₋₆ alkyl, C₄₋₈ heterocycloalkyl, or C₃₋₈ cycloalkyl is substituted with 0 to 3 substituents independently selected from halogen, -OH, -S(=O)₂Z^{b}, -OC₂₋₆ alkyl OZ^{a}, C₁₋₆ alkyl, C₁₋₆ haloalkyl, -OC₁₋₄ alkyl, -NH₂, -CN, or -NZ^{a}Z^{a}_{;}
Z^{c} is each independently hydrogen or C₁₋₆ alkyl, or the group CZ^{c}Z^{c} forms a C₃₋₈ cycloalkyl ring;
m and y are each independently an integer of 0 to 2; and
R¹ or R² is each independently hydrogen, C₁₋₆ alkyl, C₁₋₆ alkoxy, C₁₋₆ alkoxycarbonyl, halo C₁₋₆ alkyl, C₂₋₆ alkenyl, or a C₄₋₁₅ monocyclic or bicyclic ring (which is C₃₋₁₀ cycloalkyl; C₆₋₁₂ aryl; 5- to 6-membered saturated or partially saturated hetero ring containing 0 to 3 heteroatoms, independently selected from N, O and S; 5- or 6-membered aromatic hetero ring containing 0 to 3 heteroatoms (provided that two or more of the heteroatoms are not O or S), independently selected from N, O and S; or 7- to 15-membered saturated, partially saturated, or unsaturated hetero ring containing 0 to 3 heteroatoms, independently selected from N, O and S),
wherein R¹ and R^{Z} are connected to form a 5- to 12-membered monocyclic or bicyclic ring.

2. The compound of claim 1,
wherein the compound represented by Formula 1 is any one of the compounds of Formulas 2 to 10: 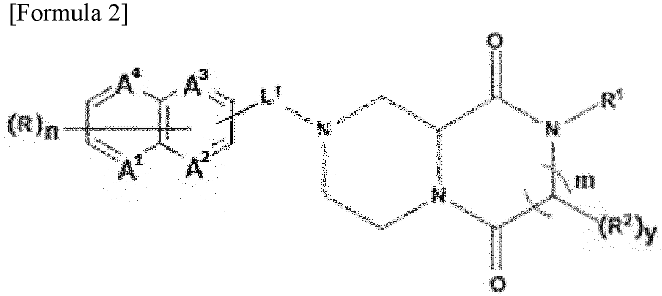 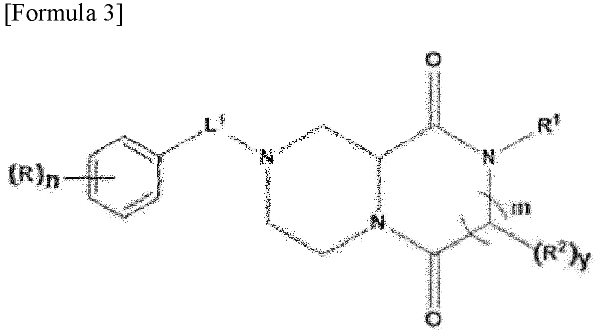 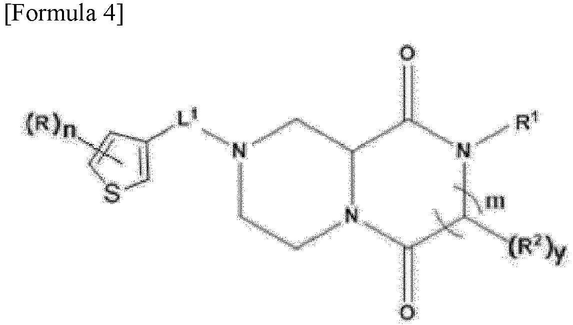 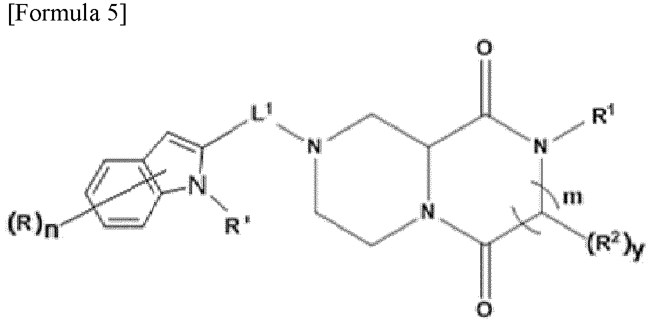 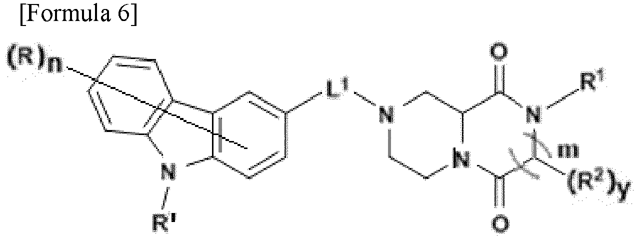 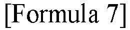 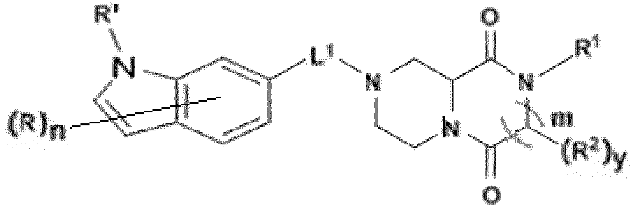 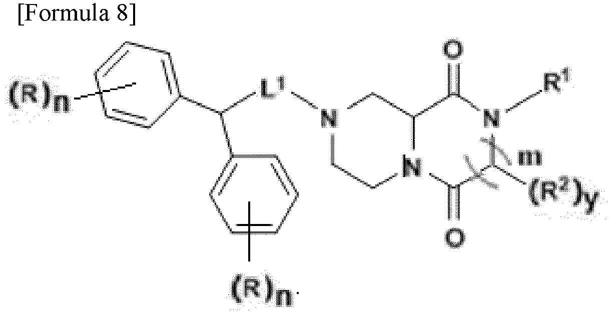 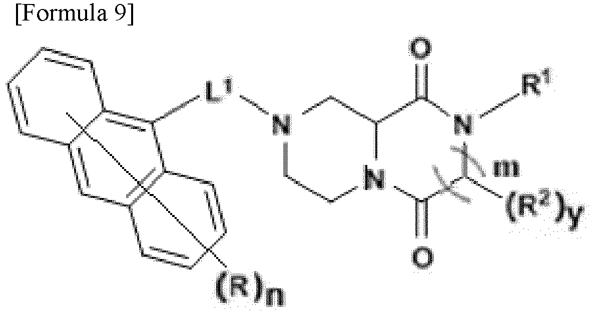 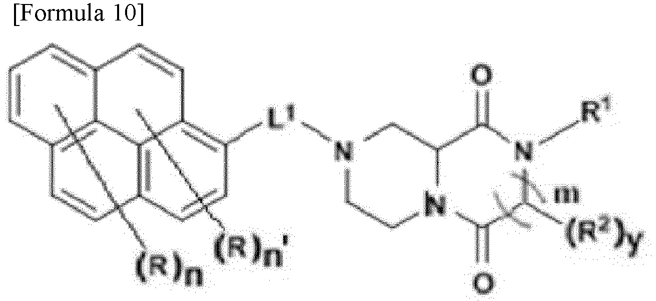 In Formulas 2 to 10,
L¹ is a direct bond or C₁₋₆ alkylene;
A¹, A², A³, or A⁴ is each independently CR or N,
R or R' is each independently hydrogen, hydroxyl, halogen, C₆₋₁₂ aryl, C₅₋₁₂ heteroaryl, - C₁₋₆ alkyl, -C₃₋₁₀cycloalkyl, -C₁₋₆ alkoxy, -C₂₋₆ alkenyl or -C₂₋₆ alkynyl (here, -C₁₋₆ alkyl, -C₃₋₁₀cycloalkyl, -C₂₋₆ alkenyl or -C₂₋₆ alkynyl is substituted with 0 to 3 substituents independently selected from C₁₋₆ haloalkyl, halogen, cyano, nitro, C₆₋₁₂ aryl, or C₅₋₁₂ heteroaryl);
n and n' are each independently an integer of 0 to 13;
m and y are each independently an integer of 0 to 2;
R¹ or R² is each independently hydrogen, C₁₋₆ alkyl, C₁₋₆ alkoxy, C₁₋₆ alkoxycarbonyl, halo C₁₋₆ alkyl, C₂₋₆ alkenyl, C₄₋₁₅ monocyclic or bicyclic ring (which is C₃₋₁₀cycloalkyl; C₆₋₁₀ aryl; 5- or 6-membered saturated or partially saturated hetero ring containing 0 to 3 heteroatoms, independently selected from N, O and S; 5- or 6-membered aromatic hetero ring containing 0 to 3 heteroatoms (provided that two or more of the heteroatoms are not O or S), independently selected from N, O and S; or 7- to 15-membered saturated, partially saturated, or unsaturated hetero ring containing 0 to 3 heteroatoms, independently selected from N, O and S);
wherein R¹ and R² are connected to form a 5- to 12-membered monocyclic or bicyclic ring.

3. The compound of claim 1,
wherein L¹ is a direct bond or C₁₋₃ alkylene;
Ar¹ is substituted with at least one of linear or branched C₁₋₆ alkyl, naphthyl, phenyl, naphthyl, anthracenyl, phenanthrenyl, pyrenyl, furanyl, indole, chromone, quinoline, carbazole or thiophenyl, which is unsubstituted or substituted with at least one of hydroxyl, halogen, C₁₋₆ alkyl, C₃₋₁₀ocycloalkyl, C₁₋₆ alkoxy, C₁₋₆ haloalkyl, C₁₋₆ haloalkoxy, C₂₋₆ alkenyl, -C₂₋₆ alkynyl, C₆₋₁₂ aryl, or C₅₋₁₂ heteroaryl;
m is 1,
y is 1 or 2,
R¹ or R₂ is each independently hydrogen, C₁₋₆ alkyl, benzyl, naphthylalkyl, benzofuranylalkyl, quinolinylalkyl, pyridinylalkyl, cyclohexyl alkyl, thiophenylalkyl, pyrrolylalkyl, furanylalkyl or benzothiophenylalkyl, which is unsubstituted or is each independently substituted with at least one of C₁₋₆ alkyl, C₃₋₆ cycloalkyl, C₆₋₁₂ aryl, hydroxyl, halogen, C₁₋₆ alkoxy, C₁₋₆ haloalkyl, and C₁₋₆ haloalkoxy, and
R¹ and R² are connected to form a 5- to 12-membered monocyclic or bicyclic ring.

4. The compound of claim 1,
wherein L¹ is C₁₋₃ alkylene;
Ar¹ is C₁₋₆ linear or branched alkyl, 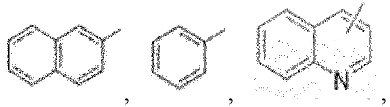 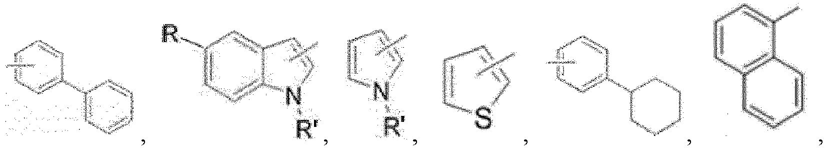 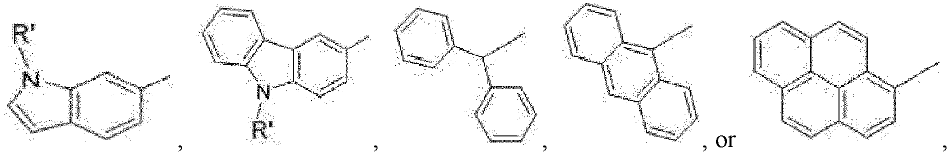
R and R' are each independently, hydrogen, halogen, C₆₋₁₂ aryl, C₅₋₁₂ heteroaryl, -C₁₋₆ alkyl, -C₃₋₁₀cycloalkyl, -C₁₋₆ alkoxy, -C₂₋₆ alkenyl or -C₂₋₆ alkynyl (here, -C₁₋₆ alkyl, -C₃₋₁₀cycloalkyl, -C₁₋₆ alkoxy, -C₂₋₆ alkenyl or -C₂₋₆ alkynyl is substituted with 0 to 3 substituents independently selected from C₁₋₆ haloalkyl, halogen, cyano, nitro, C₆₋₁₂ aryl, or C₅₋₁₂ heteroaryl);
m is 1,
y is 1 or 2,
R₁ or R₂ is each independently hydrogen, C₁₋₆ linear or branched alkyl 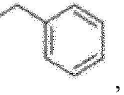 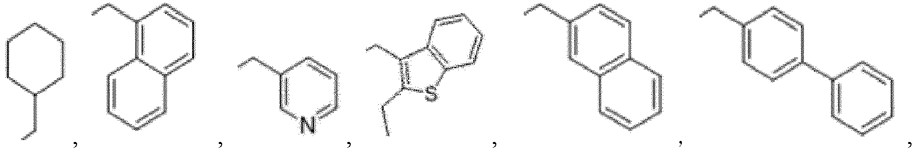 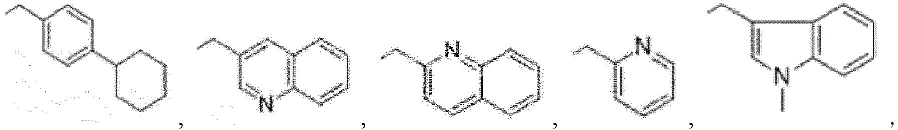 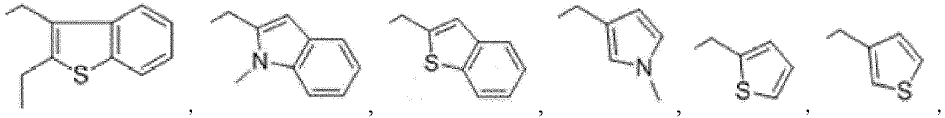 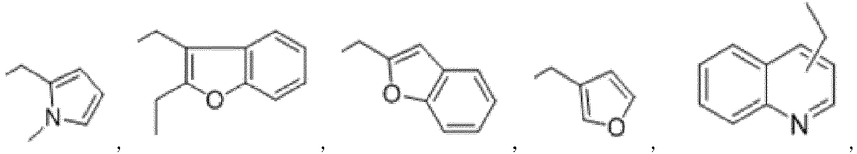 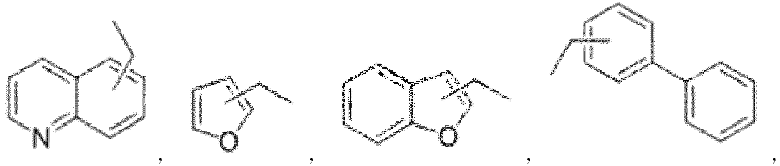 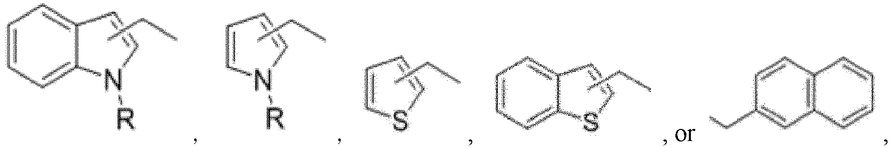
wherein R¹ and R² are connected 1q form 5- to 12-membered monocyclic or bicyclic ring.

5. The compound of claim 1,
wherein the compound is selected from the group consisting of the following compounds:

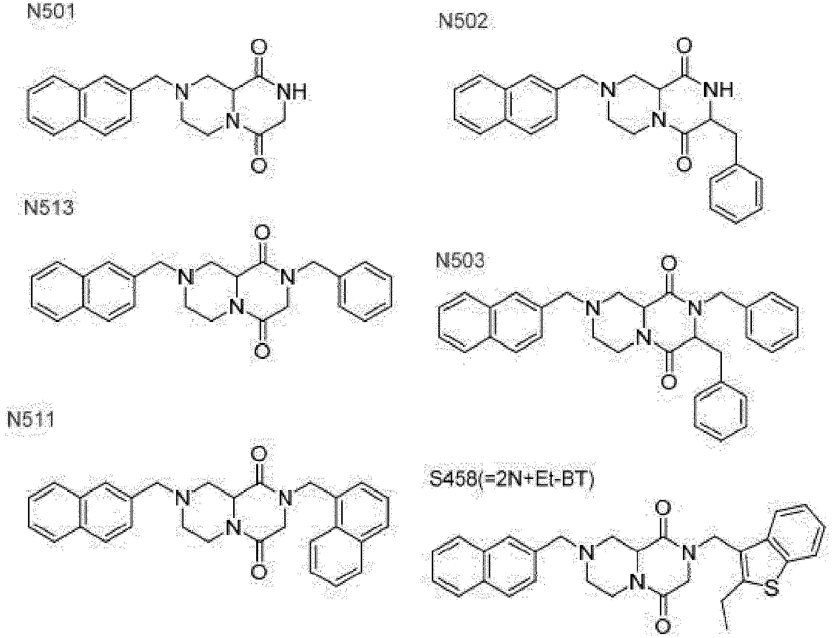

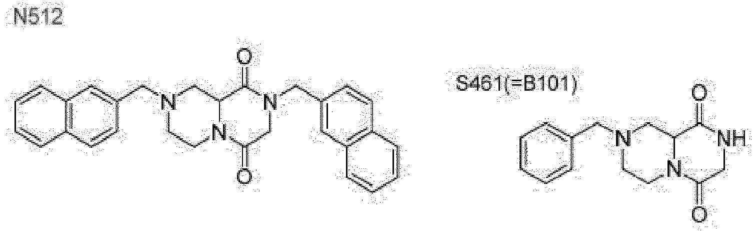

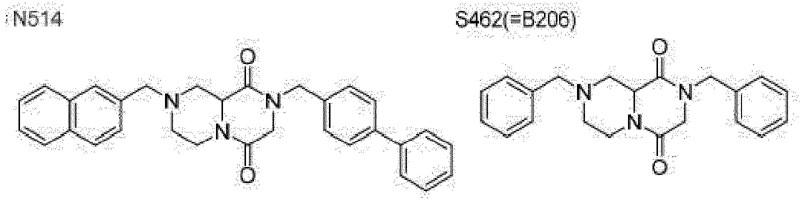

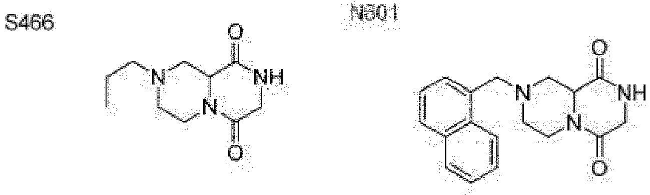

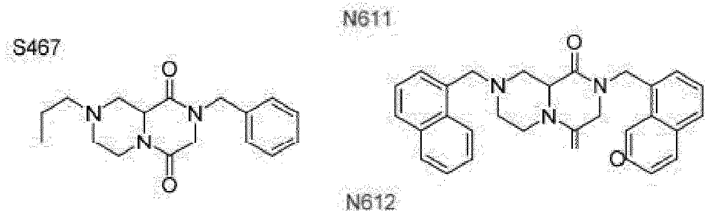

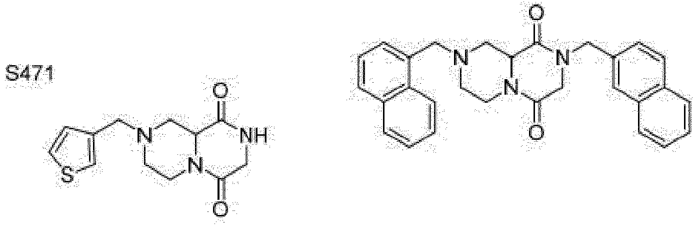

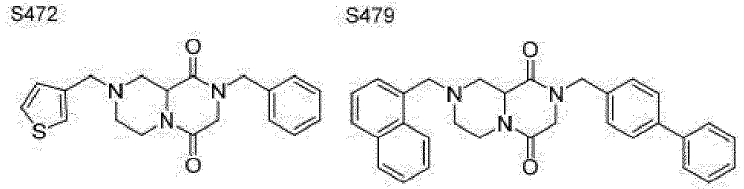

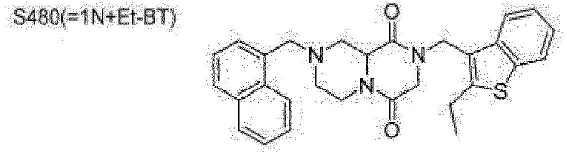

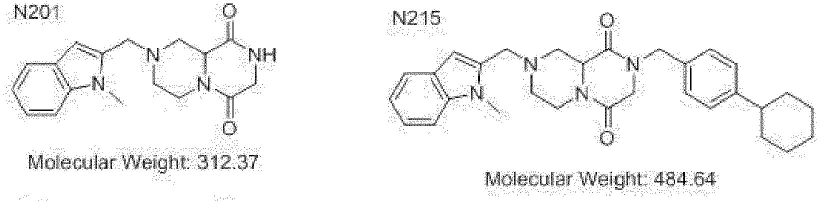

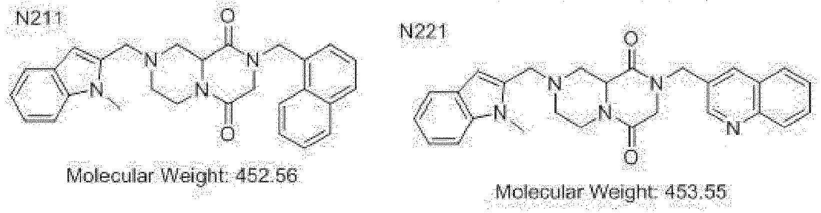

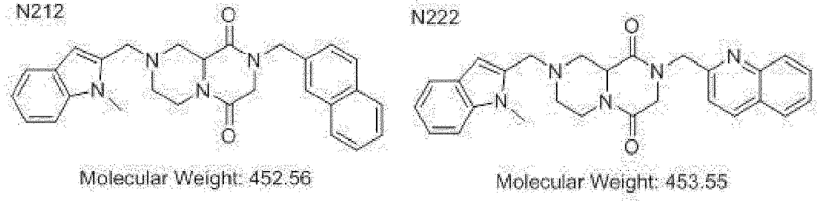

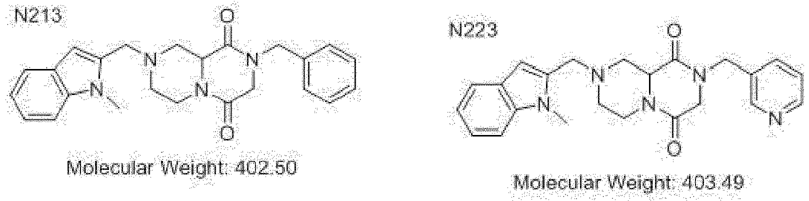

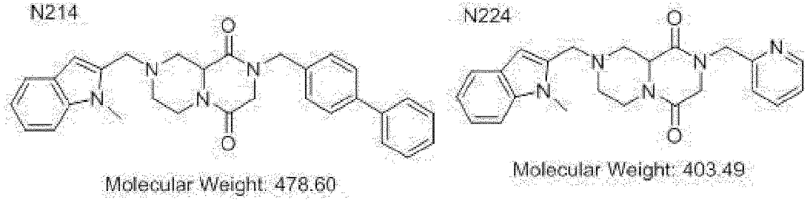

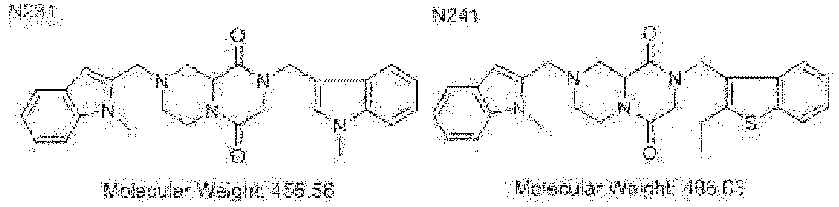

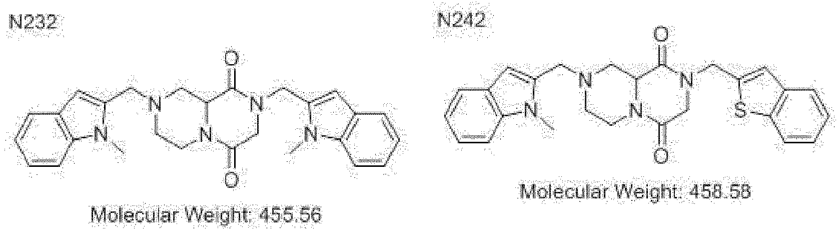

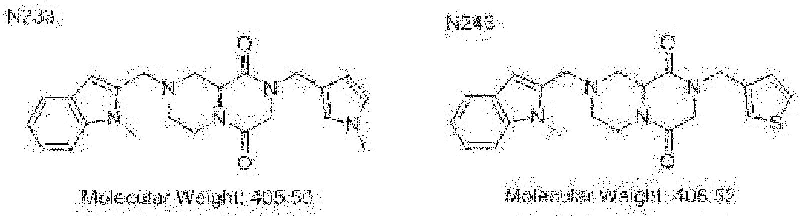

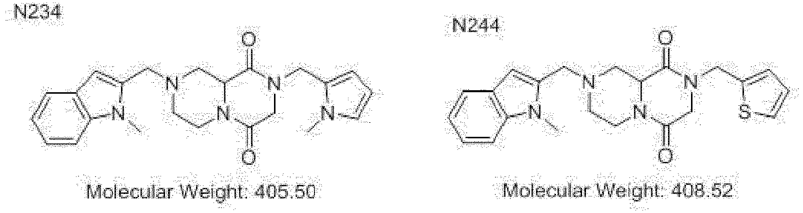

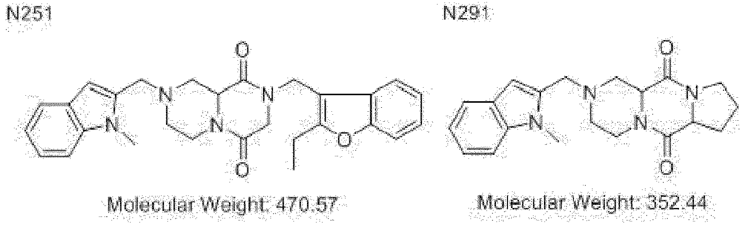

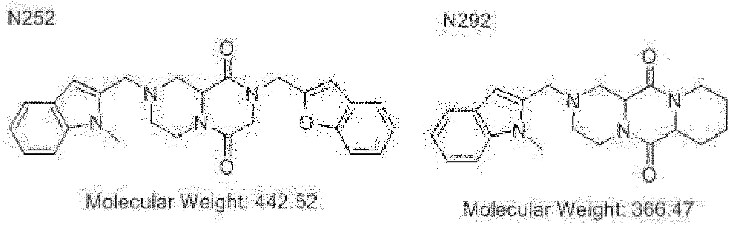

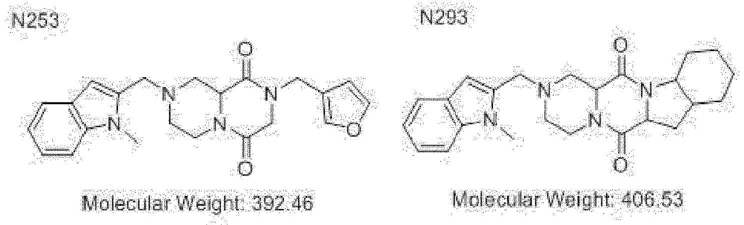

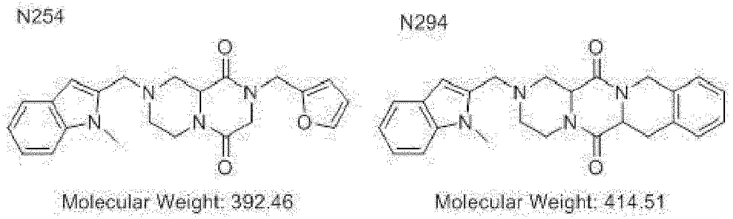

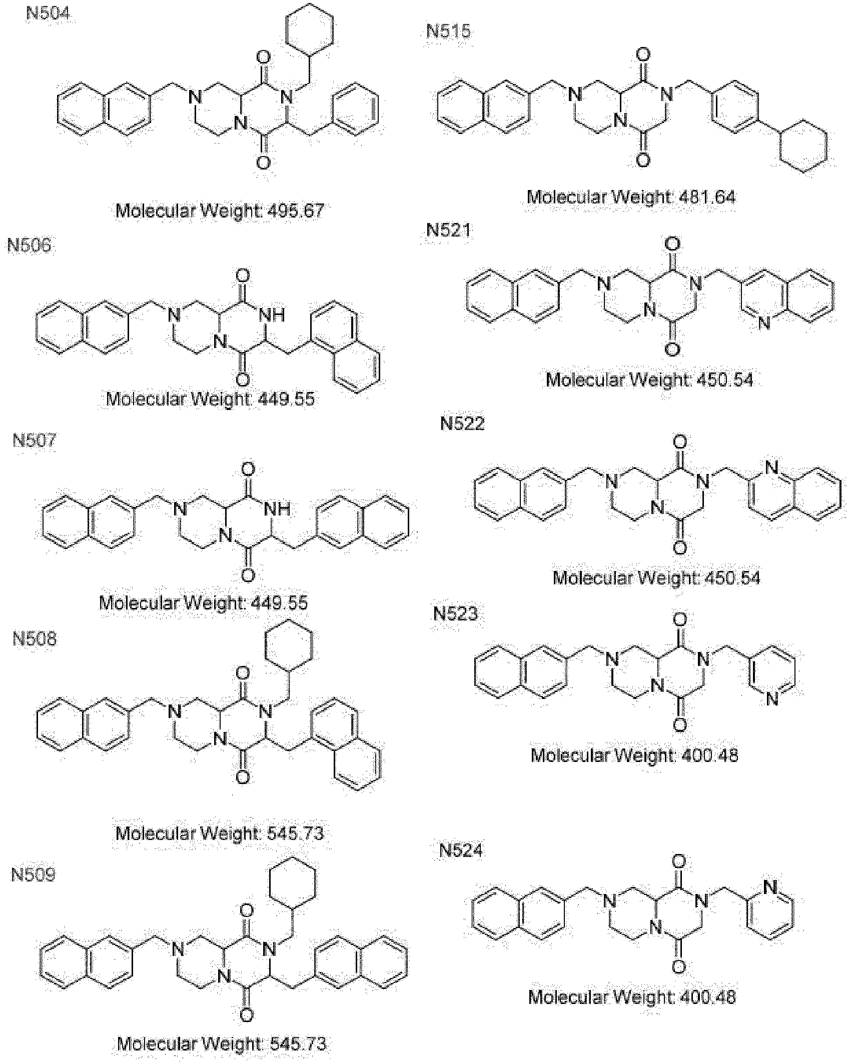

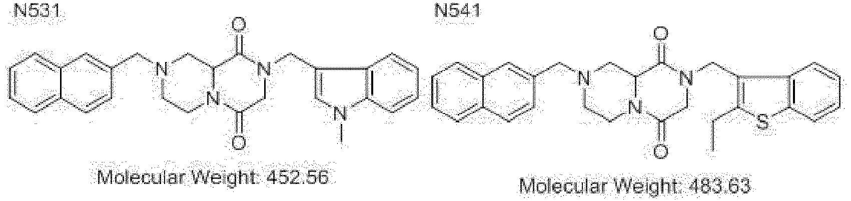

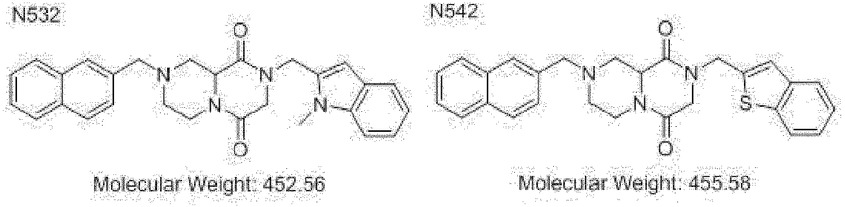

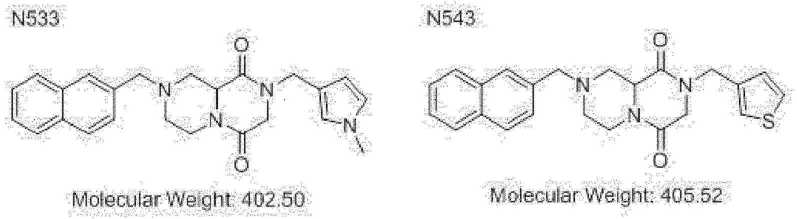

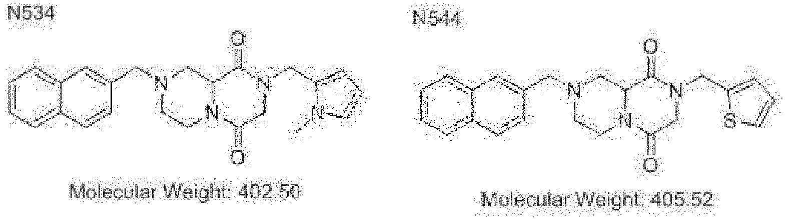

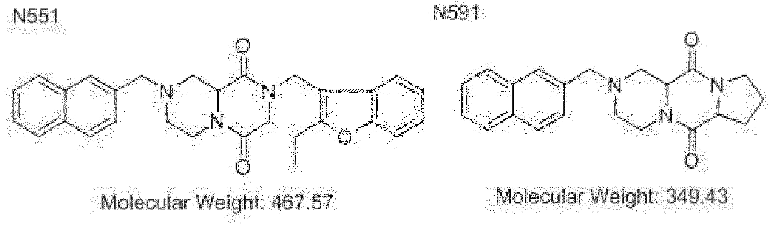

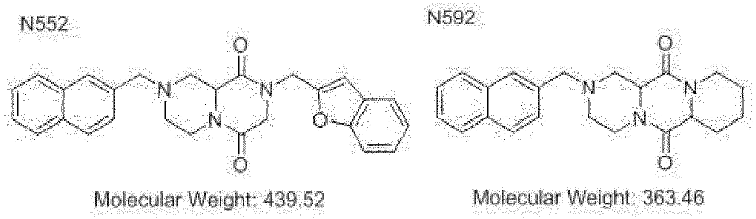

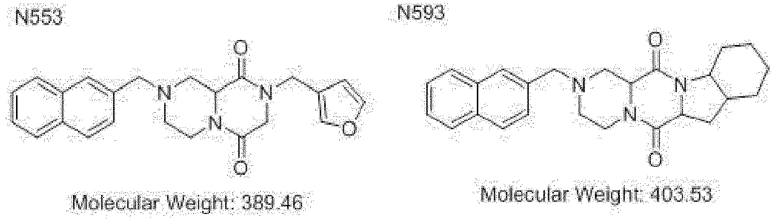

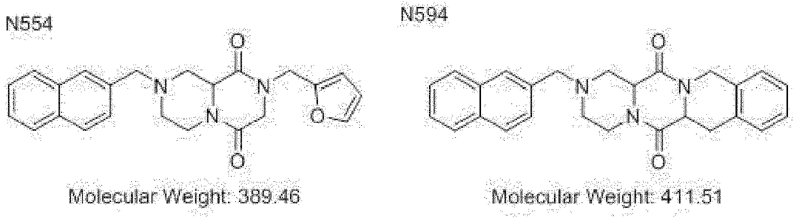

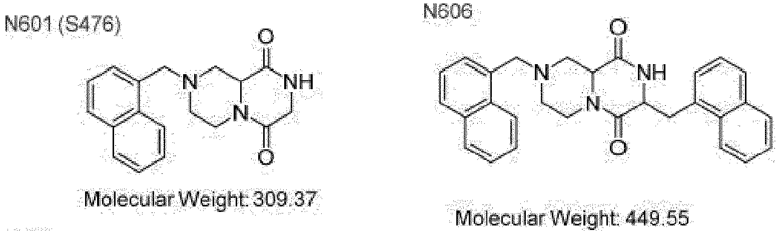

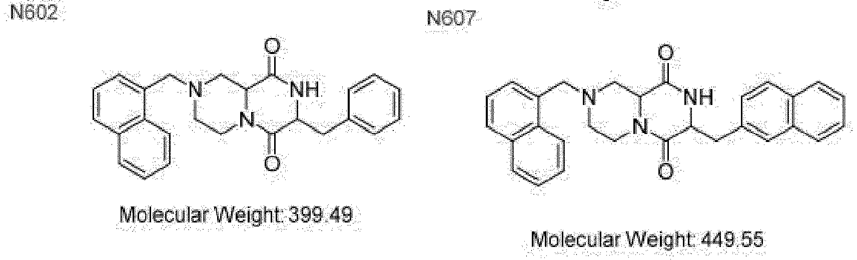

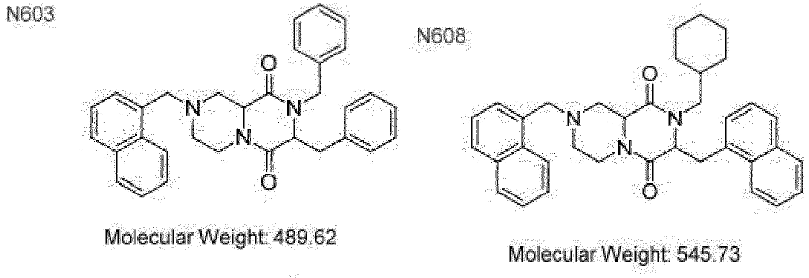

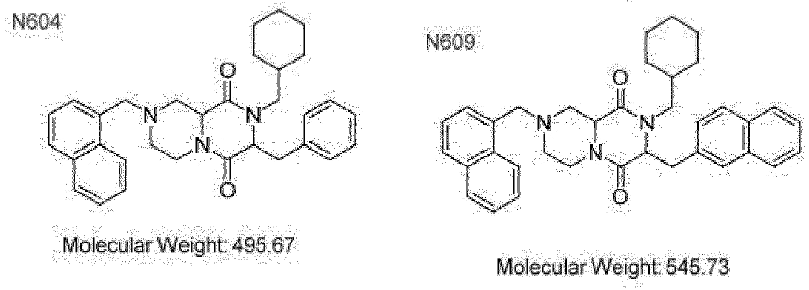

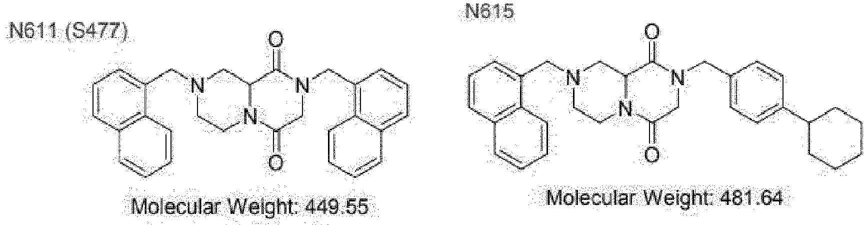

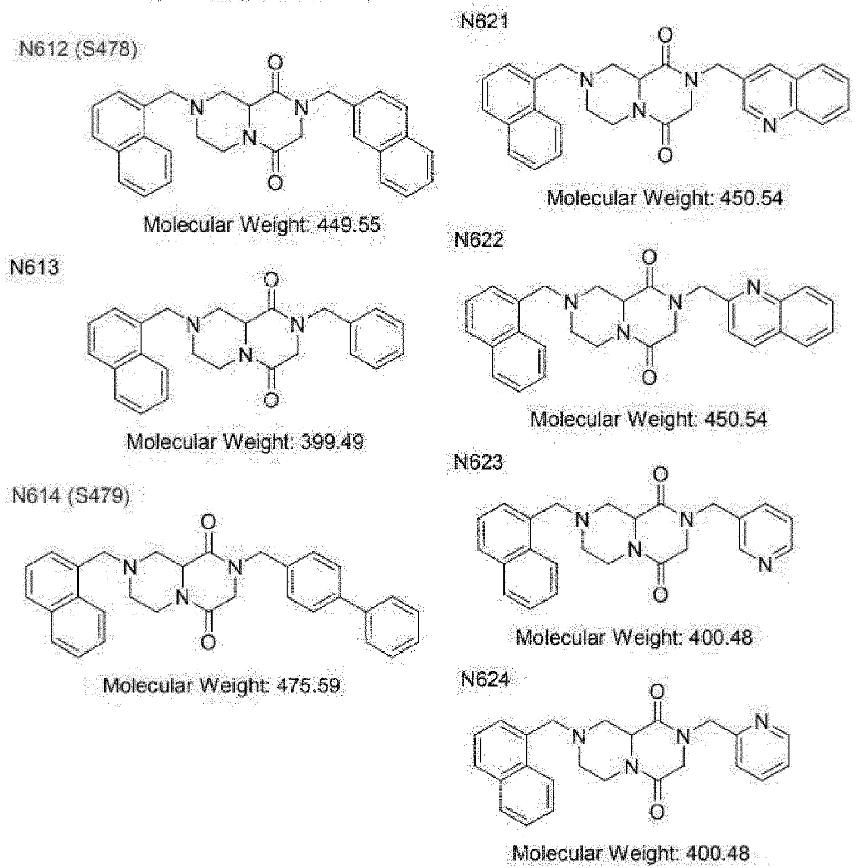

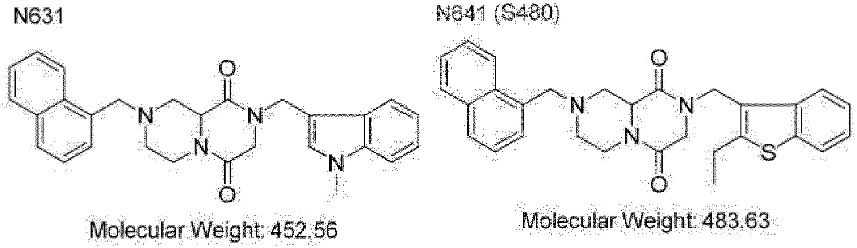

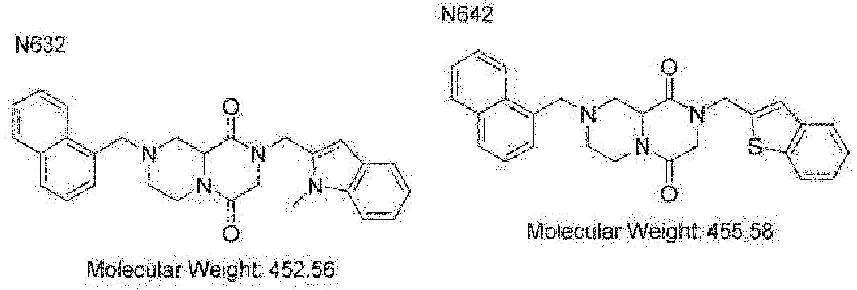

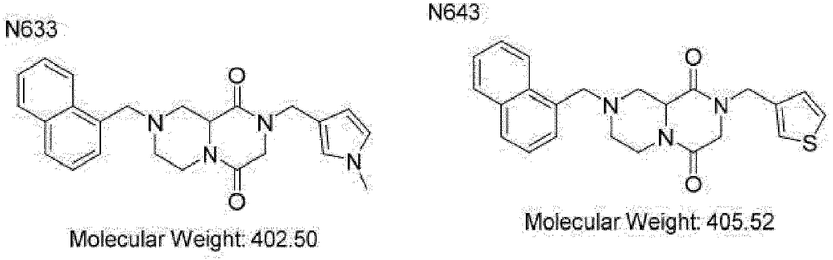

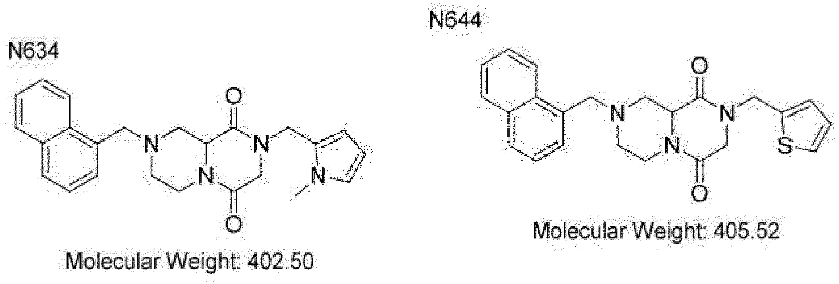

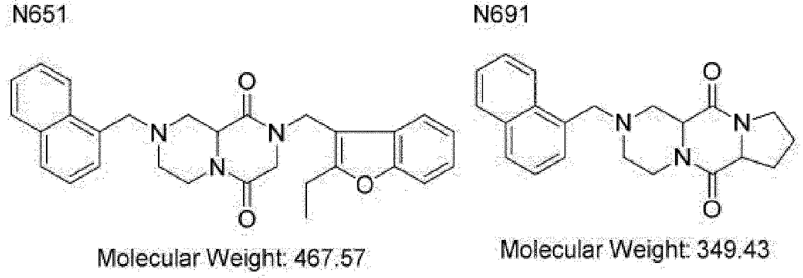

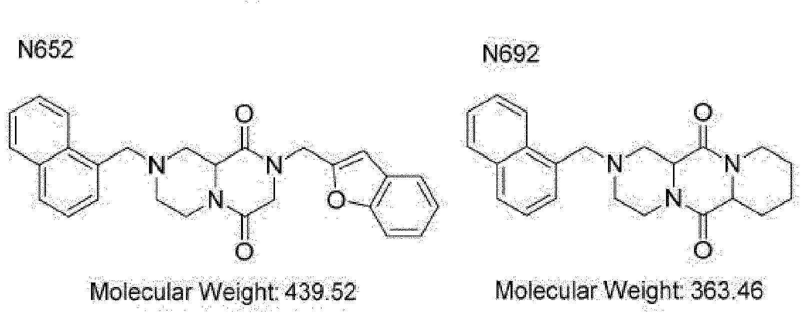

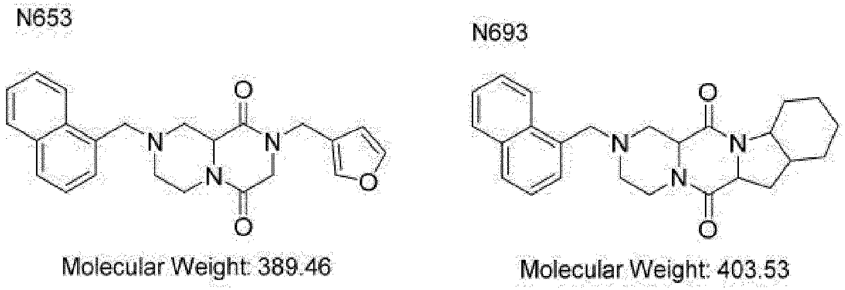

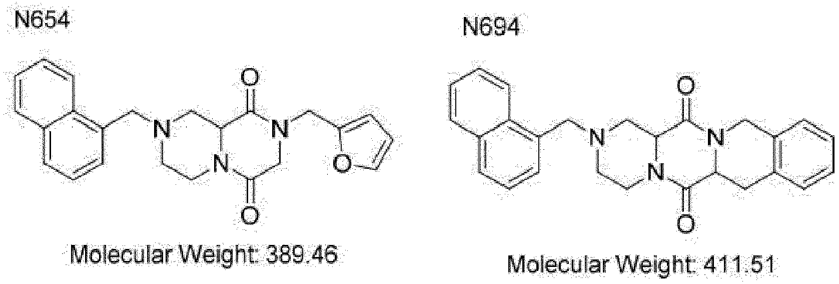

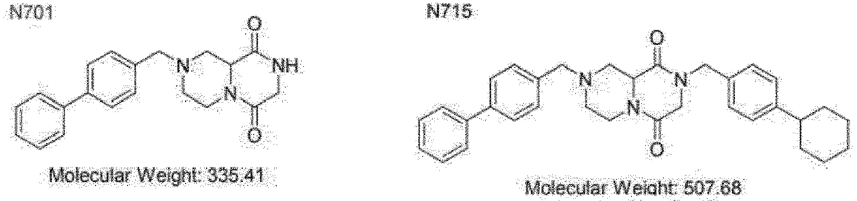

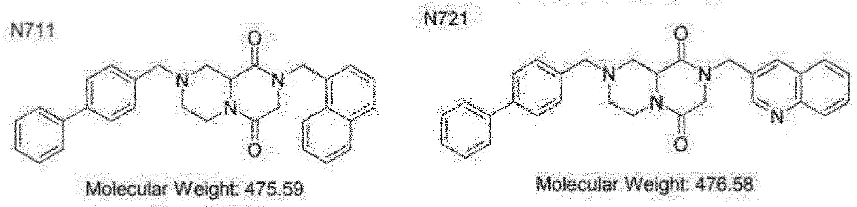

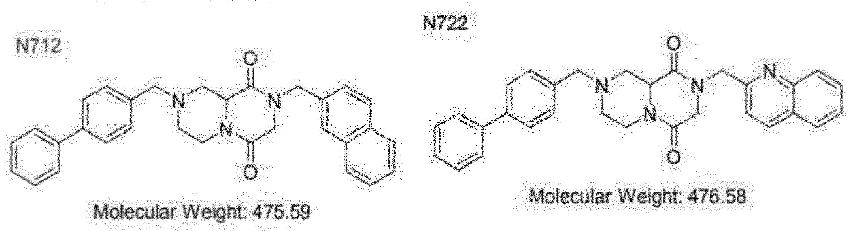

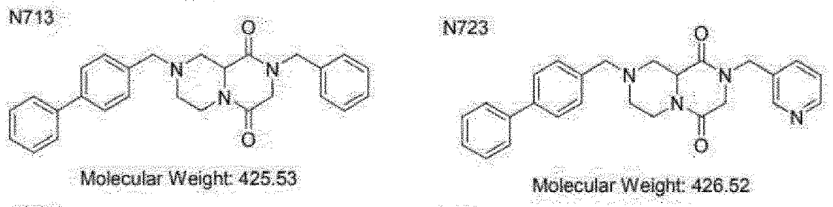

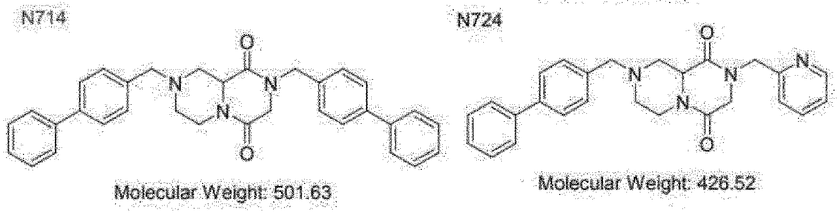

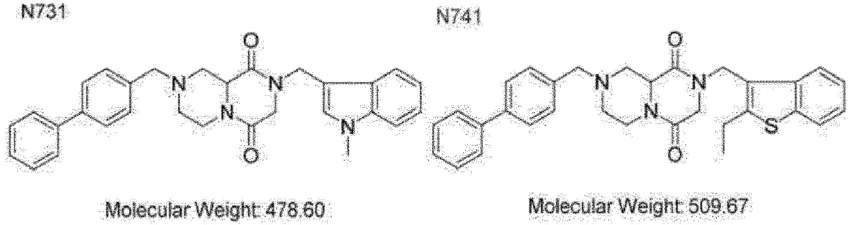

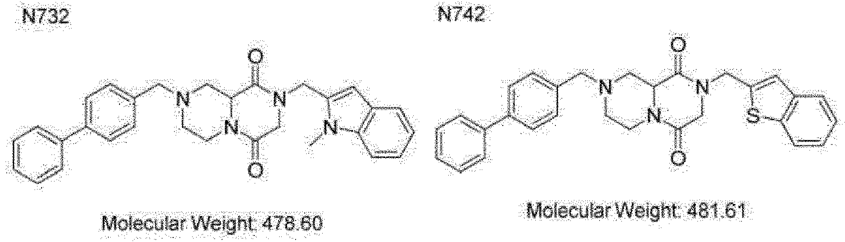

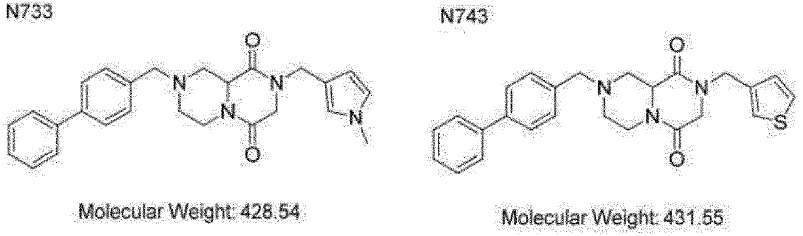

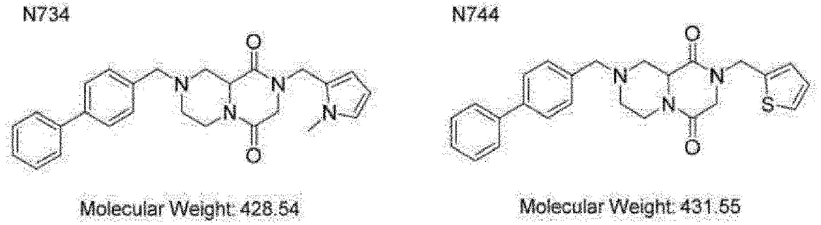

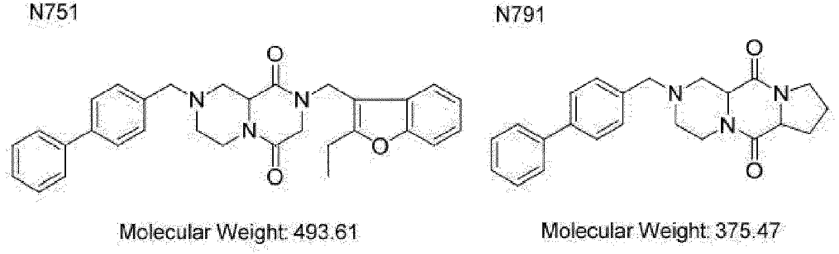

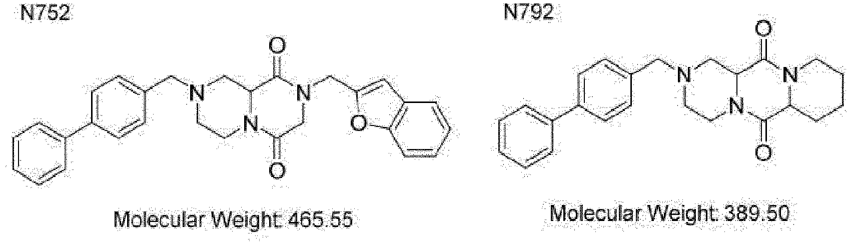

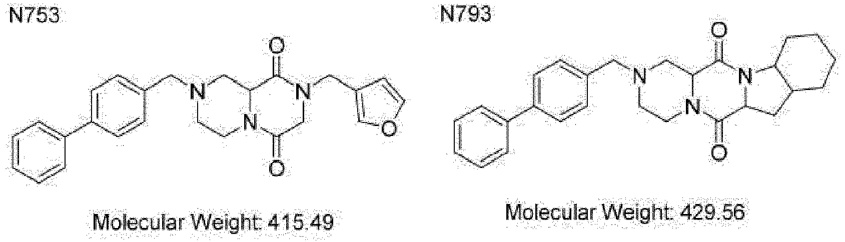

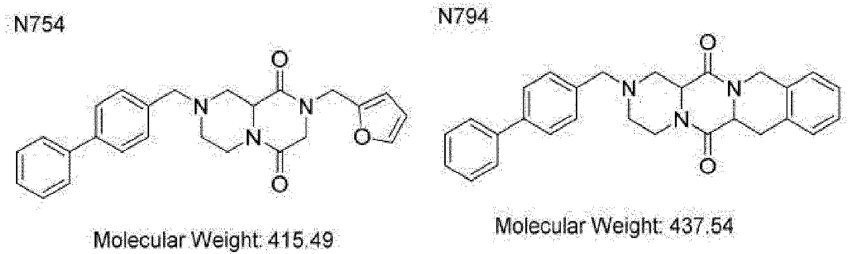

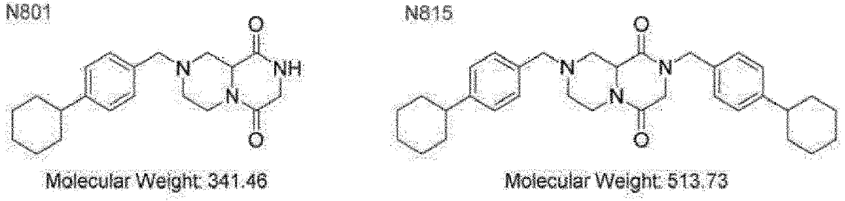

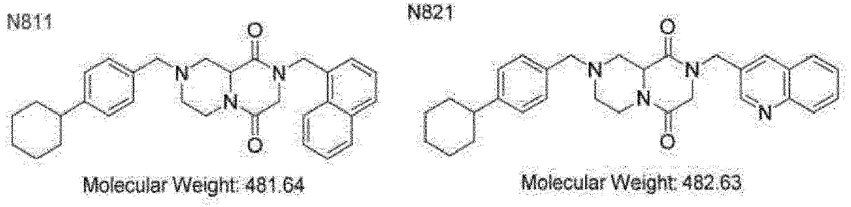

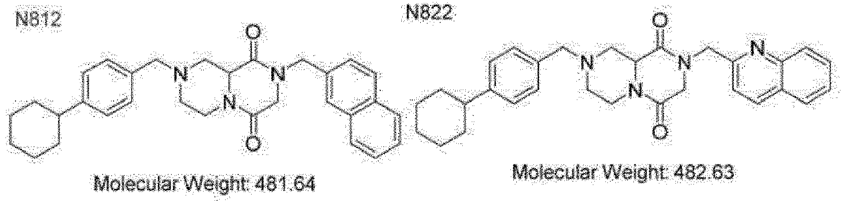

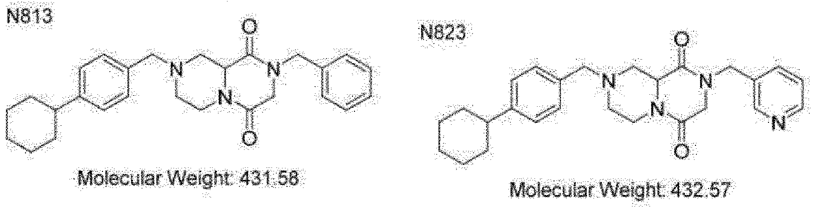

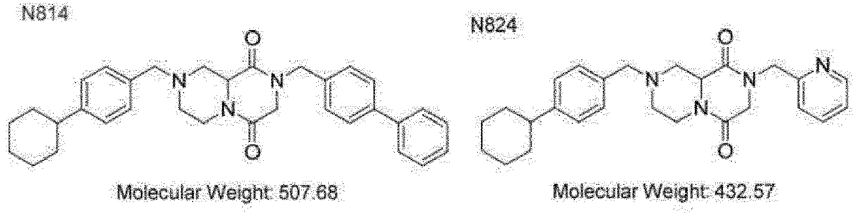

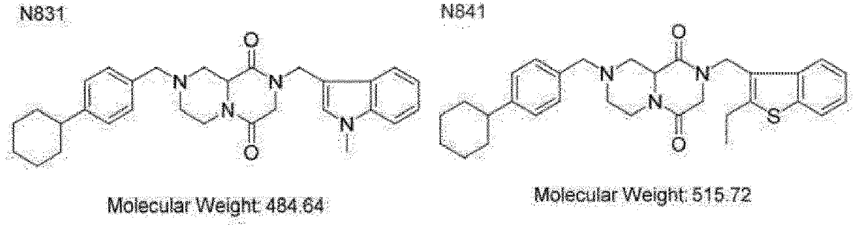

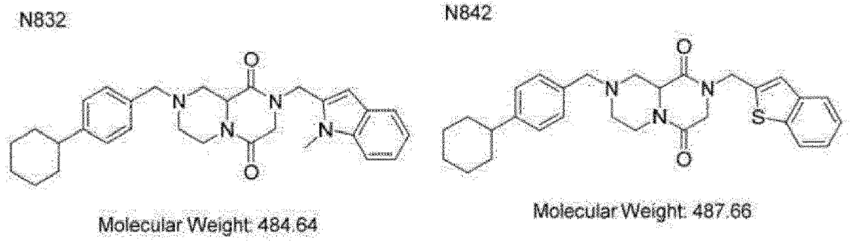

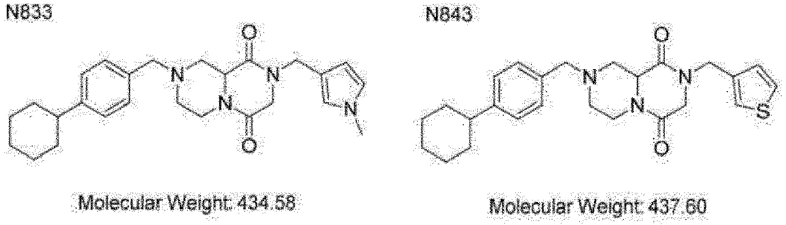

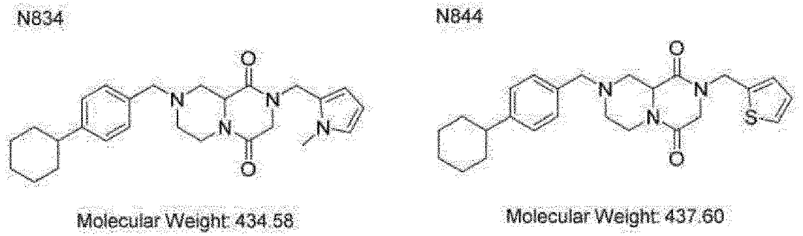

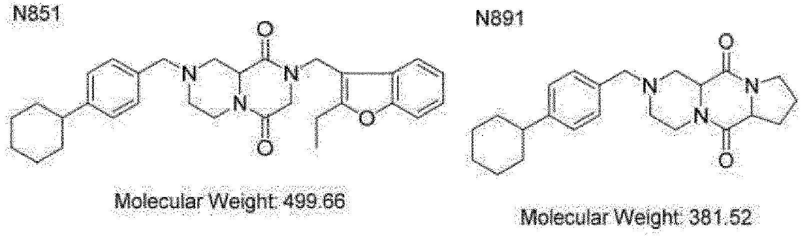

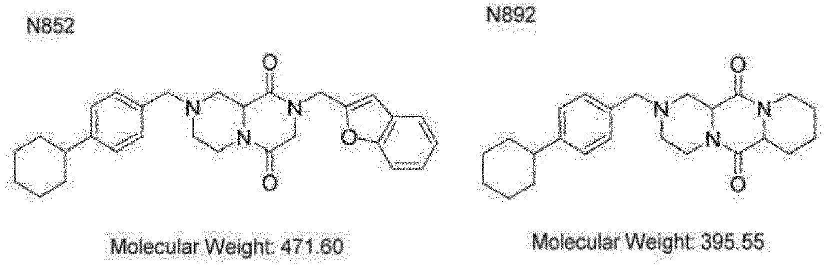

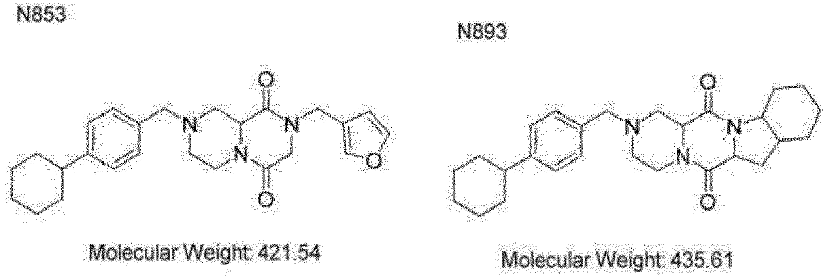

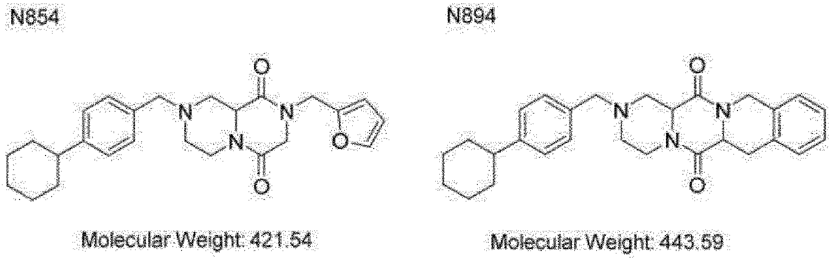

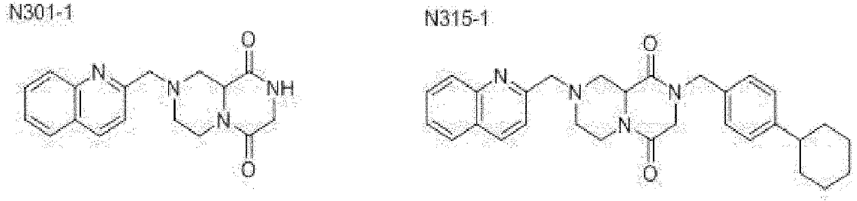

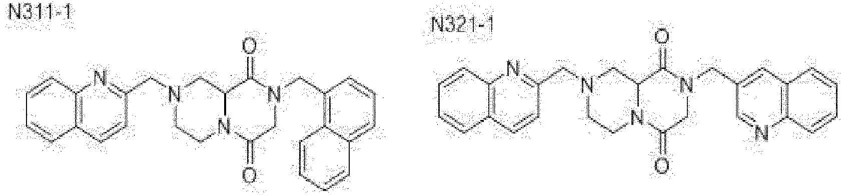

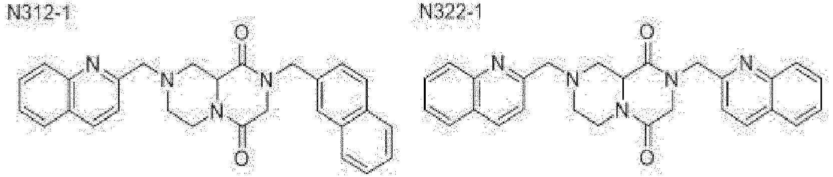

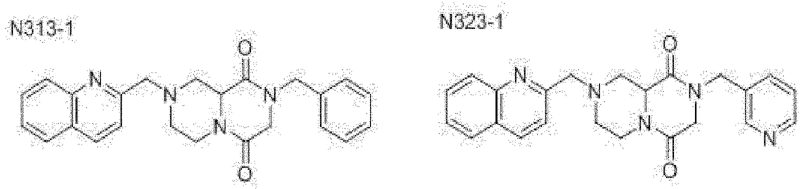

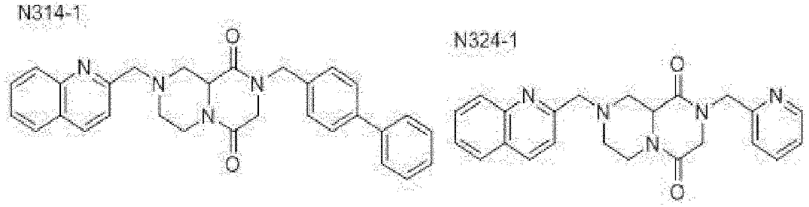

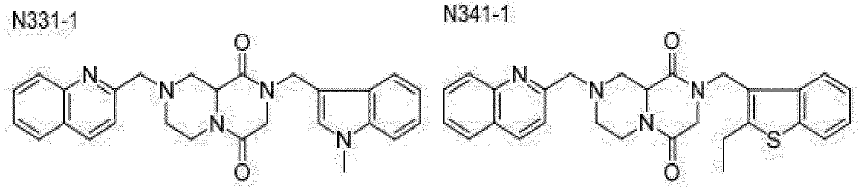

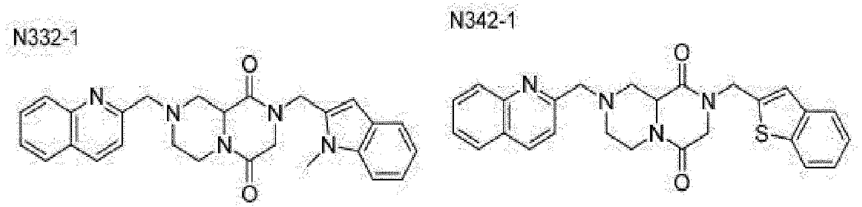

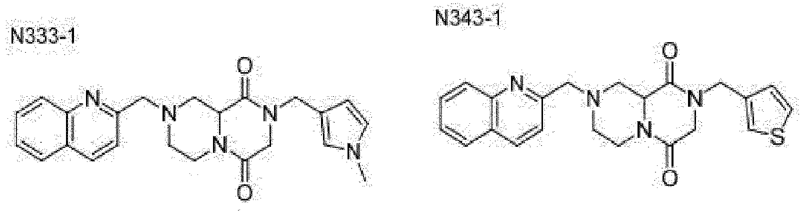

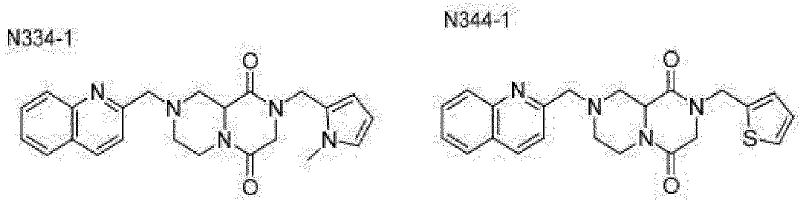

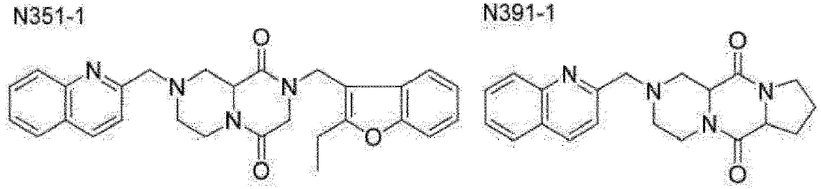

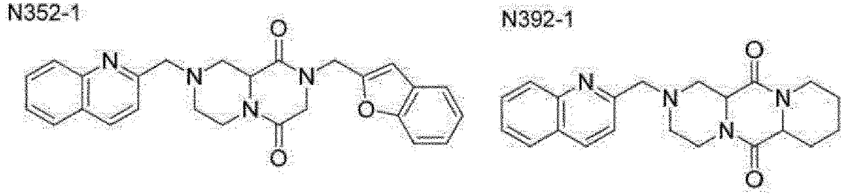

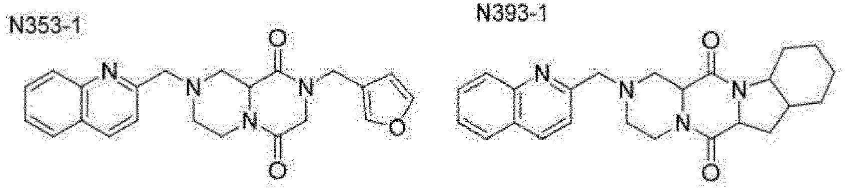

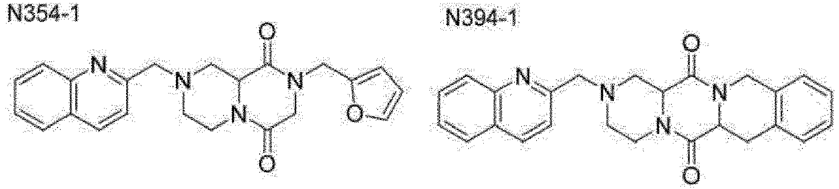

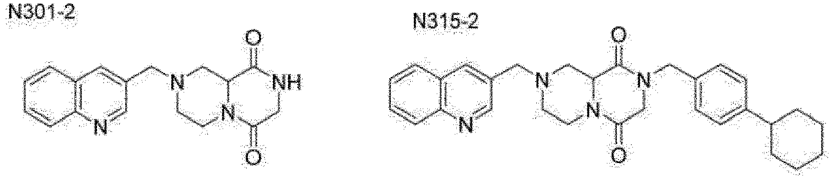

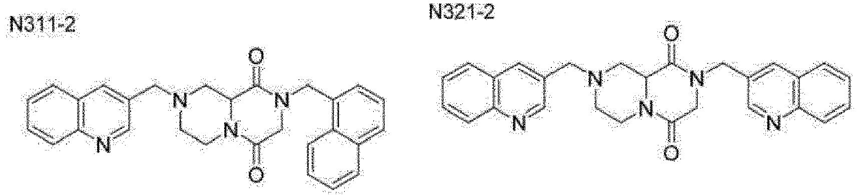

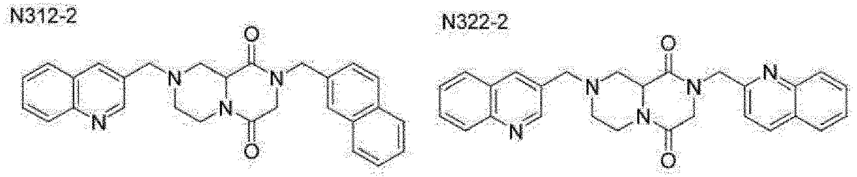

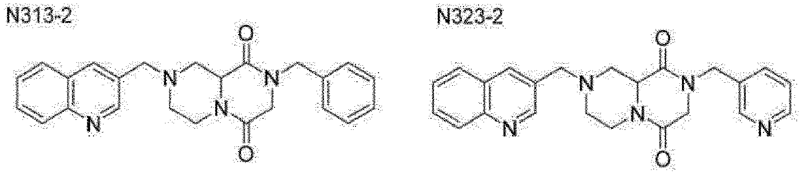

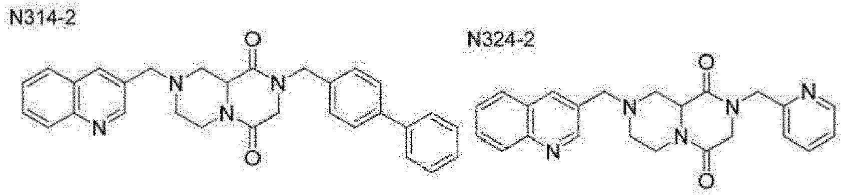

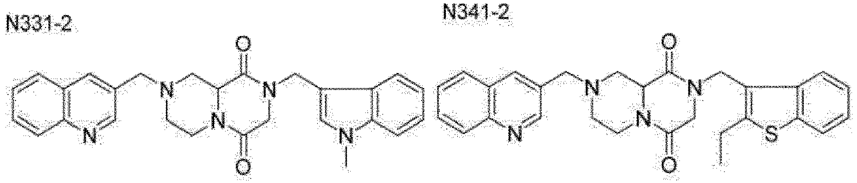

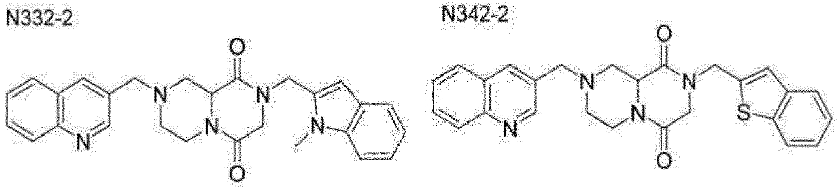

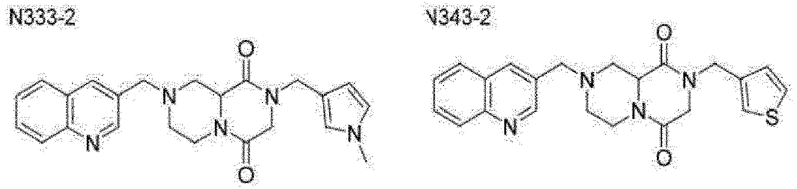

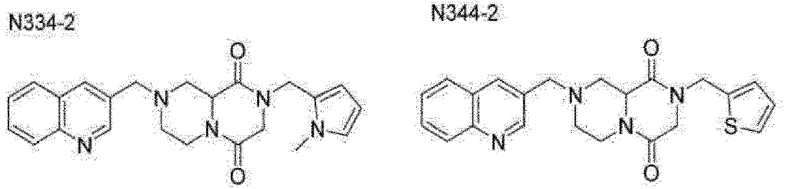

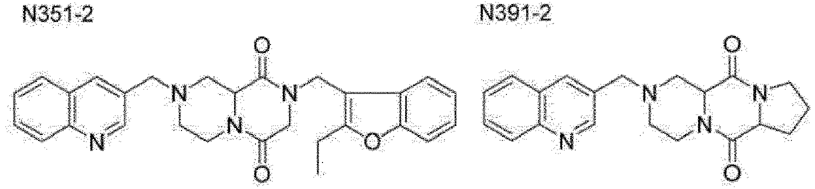

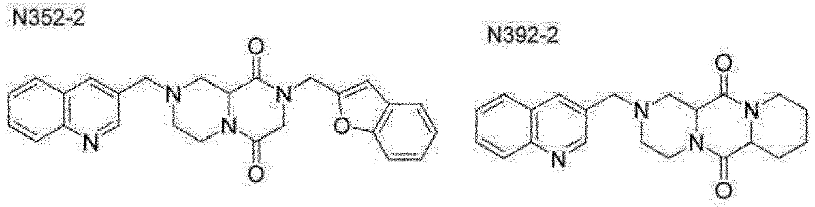

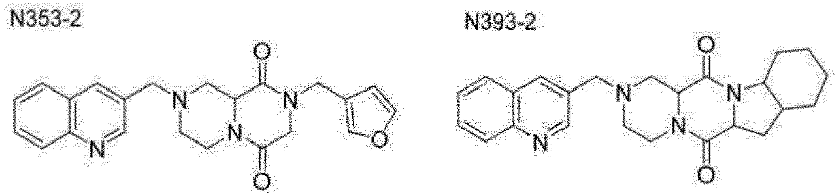

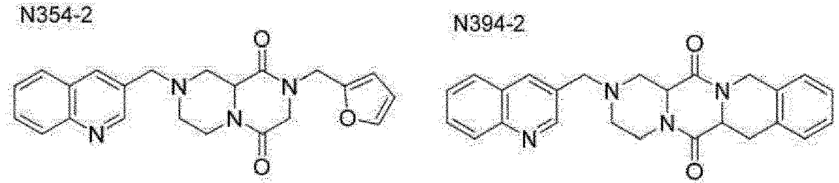

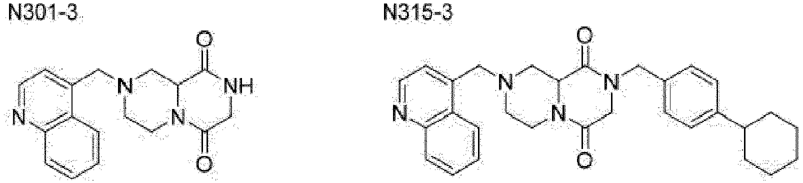

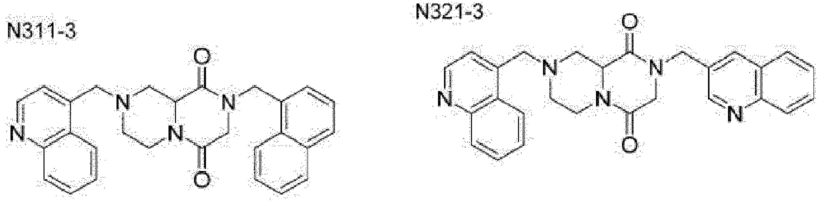

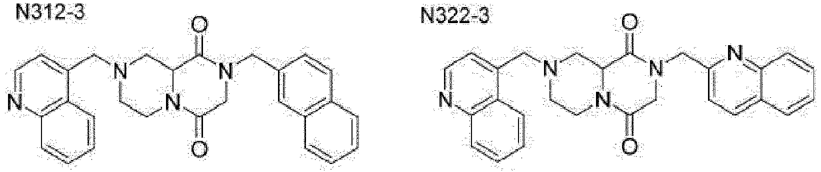

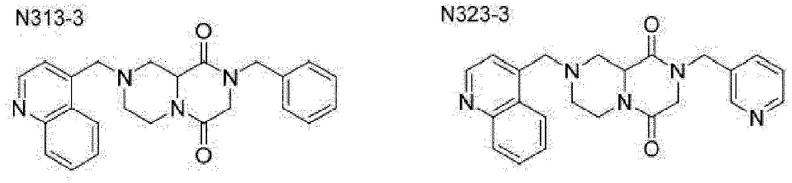

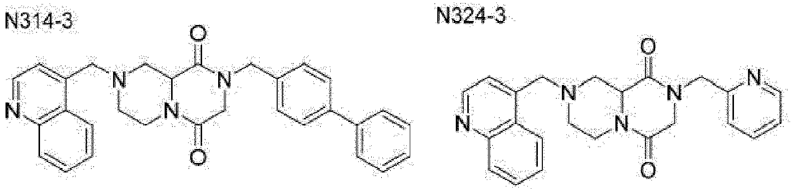

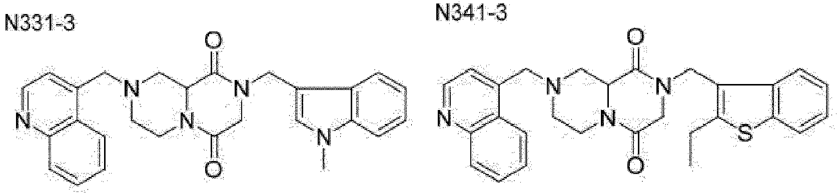

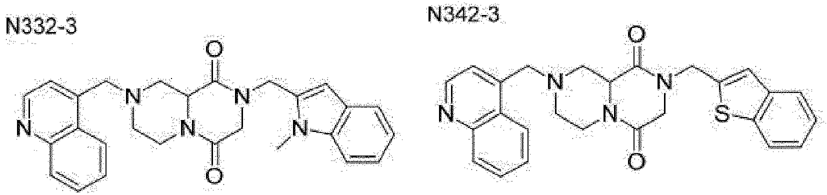

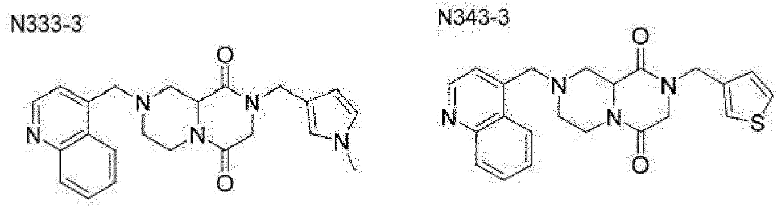

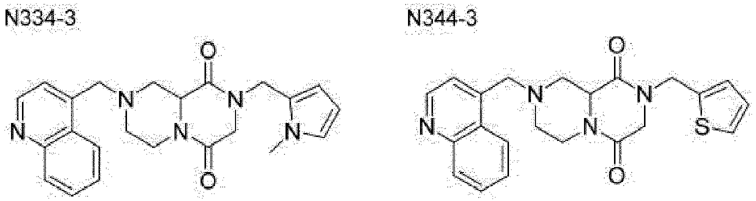

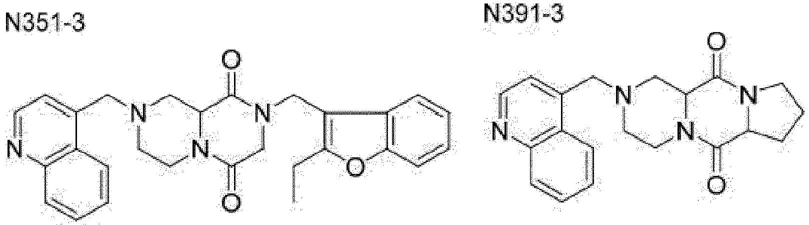

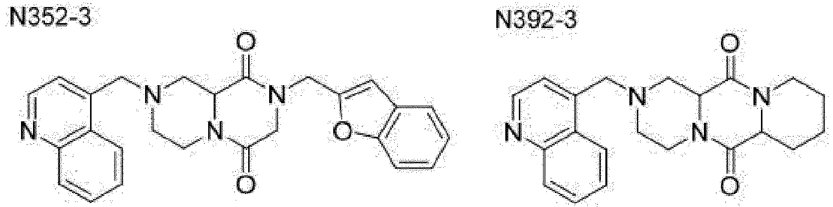

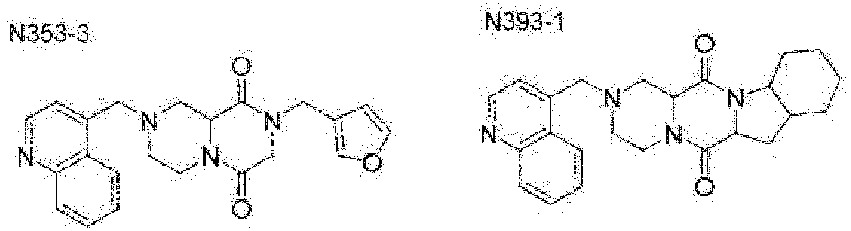

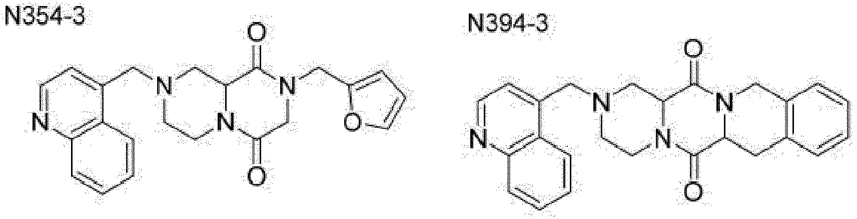

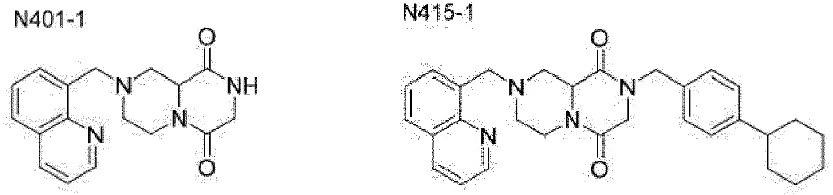

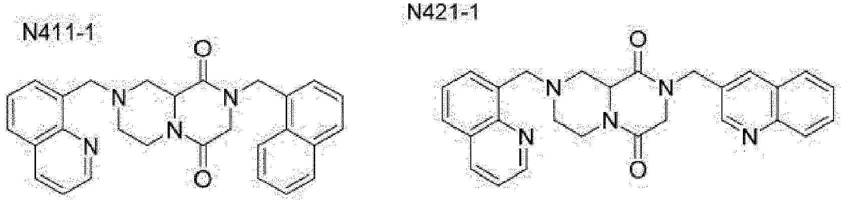

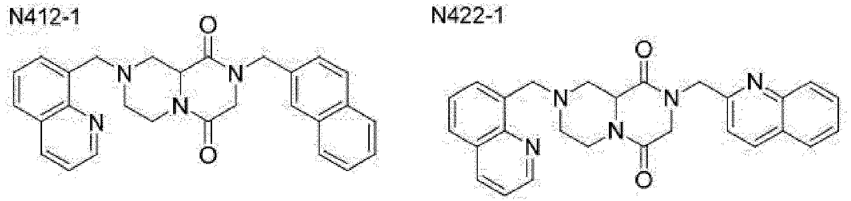

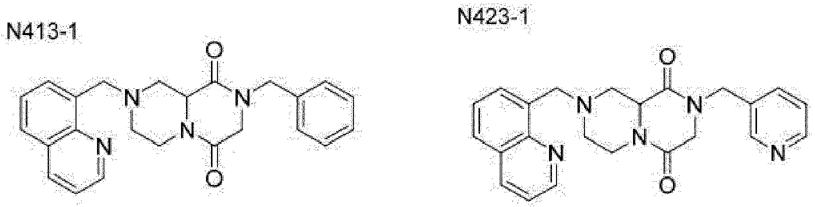

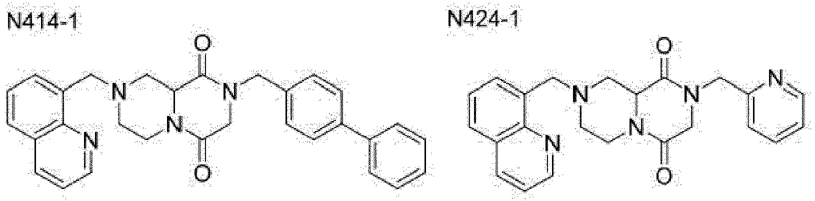

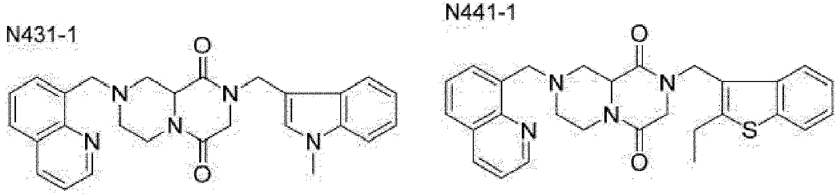

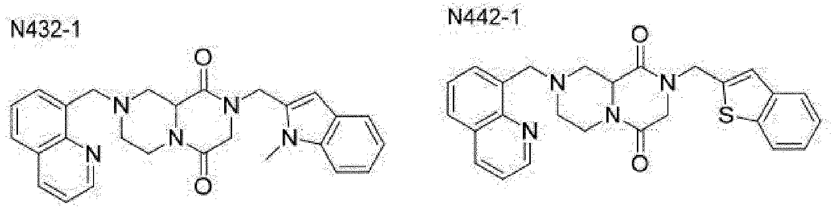

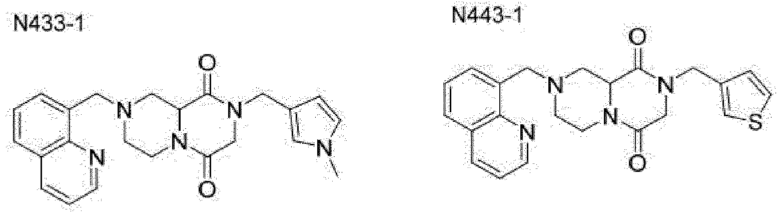

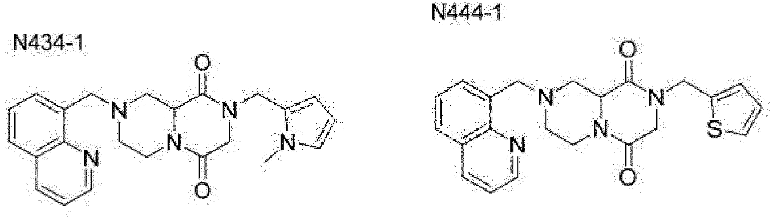

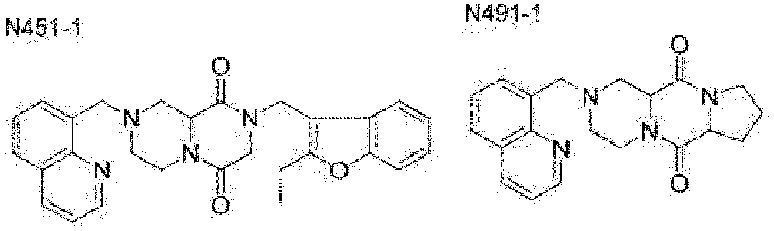

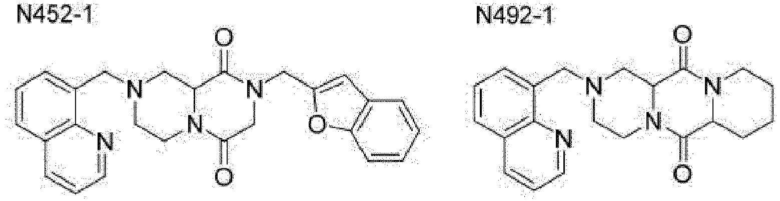

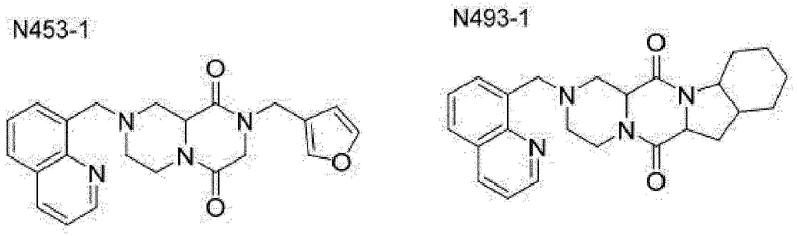

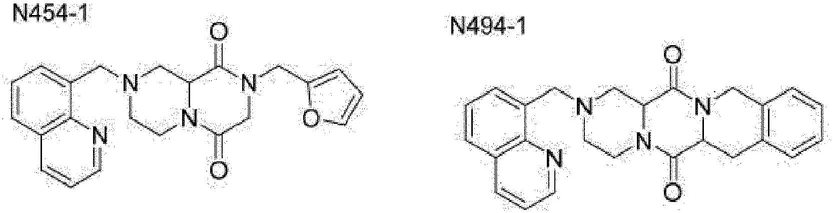

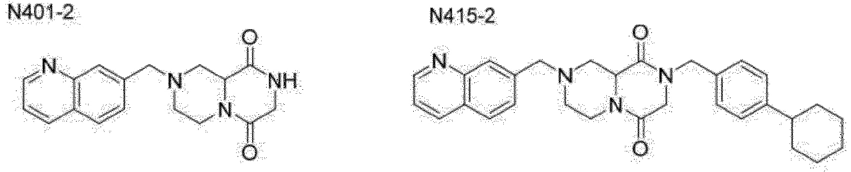

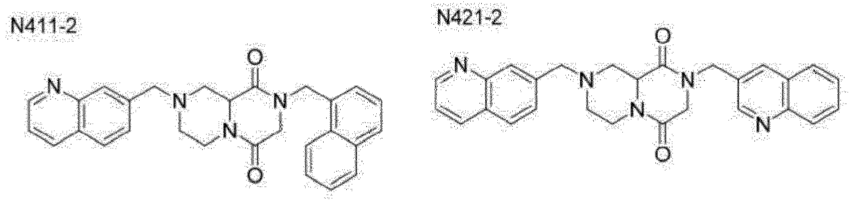

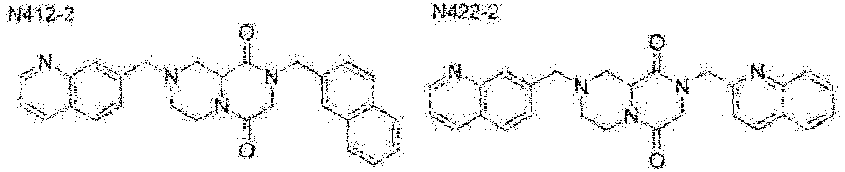

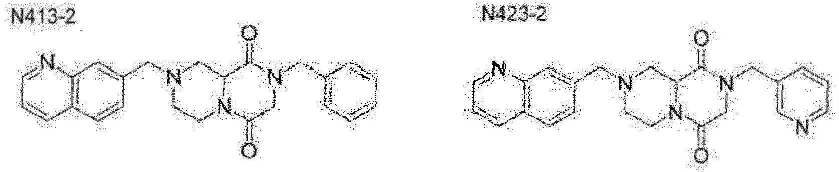

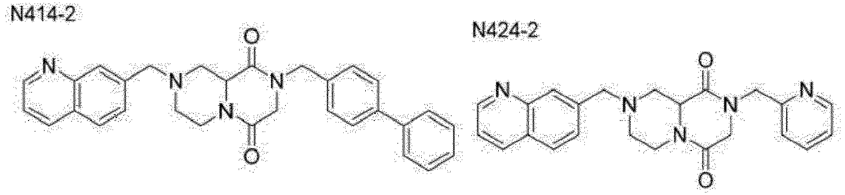

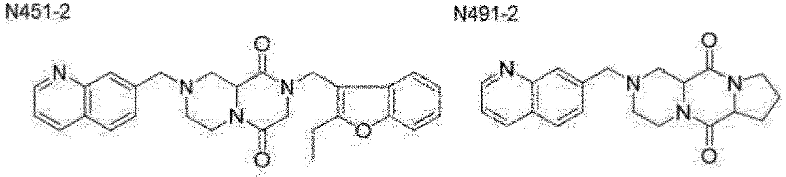

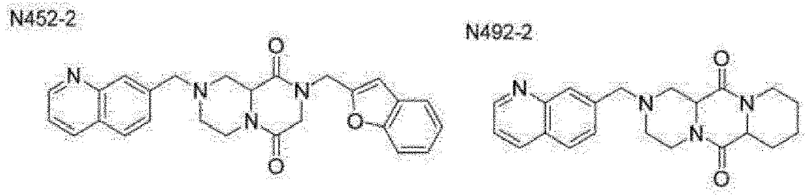

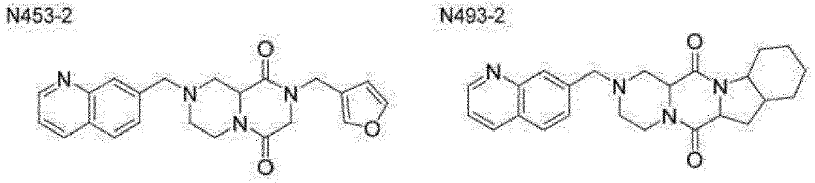

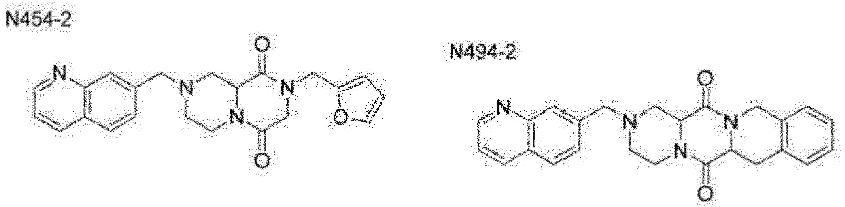

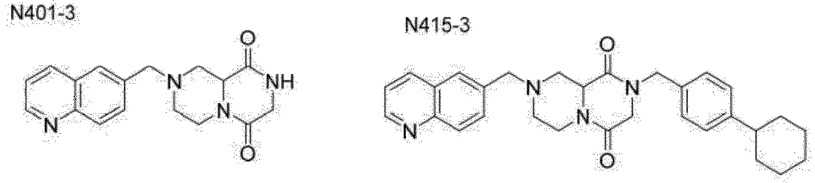

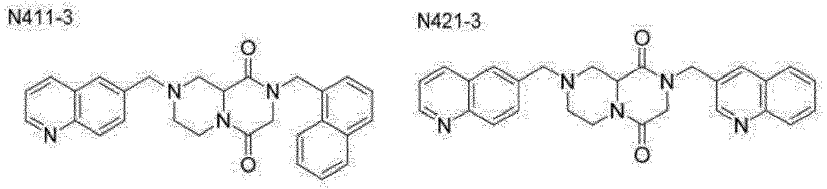

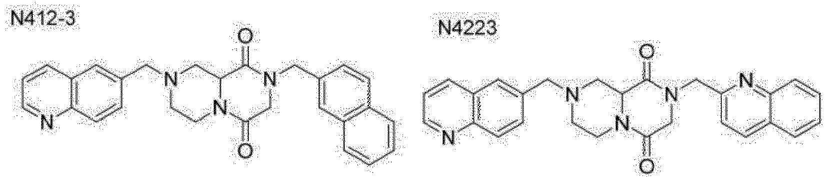

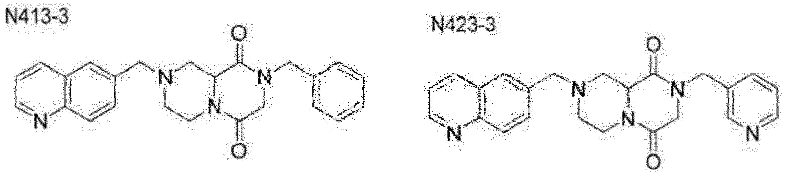

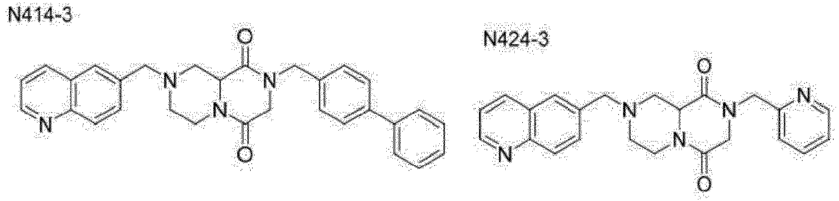

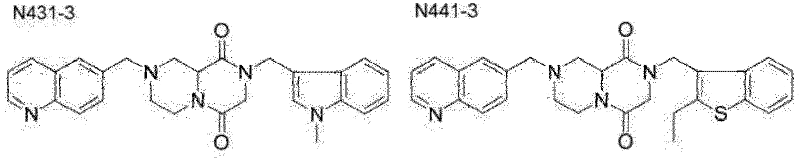

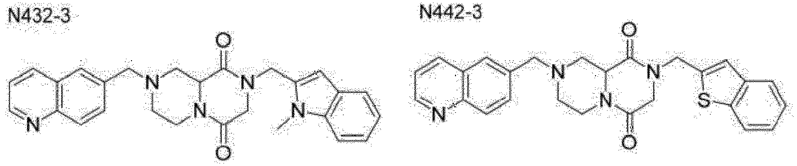

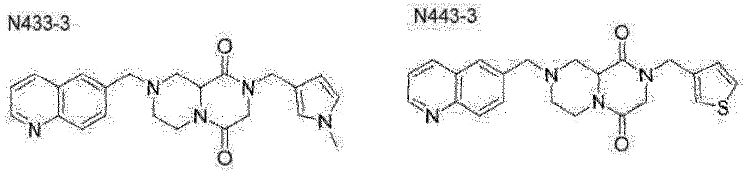

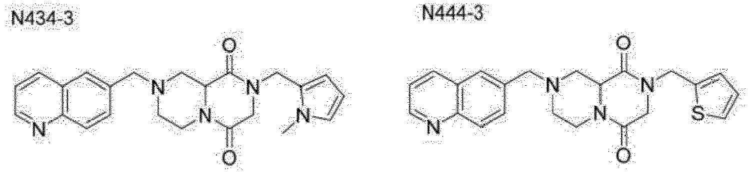

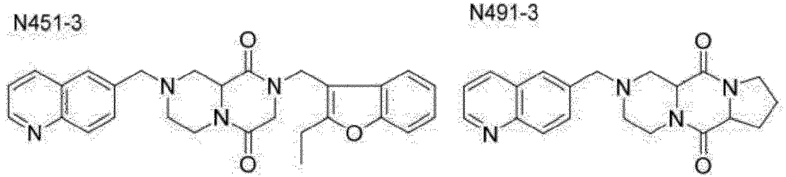

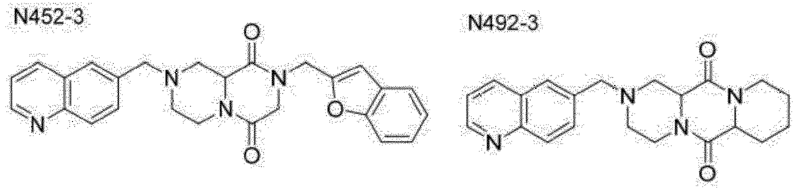

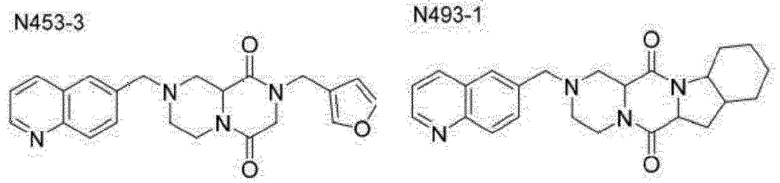

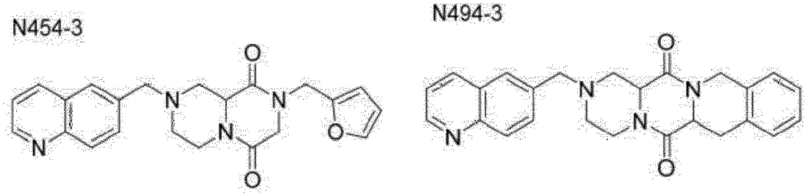

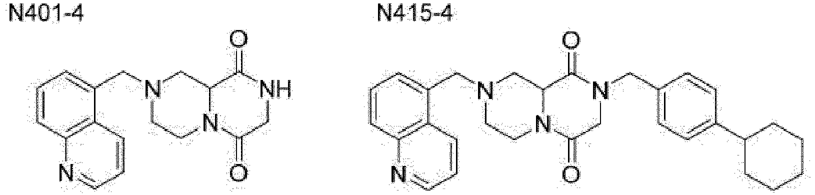

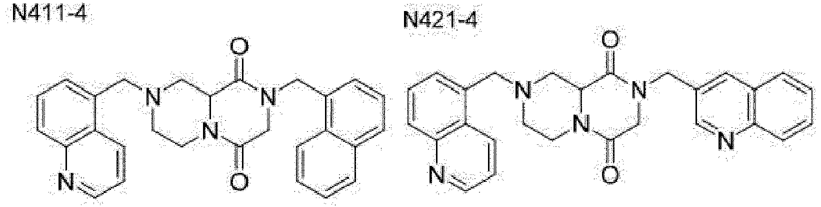

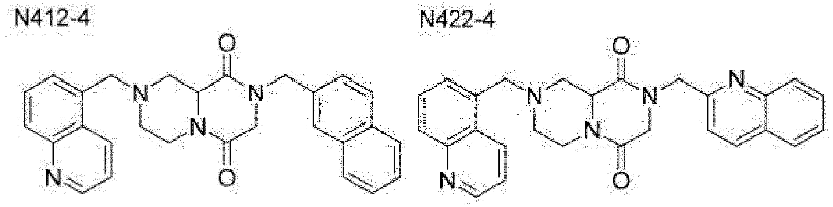

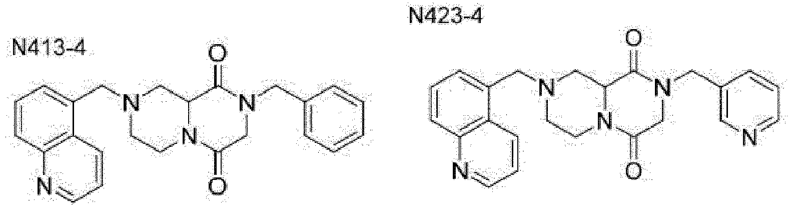

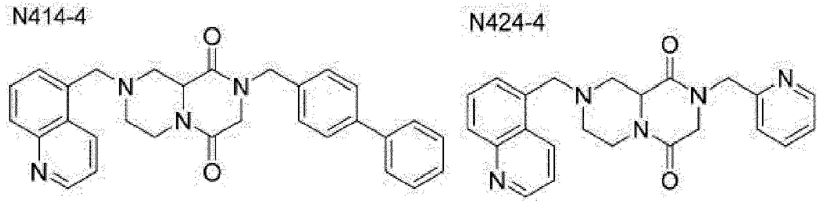

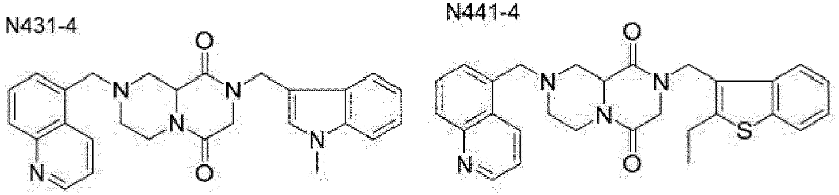

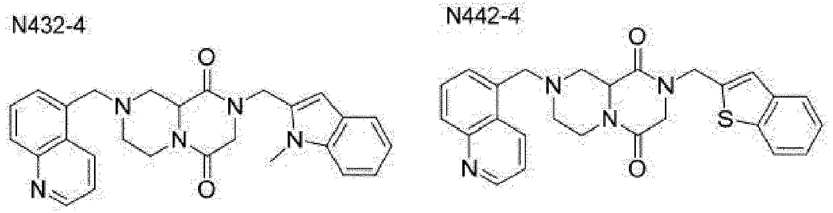

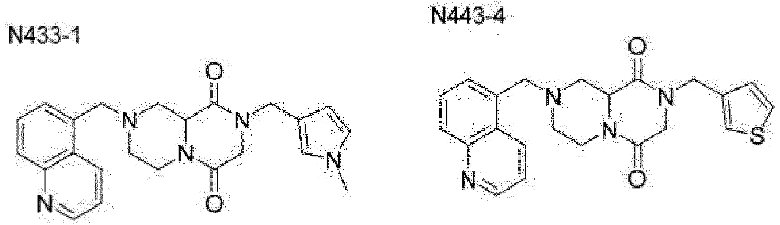

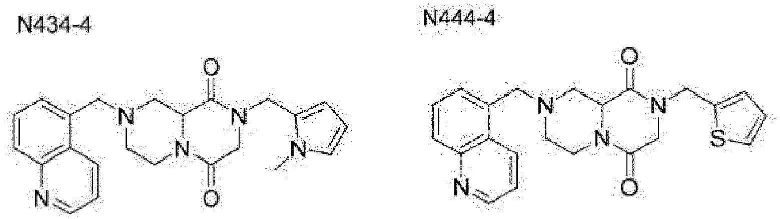

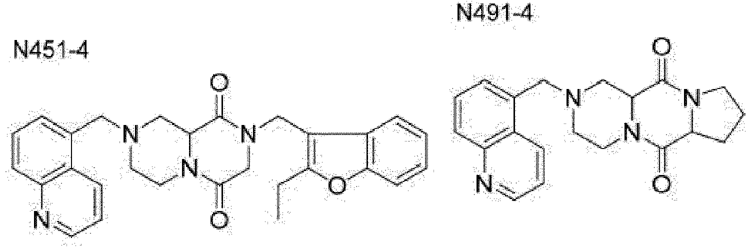

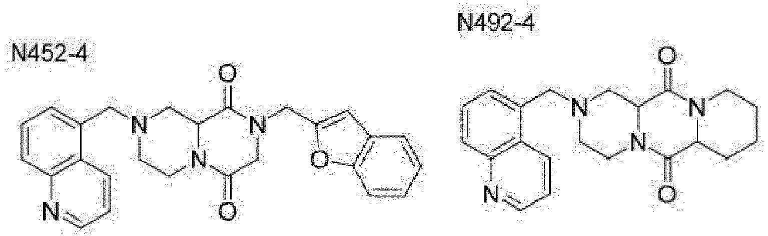

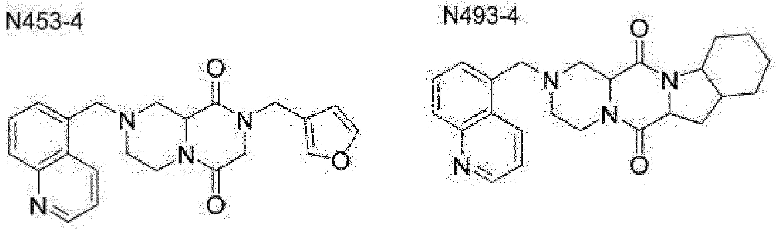

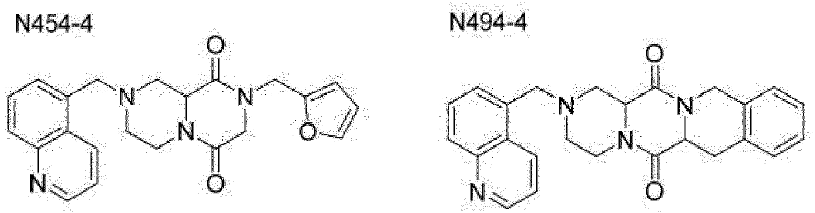

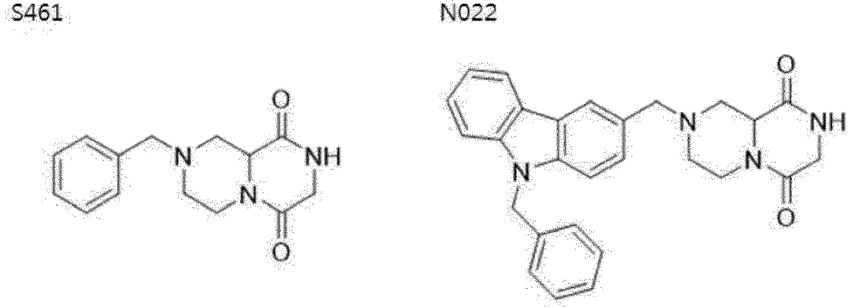

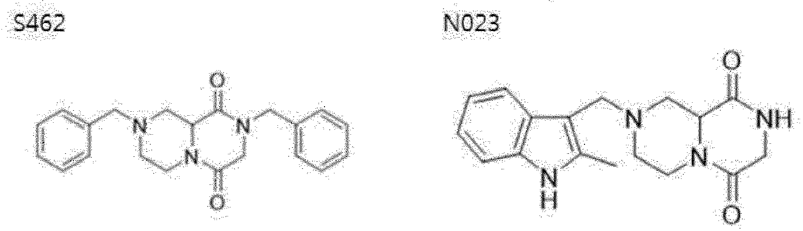

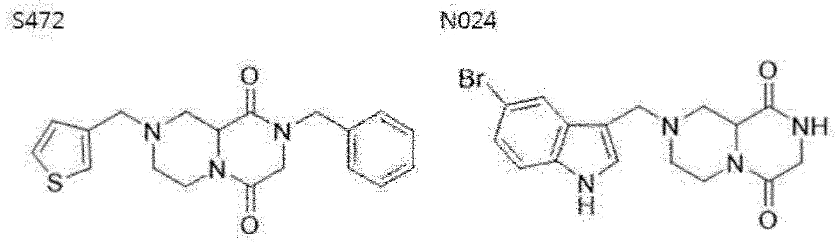

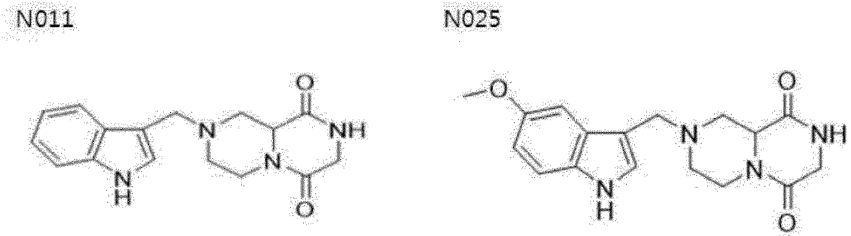

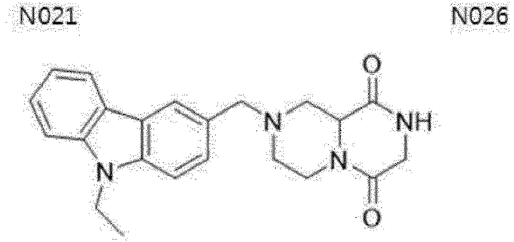

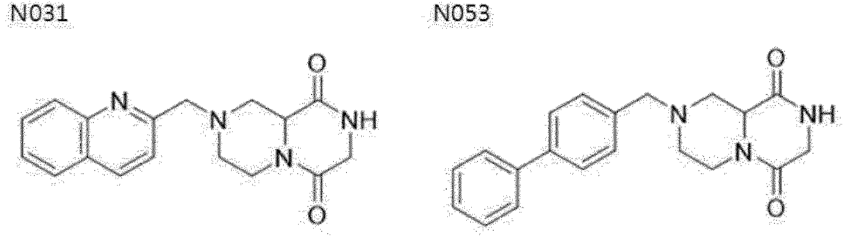

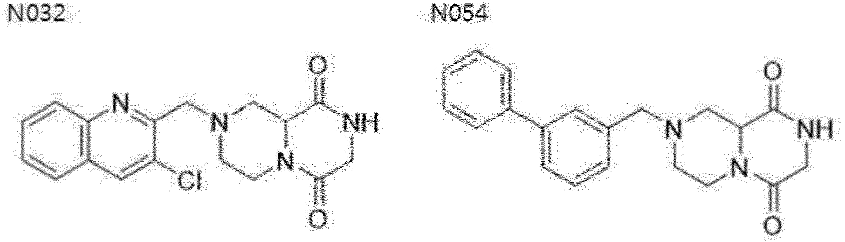

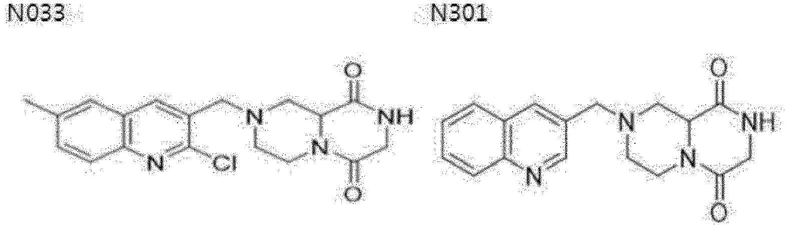

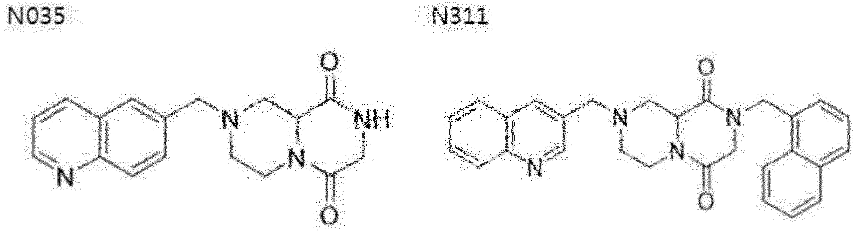

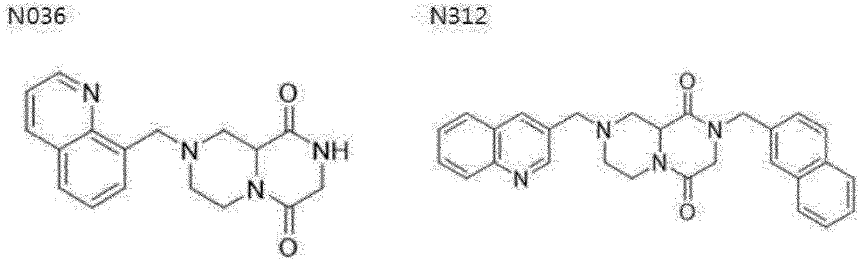

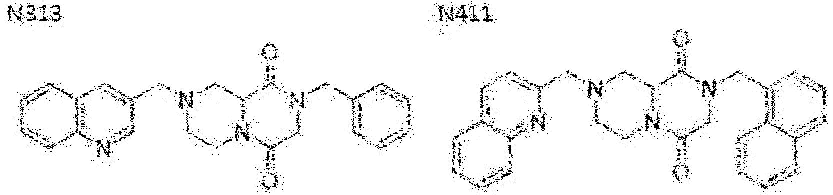

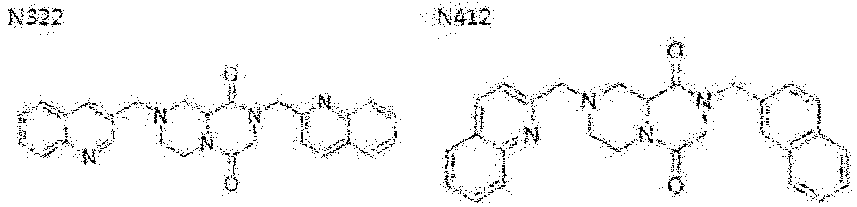

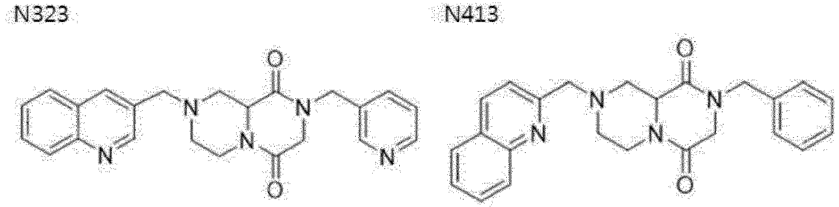

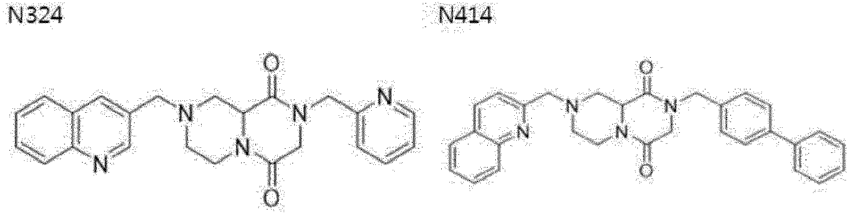

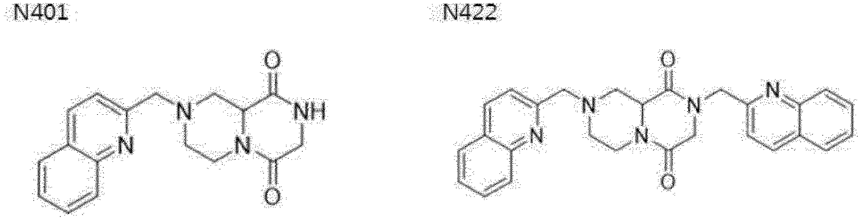

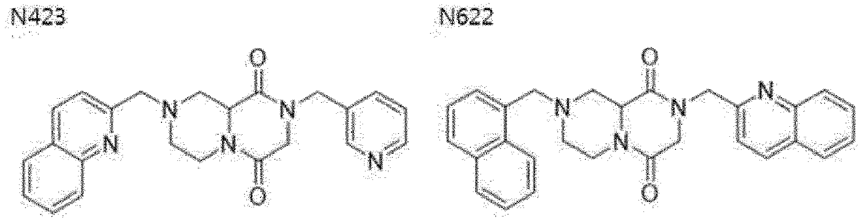

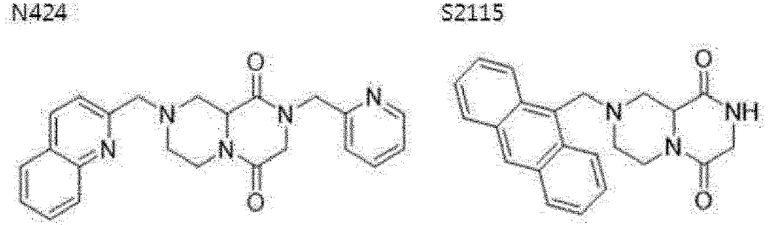

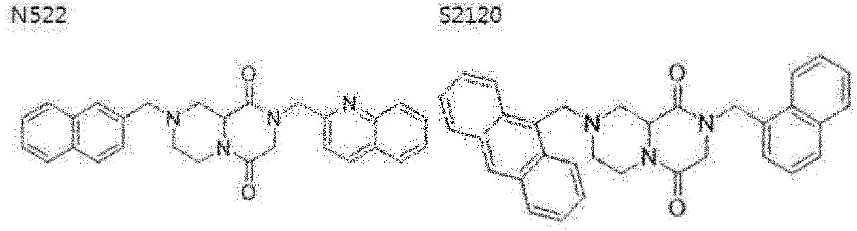

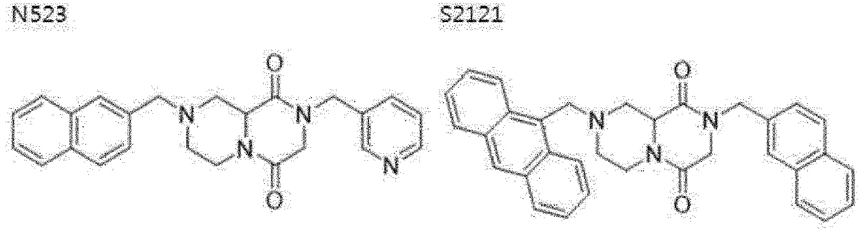

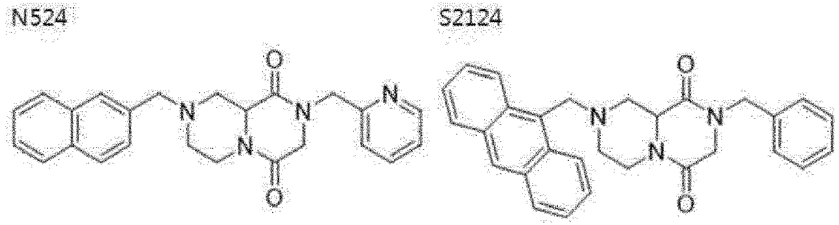

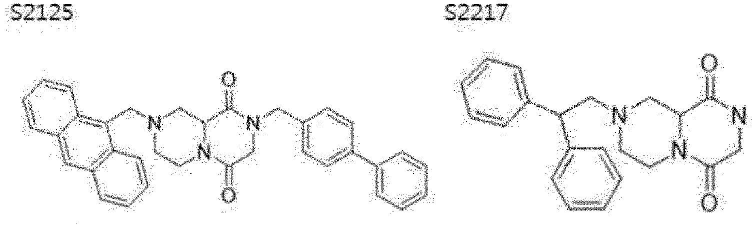

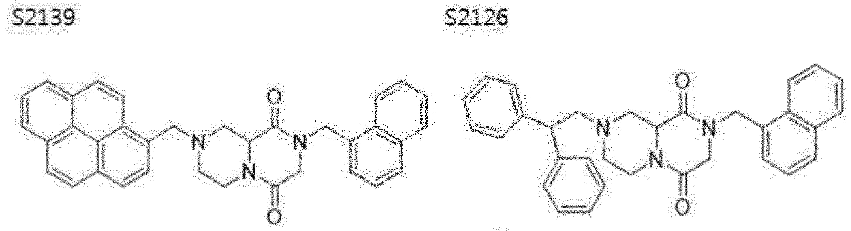

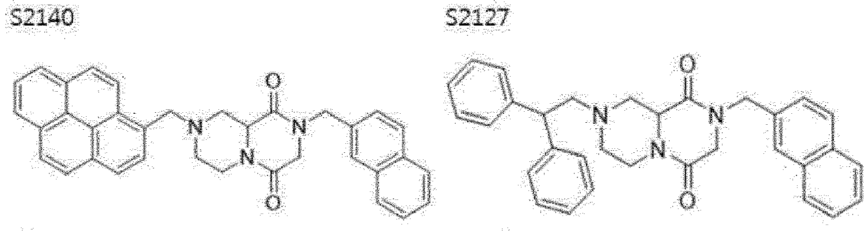

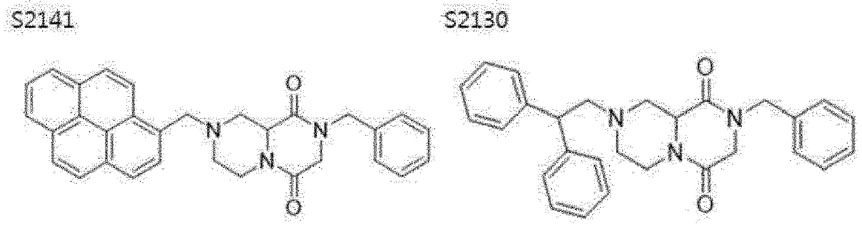

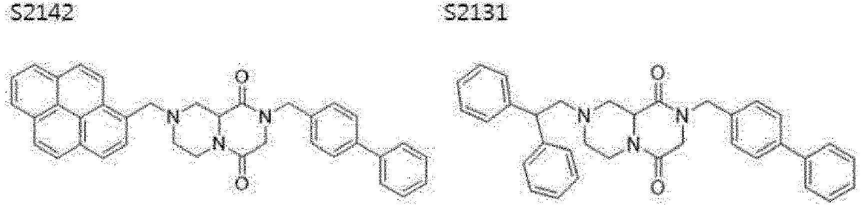

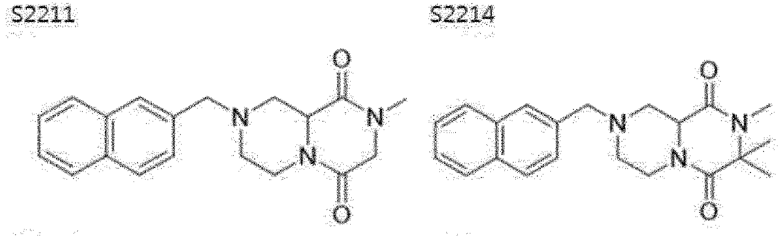

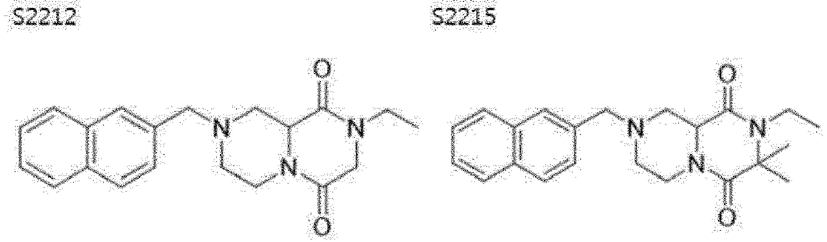

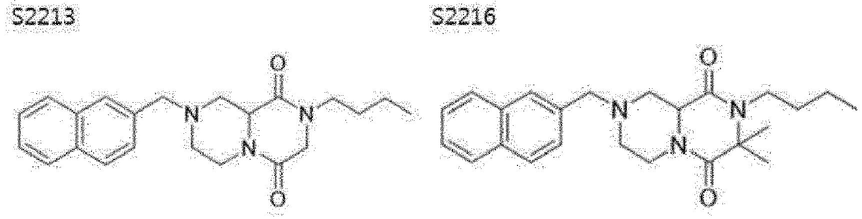

6. A pharmaceutical composition for preventing or treating cancer, comprising:
the compound or pharmaceutically acceptable salt thereof according to any one of claims 1 to 5, or a hydrate, solvate, structural isomer, optical isomer or stereoisomer thereof, or a combination thereof.

7. A pharmaceutical composition for preventing or treating resistant cancer, comprising:
the compound or pharmaceutically acceptable salt thereof according to any one of claims 1 to 5, or a hydrate, solvate, structural isomer, optical isomer or stereoisomer thereof, or a combination thereof.

8. The composition of claim 7,
wherein the composition inhibits SERCA protein expression.

9. The composition of claim 7,
wherein the resistant cancer is resistant to an anticancer drug, or to radiation.

10. The composition of claim 9,
wherein the anticancer drug is at least one selected from the group consisting of nitrogen mustard, imatinib, oxaliplatin, rituximab, erlotinib, neratinib, lapatinib, gefitinib, vandetanib, nilotinib, semaxanib, bosutinib, axitinib, masitinib, cediranib, lestaurtinib, trastuzumab, gefitinib, bortezomib, sunitinib, pazopanib, toceranib, nintedanib, regorafenib, semaxanib, tivozanib, ponatinib, cabozantinib, carboplatin, sorafenib, lenvatinib, bevacizumab, cisplatin, cetuximab, viscum album, asparaginase, tretinoin, hydroxycarbamide, dasatinib, estramustine, gemtuzumab ozogamicin, ibritumomab tiuxetan, heptaplatin, methylaminolevulinic acid, amsacrine, alemtuzumab, procarbazine, alprostadil, holmium nitrate chitosan, gemcitabine, doxifluridine, pemetrexed, tegafur, capecitabine, gimeracil, oteracil, azacytidine, methotrexate, uracil, cytarabine, 5-fluorouracil, fludarabine, enocitabine, flutamide, capecitabine, decitabine, mercaptopurine, thioguanine, cladribine, carmofur, raltitrexed, docetaxel, paclitaxel, cabazitaxel, irinotecan, belotecan, topotecan, vinorelbine, etoposide, vincristine, vinblastine, teniposide, doxorubicin, idarubicin, epirubicin, mitoxantrone, mitomycin, bleomycin, daunorubicin, dactinomycin, pirarubicin, aclarubicin, pepromycin, temsirolimus, temozolomide, busulfan, ifosfamide, cyclophosphamide, melphalan, altretamine, dacabazine, thiotepa, nimustine, chlorambucil, mitolactol, leucovorin, tretonin, exmestane, aminoglutethimide, anagrelide, olaparib, navelbine, padrazol, tamoxifen, toremifene, testolactone, anastrozole, letrozole, borozol, bicalutamide, lomustine, vorinostat, entinostat, and carmustine.

11. The composition of claim 9,
wherein the anticancer drug is comprised in a molar concentration ratio of 1:0.001 to 1:1000 with the compound represented by Formula 1 or a pharmaceutically acceptable salt thereof.

12. The composition of claim 9,
wherein the resistant cancer is at least one selected from the group consisting of ovarian cancer, colorectal cancer, pancreatic cancer, gastric cancer, liver cancer, breast cancer, cervical cancer, thyroid cancer, parathyroid cancer, lung cancer, non-small cell lung cancer, prostate cancer, gallbladder cancer, biliary tract cancer, blood cancer, bladder cancer, kidney cancer, melanoma, colon cancer, bone cancer, skin cancer, head and neck cancer, uterine cancer, rectal cancer, brain cancer, anal cancer, fallopian tube carcinoma, endometrial carcinoma, vaginal cancer, vulvar carcinoma, esophagus cancer, small intestine cancer, endocrine carcinoma, adrenal cancer, soft tissue sarcoma, urethral cancer, penile cancer, ureteral cancer, renal cell carcinoma, renal pelvic carcinoma, blood cancer, a central nervous system (CNS) tumor, a spinal cord tumor, brainstem glioma, and pituitary adenoma.

13. A method of treating cancer, comprising:
administering a therapeutically effective amount of the compound or pharmaceutically acceptable salt thereof according to any one of claims 1 to 5, or a hydrate, solvate, structural isomer, optical isomer or stereoisomer thereof, or a combination thereof to a subject with cancer.

14. A method of treating resistant cancer, comprising:
administering a therapeutically effective amount of the compound or pharmaceutically acceptable salt thereof according to any one of claims 1 to 5, or a hydrate, solvate, structural isomer, optical isomer or stereoisomer thereof, or a combination thereof to a subject with resistant cancer.

15. The method of claim 14,
which comprises simultaneously, individually or sequentially administering a chemotherapeutic agent useful for treatment of cancer or a proliferative disease.

16. A use of the compound or a pharmaceutically acceptable salt thereof according to any one of claims 1 to 5 for preparing a drug for treatment of cancer or resistant cancer.

17. A use of the compound of or a pharmaceutically acceptable salt thereof according to any one of claims 1 to 5, or a hydrate, solvate, structural isomer, optical isomer or stereoisomer thereof, or a combination thereof for preparation of a drug for treating stem cell cancer.
